# EUROPEAN PATENT APPLICATION

(11) **EP 1 988 103 A2**
(43) Date of publication of application: **05.11.2008**
(21) Application number: 08155552.6
(22) Date of filing: 01.05.2008
(51) Int. Cl.: C07K 16/30, C07K 16/44, G01N 33/574, G01N 33/577

(54) **Tyrosine phosphorylation sites and antibodies specific for them**

(30) Priority: 01.05.2007 US 927070 P; 19.10.2007 US 999628 P
(71) Applicant: Hornbeck, Peter, Magnolia, MA 01930 (US); Guo, Ailan, Burlington, MA 01803 (US); Gu, Ting-Lei, Woburn, MA 01801 (US); Rikova, Klarisa, Reading, MA 01867 (US); Moritz, Albrecht, Salem, MA 01970 (US); Farnsworth, Charles, Concord, MA 01742 (US); Stokes, Matthew, Beverly, MA 01915 (US); Jian, Yu, Hamilton, MA 01982 (US); Spek, Erik, Cambridge, MA 02138 (US); Li, Yu, Andover, MA 01810 (US); Goss, Valerie, Seabrook, NH 03874 (US); Boccalatte, Francesco, 10128 Torino (IT)
(72) Inventor: Hornbeck, Peter, Magnolia, MA 01930 (US); Guo, Ailan, Burlington, MA 01803 (US); Gu, Ting-Lei, Woburn, MA 01801 (US); Rikova, Klarisa, Reading, MA 01867 (US); Moritz, Albrecht, Salem, MA 01970 (US); Farnsworth, Charles, Concord, MA 01742 (US); Stokes, Matthew, Beverly, MA 01915 (US); Jian, Yu, Hamilton, MA 01982 (US); Spek, Erik, Cambridge, MA 02138 (US); Li, Yu, Andover, MA 01810 (US); Goss, Valerie, Seabrook, NH 03874 (US); Boccalatte, Francesco, 10128 Torino (IT)
(74) Representative: Hill, Justin John

(57) **Abstract**

The invention discloses 405 novel phosphorylation sites identified in carcinoma and/or leukemia, peptides (including AQUA peptides) comprising a phosphorylation site of the invention, antibodies specifically bind to a novel phosphorylation site of the invention, and diagnostic and therapeutic uses of the above.

SEQUENCE LISTING

<110> HORNBECK, PETER
GUO, AILAN
GU, TING-LEI
RIKOVA, KLARISA
MORITZ, ALBRECHT
FARNSWORTH, CHARLES
STOKES, MATTHEW
YU, JIAN
SPEK, ERIC
LI, YU
GOSS, VALERIE
BOCCALATTE, FRANCESCO

<120> TYROSINE PHOSPHORYLATION SITES

<130> 602231PEP

<140> EP08155552.6
<141> 2008-05-01

<150> US60/927,070
<151> 2007-05-01

<150> US60/999,628
<151> 2007-10-19

<160> 422

<170> PatentIn version 3.3

<210> 1

<400> 1
000

<210> 2

<400> 2
000

<210> 3

<400> 3
000

<210> 4
<211> 25
<212> PRT
<213> Homo sapiens

<220>
<221> MOD_RES
<222> (20)..(20)
<223> Phosphorylated Tyr

<400> 4
Ala Lys Thr Ala Phe Asp Glu Ala Ile Ala Glu Leu Asp Thr Leu Ser
1               5                   10                  15

Glu Glu Ser Tyr Lys Asp Ser Thr Leu
            20                  25

<210> 5
<211> 24
<212> PRT
<213> Homo sapiens

<220>
<221> MOD_RES
<222> (3)..(3)

<223> Phosphorylated Tyr

<400> 5
Glu Ala Tyr Ser Gly Cys Ser Gly Pro Val Asp Ser Glu Cys Pro Pro
1               5                   10                  15

Pro Pro Ser Ser Pro Val His Lys
            20

<210> 6
<211> 36
<212> PRT
<213> Homo sapiens

<220>
<221> MOD_RES
<222> (15)..(15)
<223> Phosphorylated Tyr

<400> 6
Val Ile Ser Ala Asn Pro Tyr Leu Gly Gly Thr Ser Asn Gly Tyr Ala
1               5                   10                  15

His Pro Ser Gly Thr Ala Leu His Tyr Asp Asp Val Pro Cys Ile Asn
            20                  25                  30

Gly Ser Leu Lys
        35

<210> 7
<211> 16
<212> PRT

<213> Homo sapiens

<220>
<221> MOD_RES
<222> (6)..(6)
<223> Phosphorylated Tyr

<400> 7
Ile Asp Asp Pro Ile Tyr Gly Thr Tyr Tyr Val Asp His Ile Asn Arg
1               5                   10                  15

<210> 8
<211> 10
<212> PRT
<213> Homo sapiens

<220>
<221> MOD_RES
<222> (6)..(6)
<223> Phosphorylated Tyr

<400> 8
Asn His Asp Ser Val Tyr Tyr Thr Tyr Glu
1               5                   10

<210> 9
<211> 10
<212> PRT
<213> Homo sapiens

<220>
<221> MOD_RES
<222> (9)..(9)
<223> Phosphorylated Tyr

<400> 9
Asn His Asp Ser Val Tyr Tyr Thr Tyr Glu
1               5                   10

<210> 10
<211> 14
<212> PRT
<213> Homo sapiens

<220>
<221> MOD_RES
<222> (10)..(10)
<223> Phosphorylated Tyr

<400> 10
Thr Glu Gly Thr Gln Glu Ala Asp Gln Tyr Ala Asp Glu Lys
1               5                   10

<210> 11
<211> 26
<212> PRT
<213> Homo sapiens

<220>
<221> MOD_RES
<222> (12)..(12)
<223> Phosphorylated Tyr

<400> 11
Gly Phe Ser Ser Thr Asp Gly Asp Ala Val Asn Tyr Ile Ser Ser Gln
1               5                   10                  15

Leu Pro Asp Leu Pro Ile Leu Cys Ser Arg
            20                  25

<210> 12
<211> 20
<212> PRT
<213> Homo sapiens

<220>
<221> MOD_RES
<222> (12)..(12)
<223> Phosphorylated Tyr

<400> 12
Gly Gly Ser Pro His Ser Pro Ala Trp Asn Gly Tyr Thr Pro Leu His
1               5                   10                  15

Ile Ala Ala Lys
            20

<210> 13

<400> 13
000

<210> 14
<211> 14
<212> PRT
<213> Homo sapiens

<220>
<221> MOD_RES
<222> (1)..(1)

<223> Phosphorylated Tyr

<400> 14
Tyr Leu Val Gln Asp Gly Ala Gln Val Glu Ala Lys Ala Lys
1               5                   10

<210> 15
<211> 9
<212> PRT
<213> Homo sapiens

<220>
<221> MOD_RES
<222> (3)..(3)
<223> Phosphorylated Tyr

<400> 15
His Gly Tyr Glu Asn Pro Thr Tyr Arg
1               5

<210> 16
<211> 9
<212> PRT
<213> Homo sapiens

<220>
<221> MOD_RES
<222> (8)..(8)
<223> Phosphorylated Tyr

<400> 16
His Gly Tyr Glu Asn Pro Thr Tyr Arg
1               5

<210> 17
<211> 11
<212> PRT
<213> Homo sapiens

<220>
<221> MOD_RES
<222> (1)..(1)
<223> Phosphorylated Tyr

<400> 17
Tyr Glu Val Thr Glu Asp Val Tyr Thr Ser Arg
1               5                   10

<210> 18
<211> 11
<212> PRT
<213> Homo sapiens

<220>
<221> MOD_RES
<222> (8)..(8)
<223> Phosphorylated Tyr

<400> 18
Tyr Glu Val Thr Glu Asp Val Tyr Thr Ser Arg
1               5                   10

<210> 19
<211> 16
<212> PRT
<213> Homo sapiens

<220>
<221> MOD_RES
<222> (11)..(11)
<223> Phosphorylated Tyr

<400> 19
Val His Cys Phe Cys Pro Gly Gly Ser Glu Tyr Tyr Cys Tyr Ser Lys
1               5                   10                  15

<210> 20
<211> 14
<212> PRT
<213> Homo sapiens

<220>
<221> MOD_RES
<222> (6)..(6)
<223> Phosphorylated Tyr

<400> 20
Asp Tyr Cys Asn Asn Tyr Asp Leu Thr Ser Leu Asn Val Lys
1               5                   10

<210> 21
<211> 38
<212> PRT
<213> Homo sapiens

<220>
<221> MOD_RES
<222> (10)..(10)
<223> Phosphorylated Tyr

<400> 21

Asp Thr Ile Tyr Gln Val Pro Pro Ser Tyr Gln Asn Gln Gly Ile Tyr
1               5                   10                  15

Gln Val Pro Thr Gly His Gly Thr Gln Glu Gln Glu Val Tyr Gln Val
            20                  25                  30

Pro Pro Ser Val Gln Arg
        35

<210> 22
<211> 38
<212> PRT
<213> Homo sapiens

<220>
<221> MOD_RES
<222> (30)..(30)
<223> Phosphorylated Tyr

<400> 22
Asp Thr Ile Tyr Gln Val Pro Pro Ser Tyr Gln Asn Gln Gly Ile Tyr
1               5                   10                  15

Gln Val Pro Thr Gly His Gly Thr Gln Glu Gln Glu Val Tyr Gln Val
            20                  25                  30

Pro Pro Ser Val Gln Arg
        35

<210> 23
<211> 14
<212> PRT

<213> Homo sapiens

<220>
<221> MOD_RES
<222> (3)..(3)
<223> Phosphorylated Tyr

<400> 23
Ala Met Tyr Asn Ile Gln Ser Gln Ala Pro Ser Ile Thr Glu
1               5                   10

<210> 24
<211> 12
<212> PRT
<213> Homo sapiens

<220>
<221> MOD_RES
<222> (11)..(11)
<223> Phosphorylated Tyr

<400> 24
Cys Val Lys Asp Gln Phe Gly Phe Ile Asn Tyr Glu
1               5                   10

<210> 25
<211> 30
<212> PRT
<213> Homo sapiens

<220>
<221> MOD_RES
<222> (21)..(21)

<223> Phosphorylated Tyr

<400> 25
Asp Ser Phe Gly Ser Ser Gln Ala Ser Val Ala Ser Ser Gln Pro Val
1               5                   10                  15

Ser Ser Glu Met Tyr Arg Asp Pro Phe Gly Asn Pro Phe Ala
            20                  25                  30

<210> 26
<211> 13
<212> PRT
<213> Homo sapiens

<220>
<221> MOD_RES
<222> (9)..(9)
<223> Phosphorylated Tyr

<400> 26
Asp Phe Pro Gly Leu Ser Asp Asp Tyr Tyr Gly Ala Lys
1               5                   10

<210> 27
<211> 13
<212> PRT
<213> Homo sapiens

<220>
<221> MOD_RES
<222> (10)..(10)
<223> Phosphorylated Tyr

<400> 27

Asp Phe Pro Gly Leu Ser Asp Asp Tyr Tyr Gly Ala Lys
1               5                   10

<210> 28
<211> 8
<212> PRT
<213> Homo sapiens

<220>
<221> MOD_RES
<222> (4)..(4)
<223> Phosphorylated Tyr

<400> 28
Asp Ala Val Tyr Ser Glu Tyr Lys
1               5

<210> 29

<400> 29
000

<210> 30
<211> 22
<212> PRT
<213> Homo sapiens

<220>
<221> MOD_RES
<222> (2)..(2)
<223> Phosphorylated Tyr

<400> 30

Ala Tyr Thr Ser Pro Leu Ile Asp Met Phe Asn Asn Pro Ala Thr Ala
1               5                   10                  15

Ala Pro Asn Ser Gln Arg
            20

<210> 31
<211> 13
<212> PRT
<213> Homo sapiens

<220>
<221> MOD_RES
<222> (3)..(3)
<223> Phosphorylated Tyr

<400> 31
Asp Leu Tyr Asp Val Leu Ala Lys Glu Pro Val Gln Arg
1               5                   10

<210> 32
<211> 12
<212> PRT
<213> Homo sapiens

<220>
<221> MOD_RES
<222> (10)..(10)
<223> Phosphorylated Tyr

<400> 32
Asp Ser Thr Leu Thr Cys Met Gln His Tyr Glu Glu
1               5                   10

<210> 33
<211> 31
<212> PRT
<213> Homo sapiens

<220>
<221> MOD_RES
<222> (7)..(7)
<223> Phosphorylated Tyr

<400> 33
Ala Ala Ile Asp Lys Ser Tyr Asp Lys Pro Ala Val Asp Asp Asp Asp
1               5                   10                  15

Glu Gly Met Glu Thr Leu Glu Glu Asp Thr Glu Glu Ser Ser Arg
            20                  25                  30

<210> 34
<211> 8
<212> PRT
<213> Homo sapiens

<220>
<221> MOD_RES
<222> (4)..(4)
<223> Phosphorylated Tyr

<400> 34
Ala Pro Thr Tyr Ser Val Phe Arg
1               5

<210> 35

<211> 20

<212> PRT

<213> Homo sapiens

<220>

<221> MOD_RES

<222> (4)..(4)

<223> Phosphorylated Tyr

<400> 35

Asp Glu Asn Tyr Gly Arg Val Val Leu Asn Arg His Asn Ser His Asp

1               5                   10                  15

Ala Leu Asp Arg

            20

<210> 36

<211> 18

<212> PRT

<213> Homo sapiens

<220>

<221> MOD_RES

<222> (5)..(5)

<223> Phosphorylated Tyr

<400> 36

Asp Gly Val Val Tyr Gln Glu Glu Pro Gly Gly Ser Gly Ala Val Met

1               5                   10                  15

Ser Glu

<210> 37
<211> 35
<212> PRT
<213> Homo sapiens

<220>
<221> MOD_RES
<222> (13)..(13)
<223> Phosphorylated Tyr

<400> 37
Ala Ala Cys Leu Pro Pro Gly Asp His Asn Ala Leu Tyr Val Asn Ser
1               5                   10                  15

Phe Pro Leu Leu Asp Pro Ser Asp Ala Pro Phe Pro Ser Leu Asp Ser
            20                  25                  30

Pro Gly Lys
        35

<210> 38
<211> 30
<212> PRT
<213> Homo sapiens

<220>
<221> MOD_RES
<222> (26)..(26)
<223> Phosphorylated Tyr

<400> 38
Cys Glu Phe Cys Gly Cys Asp Phe Ser Gly Glu Ala Tyr Glu Ser His
1               5                   10                  15

Glu Gly Met Cys Pro Gln Glu Ser Val Tyr Cys Glu Asn Lys
            20                  25                  30

<210> 39
<211> 14
<212> PRT
<213> Homo sapiens

<220>
<221> MOD_RES
<222> (5)..(5)
<223> Phosphorylated Tyr

<400> 39
Asp Phe Thr Ile Tyr Asn Pro Asn Leu Leu Thr Ala Ser Lys
1               5                   10

<210> 40
<211> 15
<212> PRT
<213> Homo sapiens

<220>
<221> MOD_RES
<222> (2)..(2)
<223> Phosphorylated Tyr

<400> 40
Ala Tyr Asp Pro Asp Tyr Glu Arg Ala Tyr Ser Pro Glu Tyr Arg
1               5                   10                  15

<210> 41

<211> 15
<212> PRT
<213> Homo sapiens

<220>
<221> MOD_RES
<222> (14)..(14)
<223> Phosphorylated Tyr

<400> 41
Ala Tyr Asp Pro Asp Tyr Glu Arg Ala Tyr Ser Pro Glu Tyr Arg
1               5                   10                  15

<210> 42
<211> 32
<212> PRT
<213> Homo sapiens

<220>
<221> MOD_RES
<222> (23)..(23)
<223> Phosphorylated Tyr

<400> 42
Ile Thr Ser Val Ser Thr Gly Asn Leu Cys Thr Glu Glu Gln Thr Pro
1               5                   10                  15

Pro Pro Arg Pro Glu Ala Tyr Pro Ile Pro Thr Gln Thr Tyr Thr Arg
            20                  25                  30

<210> 43
<211> 9
<212> PRT

<213> Homo sapiens

<220>
<221> MOD_RES
<222> (6)..(6)
<223> Phosphorylated Tyr

<400> 43
Arg Gln Glu Glu Gly Tyr Tyr Ser Arg
1               5

<210> 44
<211> 9
<212> PRT
<213> Homo sapiens

<220>
<221> MOD_RES
<222> (7)..(7)
<223> Phosphorylated Tyr

<400> 44
Arg Gln Glu Glu Gly Tyr Tyr Ser Arg
1               5

<210> 45
<211> 13
<212> PRT
<213> Homo sapiens

<220>
<221> MOD_RES
<222> (12)..(12)
<223> Phosphorylated Tyr

<400> 45
Ile Tyr Ala Asp Ser Leu Lys Pro Asn Ile Pro Tyr Lys
1               5                   10

<210> 46
<211> 14
<212> PRT
<213> Homo sapiens

<220>
<221> MOD_RES
<222> (6)..(6)
<223> Phosphorylated Tyr

<400> 46
Ala Asp Gly Ser Gly Tyr Gly Ser Thr Leu Pro Pro Glu Lys
1               5                   10

<210> 47
<211> 25
<212> PRT
<213> Homo sapiens

<220>
<221> MOD_RES
<222> (17)..(17)
<223> Phosphorylated Tyr

<400> 47
Thr Leu Ser Thr Asp Ala Ala Pro Gln Pro Gly Leu Ala Thr Gln Ala
1               5                   10                  15

Tyr Ser Leu Val Gly Pro Glu Val Arg
            20                  25

<210> 48
<211> 25
<212> PRT
<213> Homo sapiens

<220>
<221> MOD_RES
<222> (6)..(6)
<223> Phosphorylated Tyr

<400> 48
Asp Asn Val Tyr Tyr Tyr Asp Glu Glu Gly Gly Gly Glu Glu Asp Gln
1               5                   10                  15

Asp Phe Asp Leu Ser Gln Leu His Arg
            20                  25

<210> 49
<211> 22
<212> PRT
<213> Homo sapiens

<220>
<221> MOD_RES
<222> (12)..(12)
<223> Phosphorylated Tyr

<400> 49
Ala Thr Thr Thr Thr Ala Asn Thr Ala Pro Ala Tyr Gln Pro Pro Ala
1               5                   10                  15

Ala Tyr Lys Asp Asn Arg
            20

<210> 50
<211> 22
<212> PRT
<213> Homo sapiens

<220>
<221> MOD_RES
<222> (18)..(18)
<223> Phosphorylated Tyr

<400> 50
Ala Thr Thr Thr Thr Ala Asn Thr Ala Pro Ala Tyr Gln Pro Pro Ala
1               5                   10                  15

Ala Tyr Lys Asp Asn Arg
            20

<210> 51
<211> 13
<212> PRT
<213> Homo sapiens

<220>
<221> MOD_RES
<222> (12)..(12)
<223> Phosphorylated Tyr

<400> 51
Ala Pro Ser Val Thr Ser Ala Thr His Ser Gly Tyr Arg

1                   5                        10

<210> 52

<400> 52
000

<210> 53
<211> 29
<212> PRT
<213> Homo sapiens

<220>
<221> MOD_RES
<222> (10)..(10)
<223> Phosphorylated Tyr

<400> 53
Asp Lys Pro Ala Asn Val Gln Pro Tyr Tyr Leu Tyr Gly Ser Lys Pro
1                   5                        10                       15

Leu Asn Ile Ala Tyr Ser His Ile Tyr Ser Ser Tyr Arg
                20                       25

<210> 54
<211> 14
<212> PRT
<213> Homo sapiens

<220>
<221> MOD_RES
<222> (3)..(3)
<223> Phosphorylated Tyr

<400> 54

Asp Val Tyr Asp Lys Val Asp Tyr Leu Ser Ser Leu Gly Lys

1               5                   10

<210> 55

<211> 9

<212> PRT

<213> Homo sapiens

<220>

<221> MOD_RES

<222> (7)..(7)

<223> Phosphorylated Tyr

<400> 55

Asp His Tyr Asp Ala Thr Tyr His Lys

1               5

<210> 56

<211> 23

<212> PRT

<213> Homo sapiens

<220>

<221> MOD_RES

<222> (14)..(14)

<223> Phosphorylated Tyr

<400> 56

Ala Thr Thr Ala Gly Gly Glu Glu Asp Ala Glu Glu Asp Tyr Gln Glu

1               5                   10                  15

Pro Thr Val His Phe Ala Glu
            20

<210> 57
<211> 8
<212> PRT
<213> Homo sapiens

<220>
<221> MOD_RES
<222> (7)..(7)
<223> Phosphorylated Tyr

<400> 57
Ala Pro Thr Lys Glu Phe Tyr Ala
1               5

<210> 58

<400> 58
000

<210> 59
<211> 24
<212> PRT
<213> Homo sapiens

<220>
<221> MOD_RES
<222> (8)..(8)
<223> Phosphorylated Tyr

<400> 59
Ile Val Ser Asn Trp Asp Arg Tyr Gln Asp Ile Glu Lys Glu Val Asn

1               5                   10                  15

Asn Glu Ser Gly Glu Ser Gln Arg
            20

<210> 60
<211> 14
<212> PRT
<213> Homo sapiens

<220>
<221> MOD_RES
<222> (13)..(13)
<223> Phosphorylated Tyr

<400> 60
Gln Lys Ala Ile Asp Val Pro Gly Gln Val Gln Val Tyr Glu
1               5                   10

<210> 61
<211> 33
<212> PRT
<213> Homo sapiens

<220>
<221> MOD_RES
<222> (21)..(21)
<223> Phosphorylated Tyr

<400> 61
Val Arg Gly Gly Gly Pro Ala Glu Thr Thr Trp Leu Gly Glu Gly Gly
1               5                   10                  15

Gly Gly Asp Gly Tyr Tyr Pro Ser Gly Gly Ala Trp Pro Glu Pro Gly
            20                  25                  30

Arg

<210> 62
<211> 11
<212> PRT
<213> Homo sapiens

<220>
<221> MOD_RES
<222> (7)..(7)
<223> Phosphorylated Tyr

<400> 62
Asp Ile Ser Thr Asn Tyr Tyr Ala Ser Gln Lys
1               5                   10

<210> 63
<211> 25
<212> PRT
<213> Homo sapiens

<220>
<221> MOD_RES
<222> (4)..(4)
<223> Phosphorylated Tyr

<400> 63
Leu Asp Pro Tyr Asp Ser Ser Glu Asp Asp Lys Glu Tyr Val Gly Phe
1               5                   10                  15

Ala Thr Leu Pro Asn Gln Val His Arg
            20                  25

<210> 64
<211> 25
<212> PRT
<213> Homo sapiens

<220>
<221> MOD_RES
<222> (13)..(13)
<223> Phosphorylated Tyr

<400> 64
Leu Asp Pro Tyr Asp Ser Ser Glu Asp Asp Lys Glu Tyr Val Gly Phe
1               5                   10                  15

Ala Thr Leu Pro Asn Gln Val His Arg
            20                  25

<210> 65

<400> 65
000

<210> 66
<211> 26
<212> PRT
<213> Homo sapiens

<220>
<221> MOD_RES
<222> (12)..(12)
<223> Phosphorylated Tyr

<400> 66
Ser Ile Pro Ala Tyr Leu Ala Glu Thr Leu Tyr Tyr Ala Met Lys Gly
1               5                   10                  15

Ala Gly Thr Asp Asp His Thr Leu Ile Arg
            20                  25

<210> 67
<211> 31
<212> PRT
<213> Homo sapiens

<220>
<221> MOD_RES
<222> (21)..(21)
<223> Phosphorylated Tyr

<400> 67
Leu Ser Gly Gly Asp Asp Asp Ala Ala Gly Gln Phe Phe Pro Glu Ala
1               5                   10                  15

Ala Gln Val Ala Tyr Gln Met Trp Glu Leu Ser Ala Val Ala Arg
            20                  25                  30

<210> 68
<211> 13
<212> PRT
<213> Homo sapiens

<220>

<221> MOD_RES

<222> (2)..(2)

<223> Phosphorylated Tyr

<400> 68

Ser Tyr Gly Gln Tyr Trp Thr Gln Glu Asp Ser Ser Lys

1               5                   10

<210> 69

<211> 19

<212> PRT

<213> Homo sapiens

<220>

<221> MOD_RES

<222> (3)..(3)

<223> Phosphorylated Tyr

<400> 69

Glu Thr Tyr Tyr Leu Ser Asp Ser Pro Leu Leu Gly Pro Pro Val Gly

1               5                   10                  15

Glu Pro Arg

<210> 70

<211> 28

<212> PRT

<213> Homo sapiens

<220>

<221> MOD_RES
<222> (11)..(11)
<223> Phosphorylated Tyr

<400> 70
Cys Ser Gly Glu Gln Ser Pro Asp Thr Asn Tyr Glu Pro Pro Gly Glu
1               5                   10                  15

Asp Lys Thr Gln Gly Ser Ser Glu Cys Ile Ser Glu
            20                  25

<210> 71
<211> 16
<212> PRT
<213> Homo sapiens

<220>
<221> MOD_RES
<222> (7)..(7)
<223> Phosphorylated Tyr

<400> 71
Asp Tyr Asn Pro Tyr Pro Tyr Ser Asp Ala Ile Asn Thr Tyr Asp Lys
1               5                   10                  15

<210> 72
<211> 12
<212> PRT
<213> Homo sapiens

<220>
<221> MOD_RES
<222> (6)..(6)
<223> Phosphorylated Tyr

<400> 72
Cys Pro Leu Asp Pro Tyr Phe Ile Met Pro Asp Lys
1               5                   10

<210> 73
<211> 18
<212> PRT
<213> Homo sapiens

<220>
<221> MOD_RES
<222> (6)..(6)
<223> Phosphorylated Tyr

<400> 73
Asp Ile Asp Lys Gln Tyr Val Gly Phe Ala Thr Leu Pro Asn Gln Val
1               5                   10                  15

His Arg

<210> 74
<211> 12
<212> PRT
<213> Homo sapiens

<220>
<221> MOD_RES
<222> (9)..(9)
<223> Phosphorylated Tyr

<400> 74

Cys Pro Pro Ser Ala Gly Gln Thr Tyr Ser Gln Arg
1               5                   10

<210> 75
<211> 10
<212> PRT
<213> Homo sapiens

<220>
<221> MOD_RES
<222> (8)..(8)
<223> Phosphorylated Tyr

<400> 75
Ala Ile Cys Ile Asp Pro Ala Tyr Ser Lys
1               5                   10

<210> 76
<211> 16
<212> PRT
<213> Homo sapiens

<220>
<221> MOD_RES
<222> (2)..(2)
<223> Phosphorylated Tyr

<400> 76
Leu Tyr Val Cys Val Lys Glu Ile Gly Gly Leu Ala Gln Val Asn Lys
1               5                   10                  15

<210> 77
<211> 21
<212> PRT

<213> Homo sapiens

<220>
<221> MOD_RES
<222> (6)..(6)
<223> Phosphorylated Tyr

<400> 77
Asp Gly Lys Pro Pro Tyr Ser Tyr Ala Ser Leu Ile Thr Phe Ala Ile
1               5                   10                  15

Asn Ser Ser Pro Lys
            20

<210> 78
<211> 11
<212> PRT
<213> Homo sapiens

<220>
<221> MOD_RES
<222> (7)..(7)
<223> Phosphorylated Tyr

<400> 78
Ala Leu Ala Ala Ala Gly Tyr Asp Val Glu Lys
1               5                   10

<210> 79
<211> 21
<212> PRT
<213> Homo sapiens

<220>
<221> MOD_RES
<222> (5)..(5)
<223> Phosphorylated Tyr

<400> 79
Ala Ser Glu Ala Tyr Leu Val Gly Leu Phe Glu Asp Thr Asn Leu Cys
1               5                   10                  15

Ala Ile His Ala Lys
            20

<210> 80
<211> 11
<212> PRT
<213> Homo sapiens

<220>
<221> MOD_RES
<222> (7)..(7)
<223> Phosphorylated Tyr

<400> 80
Ala Leu Ala Ala Ala Gly Tyr Asp Val Glu Lys
1               5                   10

<210> 81
<211> 11
<212> PRT
<213> Homo sapiens

<220>
<221> MOD_RES

<222> (7)..(7)
<223> Phosphorylated Tyr

<400> 81
Ala Leu Ala Ala Ala Gly Tyr Asp Val Glu Lys
1               5                   10

<210> 82
<211> 10
<212> PRT
<213> Homo sapiens

<220>
<221> MOD_RES
<222> (5)..(5)
<223> Phosphorylated Tyr

<400> 82
Asp Ala Val Thr Tyr Thr Glu His Ala Lys
1               5                   10

<210> 83
<211> 11
<212> PRT
<213> Homo sapiens

<220>
<221> MOD_RES
<222> (2)..(2)
<223> Phosphorylated Tyr

<400> 83
Ala Tyr Leu Ile Lys Leu Ser Gly Leu Asn Lys
1               5                   10

<210> 84
<211> 18
<212> PRT
<213> Homo sapiens

<220>
<221> MOD_RES
<222> (3)..(3)
<223> Phosphorylated Tyr

<400> 84
Ala Lys Tyr Phe Tyr Ile Thr Pro Gly Ile Cys Pro Ser Leu Ser Thr
1               5                   10                  15

Met Lys

<210> 85
<211> 19
<212> PRT
<213> Homo sapiens

<220>
<221> MOD_RES
<222> (10)..(10)
<223> Phosphorylated Tyr

<400> 85
Asp Phe Arg Trp Ile Ser Ser Gly Glu Tyr Ile Gln Met Ser Gly Arg
1               5                   10                  15

Ala Gly Arg

<210> 86

<211> 18

<212> PRT

<213> Homo sapiens

<220>

<221> MOD_RES

<222> (12)..(12)

<223> Phosphorylated Tyr

<400> 86

Ala Ser Leu Phe Thr Glu Glu Glu Ala Glu Gln Tyr Lys Gln Asp Leu

1               5                   10                  15

Ile Arg

<210> 87

<211> 24

<212> PRT

<213> Homo sapiens

<220>

<221> MOD_RES

<222> (18)..(18)

<223> Phosphorylated Tyr

<400> 87

Cys Gly Tyr Gly Glu Ser Leu Gly Thr Leu Ser Pro Tyr Ser Gln Asp

1               5                   10                  15

Ile Tyr Glu Asn Leu Asp Leu Arg
            20

<210> 88
<211> 12
<212> PRT
<213> Homo sapiens

<220>
<221> MOD_RES
<222> (8)..(8)
<223> Phosphorylated Tyr

<400> 88
His Arg Pro Glu Leu Ile Glu Tyr Asp Lys Leu Arg
1               5                   10

<210> 89
<211> 28
<212> PRT
<213> Homo sapiens

<220>
<221> MOD_RES
<222> (5)..(5)
<223> Phosphorylated Tyr

<400> 89
Ser Ile Val Asp Tyr Lys Pro Asn Leu Asp Leu Leu Glu Gln Gln His
1               5                   10                  15

Gln Leu Ile Gln Glu Ala Leu Ile Phe Asp Asn Lys
            20                  25

<210> 90

<211> 12

<212> PRT

<213> Homo sapiens

<220>

<221> MOD_RES

<222> (6)..(6)

<223> Phosphorylated Tyr

<400> 90

Ser Val Ser Lys Glu Tyr Tyr Asn Gln Thr Gly Arg

1               5                   10

<210> 91

<211> 10

<212> PRT

<213> Homo sapiens

<220>

<221> MOD_RES

<222> (8)..(8)

<223> Phosphorylated Tyr

<400> 91

Phe Ser Glu Asp Asp Pro Glu Tyr Met Arg

1               5                   10

<210> 92

<211> 14

<212> PRT

<213> Homo sapiens

<220>
<221> MOD_RES
<222> (13)..(13)
<223> Phosphorylated Tyr

<400> 92
Thr Leu Asp Asn Leu Gly Tyr Arg Thr Gly Tyr Ala Tyr Arg
1               5                   10

<210> 93
<211> 30
<212> PRT
<213> Homo sapiens

<220>
<221> MOD_RES
<222> (23)..(23)
<223> Phosphorylated Tyr

<400> 93
Thr Leu Thr Gly Thr Val Ile Asp Ser Gly Asp Gly Val Thr His Val
1               5                   10                  15

Ile Pro Val Ala Glu Gly Tyr Val Ile Gly Ser Cys Ile Lys
            20                  25                  30

<210> 94
<211> 15
<212> PRT
<213> Homo sapiens

<220>

<221> MOD_RES
<222> (9)..(9)
<223> Phosphorylated Tyr

<400> 94
Pro Leu Lys Ser Ala Glu Glu Ala Tyr Glu Glu Met Met Arg Lys
1               5                   10                  15

<210> 95
<211> 24
<212> PRT
<213> Homo sapiens

<220>
<221> MOD_RES
<222> (6)..(6)
<223> Phosphorylated Tyr

<400> 95
His Ser Tyr His Asp Tyr Asp Glu Pro Pro Glu Glu Gly Leu Trp Pro
1               5                   10                  15

His Asp Glu Gly Gly Pro Gly Arg
            20

<210> 96
<211> 13
<212> PRT
<213> Homo sapiens

<220>
<221> MOD_RES
<222> (10)..(10)

<223> Phosphorylated Tyr

<400> 96
Cys Ala Ser Gln Ala Gly Met Thr Ala Tyr Gly Thr Arg
1               5                   10

<210> 97
<211> 11
<212> PRT
<213> Homo sapiens

<220>
<221> MOD_RES
<222> (9)..(9)
<223> Phosphorylated Tyr

<400> 97
Ala Glu Ile Ile Asp Gln Asp Leu Tyr Glu Arg
1               5                   10

<210> 98

<400> 98
000

<210> 99
<211> 22
<212> PRT
<213> Homo sapiens

<220>
<221> MOD_RES
<222> (9)..(9)
<223> Phosphorylated Tyr

<400> 99
Asp Ala His Ser Thr Pro Tyr Tyr Tyr Ala Arg Pro Gln Thr Leu Pro
1               5                   10                  15

Leu Ser Tyr Gln Asp Arg
            20

<210> 100
<211> 11
<212> PRT
<213> Homo sapiens

<220>
<221> MOD_RES
<222> (5)..(5)
<223> Phosphorylated Tyr

<400> 100
Ala Glu Ser Leu Tyr Gln Ser Lys Tyr Glu Glu
1               5                   10

<210> 101
<211> 11
<212> PRT
<213> Homo sapiens

<220>
<221> MOD_RES
<222> (9)..(9)
<223> Phosphorylated Tyr

<400> 101

Ala Glu Ser Leu Tyr Gln Ser Lys Tyr Glu Glu
1               5                   10

<210> 102
<211> 12
<212> PRT
<213> Homo sapiens

<220>
<221> MOD_RES
<222> (7)..(7)
<223> Phosphorylated Tyr

<400> 102
Ala Leu Glu Glu Ser Asn Tyr Glu Leu Glu Gly Lys
1               5                   10

<210> 103
<211> 9
<212> PRT
<213> Homo sapiens

<220>
<221> MOD_RES
<222> (3)..(3)
<223> Phosphorylated Tyr

<400> 103
Ala Gln Tyr Glu Glu Ile Ala Gln Arg
1               5

<210> 104
<211> 9
<212> PRT

<213> Homo sapiens

<220>
<221> MOD_RES
<222> (3)..(3)
<223> Phosphorylated Tyr

<400> 104
Ala Gln Tyr Glu Glu Ile Ala Gln Arg
1               5

<210> 105
<211> 9
<212> PRT
<213> Homo sapiens

<220>
<221> MOD_RES
<222> (3)..(3)
<223> Phosphorylated Tyr

<400> 105
Ala Gln Tyr Glu Glu Ile Ala Gln Arg
1               5

<210> 106
<211> 15
<212> PRT
<213> Homo sapiens

<220>
<221> MOD_RES
<222> (6)..(6)

<223> Phosphorylated Tyr

<400> 106
Asp Val Asp Ala Ala Tyr Met Asn Lys Val Glu Leu Gln Ala Lys
1               5                   10                  15

<210> 107
<211> 9
<212> PRT
<213> Homo sapiens

<220>
<221> MOD_RES
<222> (3)..(3)
<223> Phosphorylated Tyr

<400> 107
Ala Gln Tyr Glu Glu Ile Ala Gln Arg
1               5

<210> 108
<211> 26
<212> PRT
<213> Homo sapiens

<220>
<221> MOD_RES
<222> (18)..(18)
<223> Phosphorylated Tyr

<400> 108
Ala Ile Gly Gly Gly Leu Ser Ser Val Gly Gly Gly Ser Ser Thr Ile
1               5                   10                  15

Lys Tyr Thr Thr Thr Ser Ser Ser Ser Arg
            20                  25

<210> 109
<211> 20
<212> PRT
<213> Homo sapiens

<220>
<221> MOD_RES
<222> (12)..(12)
<223> Phosphorylated Tyr

<400> 109
Ala Val Asn Ala Ala Gly Ala Ser Glu Pro Lys Tyr Tyr Ser Gln Pro
1               5                   10                  15

Ile Leu Val Lys
            20

<210> 110
<211> 28
<212> PRT
<213> Homo sapiens

<220>
<221> MOD_RES
<222> (17)..(17)
<223> Phosphorylated Tyr

<400> 110
Ala Asn Ala Pro Ser Cys Asp Arg Leu Glu Asp Leu Ala Ser Leu Val
1               5                   10                  15

Tyr Leu Asn Glu Ser Ser Val Leu His Thr Leu Arg
            20                  25

<210> 111
<211> 14
<212> PRT
<213> Homo sapiens

<220>
<221> MOD_RES
<222> (10)..(10)
<223> Phosphorylated Tyr

<400> 111
Asp Ile Ala Ser Asp Tyr Lys Tyr Lys Tyr Asn Tyr Glu Lys
1               5                   10

<210> 112
<211> 13
<212> PRT
<213> Homo sapiens

<220>
<221> MOD_RES
<222> (6)..(6)
<223> Phosphorylated Tyr

<400> 112
Asp Ile Ala Ser Asp Tyr Lys Tyr Lys Glu Ala Tyr Arg
1               5                   10

<210> 113

<211> 13
<212> PRT
<213> Homo sapiens

<220>
<221> MOD_RES
<222> (6)..(6)
<223> Phosphorylated Tyr

<400> 113
Asp Ile Ala Ser Asp Tyr Lys Tyr Lys Glu Gly Tyr Arg
1               5                   10

<210> 114
<211> 9
<212> PRT
<213> Homo sapiens

<220>
<221> MOD_RES
<222> (6)..(6)
<223> Phosphorylated Tyr

<400> 114
Asp Ile Ala Ser Asp Tyr Lys Tyr Lys
1               5

<210> 115
<211> 12
<212> PRT
<213> Homo sapiens

<220>

<221> MOD_RES
<222> (10)..(10)
<223> Phosphorylated Tyr

<400> 115
Ala Gln Ala Leu Gln Glu Glu Cys Gly Tyr Leu Arg
1               5                   10

<210> 116
<211> 13
<212> PRT
<213> Homo sapiens

<220>
<221> MOD_RES
<222> (10)..(10)
<223> Phosphorylated Tyr

<400> 116
Ala Glu Glu Asp Pro Met Glu Asp Pro Tyr Glu Leu Lys
1               5                   10

<210> 117
<211> 12
<212> PRT
<213> Homo sapiens

<220>
<221> MOD_RES
<222> (6)..(6)
<223> Phosphorylated Tyr

<400> 117
Asp Asp Thr His Tyr Tyr Ile Gln Ala Ser Ser Lys
1               5                   10

<210> 118
<211> 16
<212> PRT
<213> Homo sapiens

<220>
<221> MOD_RES
<222> (9)..(9)
<223> Phosphorylated Tyr

<400> 118
Ala Ala Gly Gly Gly Gly Pro Leu Tyr Gly Asp Gly Tyr Gly Phe Arg
1               5                   10                  15

<210> 119
<211> 10
<212> PRT
<213> Homo sapiens

<220>
<221> MOD_RES
<222> (7)..(7)
<223> Phosphorylated Tyr

<400> 119
Asp Asp Asp Ser Asp Leu Tyr Ser Pro Arg
1               5                   10

<210> 120
<211> 22
<212> PRT
<213> Homo sapiens

<220>
<221> MOD_RES
<222> (6)..(6)
<223> Phosphorylated Tyr

<400> 120
Asp Lys Trp Asp Gln Tyr Lys Gly Val Gly Val Ala Leu Asp Asp Pro
1               5                   10                  15

Tyr Glu Asn Tyr Arg Arg
            20

<210> 121
<211> 16
<212> PRT
<213> Homo sapiens

<220>
<221> MOD_RES
<222> (14)..(14)
<223> Phosphorylated Tyr

<400> 121
Arg Ile Leu Asn Val Pro Gln Glu Leu Tyr Glu Lys Gly Tyr Val Lys
1               5                   10                  15

<210> 122
<211> 17
<212> PRT
<213> Homo sapiens

<220>

<221> MOD_RES
<222> (10)..(10)
<223> Phosphorylated Tyr

<400> 122
Asp Glu Pro Ser Val Ala Ala Met Val Tyr Pro Phe Thr Gly Asp His
1               5                   10                  15

Lys

<210> 123
<211> 12
<212> PRT
<213> Homo sapiens

<220>
<221> MOD_RES
<222> (2)..(2)
<223> Phosphorylated Tyr

<400> 123
Leu Tyr Arg Pro Gly Ser Val Ala Tyr Val Ser Arg
1               5                   10

<210> 124
<211> 14
<212> PRT
<213> Homo sapiens

<220>
<221> MOD_RES
<222> (4)..(4)

<223> Phosphorylated Tyr

<400> 124
Gly Lys Cys Tyr Gly Leu His Ala Phe Ile Val Pro Ile Arg
1               5                   10

<210> 125
<211> 11
<212> PRT
<213> Homo sapiens

<220>
<221> MOD_RES
<222> (6)..(6)
<223> Phosphorylated Tyr

<400> 125
Leu Ala Gln Gly Glu Tyr Ile Ala Pro Glu Lys
1               5                   10

<210> 126
<211> 14
<212> PRT
<213> Homo sapiens

<220>
<221> MOD_RES
<222> (13)..(13)
<223> Phosphorylated Tyr

<400> 126
Gly Leu Ala Val Ser Asp Asn Gly Pro Cys Leu Gly Tyr Arg
1               5                   10

<210> 127
<211> 11
<212> PRT
<213> Homo sapiens

<220>
<221> MOD_RES
<222> (1)..(1)
<223> Phosphorylated Tyr

<400> 127
Tyr Ala Trp Thr Ser Leu Ala Leu Thr Leu Leu
1               5                   10

<210> 128

<400> 128
000

<210> 129
<211> 11
<212> PRT
<213> Homo sapiens

<220>
<221> MOD_RES
<222> (10)..(10)
<223> Phosphorylated Tyr

<400> 129
Gln Gln Ile Gly Ser Ser Ala Met Pro Tyr Lys
1               5                   10

<210> 130

<211> 13

<212> PRT

<213> Homo sapiens

<220>

<221> MOD_RES

<222> (5)..(5)

<223> Phosphorylated Tyr

<400> 130

Gly Gly Gly Asp Tyr Leu Lys Lys Pro Gly Gly Gly Gly

1               5                   10

<210> 131

<211> 25

<212> PRT

<213> Homo sapiens

<220>

<221> MOD_RES

<222> (9)..(9)

<223> Phosphorylated Tyr

<400> 131

Thr Leu Ser Asp Leu Lys Leu Asp Tyr Leu Asp Leu Tyr Leu Ile His

1               5                   10                  15

Trp Pro Thr Gly Phe Lys Pro Gly Lys

            20                  25

<210> 132

<211> 27

<212> PRT

<213> Homo sapiens

<220>
<221> MOD_RES
<222> (6)..(6)
<223> Phosphorylated Tyr

<400> 132
Asn Leu Gln Leu Asp Tyr Val Asp Leu Tyr Leu Ile His Phe Pro Val
1               5                   10                  15

Ser Val Lys Pro Gly Glu Glu Val Ile Pro Lys
            20                  25

<210> 133
<211> 27
<212> PRT
<213> Homo sapiens

<220>
<221> MOD_RES
<222> (6)..(6)
<223> Phosphorylated Tyr

<400> 133
Asn Leu Gln Leu Asp Tyr Val Asp Leu Tyr Leu Ile His Phe Pro Val
1               5                   10                  15

Ser Val Lys Pro Gly Glu Glu Val Ile Pro Lys
            20                  25

<210> 134

<211> 22

<212> PRT

<213> Homo sapiens

<220>

<221> MOD_RES

<222> (15)..(15)

<223> Phosphorylated Tyr

<400> 134

Leu Asn Asp Gly His Phe Met Pro Val Leu Gly Phe Gly Thr Tyr Ala

1               5                   10                  15

Pro Ala Glu Val Pro Lys

            20

<210> 135

<211> 31

<212> PRT

<213> Homo sapiens

<220>

<221> MOD_RES

<222> (5)..(5)

<223> Phosphorylated Tyr

<400> 135

Ala Gln Leu Asp Tyr Val Asp Leu Tyr Leu Ile His Ser Pro Met Ser

1               5                   10                  15

Leu Lys Pro Gly Glu Glu Leu Ser Pro Thr Asp Glu Asn Gly Lys

            20                  25                  30

<210> 136
<211> 22
<212> PRT
<213> Homo sapiens

<220>
<221> MOD_RES
<222> (4)..(4)
<223> Phosphorylated Tyr

<400> 136
Asn Leu His Tyr Phe Asn Ser Asp Ser Phe Ala Ser His Pro Asn Tyr
1               5                   10                  15

Pro Tyr Ser Asp Glu Tyr
            20

<210> 137
<211> 22
<212> PRT
<213> Homo sapiens

<220>
<221> MOD_RES
<222> (18)..(18)
<223> Phosphorylated Tyr

<400> 137
Asn Leu His Tyr Phe Asn Ser Asp Ser Phe Ala Ser His Pro Asn Tyr
1               5                   10                  15

Pro Tyr Ser Asp Glu Tyr
            20

<210> 138

<211> 15

<212> PRT

<213> Homo sapiens

<220>

<221> MOD_RES

<222> (2)..(2)

<223> Phosphorylated Tyr

<400> 138

Phe Tyr Ala Tyr Asn Pro Leu Ala Gly Gly Leu Leu Thr Gly Lys

1               5                   10                  15

<210> 139

<211> 15

<212> PRT

<213> Homo sapiens

<220>

<221> MOD_RES

<222> (4)..(4)

<223> Phosphorylated Tyr

<400> 139

Phe Tyr Ala Tyr Asn Pro Leu Ala Gly Gly Leu Leu Thr Gly Lys

1               5                   10                  15

<210> 140

<211> 10

<212> PRT

<213> Homo sapiens

<220>
<221> MOD_RES
<222> (4)..(4)
<223> Phosphorylated Tyr

<400> 140
Val Leu Gly Tyr Ile Gln Leu Gly Gln Lys
1               5                   10

<210> 141
<211> 26
<212> PRT
<213> Homo sapiens

<220>
<221> MOD_RES
<222> (8)..(8)
<223> Phosphorylated Tyr

<400> 141
Gln Ala Gly Ala Phe Leu Thr Tyr Ser Ser Phe Cys Pro Pro Asp Asp
1               5                   10                  15

Leu Ala Asp Arg Gly Leu Leu Gly Val Lys
            20                  25

<210> 142
<211> 21
<212> PRT
<213> Homo sapiens

<220>

<221> MOD_RES
<222> (11)..(11)
<223> Phosphorylated Tyr

<400> 142
Asp Asp Gly Asp Gly Gly Trp Val Asp Thr Tyr His Asn Thr Gly Ile
1               5                   10                  15

Thr Gly Ile Thr Glu
            20

<210> 143
<211> 12
<212> PRT
<213> Homo sapiens

<220>
<221> MOD_RES
<222> (2)..(2)
<223> Phosphorylated Tyr

<400> 143
Tyr Tyr Ala Asp Gly Glu Asp Ala Tyr Ala Met Lys
1               5                   10

<210> 144
<211> 25
<212> PRT
<213> Homo sapiens

<220>
<221> MOD_RES
<222> (22)..(22)
<223> Phosphorylated Tyr

<400> 144
Asn Ala Arg Pro Glu Asp Leu Met Asn Met Gln His Cys Asn Leu Leu
1               5                   10                  15

Cys Leu Pro Glu Asn Tyr Gln Met Lys
            20                  25

<210> 145
<211> 13
<212> PRT
<213> Homo sapiens

<220>
<221> MOD_RES
<222> (6)..(6)
<223> Phosphorylated Tyr

<400> 145
Lys Thr Ser Arg Ser Tyr Pro Glu Ile Leu Thr Leu Lys
1               5                   10

<210> 146
<211> 10
<212> PRT
<213> Homo sapiens

<220>
<221> MOD_RES
<222> (8)..(8)
<223> Phosphorylated Tyr

<400> 146

Ala Leu Leu His Gly Asn Cys Tyr Asn Arg
1               5                   10

<210> 147
<211> 21
<212> PRT
<213> Homo sapiens

<220>
<221> MOD_RES
<222> (12)..(12)
<223> Phosphorylated Tyr

<400> 147
Ala Glu Asn Asn Ser Glu Val Gly Ala Ser Gly Tyr Gly Val Pro Gly
1               5                   10                  15

Pro Thr Trp Asp Arg
            20

<210> 148
<211> 13
<212> PRT
<213> Homo sapiens

<220>
<221> MOD_RES
<222> (9)..(9)
<223> Phosphorylated Tyr

<400> 148
Cys Leu Gly His Pro Glu Glu Phe Tyr Asn Leu Val Arg
1               5                   10

<210> 149

<211> 13

<212> PRT

<213> Homo sapiens

<220>

<221> MOD_RES

<222> (7)..(7)

<223> Phosphorylated Tyr

<400> 149

Asp Asp Asn Gly Lys Pro Tyr Val Leu Pro Ser Val Arg

1               5                   10

<210> 150

<211> 9

<212> PRT

<213> Homo sapiens

<220>

<221> MOD_RES

<222> (6)..(6)

<223> Phosphorylated Tyr

<400> 150

Ala Leu Glu Arg Pro Tyr Thr Ser Lys

1               5

<210> 151

<211> 22

<212> PRT

<213> Homo sapiens

<220>

<221> MOD_RES

<222> (14)..(14)

<223> Phosphorylated Tyr

<400> 151

Asp Thr Leu Ser Asn Pro Gln Ser Pro Gln Pro Ser Pro Tyr Asn Ser

1               5                   10                  15

Pro Lys Pro Gln His Lys

            20

<210> 152

<211> 27

<212> PRT

<213> Homo sapiens

<220>

<221> MOD_RES

<222> (4)..(4)

<223> Phosphorylated Tyr

<400> 152

Cys Tyr Tyr Tyr Glu Asp Thr Ile Ser Thr Ile Lys Asp Ile Ala Val

1               5                   10                  15

Glu Glu Asp Leu Ser Ser Thr Pro Leu Leu Lys

            20                  25

<210> 153

<211> 10

<212> PRT

<213> Homo sapiens

<220>
<221> MOD_RES
<222> (7)..(7)
<223> Phosphorylated Tyr

<400> 153
Ala Ala Ala Ser Thr Asp Tyr Tyr Lys Arg
1               5                   10

<210> 154
<211> 10
<212> PRT
<213> Homo sapiens

<220>
<221> MOD_RES
<222> (8)..(8)
<223> Phosphorylated Tyr

<400> 154
Ala Ala Ala Ser Thr Asp Tyr Tyr Lys Arg
1               5                   10

<210> 155
<211> 12
<212> PRT
<213> Homo sapiens

<220>
<221> MOD_RES
<222> (3)..(3)
<223> Phosphorylated Tyr

<400> 155

Ala Ile Tyr Val Glu Tyr Gly Tyr His Ile Thr Lys

1               5                   10

<210> 156

<211> 12

<212> PRT

<213> Homo sapiens

<220>

<221> MOD_RES

<222> (6)..(6)

<223> Phosphorylated Tyr

<400> 156

Ala Ile Tyr Val Glu Tyr Gly Tyr His Ile Thr Lys

1               5                   10

<210> 157

<211> 15

<212> PRT

<213> Homo sapiens

<220>

<221> MOD_RES

<222> (13)..(13)

<223> Phosphorylated Tyr

<400> 157

Asp Tyr Met Pro Ile Lys Asn Thr Asn Gln Asp Ile Tyr Arg Glu

1               5                   10                  15

<210> 158

<211> 23
<212> PRT
<213> Homo sapiens

<220>
<221> MOD_RES
<222> (12)..(12)
<223> Phosphorylated Tyr

<400> 158
Ala Arg Pro Glu Phe Thr Leu Pro Val His Phe Tyr Gly His Val Glu
1               5                   10                  15

His Thr Ser Gln Gly Ala Lys
            20

<210> 159
<211> 15
<212> PRT
<213> Homo sapiens

<220>
<221> MOD_RES
<222> (6)..(6)
<223> Phosphorylated Tyr

<400> 159
Trp Asn Ser Lys Leu Tyr Asp Leu Pro Asp Glu Pro Phe Thr Arg
1               5                   10                  15

<210> 160
<211> 26
<212> PRT

<213> Homo sapiens

<220>
<221> MOD_RES
<222> (25)..(25)
<223> Phosphorylated Tyr

<400> 160
Ala Gln Lys Lys Phe Gly Asn Val Lys Ala Ile Ser Ser Asp Met Tyr
1               5                   10                  15

Phe Gly Arg Gln Ser Gln Ala Asp Tyr Glu
            20                  25

<210> 161
<211> 29
<212> PRT
<213> Homo sapiens

<220>
<221> MOD_RES
<222> (18)..(18)
<223> Phosphorylated Tyr

<400> 161
His Arg Asn Trp Tyr Ile Gln Ala Thr Cys Ala Thr Ser Gly Asp Gly
1               5                   10                  15

Leu Tyr Glu Gly Leu Asp Trp Leu Ser Asn Gln Leu Arg
            20                  25

<210> 162
<211> 29

<212> PRT
<213> Homo sapiens

<220>
<221> MOD_RES
<222> (18)..(18)
<223> Phosphorylated Tyr

<400> 162
His Arg Asn Trp Tyr Ile Gln Ala Thr Cys Ala Thr Ser Gly Asp Gly
1               5                   10                  15

Leu Tyr Glu Gly Leu Asp Trp Leu Ala Asn Gln Leu Lys
            20                  25

<210> 163

<400> 163
000

<210> 164
<211> 17
<212> PRT
<213> Homo sapiens

<220>
<221> MOD_RES
<222> (9)..(9)
<223> Phosphorylated Tyr

<400> 164
His Val Asp Asp Gln Gly Arg Gln Tyr Tyr Tyr Ser Ala Asp Gly Ser
1               5                   10                  15

Arg

<210> 165
<211> 17
<212> PRT
<213> Homo sapiens

<220>
<221> MOD_RES
<222> (10)..(10)
<223> Phosphorylated Tyr

<400> 165
His Val Asp Asp Gln Gly Arg Gln Tyr Tyr Tyr Ser Ala Asp Gly Ser
1               5                   10                  15

Arg

<210> 166
<211> 17
<212> PRT
<213> Homo sapiens

<220>
<221> MOD_RES
<222> (11)..(11)
<223> Phosphorylated Tyr

<400> 166
His Val Asp Asp Gln Gly Arg Gln Tyr Tyr Tyr Ser Ala Asp Gly Ser

1               5                   10                  15

Arg

<210> 167
<211> 10
<212> PRT
<213> Homo sapiens

<220>
<221> MOD_RES
<222> (3)..(3)
<223> Phosphorylated Tyr

<400> 167
Ala Phe Tyr Val Pro Ala Gln Tyr Val Lys
1               5                   10

<210> 168
<211> 15
<212> PRT
<213> Homo sapiens

<220>
<221> MOD_RES
<222> (8)..(8)
<223> Phosphorylated Tyr

<400> 168
Ala Phe Tyr Val Pro Ala Gln Tyr Val Lys Glu Val Thr Arg Lys
1               5                   10                  15

<210> 169

<211> 15

<212> PRT

<213> Homo sapiens

<220>

<221> MOD_RES

<222> (4)..(4)

<223> Phosphorylated Tyr

<400> 169

Ser Gly Asn Tyr Ser Gly His Ser Asp Gly Ile Ser Ser Ser Arg

1               5                   10                  15

<210> 170

<211> 10

<212> PRT

<213> Homo sapiens

<220>

<221> MOD_RES

<222> (5)..(5)

<223> Phosphorylated Tyr

<400> 170

Thr Tyr Lys Glu Tyr Ile Asp Asn Arg Arg

1               5                   10

<210> 171

<211> 13

<212> PRT

<213> Homo sapiens

<220>
<221> MOD_RES
<222> (10)..(10)
<223> Phosphorylated Tyr

<400> 171
Thr Gly Thr Gly Asp Ile Ala Thr Cys Tyr Ser Pro Arg
1               5                   10

<210> 172
<211> 20
<212> PRT
<213> Homo sapiens

<220>
<221> MOD_RES
<222> (5)..(5)
<223> Phosphorylated Tyr

<400> 172
Val Ile Glu Ala Tyr Cys Thr Ser Ala Asn Phe Gln Gln Gly His Gly
1               5                   10                  15

Ser Ser Thr Arg
            20

<210> 173
<211> 28
<212> PRT
<213> Homo sapiens

<220>
<221> MOD_RES

<222> (8)..(8)
<223> Phosphorylated Tyr

<400> 173
Ile Phe Asp Pro Asp Asp Leu Tyr Ser Gly Val Asn Phe Ser Lys Val
1               5                   10                  15

Leu Ser Thr Leu Leu Ala Val Asn Lys Ala Thr Glu
            20                  25

<210> 174
<211> 24
<212> PRT
<213> Homo sapiens

<220>
<221> MOD_RES
<222> (13)..(13)
<223> Phosphorylated Tyr

<400> 174
Asp Leu Leu Gly Ala Ile Ser Gly Ile Ala Ser Pro Tyr Thr Thr Ser
1               5                   10                  15

Thr Pro Asn Ile Asn Ser Val Arg
            20

<210> 175
<211> 15
<212> PRT
<213> Homo sapiens

<220>

<221> MOD_RES
<222> (13)..(13)
<223> Phosphorylated Tyr

<400> 175
Asp Ser Ser Ser Ser Leu Thr Asp Pro Gln Val Ser Tyr Val Lys
1               5                   10                  15

<210> 176
<211> 13
<212> PRT
<213> Homo sapiens

<220>
<221> MOD_RES
<222> (2)..(2)
<223> Phosphorylated Tyr

<400> 176
Ala Tyr Ser Thr Glu Asn Tyr Ser Leu Glu Ser Gln Lys
1               5                   10

<210> 177
<211> 18
<212> PRT
<213> Homo sapiens

<220>
<221> MOD_RES
<222> (9)..(9)
<223> Phosphorylated Tyr

<400> 177
Ala Met Asp Gly Arg Glu Pro Val Tyr Asn Ser Asn Lys Asp Ser Gln

1               5                   10                  15

Ser Glu

<210> 178

<400> 178
000

<210> 179
<211> 21
<212> PRT
<213> Homo sapiens

<220>
<221> MOD_RES
<222> (18)..(18)
<223> Phosphorylated Tyr

<400> 179
Ala Leu Asn Arg Asp Leu Val Pro Thr Gly Val Leu Val Thr Asn Gly
1               5                   10                  15

Asp Tyr Asp Gln Glu
            20

<210> 180
<211> 11
<212> PRT
<213> Homo sapiens

<220>
<221> MOD_RES
<222> (8)..(8)
<223> Phosphorylated Tyr

<400> 180
Asp Thr Ala Gly Gln Glu Asp Tyr Asp Arg Leu
1               5                   10

<210> 181
<211> 11
<212> PRT
<213> Homo sapiens

<220>
<221> MOD_RES
<222> (8)..(8)
<223> Phosphorylated Tyr

<400> 181
Asp Thr Ala Gly Gln Glu Asp Tyr Asp Arg Leu
1               5                   10

<210> 182
<211> 10
<212> PRT
<213> Homo sapiens

<220>
<221> MOD_RES
<222> (4)..(4)
<223> Phosphorylated Tyr

<400> 182

Asp Ser Gly Tyr Asp Ser Leu Ser Asn Arg
1               5                   10

<210> 183
<211> 17
<212> PRT
<213> Homo sapiens

<220>
<221> MOD_RES
<222> (3)..(3)
<223> Phosphorylated Tyr

<400> 183
Ala Gly Tyr Val Gly Leu Val Thr Val Pro Val Ala Thr Leu Ala Gly
1               5                   10                  15

Arg

<210> 184
<211> 11
<212> PRT
<213> Homo sapiens

<220>
<221> MOD_RES
<222> (4)..(4)
<223> Phosphorylated Tyr

<400> 184
Val Gly Phe Tyr Glu Ser Asp Val Met Gly Arg
1               5                   10

<210> 185
<211> 23
<212> PRT
<213> Homo sapiens

<220>
<221> MOD_RES
<222> (10)..(10)
<223> Phosphorylated Tyr

<400> 185
Ala Lys Phe Glu Ser Leu Phe Arg Thr Tyr Asp Lys Asp Ile Thr Phe
1               5                   10                  15

Gln Tyr Phe Lys Ser Phe Lys
            20

<210> 186
<211> 16
<212> PRT
<213> Homo sapiens

<220>
<221> MOD_RES
<222> (7)..(7)
<223> Phosphorylated Tyr

<400> 186
Ala Leu Gly Asn Asn Phe Tyr Glu Tyr Tyr Val Asp Asp Pro Pro Arg
1               5                   10                  15

<210> 187

<211> 16

<212> PRT

<213> Homo sapiens

<220>

<221> MOD_RES

<222> (9)..(9)

<223> Phosphorylated Tyr

<400> 187

Ala Leu Gly Asn Asn Phe Tyr Glu Tyr Tyr Val Asp Asp Pro Pro Arg

1               5                   10                  15

<210> 188

<211> 30

<212> PRT

<213> Homo sapiens

<220>

<221> MOD_RES

<222> (29)..(29)

<223> Phosphorylated Tyr

<400> 188

Ala Pro Leu Asn Ser Ala Val Pro Ser Ala Gly Ala Ser Val Ile Gln

1               5                   10                  15

Pro Ser Ser Ser Pro Leu Glu Ala Ser Ser Val Asn Tyr Glu

            20                  25                  30

<210> 189

<211> 12

<212> PRT

<213> Homo sapiens

<220>
<221> MOD_RES
<222> (5)..(5)
<223> Phosphorylated Tyr

<400> 189
Ala Gly Cys Val Tyr Phe Gly Thr Pro Glu Asn Lys
1               5                   10

<210> 190
<211> 12
<212> PRT
<213> Homo sapiens

<220>
<221> MOD_RES
<222> (4)..(4)
<223> Phosphorylated Tyr

<400> 190
Cys Asn Gly Tyr Cys Asn Glu Cys Phe Gln Phe Lys
1               5                   10

<210> 191
<211> 23
<212> PRT
<213> Homo sapiens

<220>
<221> MOD_RES
<222> (18)..(18)
<223> Phosphorylated Tyr

<400> 191
Thr Asp Leu Asn Pro Asp Asn Leu Gln Gly Gly Asp Asp Leu Asp Pro
1               5                   10                  15

Asn Tyr Val Leu Ser Ser Arg
            20

<210> 192
<211> 11
<212> PRT
<213> Homo sapiens

<220>
<221> MOD_RES
<222> (7)..(7)
<223> Phosphorylated Tyr

<400> 192
Val Leu Thr Pro Glu Leu Tyr Ala Glu Leu Arg
1               5                   10

<210> 193
<211> 26
<212> PRT
<213> Homo sapiens

<220>
<221> MOD_RES
<222> (13)..(13)
<223> Phosphorylated Tyr

<400> 193
Ala Gly His Pro Phe Met Trp Asn Gln His Leu Gly Tyr Val Leu Thr

1               5                   10                  15

Cys Pro Ser Asn Leu Gly Thr Gly Leu Arg
            20                  25

<210> 194
<211> 25
<212> PRT
<213> Homo sapiens

<220>
<221> MOD_RES
<222> (6)..(6)
<223> Phosphorylated Tyr

<400> 194
Ala Gln Pro Pro Asp Tyr Thr Phe Pro Ser Gly Ser Gly Ala His Phe
1               5                   10                  15

Pro Gln Val Pro Gly Gly Ala Val Arg
            20                  25

<210> 195
<211> 13
<212> PRT
<213> Homo sapiens

<220>
<221> MOD_RES
<222> (3)..(3)
<223> Phosphorylated Tyr

<400> 195
Asp Phe Tyr Glu Leu Glu Pro Glu Lys Phe Gln Asn Lys
1               5                   10

<210> 196
<211> 14
<212> PRT
<213> Homo sapiens

<220>
<221> MOD_RES
<222> (7)..(7)
<223> Phosphorylated Tyr

<400> 196
Ala Asp Ser Ile Glu Ile Tyr Ile Pro Glu Ala Gln Thr Arg
1               5                   10

<210> 197
<211> 14
<212> PRT
<213> Homo sapiens

<220>
<221> MOD_RES
<222> (7)..(7)
<223> Phosphorylated Tyr

<400> 197
Ala Asp Ser Ile Glu Ile Tyr Ile Pro Glu Ala Gln Thr Arg
1               5                   10

<210> 198
<211> 18

<212> PRT
<213> Homo sapiens

<220>
<221> MOD_RES
<222> (10)..(10)
<223> Phosphorylated Tyr

<400> 198
Asp Ser Cys Asn Asp Ser Met Gln Ser Tyr Asp Leu Asp Tyr Pro Glu
1               5                   10                  15

Arg Arg

<210> 199
<211> 18
<212> PRT
<213> Homo sapiens

<220>
<221> MOD_RES
<222> (14)..(14)
<223> Phosphorylated Tyr

<400> 199
Asp Ser Cys Asn Asp Ser Met Gln Ser Tyr Asp Leu Asp Tyr Pro Glu
1               5                   10                  15

Arg Arg

<210> 200

<211> 15

<212> PRT

<213> Homo sapiens

<220>

<221> MOD_RES

<222> (7)..(7)

<223> Phosphorylated Tyr

<400> 200

Phe Asn Pro Glu Thr Asp Tyr Leu Thr Gly Thr Asp Gly Lys Lys

1               5                   10                  15

<210> 201

<211> 15

<212> PRT

<213> Homo sapiens

<220>

<221> MOD_RES

<222> (12)..(12)

<223> Phosphorylated Tyr

<400> 201

Ala Tyr Glu Asp Gln Thr Lys Pro Val Leu Glu Tyr Tyr Gln Lys

1               5                   10                  15

<210> 202

<211> 10

<212> PRT

<213> Homo sapiens

<220>

<221> MOD_RES
<222> (4)..(4)
<223> Phosphorylated Tyr

<400> 202
Cys Val Ala Tyr Thr Gly Asn Asn Met Arg
1               5                   10

<210> 203
<211> 12
<212> PRT
<213> Homo sapiens

<220>
<221> MOD_RES
<222> (10)..(10)
<223> Phosphorylated Tyr

<400> 203
Ala Arg Leu Gln Thr Glu Ser Gly Glu Tyr Ser Arg
1               5                   10

<210> 204
<211> 14
<212> PRT
<213> Homo sapiens

<220>
<221> MOD_RES
<222> (3)..(3)
<223> Phosphorylated Tyr

<400> 204
Ala Ala Tyr Leu Gln Asn Leu Asn Ser Ala Asp Leu Leu Lys

1                5                   10

<210> 205
<211> 10
<212> PRT
<213> Homo sapiens

<220>
<221> MOD_RES
<222> (9)..(9)
<223> Phosphorylated Tyr

<400> 205
Ala Leu Glu Leu Asp Pro Asn Leu Tyr Arg
1                5                   10

<210> 206
<211> 10
<212> PRT
<213> Homo sapiens

<220>
<221> MOD_RES
<222> (9)..(9)
<223> Phosphorylated Tyr

<400> 206
Ala Leu Glu Leu Asp Pro Asn Leu Tyr Arg
1                5                   10

<210> 207
<211> 10
<212> PRT
<213> Homo sapiens

<220>
<221> MOD_RES
<222> (9)..(9)
<223> Phosphorylated Tyr

<400> 207
Ala Leu Glu Leu Asp Pro Asn Leu Tyr Arg
1               5                   10

<210> 208
<211> 14
<212> PRT
<213> Homo sapiens

<220>
<221> MOD_RES
<222> (3)..(3)
<223> Phosphorylated Tyr

<400> 208
Ala Ala Tyr Leu Gln Ser Leu Asn Ser Ala Asp Leu Leu Lys
1               5                   10

<210> 209
<211> 14
<212> PRT
<213> Homo sapiens

<220>
<221> MOD_RES
<222> (3)..(3)
<223> Phosphorylated Tyr

<400> 209

Ala Ala Tyr Leu Gln Ser Leu Asn Ser Ala Asp Leu Leu Lys

1               5                   10

<210> 210

<211> 13

<212> PRT

<213> Homo sapiens

<220>

<221> MOD_RES

<222> (12)..(12)

<223> Phosphorylated Tyr

<400> 210

Asn Trp Arg Val Asp Ser Ala Ala Thr Ser Gly Tyr Glu

1               5                   10

<210> 211

<211> 13

<212> PRT

<213> Homo sapiens

<220>

<221> MOD_RES

<222> (4)..(4)

<223> Phosphorylated Tyr

<400> 211

Cys Ala Glu Tyr Trp Pro Ser Met Glu Glu Gly Thr Arg

1               5                   10

<210> 212

<211> 15

<212> PRT

<213> Homo sapiens

<220>

<221> MOD_RES

<222> (15)..(15)

<223> Phosphorylated Tyr

<400> 212

Ala Lys Tyr Pro Asp Tyr Glu Val Thr Trp Ala Asn Asp Gly Tyr

1               5                   10                  15

<210> 213

<211> 15

<212> PRT

<213> Homo sapiens

<220>

<221> MOD_RES

<222> (9)..(9)

<223> Phosphorylated Tyr

<400> 213

Ala His Gln Leu Val Met Glu Gly Tyr Asn Trp Cys His Asp Arg

1               5                   10                  15

<210> 214

<211> 15

<212> PRT

<213> Homo sapiens

<220>

<221> MOD_RES
<222> (9)..(9)
<223> Phosphorylated Tyr

<400> 214
Ala His Gln Leu Val Met Glu Gly Tyr Asn Trp Cys His Asp Arg
1               5                   10                  15

<210> 215
<211> 15
<212> PRT
<213> Homo sapiens

<220>
<221> MOD_RES
<222> (11)..(11)
<223> Phosphorylated Tyr

<400> 215
Ala Phe Ser Ile Gln Thr Pro Glu Gln Tyr Tyr Phe Cys Tyr Lys
1               5                   10                  15

<210> 216
<211> 16
<212> PRT
<213> Homo sapiens

<220>
<221> MOD_RES
<222> (14)..(14)
<223> Phosphorylated Tyr

<400> 216
Asp Ser Asn Ile Pro Gly Ser Asp Tyr Ile Asn Ala Asn Tyr Ile Lys
1               5                   10                  15

<210> 217
<211> 22
<212> PRT
<213> Homo sapiens

<220>
<221> MOD_RES
<222> (20)..(20)
<223> Phosphorylated Tyr

<400> 217
Arg Pro Pro Pro Ala Pro Pro Val Thr Val Ser Ser Pro Pro Phe Pro
1               5                   10                  15

Val Pro Val Tyr Thr Arg
            20

<210> 218
<211> 9
<212> PRT
<213> Homo sapiens

<220>
<221> MOD_RES
<222> (6)..(6)
<223> Phosphorylated Tyr

<400> 218
Phe Ala Val Pro Thr Tyr Ala Ala Lys
1               5

<210> 219
<211> 12
<212> PRT
<213> Homo sapiens

<220>
<221> MOD_RES
<222> (8)..(8)
<223> Phosphorylated Tyr

<400> 219
Ala Thr Ala Met Gly Lys Asn Tyr Val Asn Gly Lys
1               5                   10

<210> 220
<211> 14
<212> PRT
<213> Homo sapiens

<220>
<221> MOD_RES
<222> (11)..(11)
<223> Phosphorylated Tyr

<400> 220
Asp Ala Tyr Ser Gly Gly Ala Val Asn Leu Tyr His Val Arg
1               5                   10

<210> 221
<211> 11
<212> PRT
<213> Homo sapiens

<220>

<221> MOD_RES
<222> (6)..(6)
<223> Phosphorylated Tyr

<400> 221
Met Leu Asn His Leu Tyr Ala Leu Ser Ile Lys
1               5                   10

<210> 222
<211> 9
<212> PRT
<213> Homo sapiens

<220>
<221> MOD_RES
<222> (5)..(5)
<223> Phosphorylated Tyr

<400> 222
Thr Asp Thr Arg Tyr Phe Asp Glu Glu
1               5

<210> 223
<211> 9
<212> PRT
<213> Homo sapiens

<220>
<221> MOD_RES
<222> (5)..(5)
<223> Phosphorylated Tyr

<400> 223
Thr Asp Thr Arg Tyr Phe Asp Glu Glu

1                   5

<210> 224
<211> 20
<212> PRT
<213> Homo sapiens

<220>
<221> MOD_RES
<222> (9)..(9)
<223> Phosphorylated Tyr

<400> 224
Tyr Leu Phe Pro Glu Asp Pro Ser Tyr Ser Ser Thr Met Ile Asp Asp
1               5                   10                  15

Glu Ala Leu Lys
            20

<210> 225
<211> 15
<212> PRT
<213> Homo sapiens

<220>
<221> MOD_RES
<222> (4)..(4)
<223> Phosphorylated Tyr

<400> 225
Gln Gln Phe Tyr His Ser Val Gln Asp Leu Ser Gly Gly Ser Arg
1               5                   10                  15

<210> 226

<211> 11

<212> PRT

<213> Homo sapiens

<220>

<221> MOD_RES

<222> (6)..(6)

<223> Phosphorylated Tyr

<400> 226

Asp Gly Met Ser Val Tyr Asp Ser Leu Asp Lys

1               5                   10

<210> 227

<211> 10

<212> PRT

<213> Homo sapiens

<220>

<221> MOD_RES

<222> (3)..(3)

<223> Phosphorylated Tyr

<400> 227

Met Ile Tyr Phe Leu Leu Leu Asp Arg Lys

1               5                   10

<210> 228

<211> 18

<212> PRT

<213> Homo sapiens

<220>
<221> MOD_RES
<222> (3)..(3)
<223> Phosphorylated Tyr

<400> 228
Ala Glu Tyr Glu Phe Asp Ser Pro Tyr Trp Asp Asp Ile Ser Asp Ser
1               5                   10                  15

Ala Lys

<210> 229
<211> 18
<212> PRT
<213> Homo sapiens

<220>
<221> MOD_RES
<222> (3)..(3)
<223> Phosphorylated Tyr

<400> 229
Ala Glu Tyr Glu Phe Asp Ser Pro Tyr Trp Asp Asp Ile Ser Asp Ser
1               5                   10                  15

Ala Lys

<210> 230
<211> 19
<212> PRT
<213> Homo sapiens

<220>
<221> MOD_RES
<222> (4)..(4)
<223> Phosphorylated Tyr

<400> 230
Ala Gly Ala Tyr Asp Phe Pro Ser Pro Glu Trp Asp Thr Val Thr Pro
1               5                   10                  15

Glu Ala Lys

<210> 231
<211> 19
<212> PRT
<213> Homo sapiens

<220>
<221> MOD_RES
<222> (4)..(4)
<223> Phosphorylated Tyr

<400> 231
Ala Gly Ala Tyr Asp Phe Pro Ser Pro Glu Trp Asp Thr Val Thr Pro
1               5                   10                  15

Glu Ala Lys

<210> 232
<211> 19
<212> PRT

<213> Homo sapiens

<220>
<221> MOD_RES
<222> (4)..(4)
<223> Phosphorylated Tyr

<400> 232
Ala Gly Ala Tyr Asp Phe Pro Ser Pro Glu Trp Asp Thr Val Thr Pro
1               5                   10                  15

Glu Ala Lys

<210> 233
<211> 19
<212> PRT
<213> Homo sapiens

<220>
<221> MOD_RES
<222> (4)..(4)
<223> Phosphorylated Tyr

<400> 233
Ala Gly Ala Tyr Asp Phe Pro Ser Pro Glu Trp Asp Thr Val Thr Pro
1               5                   10                  15

Glu Ala Lys

<210> 234
<211> 19

<212> PRT
<213> Homo sapiens

<220>
<221> MOD_RES
<222> (9)..(9)
<223> Phosphorylated Tyr

<400> 234
Asp Leu Lys Pro Glu Asn Leu Leu Tyr Ala Thr Pro Ala Pro Asp Ala
1               5                   10                  15

Pro Leu Lys

<210> 235
<211> 14
<212> PRT
<213> Homo sapiens

<220>
<221> MOD_RES
<222> (7)..(7)
<223> Phosphorylated Tyr

<400> 235
Asp Ala Ser Gly Met Leu Tyr Asn Leu Ala Ser Ala Ile Lys
1               5                   10

<210> 236
<211> 18
<212> PRT
<213> Homo sapiens

<220>
<221> MOD_RES
<222> (2)..(2)
<223> Phosphorylated Tyr

<400> 236
Ala Tyr Ser Phe Cys Gly Thr Val Glu Tyr Met Ala Pro Glu Val Val
1               5                   10                  15

Asn Arg

<210> 237
<211> 18
<212> PRT
<213> Homo sapiens

<220>
<221> MOD_RES
<222> (10)..(10)
<223> Phosphorylated Tyr

<400> 237
Ala Tyr Ser Phe Cys Gly Thr Val Glu Tyr Met Ala Pro Glu Val Val
1               5                   10                  15

Asn Arg

<210> 238
<211> 18
<212> PRT

<213> Homo sapiens

<220>
<221> MOD_RES
<222> (3)..(3)
<223> Phosphorylated Tyr

<400> 238
Ala Leu Tyr Leu Ile Ala Thr Asn Gly Thr Pro Glu Leu Gln Asn Pro
1               5                   10                  15

Glu Lys

<210> 239
<211> 18
<212> PRT
<213> Homo sapiens

<220>
<221> MOD_RES
<222> (3)..(3)
<223> Phosphorylated Tyr

<400> 239
Ala Leu Tyr Leu Ile Ala Thr Asn Gly Thr Pro Glu Leu Gln Asn Pro
1               5                   10                  15

Glu Lys

<210> 240

<211> 8

<212> PRT

<213> Homo sapiens

<220>

<221> MOD_RES

<222> (7)..(7)

<223> Phosphorylated Tyr

<400> 240

Ala Pro Val Asp Gly Trp Tyr Lys

1               5

<210> 241

<211> 18

<212> PRT

<213> Homo sapiens

<220>

<221> MOD_RES

<222> (2)..(2)

<223> Phosphorylated Tyr

<400> 241

Ala Tyr Ser Phe Cys Gly Thr Val Glu Tyr Met Ala Pro Glu Val Val

1               5                   10                  15

Asn Arg

<210> 242

<211> 18

<212> PRT

<213> Homo sapiens

<220>
<221> MOD_RES
<222> (10)..(10)
<223> Phosphorylated Tyr

<400> 242
Ala Tyr Ser Phe Cys Gly Thr Val Glu Tyr Met Ala Pro Glu Val Val
1               5                   10                  15

Asn Arg

<210> 243
<211> 20
<212> PRT
<213> Homo sapiens

<220>
<221> MOD_RES
<222> (9)..(9)
<223> Phosphorylated Tyr

<400> 243
Asp Leu Lys Pro Ser Asn Ile Leu Tyr Val Asp Glu Ser Gly Asn Pro
1               5                   10                  15

Glu Ser Ile Arg
            20

<210> 244
<211> 18

<212> PRT
<213> Homo sapiens

<220>
<221> MOD_RES
<222> (2)..(2)
<223> Phosphorylated Tyr

<400> 244
Ala Tyr Ser Phe Cys Gly Thr Ile Glu Tyr Met Ala Pro Glu Val Val
1               5                   10                  15

Asn Arg

<210> 245
<211> 22
<212> PRT
<213> Homo sapiens

<220>
<221> MOD_RES
<222> (11)..(11)
<223> Phosphorylated Tyr

<400> 245
Ala Gly Asn Gly Leu Leu Met Thr Pro Cys Tyr Thr Ala Asn Phe Val
1               5                   10                  15

Ala Pro Glu Val Leu Lys
            20

<210> 246

<211> 18
<212> PRT
<213> Homo sapiens

<220>
<221> MOD_RES
<222> (2)..(2)
<223> Phosphorylated Tyr

<400> 246
Ala Tyr Ser Phe Cys Gly Thr Val Glu Tyr Met Ala Pro Glu Val Val
1               5                   10                  15

Asn Arg

<210> 247
<211> 18
<212> PRT
<213> Homo sapiens

<220>
<221> MOD_RES
<222> (10)..(10)
<223> Phosphorylated Tyr

<400> 247
Ala Tyr Ser Phe Cys Gly Thr Val Glu Tyr Met Ala Pro Glu Val Val
1               5                   10                  15

Asn Arg

<210> 248

<211> 11

<212> PRT

<213> Homo sapiens

<220>

<221> MOD_RES

<222> (10)..(10)

<223> Phosphorylated Tyr

<400> 248

Thr Ile Val Asn Gly Ile Val Glu Asp Tyr Arg

1               5                   10

<210> 249

<211> 24

<212> PRT

<213> Homo sapiens

<220>

<221> MOD_RES

<222> (7)..(7)

<223> Phosphorylated Tyr

<400> 249

Thr Leu Gln Asp Leu Val Tyr Asp Leu Ser Thr Ser Gly Ser Gly Ser

1               5                   10                  15

Gly Leu Pro Leu Phe Val Gln Arg

            20

<210> 250

<211> 11

<212> PRT
<213> Homo sapiens

<220>
<221> MOD_RES
<222> (3)..(3)
<223> Phosphorylated Tyr

<400> 250
Asp Phe Tyr Thr Ala Thr Glu Arg Gln Tyr Glu
1               5                   10

<210> 251
<211> 20
<212> PRT
<213> Homo sapiens

<220>
<221> MOD_RES
<222> (15)..(15)
<223> Phosphorylated Tyr

<400> 251
Asp Gly Ser Ser Gln Gln Leu Gln Gly Tyr Ile Pro Ser Asn Tyr Val
1               5                   10                  15

Ala Glu Asp Arg
            20

<210> 252
<211> 20
<212> PRT
<213> Homo sapiens

<220>
<221> MOD_RES
<222> (10)..(10)
<223> Phosphorylated Tyr

<400> 252
Asp Gly Ser Ser Gln Gln Leu Gln Gly Tyr Ile Pro Ser Asn Tyr Val
1               5                   10                  15

Ala Glu Asp Arg
            20

<210> 253
<211> 17
<212> PRT
<213> Homo sapiens

<220>
<221> MOD_RES
<222> (8)..(8)
<223> Phosphorylated Tyr

<400> 253
Asp Lys Tyr Gly Asn Glu Gly Tyr Ile Pro Ser Asn Tyr Val Thr Gly
1               5                   10                  15

Lys

<210> 254
<211> 18
<212> PRT

<213> Homo sapiens

<220>
<221> MOD_RES
<222> (16)..(16)
<223> Phosphorylated Tyr

<400> 254
Gly Gln Thr Pro Tyr Pro Gly Val Glu Asn Ser Glu Ile Tyr Asp Tyr
1               5                   10                  15

Leu Arg

<210> 255
<211> 11
<212> PRT
<213> Homo sapiens

<220>
<221> MOD_RES
<222> (7)..(7)
<223> Phosphorylated Tyr

<400> 255
Asp Leu Val Ser Cys Thr Tyr Gln Leu Ala Arg
1               5                   10

<210> 256
<211> 9
<212> PRT
<213> Homo sapiens

<220>
<221> MOD_RES
<222> (7)..(7)
<223> Phosphorylated Tyr

<400> 256
Asp Ile Asn Asn Ile Asp Tyr Tyr Lys
1               5

<210> 257
<211> 9
<212> PRT
<213> Homo sapiens

<220>
<221> MOD_RES
<222> (8)..(8)
<223> Phosphorylated Tyr

<400> 257
Asp Ile Asn Asn Ile Asp Tyr Tyr Lys
1               5

<210> 258
<211> 9
<212> PRT
<213> Homo sapiens

<220>
<221> MOD_RES
<222> (7)..(7)
<223> Phosphorylated Tyr

<400> 258

Asp Ile Tyr Arg Ala Ser Tyr Tyr Arg
1               5

<210> 259
<211> 9
<212> PRT
<213> Homo sapiens

<220>
<221> MOD_RES
<222> (8)..(8)
<223> Phosphorylated Tyr

<400> 259
Asp Ile Tyr Arg Ala Ser Tyr Tyr Arg
1               5

<210> 260
<211> 10
<212> PRT
<213> Homo sapiens

<220>
<221> MOD_RES
<222> (3)..(3)
<223> Phosphorylated Tyr

<400> 260
Asp Ile Tyr Lys Asp Pro Asp Tyr Val Arg
1               5                   10

<210> 261
<211> 10

<212> PRT
<213> Homo sapiens

<220>
<221> MOD_RES
<222> (8)..(8)
<223> Phosphorylated Tyr

<400> 261
Asp Ile Tyr Lys Asp Pro Asp Tyr Val Arg
1               5                   10

<210> 262
<211> 28
<212> PRT
<213> Homo sapiens

<220>
<221> MOD_RES
<222> (3)..(3)
<223> Phosphorylated Tyr

<400> 262
Ile Leu Tyr Ala Pro Tyr Asn Pro Val Thr Lys Ala Ile Met Glu Lys
1               5                   10                  15

Ser Asn Val Thr Leu Arg Gln Leu Ala Glu Leu Arg
            20                  25

<210> 263
<211> 28
<212> PRT
<213> Homo sapiens

<220>
<221> MOD_RES
<222> (6)..(6)
<223> Phosphorylated Tyr

<400> 263
Ile Leu Tyr Ala Pro Tyr Asn Pro Val Thr Lys Ala Ile Met Glu Lys
1               5                   10                  15

Ser Asn Val Thr Leu Arg Gln Leu Ala Glu Leu Arg
            20                  25

<210> 264

<400> 264
000

<210> 265

<400> 265
000

<210> 266
<211> 13
<212> PRT
<213> Homo sapiens

<220>
<221> MOD_RES
<222> (2)..(2)
<223> Phosphorylated Tyr

<400> 266
Glu Tyr Asp Asp Lys Lys Asp Phe Leu Leu Ser Arg Lys
1               5                   10

<210> 267
<211> 10
<212> PRT
<213> Homo sapiens

<220>
<221> MOD_RES
<222> (2)..(2)
<223> Phosphorylated Tyr

<400> 267
Ala Tyr Tyr Pro Ser Ile Val Ala Asn Arg
1               5                   10

<210> 268
<211> 19
<212> PRT
<213> Homo sapiens

<220>
<221> MOD_RES
<222> (19)..(19)
<223> Phosphorylated Tyr

<400> 268
Leu Asn Pro Leu Phe Lys Ile Gly His Lys Arg Arg Leu Glu Glu Asp
1               5                   10                  15

Asp Met Tyr

<210> 269

<211> 11

<212> PRT

<213> Homo sapiens

<220>

<221> MOD_RES

<222> (1)..(1)

<223> Phosphorylated Tyr

<400> 269

Tyr Arg Glu Asn Leu Pro Leu Val Leu Lys Lys

1               5                   10

<210> 270

<211> 28

<212> PRT

<213> Homo sapiens

<220>

<221> MOD_RES

<222> (15)..(15)

<223> Phosphorylated Tyr

<400> 270

Gln Leu Gln Glu Gln His Tyr Gln Gln Tyr Met Gln Gln Leu Tyr Gln

1               5                   10                  15

Val Gln Leu Ala Gln Gln Gln Ala Ala Leu Gln Lys

            20                  25

<210> 271

<211> 15

<212> PRT

<213> Homo sapiens

<220>

<221> MOD_RES

<222> (6)..(6)

<223> Phosphorylated Tyr

<400> 271

Arg His Pro Tyr Phe Tyr Ala Pro Glu Leu Leu Phe Phe Ala Lys

1               5                   10                  15

<210> 272

<400> 272

000

<210> 273

<211> 13

<212> PRT

<213> Homo sapiens

<220>

<221> MOD_RES

<222> (11)..(11)

<223> Phosphorylated Tyr

<400> 273

Cys Cys Ala Ala Ala Asp Pro His Glu Cys Tyr Ala Lys

1               5                   10

<210> 274

<211> 12

<212> PRT

<213> Homo sapiens

<220>

<221> MOD_RES

<222> (4)..(4)

<223> Phosphorylated Tyr

<400> 274

Cys Gly Asp Tyr His Pro Glu Gly Glu Ala Pro Arg

1               5                   10

<210> 275

<211> 10

<212> PRT

<213> Homo sapiens

<220>

<221> MOD_RES

<222> (3)..(3)

<223> Phosphorylated Tyr

<400> 275

Gly Ser Tyr Met Glu Val Glu Asp Asn Arg

1               5                   10

<210> 276

<211> 16

<212> PRT

<213> Homo sapiens

<220>

<221> MOD_RES
<222> (3)..(3)
<223> Phosphorylated Tyr

<400> 276
Gly Thr Tyr Glu Pro Asp Glu Asp Trp Glu Glu Gln Arg Glu Glu Arg
1               5                   10                  15

<210> 277
<211> 10
<212> PRT
<213> Homo sapiens

<220>
<221> MOD_RES
<222> (5)..(5)
<223> Phosphorylated Tyr

<400> 277
Asn Val Ala Asp Tyr Tyr Pro Glu Tyr Lys
1               5                   10

<210> 278
<211> 21
<212> PRT
<213> Homo sapiens

<220>
<221> MOD_RES
<222> (20)..(20)
<223> Phosphorylated Tyr

<400> 278
Cys Arg Lys Gln Asp Phe Pro Leu Val Lys Ala Ala Val Gln Lys Ala
1               5                   10                  15

Ile Pro Met Tyr Lys
            20

<210> 279
<211> 12
<212> PRT
<213> Homo sapiens

<220>
<221> MOD_RES
<222> (1)..(1)
<223> Phosphorylated Tyr

<400> 279
Tyr Asp Val Asp Thr Gln Met Trp Thr Ile Leu Lys
1               5                   10

<210> 280
<211> 19
<212> PRT
<213> Homo sapiens

<220>
<221> MOD_RES
<222> (9)..(9)
<223> Phosphorylated Tyr

<400> 280
Gly Thr Asn Pro Glu Gly Leu Ser Tyr Ser Thr Leu Pro Gly Asn Val
1               5                   10                  15

Ile Ser Lys

<210> 281
<211> 12
<212> PRT
<213> Homo sapiens

<220>
<221> MOD_RES
<222> (4)..(4)
<223> Phosphorylated Tyr

<400> 281
Ala Thr Asp Tyr Leu Gln Ala Ser Ala Ile Thr Arg
1               5                   10

<210> 282
<211> 26
<212> PRT
<213> Homo sapiens

<220>
<221> MOD_RES
<222> (9)..(9)
<223> Phosphorylated Tyr

<400> 282
Ala Ala Thr Thr Pro Thr Ala Thr Tyr Asn Ser Asp Arg Asp Asn Ser
1               5                   10                  15

Phe Leu Gln Val His Asn Cys Ile Gln Lys
            20                  25

<210> 283

<211> 13

<212> PRT

<213> Homo sapiens

<220>

<221> MOD_RES

<222> (5)..(5)

<223> Phosphorylated Tyr

<400> 283

Asp Lys Gly Leu Tyr Gln Cys Ile Ile His His Lys Lys

1               5                   10

<210> 284

<211> 16

<212> PRT

<213> Homo sapiens

<220>

<221> MOD_RES

<222> (3)..(3)

<223> Phosphorylated Tyr

<400> 284

Ala Gly Tyr Asp Gly Glu Ser Ile Gly Asn Cys Pro Phe Ser Gln Arg

1               5                   10                  15

<210> 285

<211> 15

<212> PRT

<213> Homo sapiens

<220>

<221> MOD_RES

<222> (13)..(13)

<223> Phosphorylated Tyr

<400> 285

Ala Leu Pro His Leu Ile Gln Asp Ala Gly Gln Phe Tyr Ala Lys

1               5                   10                  15

<210> 286

<211> 27

<212> PRT

<213> Homo sapiens

<220>

<221> MOD_RES

<222> (16)..(16)

<223> Phosphorylated Tyr

<400> 286

Cys Gln Ala Leu His Pro Asp Pro Glu Asp Glu Asp Ser Asp Asp Tyr

1               5                   10                  15

Asp Gly Glu Glu Tyr Asp Val Glu Ala His Glu

            20                  25

<210> 287

<211> 27

<212> PRT

<213> Homo sapiens

<220>

<221> MOD_RES

<222> (21)..(21)
<223> Phosphorylated Tyr

<400> 287
Cys Gln Ala Leu His Pro Asp Pro Glu Asp Glu Asp Ser Asp Asp Tyr
1               5                   10                  15

Asp Gly Glu Glu Tyr Asp Val Glu Ala His Glu
            20                  25

<210> 288
<211> 11
<212> PRT
<213> Homo sapiens

<220>
<221> MOD_RES
<222> (4)..(4)
<223> Phosphorylated Tyr

<400> 288
Asp Ser Leu Tyr Thr Ser Met Pro Asn Leu Arg
1               5                   10

<210> 289

<400> 289
000

<210> 290
<211> 18
<212> PRT
<213> Homo sapiens

<220>
<221> MOD_RES
<222> (14)..(14)
<223> Phosphorylated Tyr

<400> 290
Ala Thr Ser Tyr Thr Leu Val Glu Ser Phe Ser Gly Lys Tyr Val Arg
1               5                   10                  15

Leu Lys

<210> 291
<211> 22
<212> PRT
<213> Homo sapiens

<220>
<221> MOD_RES
<222> (10)..(10)
<223> Phosphorylated Tyr

<400> 291
Ala Val Phe Gln Asp Gln Phe Ser Glu Tyr Lys Glu Leu Ser Ala Glu
1               5                   10                  15

Val Gln Ala Val Leu Arg
            20

<210> 292
<211> 13
<212> PRT

<213> Homo sapiens

<220>
<221> MOD_RES
<222> (6)..(6)
<223> Phosphorylated Tyr

<400> 292
Asp Met Ala Ser Asn Tyr Lys Glu Leu Gly Phe Gln Gly
1               5                   10

<210> 293
<211> 25
<212> PRT
<213> Homo sapiens

<220>
<221> MOD_RES
<222> (9)..(9)
<223> Phosphorylated Tyr

<400> 293
Ala Ser Pro Gly His Ser Pro His Tyr Phe Ala Ala Ser Ser Pro Thr
1               5                   10                  15

Ser Pro Asn Ala Leu Pro Pro Ala Arg
            20                  25

<210> 294
<211> 12
<212> PRT
<213> Homo sapiens

<220>
<221> MOD_RES
<222> (6)..(6)
<223> Phosphorylated Tyr

<400> 294
Ala Ser Thr Ser Asp Tyr Gln Val Ile Ser Asp Arg
1               5                   10

<210> 295
<211> 8
<212> PRT
<213> Homo sapiens

<220>
<221> MOD_RES
<222> (5)..(5)
<223> Phosphorylated Tyr

<400> 295
Cys Ser Ser Leu Tyr Glu Glu Arg
1               5

<210> 296
<211> 14
<212> PRT
<213> Homo sapiens

<220>
<221> MOD_RES
<222> (8)..(8)
<223> Phosphorylated Tyr

<400> 296

Ala Glu Gln Trp Asn Val Asn Tyr Val Glu Thr Ser Ala Lys
1               5                   10

<210> 297
<211> 20
<212> PRT
<213> Homo sapiens

<220>
<221> MOD_RES
<222> (15)..(15)
<223> Phosphorylated Tyr

<400> 297
Ala Cys Pro Asp Ser Leu Gly Ser Pro Ala Pro Ser His Ala Tyr His
1               5                   10                  15

Gly Gly Val Leu
            20

<210> 298
<211> 11
<212> PRT
<213> Homo sapiens

<220>
<221> MOD_RES
<222> (7)..(7)
<223> Phosphorylated Tyr

<400> 298
Ala Asp His Thr Pro His Tyr Pro Glu Gln Glu
1               5                   10

<210> 299

<211> 14

<212> PRT

<213> Homo sapiens

<220>

<221> MOD_RES

<222> (3)..(3)

<223> Phosphorylated Tyr

<400> 299

Ser Ser Tyr Val Asn Met Gln Ala Phe Asp Tyr Glu Gln Lys

1               5                   10

<210> 300

<211> 9

<212> PRT

<213> Homo sapiens

<220>

<221> MOD_RES

<222> (4)..(4)

<223> Phosphorylated Tyr

<400> 300

Ala Leu Gly Tyr Lys Asp Val Ser Arg

1               5

<210> 301

<211> 15

<212> PRT

<213> Homo sapiens

<220>
<221> MOD_RES
<222> (6)..(6)
<223> Phosphorylated Tyr

<400> 301
Asp Trp Gln Ser Tyr Tyr Tyr His His Pro Gln Asp Arg Asp Arg
1               5                   10                  15

<210> 302
<211> 15
<212> PRT
<213> Homo sapiens

<220>
<221> MOD_RES
<222> (7)..(7)
<223> Phosphorylated Tyr

<400> 302
Asp Trp Gln Ser Tyr Tyr Tyr His His Pro Gln Asp Arg Asp Arg
1               5                   10                  15

<210> 303
<211> 19
<212> PRT
<213> Homo sapiens

<220>
<221> MOD_RES
<222> (10)..(10)
<223> Phosphorylated Tyr

<400> 303
Asp Lys Val Ala Gly His Ser Leu Gly Tyr Gly Phe Val Asn Tyr Val
1               5                   10                  15

Thr Ala Lys

<210> 304
<211> 11
<212> PRT
<213> Homo sapiens

<220>
<221> MOD_RES
<222> (10)..(10)
<223> Phosphorylated Tyr

<400> 304
Ala Thr Glu Lys Asp Lys Val Glu Ile Tyr Lys
1               5                   10

<210> 305
<211> 12
<212> PRT
<213> Homo sapiens

<220>
<221> MOD_RES
<222> (2)..(2)
<223> Phosphorylated Tyr

<400> 305
Asp Tyr Ala Pro Pro Pro Arg Asp Tyr Thr Tyr Arg
1               5                   10

<210> 306
<211> 13
<212> PRT
<213> Homo sapiens

<220>
<221> MOD_RES
<222> (4)..(4)
<223> Phosphorylated Tyr

<400> 306
Asp Tyr Thr Tyr Arg Asp Tyr Gly His Ser Ser Ser Arg
1               5                   10

<210> 307
<211> 10
<212> PRT
<213> Homo sapiens

<220>
<221> MOD_RES
<222> (6)..(6)
<223> Phosphorylated Tyr

<400> 307
Asp Ser Tyr Glu Ser Tyr Gly Asn Ser Arg
1               5                   10

<210> 308
<211> 17
<212> PRT
<213> Homo sapiens

<220>
<221> MOD_RES
<222> (7)..(7)
<223> Phosphorylated Tyr

<400> 308
Ala Thr Glu Asn Asp Ile Tyr Asn Phe Phe Ser Pro Leu Asn Pro Met
1               5                   10                  15

Arg

<210> 309
<211> 12
<212> PRT
<213> Homo sapiens

<220>
<221> MOD_RES
<222> (2)..(2)
<223> Phosphorylated Tyr

<400> 309
Asp Tyr Tyr Asp Arg Met Tyr Ser Tyr Pro Ala Arg
1               5                   10

<210> 310
<211> 15
<212> PRT
<213> Homo sapiens

<220>

<221> MOD_RES
<222> (4)..(4)
<223> Phosphorylated Tyr

<400> 310
Cys Leu Asn Tyr Gln His Tyr Lys Gly Ser Asp Phe Asp Cys Glu
1               5                   10                  15

<210> 311
<211> 14
<212> PRT
<213> Homo sapiens

<220>
<221> MOD_RES
<222> (5)..(5)
<223> Phosphorylated Tyr

<400> 311
Ala Phe Glu Lys Tyr Gly Ile Ile Glu Glu Val Val Ile Lys
1               5                   10

<210> 312
<211> 28
<212> PRT
<213> Homo sapiens

<220>
<221> MOD_RES
<222> (19)..(19)
<223> Phosphorylated Tyr

<400> 312
Ala Glu Glu Thr Arg Tyr Pro Gln Gln Tyr Gln Pro Ser Pro Leu Pro

1               5                   10                  15

Val His Tyr Glu Leu Leu Thr Asp Gly Tyr Thr Arg
            20                  25

<210> 313
<211> 23
<212> PRT
<213> Homo sapiens

<220>
<221> MOD_RES
<222> (19)..(19)
<223> Phosphorylated Tyr

<400> 313
Ala Val Leu Ile Asp Glu Val Cys Thr Met Asn Asp Gly Pro His Ser
1               5                   10                  15

Ala Leu Tyr Thr Met Met Lys
            20

<210> 314
<211> 18
<212> PRT
<213> Homo sapiens

<220>
<221> MOD_RES
<222> (8)..(8)
<223> Phosphorylated Tyr

<400> 314

Cys Tyr Cys Ala Asp Val Ile Tyr Pro Met Ala Val Val Ala Thr Ala
1               5                   10                  15

Glu Arg

<210> 315
<211> 16
<212> PRT
<213> Homo sapiens

<220>
<221> MOD_RES
<222> (7)..(7)
<223> Phosphorylated Tyr

<400> 315
Ala Tyr Val Glu Ile Glu Tyr Glu Gln Glu Thr Glu Pro Val Ala Lys
1               5                   10                  15

<210> 316
<211> 15
<212> PRT
<213> Homo sapiens

<220>
<221> MOD_RES
<222> (14)..(14)
<223> Phosphorylated Tyr

<400> 316
Ala Ser Tyr Val Ala Pro Leu Thr Ala Gln Pro Ala Thr Tyr Arg
1               5                   10                  15

<210> 317
<211> 12
<212> PRT
<213> Homo sapiens

<220>
<221> MOD_RES
<222> (11)..(11)
<223> Phosphorylated Tyr

<400> 317
Ala Gln Pro Ser Val Ser Leu Gly Ala Pro Tyr Arg
1               5                   10

<210> 318
<211> 16
<212> PRT
<213> Homo sapiens

<220>
<221> MOD_RES
<222> (7)..(7)
<223> Phosphorylated Tyr

<400> 318
Ala Ser Glu Asn Gly His Tyr Glu Leu Glu His Leu Ser Thr Pro Lys
1               5                   10                  15

<210> 319
<211> 14
<212> PRT
<213> Homo sapiens

<220>
<221> MOD_RES
<222> (9)..(9)
<223> Phosphorylated Tyr

<400> 319
Asp Leu Asp Lys Glu Leu Asp Glu Tyr Met His Gly Gly Lys
1               5                   10

<210> 320
<211> 15
<212> PRT
<213> Homo sapiens

<220>
<221> MOD_RES
<222> (11)..(11)
<223> Phosphorylated Tyr

<400> 320
Ala Gln Gly Val Gly Leu Asp Ser Thr Gly Tyr Tyr Asp Gln Glu
1               5                   10                  15

<210> 321
<211> 15
<212> PRT
<213> Homo sapiens

<220>
<221> MOD_RES
<222> (12)..(12)
<223> Phosphorylated Tyr

<400> 321
Ala Gln Gly Val Gly Leu Asp Ser Thr Gly Tyr Tyr Asp Gln Glu
1               5                   10                  15

<210> 322
<211> 10
<212> PRT
<213> Homo sapiens

<220>
<221> MOD_RES
<222> (4)..(4)
<223> Phosphorylated Tyr

<400> 322
Asp Lys Lys Tyr Tyr Pro Thr Ala Glu Glu
1               5                   10

<210> 323
<211> 15
<212> PRT
<213> Homo sapiens

<220>
<221> MOD_RES
<222> (9)..(9)
<223> Phosphorylated Tyr

<400> 323
Ala Val Ser Ser Asn Val Gly Ala Tyr Met Gln Pro Gly Ala Arg
1               5                   10                  15

<210> 324
<211> 12

<212> PRT
<213> Homo sapiens

<220>
<221> MOD_RES
<222> (6)..(6)
<223> Phosphorylated Tyr

<400> 324
Asp Arg Phe Asp Gly Tyr Val Phe Leu Asp Asn Lys
1               5                   10

<210> 325
<211> 17
<212> PRT
<213> Homo sapiens

<220>
<221> MOD_RES
<222> (3)..(3)
<223> Phosphorylated Tyr

<400> 325
Ala Gly Tyr Ser Gln Gly Ala Thr Gln Tyr Thr Gln Ala Gln Gln Thr
1               5                   10                  15

Arg

<210> 326
<211> 17
<212> PRT
<213> Homo sapiens

<220>
<221> MOD_RES
<222> (10)..(10)
<223> Phosphorylated Tyr

<400> 326
Ala Gly Tyr Ser Gln Gly Ala Thr Gln Tyr Thr Gln Ala Gln Gln Thr
1               5                   10                  15

Arg

<210> 327
<211> 13
<212> PRT
<213> Homo sapiens

<220>
<221> MOD_RES
<222> (1)..(1)
<223> Phosphorylated Tyr

<400> 327
Tyr Leu Ala Ala Val Leu Gly Lys Arg Tyr Lys Gln Arg
1               5                   10

<210> 328
<211> 13
<212> PRT
<213> Homo sapiens

<220>

<221> MOD_RES
<222> (10)..(10)
<223> Phosphorylated Tyr

<400> 328
Tyr Leu Ala Ala Val Leu Gly Lys Arg Tyr Lys Gln Arg
1               5                   10

<210> 329
<211> 17
<212> PRT
<213> Homo sapiens

<220>
<221> MOD_RES
<222> (12)..(12)
<223> Phosphorylated Tyr

<400> 329
Cys Ala Phe Ser Ser Gln Glu Pro Tyr Phe Ser Tyr Ser Gly Ala Phe
1               5                   10                  15

Lys

<210> 330
<211> 11
<212> PRT
<213> Homo sapiens

<220>
<221> MOD_RES
<222> (6)..(6)

<223> Phosphorylated Tyr

<400> 330
Trp Ser Ser Asn Gly Tyr Phe Tyr Ser Gly Lys
1               5                   10

<210> 331
<211> 15
<212> PRT
<213> Homo sapiens

<220>
<221> MOD_RES
<222> (8)..(8)
<223> Phosphorylated Tyr

<400> 331
Met Ile Arg Leu Leu Ile Met Tyr Gly Ala Asp Leu Asn Ile Lys
1               5                   10                  15

<210> 332
<211> 15
<212> PRT
<213> Homo sapiens

<220>
<221> MOD_RES
<222> (4)..(4)
<223> Phosphorylated Tyr

<400> 332
Phe Lys Ser Tyr Leu Tyr Phe Glu Glu Lys Asp Phe Val Asp Lys
1               5                   10                  15

<210> 333
<211> 15
<212> PRT
<213> Homo sapiens

<220>
<221> MOD_RES
<222> (6)..(6)
<223> Phosphorylated Tyr

<400> 333
Phe Lys Ser Tyr Leu Tyr Phe Glu Glu Lys Asp Phe Val Asp Lys
1               5                   10                  15

<210> 334
<211> 18
<212> PRT
<213> Homo sapiens

<220>
<221> MOD_RES
<222> (13)..(13)
<223> Phosphorylated Tyr

<400> 334
Asp Ala Leu Gln Lys Glu Glu Glu Met Glu Gly Asp Tyr Gln Glu Thr
1               5                   10                  15

Trp Lys

<210> 335
<211> 16

<212> PRT
<213> Homo sapiens

<220>
<221> MOD_RES
<222> (11)..(11)
<223> Phosphorylated Tyr

<400> 335
Ala Asp Met Thr Ala Ser Gly Ser Pro Asp Tyr Gly Gln Pro His Lys
1               5                   10                  15

<210> 336
<211> 16
<212> PRT
<213> Homo sapiens

<220>
<221> MOD_RES
<222> (14)..(14)
<223> Phosphorylated Tyr

<400> 336
Asp Arg Thr Gly His Tyr Leu Cys Asn Ala Cys Gly Leu Tyr His Lys
1               5                   10                  15

<210> 337
<211> 16
<212> PRT
<213> Homo sapiens

<220>
<221> MOD_RES
<222> (5)..(5)

<223> Phosphorylated Tyr

<400> 337
Ala Ser Phe Ala Tyr Ala Trp Val Leu Asp Glu Thr Gly Glu Glu Arg
1               5                   10                  15

<210> 338
<211> 25
<212> PRT
<213> Homo sapiens

<220>
<221> MOD_RES
<222> (14)..(14)
<223> Phosphorylated Tyr

<400> 338
Asp Lys Leu Pro Ser Thr Glu Val Lys Glu Asp Ser Ala Tyr Gly Ser
1               5                   10                  15

Gln Ser Val Glu Gln Glu Ala Glu Lys
            20                  25

<210> 339

<400> 339
000

<210> 340
<211> 20
<212> PRT
<213> Homo sapiens

<220>
<221> MOD_RES
<222> (12)..(12)
<223> Phosphorylated Tyr

<400> 340
Ala Met Glu Val Asp Glu Arg Pro Thr Glu Gln Tyr Ser Asp Ile Gly
1               5                   10                  15

Gly Leu Asp Lys
            20

<210> 341
<211> 16
<212> PRT
<213> Homo sapiens

<220>
<221> MOD_RES
<222> (6)..(6)
<223> Phosphorylated Tyr

<400> 341
Asp Cys Arg Asp Gly Tyr Tyr Glu Ala Glu Phe Gly Gln Glu Arg Arg
1               5                   10                  15

<210> 342
<211> 16
<212> PRT
<213> Homo sapiens

<220>
<221> MOD_RES

<222> (7)..(7)
<223> Phosphorylated Tyr

<400> 342
Asp Cys Arg Asp Gly Tyr Tyr Glu Ala Glu Phe Gly Gln Glu Arg Arg
1               5                   10                  15

<210> 343
<211> 12
<212> PRT
<213> Homo sapiens

<220>
<221> MOD_RES
<222> (3)..(3)
<223> Phosphorylated Tyr

<400> 343
Asp Leu Tyr Pro Cys Pro Gly Thr Asp Cys Ser Arg
1               5                   10

<210> 344
<211> 15
<212> PRT
<213> Homo sapiens

<220>
<221> MOD_RES
<222> (8)..(8)
<223> Phosphorylated Tyr

<400> 344
Ala Tyr His Asn Ser Pro Ala Tyr Leu Ala Tyr Ile Asn Ala Lys
1               5                   10                  15

<210> 345

<211> 32

<212> PRT

<213> Homo sapiens

<220>

<221> MOD_RES

<222> (4)..(4)

<223> Phosphorylated Tyr

<400> 345

Ala Leu Leu Tyr Ser Leu Ser Gln Pro Leu Ala Ser Leu Gly Ser Gly

1               5                   10                  15

Phe Phe Gly Glu Pro Asp Ala Phe Pro Met Phe Thr Thr Asn Asn Arg

            20                  25                  30

<210> 346

<211> 15

<212> PRT

<213> Homo sapiens

<220>

<221> MOD_RES

<222> (11)..(11)

<223> Phosphorylated Tyr

<400> 346

Ala Glu Ile Tyr Glu Ala Phe Glu Asn Ile Tyr Pro Ile Leu Lys

1               5                   10                  15

<210> 347

<211> 17

<212> PRT

<213> Homo sapiens

<220>

<221> MOD_RES

<222> (13)..(13)

<223> Phosphorylated Tyr

<400> 347

Asp Glu Ser Thr Asp Ser Gly Leu Ser Met Ser Ser Tyr Ser Val Pro

1               5                   10                  15

Arg

<210> 348

<211> 17

<212> PRT

<213> Homo sapiens

<220>

<221> MOD_RES

<222> (13)..(13)

<223> Phosphorylated Tyr

<400> 348

Gly Ala Asp Asn Asp Gly Ser Gly Ser Glu Ser Gly Tyr Thr Thr Pro

1               5                   10                  15

Lys

<210> 349

<211> 25

<212> PRT

<213> Homo sapiens

<220>

<221> MOD_RES

<222> (10)..(10)

<223> Phosphorylated Tyr

<400> 349

Ala Thr Met Lys Asp Asp Leu Ala Asp Tyr Gly Gly Tyr Asp Gly Gly

1               5                   10                  15

Tyr Val Gln Asp Tyr Glu Asp Phe Met

            20                  25

<210> 350

<211> 25

<212> PRT

<213> Homo sapiens

<220>

<221> MOD_RES

<222> (13)..(13)

<223> Phosphorylated Tyr

<400> 350

Ala Thr Met Lys Asp Asp Leu Ala Asp Tyr Gly Gly Tyr Asp Gly Gly

1               5                   10                  15

Tyr Val Gln Asp Tyr Glu Asp Phe Met

            20                  25

<210> 351
<211> 25
<212> PRT
<213> Homo sapiens

<220>
<221> MOD_RES
<222> (17)..(17)
<223> Phosphorylated Tyr

<400> 351
Ala Thr Met Lys Asp Asp Leu Ala Asp Tyr Gly Gly Tyr Asp Gly Gly
1               5                   10                  15

Tyr Val Gln Asp Tyr Glu Asp Phe Met
            20                  25

<210> 352
<211> 25
<212> PRT
<213> Homo sapiens

<220>
<221> MOD_RES
<222> (21)..(21)
<223> Phosphorylated Tyr

<400> 352
Ala Thr Met Lys Asp Asp Leu Ala Asp Tyr Gly Gly Tyr Asp Gly Gly
1               5                   10                  15

Tyr Val Gln Asp Tyr Glu Asp Phe Met
            20                  25

<210> 353
<211> 26
<212> PRT
<213> Homo sapiens

<220>
<221> MOD_RES
<222> (3)..(3)
<223> Phosphorylated Tyr

<400> 353
Ala Ala Tyr Asp Pro Tyr Ala Tyr Pro Ser Asp Tyr Asp Met His Thr
1               5                   10                  15

Gly Asp Pro Lys Gln Asp Leu Ala Tyr Glu
            20                  25

<210> 354
<211> 20
<212> PRT
<213> Homo sapiens

<220>
<221> MOD_RES
<222> (10)..(10)
<223> Phosphorylated Tyr

<400> 354
Asp Gly Gly Thr Gly Gly Gly Ser Thr Tyr Val Ser Lys Pro Val Ser
1               5                   10                  15

Trp Ala Asp Glu
            20

<210> 355
<211> 16
<212> PRT
<213> Homo sapiens

<220>
<221> MOD_RES
<222> (8)..(8)
<223> Phosphorylated Tyr

<400> 355
Ala Pro Gly Ala Ile Gly Pro Tyr Ser Gln Ala Val Leu Val Asp Arg
1               5                   10                  15

<210> 356
<211> 8
<212> PRT
<213> Homo sapiens

<220>
<221> MOD_RES
<222> (3)..(3)
<223> Phosphorylated Tyr

<400> 356
Met Ser Tyr Thr Ser Pro Gly Arg
1               5

<210> 357

<211> 32

<212> PRT

<213> Homo sapiens

<220>

<221> MOD_RES

<222> (9)..(9)

<223> Phosphorylated Tyr

<400> 357

Val Pro Val Arg Pro Pro Gln Gln Tyr Ser Asp Asp Glu Asp Asp Tyr

1               5                   10                  15

Glu Asp Asp Glu Glu Asp Asp Val Gln Asn Thr Asn Ser Ala Leu Arg

            20                  25                  30

<210> 358

<211> 32

<212> PRT

<213> Homo sapiens

<220>

<221> MOD_RES

<222> (16)..(16)

<223> Phosphorylated Tyr

<400> 358

Val Pro Val Arg Pro Pro Gln Gln Tyr Ser Asp Asp Glu Asp Asp Tyr

1               5                   10                  15

Glu Asp Asp Glu Glu Asp Asp Val Gln Asn Thr Asn Ser Ala Leu Arg

            20                  25                  30

<210> 359
<211> 11
<212> PRT
<213> Homo sapiens

<220>
<221> MOD_RES
<222> (10)..(10)
<223> Phosphorylated Tyr

<400> 359
Glu Ala Met Val Met Ala Asn Asn Val Tyr Lys
1               5                   10

<210> 360
<211> 11
<212> PRT
<213> Homo sapiens

<220>
<221> MOD_RES
<222> (7)..(7)
<223> Phosphorylated Tyr

<400> 360
Gln Ser Gly Glu Leu Val Tyr Glu Ala Pro Glu
1               5                   10

<210> 361
<211> 19
<212> PRT
<213> Homo sapiens

<220>
<221> MOD_RES
<222> (4)..(4)
<223> Phosphorylated Tyr

<400> 361
Ala Met Ser Tyr Lys Pro Ile Cys Pro Thr Cys Gln Thr Ser Tyr Gly
1               5                   10                  15

Ile Gln Lys

<210> 362
<211> 22
<212> PRT
<213> Homo sapiens

<220>
<221> MOD_RES
<222> (15)..(15)
<223> Phosphorylated Tyr

<400> 362
Thr Val Glu Asp Cys Gly Ser Leu Val Asn Gly Gln Pro Gln Tyr Val
1               5                   10                  15

Met Gln Val Ser Ala Lys
            20

<210> 363
<211> 11
<212> PRT
<213> Homo sapiens

<220>
<221> MOD_RES
<222> (8)..(8)
<223> Phosphorylated Tyr

<400> 363
Gly Ser Ser Leu Asn Asp Thr Tyr His Ser Arg
1               5                   10

<210> 364
<211> 17
<212> PRT
<213> Homo sapiens

<220>
<221> MOD_RES
<222> (6)..(6)
<223> Phosphorylated Tyr

<400> 364
Ala Ser Val Cys His Tyr His Gly Ile Glu Tyr Cys Leu Asp Asp Arg
1               5                   10                  15

Lys

<210> 365
<211> 17
<212> PRT
<213> Homo sapiens

<220>

<221> MOD_RES
<222> (11)..(11)
<223> Phosphorylated Tyr

<400> 365
Ala Ser Val Cys His Tyr His Gly Ile Glu Tyr Cys Leu Asp Asp Arg
1               5                   10                  15

Lys

<210> 366
<211> 17
<212> PRT
<213> Homo sapiens

<220>
<221> MOD_RES
<222> (12)..(12)
<223> Phosphorylated Tyr

<400> 366
Ala Ile Ser Tyr Val Asp Pro Gln Phe Leu Thr Tyr Met Ala Leu Glu
1               5                   10                  15

Glu

<210> 367
<211> 20
<212> PRT
<213> Homo sapiens

<220>
<221> MOD_RES
<222> (5)..(5)
<223> Phosphorylated Tyr

<400> 367
Glu Ala Glu Ala Tyr Thr Gly Leu Ser Pro Pro Val Pro Ser Phe Asn
1               5                   10                  15

Cys Glu Val Arg
            20

<210> 368
<211> 17
<212> PRT
<213> Homo sapiens

<220>
<221> MOD_RES
<222> (12)..(12)
<223> Phosphorylated Tyr

<400> 368
Ala Ile Ser Tyr Val Asp Pro Gln Phe Leu Thr Tyr Met Ala Leu Glu
1               5                   10                  15

Glu

<210> 369
<211> 15
<212> PRT
<213> Homo sapiens

<220>
<221> MOD_RES
<222> (5)..(5)
<223> Phosphorylated Tyr

<400> 369
Ala Gly Asp Asp Tyr Glu Val Leu Glu Leu Asp Asp Val Pro Lys
1               5                   10                  15

<210> 370
<211> 17
<212> PRT
<213> Homo sapiens

<220>
<221> MOD_RES
<222> (3)..(3)
<223> Phosphorylated Tyr

<400> 370
Cys Val Tyr Tyr Arg Lys Pro Leu Leu Glu Ser Gly Thr Leu Gly Thr
1               5                   10                  15

Lys

<210> 371
<211> 16
<212> PRT
<213> Homo sapiens

<220>

<221> MOD_RES
<222> (12)..(12)
<223> Phosphorylated Tyr

<400> 371
Ala Leu Ser Asn Leu Glu Ser Ile Pro Gly Gly Tyr Asn Ala Leu Arg
1               5                   10                  15

<210> 372
<211> 19
<212> PRT
<213> Homo sapiens

<220>
<221> MOD_RES
<222> (11)..(11)
<223> Phosphorylated Tyr

<400> 372
Arg Met Glu Ala Gln Ala Leu Leu Gln Asp Tyr Ile Ser Thr Gln Ser
1               5                   10                  15

Ala Lys Glu

<210> 373
<211> 28
<212> PRT
<213> Homo sapiens

<220>
<221> MOD_RES
<222> (26)..(26)

<223> Phosphorylated Tyr

<400> 373
Met Arg Pro Ile Gly His Asp Gly Tyr His Pro Thr Ser Val Ala Glu
1               5                   10                  15

Trp Leu Asp Ser Ile Glu Leu Gly Asp Tyr Thr Lys
            20                  25

<210> 374
<211> 19
<212> PRT
<213> Homo sapiens

<220>
<221> MOD_RES
<222> (18)..(18)
<223> Phosphorylated Tyr

<400> 374
Ser Tyr Tyr Val Gln Asp Asn Gly Arg Asn Val His Leu Gln Asp Glu
1               5                   10                  15

Asp Tyr Glu

<210> 375
<211> 9
<212> PRT
<213> Homo sapiens

<220>
<221> MOD_RES

<222> (4)..(4)
<223> Phosphorylated Tyr

<400> 375
His Ser Ala Tyr Ser Tyr Cys Gly Arg
1               5

<210> 376
<211> 13
<212> PRT
<213> Homo sapiens

<220>
<221> MOD_RES
<222> (1)..(1)
<223> Phosphorylated Tyr

<400> 376
Tyr Phe Pro Leu Thr Ala Ser Glu Val Leu Ser Met Arg
1               5                   10

<210> 377
<211> 10
<212> PRT
<213> Homo sapiens

<220>
<221> MOD_RES
<222> (6)..(6)
<223> Phosphorylated Tyr

<400> 377
Ile Asp Leu His Lys Tyr Leu Thr Val Lys
1               5                   10

<210> 378
<211> 15
<212> PRT
<213> Homo sapiens

<220>
<221> MOD_RES
<222> (12)..(12)
<223> Phosphorylated Tyr

<400> 378
Ala Ala Leu Asp Gln Glu Gln Ile Thr Ala Ala Tyr Ser Val Glu
1               5                   10                  15

<210> 379
<211> 9
<212> PRT
<213> Homo sapiens

<220>
<221> MOD_RES
<222> (8)..(8)
<223> Phosphorylated Tyr

<400> 379
Asn Ser Asn Ile Ala Gln Asn Tyr Arg
1               5

<210> 380
<211> 14
<212> PRT
<213> Homo sapiens

<220>
<221> MOD_RES
<222> (13)..(13)
<223> Phosphorylated Tyr

<400> 380
Val Thr Ile Asp Lys Leu Ser Glu Asp Asn Glu Leu Tyr Arg
1               5                   10

<210> 381
<211> 17
<212> PRT
<213> Homo sapiens

<220>
<221> MOD_RES
<222> (15)..(15)
<223> Phosphorylated Tyr

<400> 381
Asp Cys Asn Leu Ala Ala Gln Leu Leu Gln Cys Ser Gln Thr Tyr Gly
1               5                   10                  15

Arg

<210> 382
<211> 13
<212> PRT
<213> Homo sapiens

<220>

<221> MOD_RES
<222> (6)..(6)
<223> Phosphorylated Tyr

<400> 382
Thr Thr Asn Arg Glu Tyr Phe Leu Ile Gly His Asp Arg
1               5                   10

<210> 383
<211> 18
<212> PRT
<213> Homo sapiens

<220>
<221> MOD_RES
<222> (4)..(4)
<223> Phosphorylated Tyr

<400> 383
Leu Arg Glu Tyr Phe Asn Ser Glu Lys Pro Glu Gly Arg Ile Ile Met
1               5                   10                  15

Thr Arg

<210> 384
<211> 16
<212> PRT
<213> Homo sapiens

<220>
<221> MOD_RES
<222> (8)..(8)
<223> Phosphorylated Tyr

<400> 384
Ala Ser Val Glu Lys Gly Asp Tyr Tyr Glu Ala His Gln Met Tyr Arg
1               5                   10                  15

<210> 385
<211> 14
<212> PRT
<213> Homo sapiens

<220>
<221> MOD_RES
<222> (4)..(4)
<223> Phosphorylated Tyr

<400> 385
Asp Glu Glu Tyr Asp Glu Glu Asp Tyr Glu Arg Glu Lys Glu
1               5                   10

<210> 386
<211> 14
<212> PRT
<213> Homo sapiens

<220>
<221> MOD_RES
<222> (9)..(9)
<223> Phosphorylated Tyr

<400> 386
Asp Glu Glu Tyr Asp Glu Glu Asp Tyr Glu Arg Glu Lys Glu
1               5                   10

<210> 387
<211> 17
<212> PRT
<213> Homo sapiens

<220>
<221> MOD_RES
<222> (7)..(7)
<223> Phosphorylated Tyr

<400> 387
Cys His Leu Gln Asp His Tyr Gly Pro Tyr Pro Leu Ala Glu Ser Glu
1               5                   10                  15

Arg

<210> 388
<211> 16
<212> PRT
<213> Homo sapiens

<220>
<221> MOD_RES
<222> (12)..(12)
<223> Phosphorylated Tyr

<400> 388
Asp Leu Leu His Asn Glu Asp Arg His Asp Asp Tyr Phe Gln Glu Arg
1               5                   10                  15

<210> 389
<211> 15
<212> PRT

<213> Homo sapiens

<220>
<221> MOD_RES
<222> (10)..(10)
<223> Phosphorylated Tyr

<400> 389
Asp Lys Gln Pro Asn Ala Gln Pro Gln Tyr Leu His Gly Ser Lys
1               5                   10                  15

<210> 390
<211> 14
<212> PRT
<213> Homo sapiens

<220>
<221> MOD_RES
<222> (7)..(7)
<223> Phosphorylated Tyr

<400> 390
Asp Gly Thr Asn Val Leu Tyr Ser Ala Leu Ile Ser Leu Lys
1               5                   10

<210> 391
<211> 12
<212> PRT
<213> Homo sapiens

<220>
<221> MOD_RES
<222> (11)..(11)

<223> Phosphorylated Tyr

<400> 391
Ala Glu Thr Arg Val Gly Ser Ala Leu Lys Tyr Glu
1               5                   10

<210> 392
<211> 12
<212> PRT
<213> Homo sapiens

<220>
<221> MOD_RES
<222> (11)..(11)
<223> Phosphorylated Tyr

<400> 392
Ala Ile Val Ala Gly Asp Gln Asn Val Glu Tyr Lys
1               5                   10

<210> 393
<211> 26
<212> PRT
<213> Homo sapiens

<220>
<221> MOD_RES
<222> (18)..(18)
<223> Phosphorylated Tyr

<400> 393
Ala Leu Ile Ala Pro Asp His Val Val Pro Ala Pro Glu Glu Cys Tyr
1               5                   10                  15

Val Tyr Ser Pro Leu Gly Ser Ala Tyr Lys
            20                  25

<210> 394
<211> 10
<212> PRT
<213> Homo sapiens

<220>
<221> MOD_RES
<222> (3)..(3)
<223> Phosphorylated Tyr

<400> 394
Asp Ala Tyr Thr Phe Pro Gln Glu Met Lys
1               5                   10

<210> 395
<211> 15
<212> PRT
<213> Homo sapiens

<220>
<221> MOD_RES
<222> (7)..(7)
<223> Phosphorylated Tyr

<400> 395
Ala Cys Leu Asp Asp Ser Tyr Ala Ser Gly Glu Gly Leu Lys Arg
1               5                   10                  15

<210> 396
<211> 15

<212> PRT
<213> Homo sapiens

<220>
<221> MOD_RES
<222> (6)..(6)
<223> Phosphorylated Tyr

<400> 396
Ala Leu Ser Asp Ser Tyr Glu Leu Ser Thr Asp Leu Gln Asp Lys
1               5                   10                  15

<210> 397
<211> 9
<212> PRT
<213> Homo sapiens

<220>
<221> MOD_RES
<222> (7)..(7)
<223> Phosphorylated Tyr

<400> 397
Asp Leu Leu Val Gly Ile Tyr Gln Arg
1               5

<210> 398
<211> 10
<212> PRT
<213> Homo sapiens

<220>
<221> MOD_RES
<222> (4)..(4)

<223> Phosphorylated Tyr

<400> 398
Asp Gly Val Tyr Val Ser Ala Asn Gly Arg
1               5                   10

<210> 399
<211> 14
<212> PRT
<213> Homo sapiens

<220>
<221> MOD_RES
<222> (3)..(3)
<223> Phosphorylated Tyr

<400> 399
Ala Arg Tyr Val Asp Val Leu Asn Pro Ser Gly Thr Gln Arg
1               5                   10

<210> 400
<211> 17
<212> PRT
<213> Homo sapiens

<220>
<221> MOD_RES
<222> (13)..(13)
<223> Phosphorylated Tyr

<400> 400
Ala Glu Pro Ser Ile Ser Tyr Ser Cys Asn Pro Leu Tyr Phe Asp Phe
1               5                   10                  15

Lys

<210> 401
<211> 23
<212> PRT
<213> Homo sapiens

<220>
<221> MOD_RES
<222> (18)..(18)
<223> Phosphorylated Tyr

<400> 401
Ala Gly Ala Ser Gly Gln Pro Pro Ser Gly Pro Pro Gly Thr Gly Asn
1               5                   10                  15

Glu Tyr Gly Val Ser Pro Arg
            20

<210> 402
<211> 8
<212> PRT
<213> Homo sapiens

<220>
<221> MOD_RES
<222> (4)..(4)
<223> Phosphorylated Tyr

<400> 402
Asp Leu Glu Tyr Leu Asp Leu Lys
1               5

<210> 403
<211> 23
<212> PRT
<213> Homo sapiens

<220>
<221> MOD_RES
<222> (5)..(5)
<223> Phosphorylated Tyr

<400> 403
Asp Thr Glu Gln Tyr Glu Arg Glu Asp Phe Leu Ile Lys Pro Ser Asp
1               5                   10                  15

Asn Leu Ile Val Cys Gly Arg
            20

<210> 404
<211> 18
<212> PRT
<213> Homo sapiens

<220>
<221> MOD_RES
<222> (5)..(5)
<223> Phosphorylated Tyr

<400> 404
Ala Asp Ser Ala Tyr Ser Ser Phe Ser Ala Ala Ser Gly Gly Pro Glu
1               5                   10                  15

Pro Arg

<210> 405
<211> 21
<212> PRT
<213> Homo sapiens

<220>
<221> MOD_RES
<222> (12)..(12)
<223> Phosphorylated Tyr

<400> 405
Asp Ala Ala His Met Leu Gln Ala Asn Lys Thr Tyr Gly Cys Val Pro
1               5                   10                  15

Val Ala Asn Lys Arg
            20

<210> 406
<211> 16
<212> PRT
<213> Homo sapiens

<220>
<221> MOD_RES
<222> (5)..(5)
<223> Phosphorylated Tyr

<400> 406
Cys Thr Lys Ala Tyr Phe His Met Gly Lys Ala Asn Leu Ala Leu Lys
1               5                   10                  15

<210> 407

<211> 15

<212> PRT

<213> Homo sapiens

<220>

<221> MOD_RES

<222> (13)..(13)

<223> Phosphorylated Tyr

<400> 407

Ala Ala Glu Asp Ser Pro Tyr Trp Val Ser Pro Ala Tyr Ser Lys

1               5                   10                  15

<210> 408

<211> 32

<212> PRT

<213> Homo sapiens

<220>

<221> MOD_RES

<222> (23)..(23)

<223> Phosphorylated Tyr

<400> 408

Arg Ala Gly Leu Gly Glu Gly Val Pro Pro Gly Asn Tyr Gly Asn Tyr

1               5                   10                  15

Gly Tyr Ala Asn Ser Gly Tyr Ser Ala Cys Glu Glu Glu Asn Glu Arg

            20                  25                  30

<210> 409

<211> 17
<212> PRT
<213> Homo sapiens

<220>
<221> MOD_RES
<222> (16)..(16)
<223> Phosphorylated Tyr

<400> 409
Leu Asp Gly Glu Lys Gly Arg Asp Ser Gly Glu Glu Ala His Asp Tyr
1               5                   10                  15

Glu

<210> 410
<211> 16
<212> PRT
<213> Homo sapiens

<220>
<221> MOD_RES
<222> (3)..(3)
<223> Phosphorylated Tyr

<400> 410
Asn Lys Tyr Glu Asn Glu Lys Ala Met Val Thr Glu Thr Met Thr Lys
1               5                   10                  15

<210> 411
<211> 22
<212> PRT
<213> Homo sapiens

<220>
<221> MOD_RES
<222> (8)..(8)
<223> Phosphorylated Tyr

<400> 411
Ala Asn Gly Pro Ala Asp Gly Tyr Ala Ala Ile Ala Gln Ala Asp Arg
1               5                   10                  15

Leu Thr Gln Glu Pro Glu
            20

<210> 412
<211> 17
<212> PRT
<213> Homo sapiens

<220>
<221> MOD_RES
<222> (16)..(16)
<223> Phosphorylated Tyr

<400> 412
Ala Tyr Gly Glu Val Tyr Ala Ala Val Met Asn Pro Ile Asn Glu Tyr
1               5                   10                  15

Lys

<210> 413
<211> 16

<212> PRT
<213> Homo sapiens

<220>
<221> MOD_RES
<222> (7)..(7)
<223> Phosphorylated Tyr

<400> 413
Asp Thr Cys Thr Ala Ala Tyr Arg Gly Ile Val Gln Ser Glu Glu Lys
1               5                   10                  15

<210> 414
<211> 13
<212> PRT
<213> Homo sapiens

<220>
<221> MOD_RES
<222> (9)..(9)
<223> Phosphorylated Tyr

<400> 414
Ala Ala Thr Ser Asp Leu Glu His Tyr Asp Lys Thr Arg
1               5                   10

<210> 415
<211> 10
<212> PRT
<213> Homo sapiens

<220>
<221> MOD_RES
<222> (5)..(5)

<223> Phosphorylated Tyr

<400> 415
Asp Leu Gln Gln Tyr Gln Ser Gln Ala Lys
1               5                   10

<210> 416
<211> 15
<212> PRT
<213> Homo sapiens

<220>
<221> MOD_RES
<222> (14)..(14)
<223> Phosphorylated Tyr

<400> 416
Ala Thr Ala Ala Ser Thr Ser Arg Ser Leu Ala Leu Met Tyr Glu
1               5                   10                  15

<210> 417
<211> 23
<212> PRT
<213> Homo sapiens

<220>
<221> MOD_RES
<222> (11)..(11)
<223> Phosphorylated Tyr

<400> 417
Ala Ile Ala His Tyr Glu Gln Ser Ala Asp Tyr Tyr Lys Gly Glu Glu
1               5                   10                  15

Ser Asn Ser Ser Ala Asn Lys
            20

<210> 418
<211> 20
<212> PRT
<213> Homo sapiens

<220>
<221> MOD_RES
<222> (11)..(11)
<223> Phosphorylated Tyr

<400> 418
Ala Thr Asn Ser Ser Lys Pro Gln Pro Thr Tyr Glu Glu Leu Glu Glu
1               5                   10                  15

Glu Glu Gln Glu
            20

<210> 419
<211> 18
<212> PRT
<213> Homo sapiens

<220>
<221> MOD_RES
<222> (3)..(3)
<223> Phosphorylated Tyr

<400> 419
Asp Ala Tyr Gly Thr Ser Ser Leu Ser Ser Ser Ser Asn Ser Gly Ser
1               5                   10                  15

Tyr Lys

<210> 420
<211> 18
<212> PRT
<213> Homo sapiens

<220>
<221> MOD_RES
<222> (17)..(17)
<223> Phosphorylated Tyr

<400> 420
Asp Ala Tyr Gly Thr Ser Ser Leu Ser Ser Ser Ser Asn Ser Gly Ser
1               5                   10                  15

Tyr Lys

<210> 421
<211> 28
<212> PRT
<213> Homo sapiens

<220>
<221> MOD_RES
<222> (6)..(6)
<223> Phosphorylated Tyr

<400> 421

Asp Ile Leu Ser Thr Tyr Cys Val Asp Asn Asn Gln Gly Gly Pro Gly
1               5                   10                  15

Glu Asp Gly Ala Gln Gly Glu Pro Ala Glu Pro Glu
            20                  25

<210> 422
<211> 13
<212> PRT
<213> Homo sapiens

<220>
<221> MOD_RES
<222> (8)..(8)
<223> Phosphorylated Tyr

<400> 422
Asp Thr Asn Asn Asn Val Ala Tyr Trp Asp Val Leu Lys
1               5                   10

## Description

### RELATED APPLICATIONS

Pursuant to 35 U.S.C. § 119(e) this application claims the benefit of, and priority to, provisional application U.S.S.N. 60/927,070, filed May 1, 2007, and to provisional application U.S.S.N. 60/999,628, filed October 19, 2007, the disclosures of which are incorporated herein, in their entirety, by reference.

### FIELD OF THE INVENTION

The invention relates generally to novel tyrosine phosphorylation sites, methods and compositions for detecting, quantitating and modulating same.

### BACKGROUND OF THE INVENTION

The activation of proteins by post-translational modification is an important cellular mechanism for regulating most aspects of biological organization and control, including growth, development, homeostasis, and cellular communication. Protein phosphorylation, for example, plays a critical role in the etiology of many pathological conditions and diseases, including to mention but a few: cancer, developmental disorders, autoimmune diseases, and diabetes. Yet, in spite of the importance of protein modification, it is not yet well understood at the molecular level, due to the extraordinary complexity of signaling pathways, and the slow development of technology necessary to unravel it.

Protein phosphorylation on a proteome-wide scale is extremely complex as a result of three factors: the large number of modifying proteins, e.g., kinases, encoded in the genome, the much larger number of sites on substrate proteins that are modified by these enzymes, and the dynamic nature of protein expression during growth, development, disease states, and aging. The human genome, for example, encodes over 520 different protein kinases, making them the most abundant class of enzymes known. (Hunter, Nature 411: 355-65 (2001)). Most kinases phosphorylate many different substrate proteins, at distinct tyrosine, serine, and/or threonine residues. Indeed, it is estimated that one-third of all proteins encoded by the human genome are phosphorylated, and many are phosphorylated at multiple sites by different kinases.

Many of these phosphorylation sites regulate critical biological processes and may prove to be important diagnostic or therapeutic targets for molecular medicine. For example, of the more than 100 dominant oncogenes identified to date, 46 are protein kinases. *See* Hunter, *supra.* Understanding which proteins are modified by these kinases will greatly expand our understanding of the molecular mechanisms underlying oncogenic transformation. Therefore, the identification of, and ability to detect, phosphorylation sites on a wide variety of cellular proteins is crucially important to understanding the key signaling proteins and pathways implicated in the progression of disease states like cancer.

Carcinoma and/or leukemia is one of the two main categories of cancer, and is generally characterized by the formation of malignant tumors or cells of epithelial tissue original, such as skin, digestive tract, glands, *etc.* Carcinoma and/or leukemias are malignant by definition, and tend to metastasize to other areas of the body. The most common forms of carcinoma and/or leukemia are skin cancer, lung cancer, breast cancer, and colon cancer, as well as other numerous but less prevalent carcinoma and/or leukemias. Current estimates show that, collectively, various carcinoma and/or leukemias will account for approximately 1.65 million cancer diagnoses in the United States alone, and more than 300,000 people will die from some type of carcinoma and/or leukemia during 2005. (Source: American Cancer Society (2005)). The worldwide incidence of carcinoma and/or leukemia is much higher.

As with many cancers, deregulation of receptor tyrosine kinases (RTKs) appears to be a central theme in the etiology of carcinoma and/or leukemias. Constitutively active RTKs can contribute not only to unrestricted cell proliferation, but also to other important features of malignant tumors, such as evading apoptosis, the ability to promote blood vessel growth, the ability to invade other tissues and build metastases at distant sites *(see* Blume-Jensen et al., Nature 411: 355-365 (2001)). These effects are mediated not only through aberrant activity of RTKs themselves, but, in turn, by aberrant activity of their downstream signaling molecules and substrates.

The importance of RTKs in carcinoma and/or leukemia progression has led to a very active search for pharmacological compounds that can inhibit RTK activity in tumor cells, and more recently to significant efforts aimed at identifying genetic mutations in RTKs that may occur in, and affect progression of, different types of carcinoma and/or leukemias (*see, e.g.,* Bardell et al., Science 300: 949(2003); Lynch et al., N. Eng. J. Med. 350:2129-2139 (2004)). For example, non-small cell lung carcinoma and/or leukemia patients carrying activating mutations in the epidermal growth factor receptor (EGFR), an RTK, appear to respond better to specific EGFR inhibitors than do patients without such mutations (Lynch *et al., supra.;* Paez et al., Science 304: 1497-1500 (2004)).

Clearly, identifying activated RTKs and downstream signaling molecules driving the oncogenic phenotype of carcinoma and/or leukemias would be highly beneficial for understanding the underlying mechanisms of this prevalent form of cancer, identifying novel drug targets for the treatment of such disease, and for assessing appropriate patient treatment with selective kinase inhibitors of relevant targets when and if they become available. The identification of key signaling mechanisms is highly desirable in many contexts in addition to cancer.

Leukemia, another form of cancer, is a disease in which a number of underlying signal transduction events have been elucidated and which has become a disease model for phosphoproteomic research and development efforts. As such, it represent a paradigm leading the way for many other programs seeking to address many classes of diseases (See, Harrison's Principles of Internal Medicine, McGraw-Hill, New York, N.Y.).

Most varieties of leukemia are generally characterized by genetic alterations e.g., chromosomal translocations, deletions or point mutations resulting in the constitutive activation of protein kinase genes, and their products, particularly tyrosine kinases. The most well known alteration is the oncogenic role of the chimeric BCR-Abl gene. *See* Nowell, Science 132: 1497 (1960)). The resulting BCR-Abl kinase protein is constitutively active and elicits characteristic signaling pathways that have been shown to drive the proliferation and survival of CML cells *(see* Daley, Science 247: 824-830 (1990); Raitano et al., Biochim. Biophys. Acta. Dec 9; 1333(3): F201-16 (1997)).

The recent success of *Imanitib* (also known as STI571 or Gleevec®), the first molecularly targeted compound designed to specifically inhibit the tyrosine kinase activity of BCR-Abl; provided critical confirmation of the central role of BCR-Abl signaling in the progression of CML *(see* Schindler et al., Science289: 1938-1942 (2000); Nardi et al., Curr. Opin. Hematol.11: 35-43 (2003)).

The success of Gleevec® now serves as a paradigm for the development of targeted drugs designed to block the activity of other tyrosine kinases known to be involved in many diseased including leukemias and other malignancies *(see,* e.g., Sawyers, Curr. Opin. Genet. Dev. Feb; 12(1): 111-5 (2002); Druker, Adv. Cancer Res.91:1-30 (2004)). For example, recent studies have demonstrated that mutations in the FLT3 gene occur in one third of adult patients with AML. FLT3 (Fms-like tyrosine kinase 3) is a member of the class III receptor tyrosine kinase (RTK) family including FMS, platelet-derived growth factor receptor (PDGFR) and c-KIT (*see* Rosnet et al., Crit. Rev. Oncog. 4: 595-613 (1993). In 20-27% of patients with AML, internal tandem duplication in the juxta-membrane region of FLT3 can be detected (see Yokota et al., Leukemia 11: 1605-1609 (1997)). Another 7% of patients have mutations within the active loop of the second kinase domain, predominantly substitutions of aspartate residue 835 (D835), while additional mutations have been described *(see* Yamamoto et al., Blood 97: 2434-2439 (2001); Abu-Duhier et al., Br. J. Haematol. 113: 983-988 (2001)). Expression of mutated FLT3 receptors results in constitutive tyrosine phosphorylation of FLT3, and subsequent phosphorylation and activation of downstream molecules such as STAT5, Akt and MAPK, resulting in factor-independent growth of hematopoietic cell lines.

Altogether, FLT3 is the single most common activated gene in AML known to date. This evidence has triggered an intensive search for FLT3 inhibitors for clinical use leading to at least four compounds in advanced stages of clinical development, including: PKC412 (by Novartis), CEP-701 (by Cephalon), MLN518 (by Millenium Pharmaceuticals), and SU5614 (by Sugen/Pfizer) *(see* Stone et al., Blood (in press)(2004); Smith et al., Blood 103: 3669-3676 (2004); Clark et al., Blood 104: 2867-2872 (2004); and Spiekerman et al., Blood 101: 1494-1504 (2003)).

There is also evidence indicating that kinases such as FLT3, c-KIT and Abl are implicated in some cases of ALL *(see* Cools et al., Cancer Res. 64: 6385-6389 (2004); Hu, Nat.Genet. 36: 453-461 (2004); and Graux et al., Nat. Genet. 36: 1084-1089 (2004)). In contrast, very little is know regarding any causative role of protein kinases in CLL, except for a high correlation between high expression of the tyrosine kinase ZAP70 and the more aggressive form of the disease *(see* Rassenti et al., N. Eng. J. Med. 351: 893-901 (2004)).

Although a few key RTKs and various other signaling proteins involved in carcinoma and/or leukemia and leukemia progression are known, there is relatively scarce information about kinase-driven signaling pathways and phosphorylation sites that underlie the different types of cancer. Therefore there is presently an incomplete and inaccurate understanding of how protein activation within signaling pathways is driving these complex cancers. Accordingly, there is a continuing and pressing need to unravel the molecular mechanisms of kinase-driven ontogenesis in cancer by identifying the downstream signaling proteins mediating cellular transformation in these cancers.

Presently, diagnosis of many types of cancer is often made by tissue biopsy and detection of different cell surface markers. However, misdiagnosis can occur since certain types of cancer can be negative for certain markers and because these markers may not indicate which genes or protein kinases may be deregulated. Although the genetic translocations and/or mutations characteristic of a particular form of cancer can be sometimes detected, it is clear that other downstream effectors of constitutively active kinases having potential diagnostic, predictive, or therapeutic value, remain to be elucidated.

Accordingly, identification of downstream signaling molecules and phosphorylation sites involved in different types of diseases including for example, carcinoma and/or leukemia and development of new reagents to detect and quantify these sites and proteins may lead to improved diagnostic/prognostic markers, as well as novel drug targets, for the detection and treatment of many diseases.

### SUMMARY OF THE INVENTION

The present invention provides in one aspect novel tyrosine phosphorylation sites (Table 1) identified in carcinoma and/or leukemia. The novel sites occur in proteins such as: enzyme proteins, adaptor/scaffold, protein kinase, receptor/channel/transportercell surface protein, cytoskeletal protein, RNA processing protein, G protein or regulator protein, transcriptional regulator protein, adhesion or extracellular matrix protein, vesicle protein, ubiquitin conjugating system protein, chromatin or DNA binding/repair/replication protein, motor or contractile protein, translational regulator, phosphatase, apoptosis protein, inhibitor protein, kinase (non-protein), cell cycle regulation protein, protease, tumor suppressor protein, secreted protein, calcium-binding protein, chaperone protein, lipid binding protein, mitochondrial protein, endoplasmic reticulum or golgi apparatus protein, vesicle proteins and proteins of unknown function.

In another aspect, the invention provides peptides comprising the novel phosphorylation sites of the invention, and proteins and peptides that are mutated to eliminate the novel phosphorylation sites.

In another aspect, the invention provides modulators that modulate tyrosine phosphorylation at a novel phosphorylation site of the invention, including small molecules, peptides comprising a novel phosphorylation site, and binding molecules that specifically bind at a novel phosphorylation site, including but not limited to antibodies or antigen-binding fragments thereof.

In another aspect, the invention provides compositions for detecting, quantitating or modulating a novel phosphorylation site of the invention, including peptides comprising a novel phosphorylation site and antibodies or antigen-binding fragments thereof that specifically bind at a novel phosphorylation site. In certain embodiments, the compositions for detecting, quantitating or modulating a novel phosphorylation site of the invention are Heavy-Isotype Labeled Peptides (AQUA peptides) comprising a novel phosphorylation site.

In another aspect, the invention discloses phosphorylation site specific antibodies or antigen-binding fragments thereof. In one embodiment, the antibodies specifically bind to an amino acid sequence comprising a phosphorylation site identified in Table 1 when the tyrosine identified in Column D is phosphorylated, and do not significantly bind when the tyrosine is not phosphorylated. In another embodiment, the antibodies specifically bind to an amino acid sequence comprising a phosphorylation site when the tyrosine is not phosphorylated, and do not significantly bind when the tyrosine is phosphorylated.

In another aspect, the invention provides a method for making phosphorylation site-specific antibodies.

In another aspect, the invention provides compositions comprising a peptide, protein, or antibody of the invention, including pharmaceutical compositions.

In a further aspect, the invention provides methods of treating or preventing carcinoma and/or leukemia in a subject, wherein the carcinoma and/or leukemia is associated with the phosphorylation state of a novel phosphorylation site in Table 1, whether phosphorylated or dephosphorylated. In certain embodiments, the methods comprise administering to a subject a therapeutically effective amount of a peptide comprising a novel phosphorylation site of the invention. In certain embodiments, the methods comprise administering to a subject a therapeutically effective amount of an antibody or antigen-binding fragment thereof that specifically binds at a novel phosphorylation site of the invention.

In a further aspect, the invention provides methods for detecting and quantitating phosphorylation at a novel tyrosine phosphorylation site of the invention.

In another aspect, the invention provides a method for identifying an agent that modulates tyrosine phosphorylation at a novel phosphorylation site of the invention, comprising: contacting a peptide or protein comprising a novel phosphorylation site of the invention with a candidate agent, and determining the phosphorylation state or level at the novel phosphorylation site. A change in the phosphorylation state or level at the specified tyrosine in the presence of the test agent, as compared to a control, indicates that the candidate agent potentially modulates tyrosine phosphorylation at a novel phosphorylation site of the invention.

In another aspect, the invention discloses immunoassays for binding, purifying, quantifying and otherwise generally detecting the phosphorylation of a protein or peptide at a novel phosphorylation site of the invention.

Also provided are pharmaceutical compositions and kits comprising one or more antibodies or peptides of the invention and methods of using them.

### BRIEF DESCRIPTION OF THE DRAWINGS

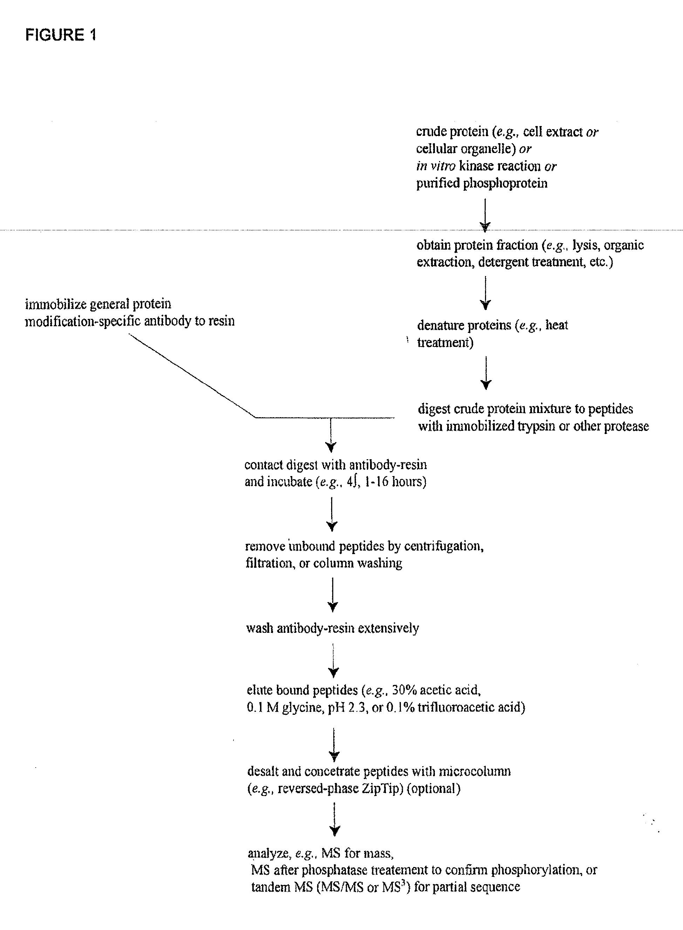

**FIGURE 1** is a diagram depicting the immuno-affinity isolation and mass-spectrometric characterization methodology (IAP) used in the Examples to identify the novel phosphorylation sites disclosed herein.

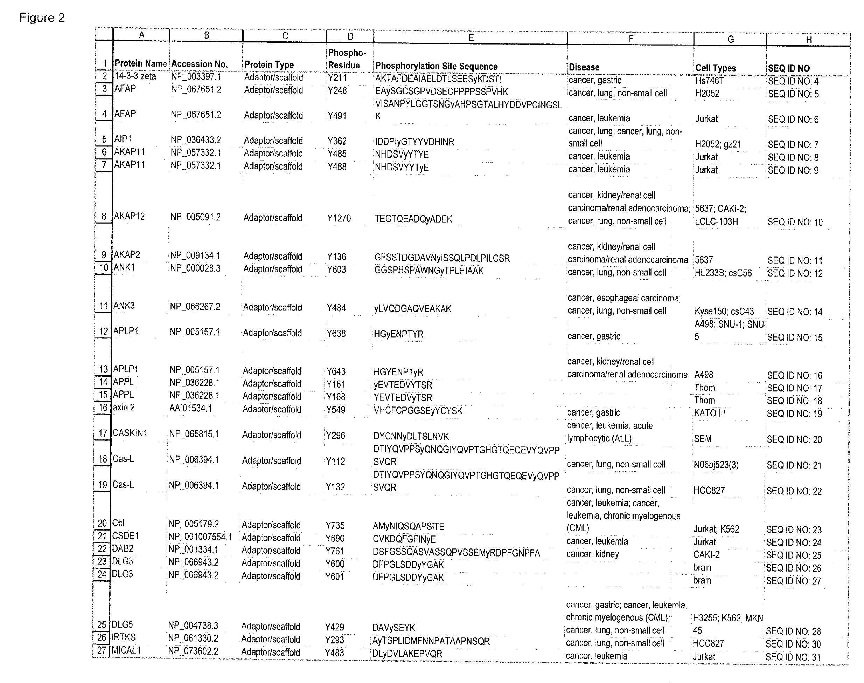

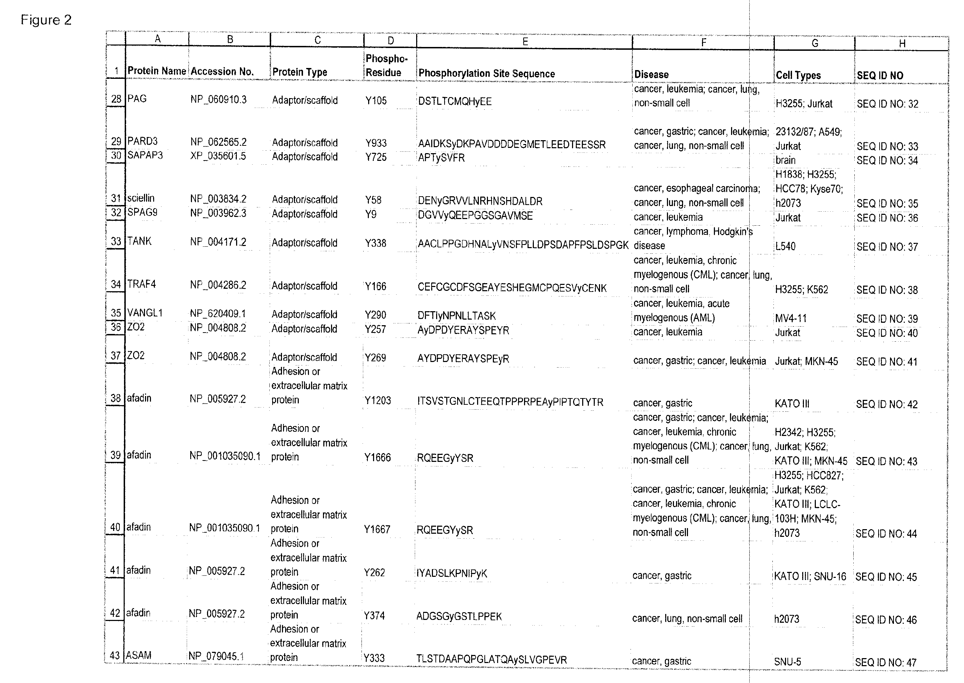

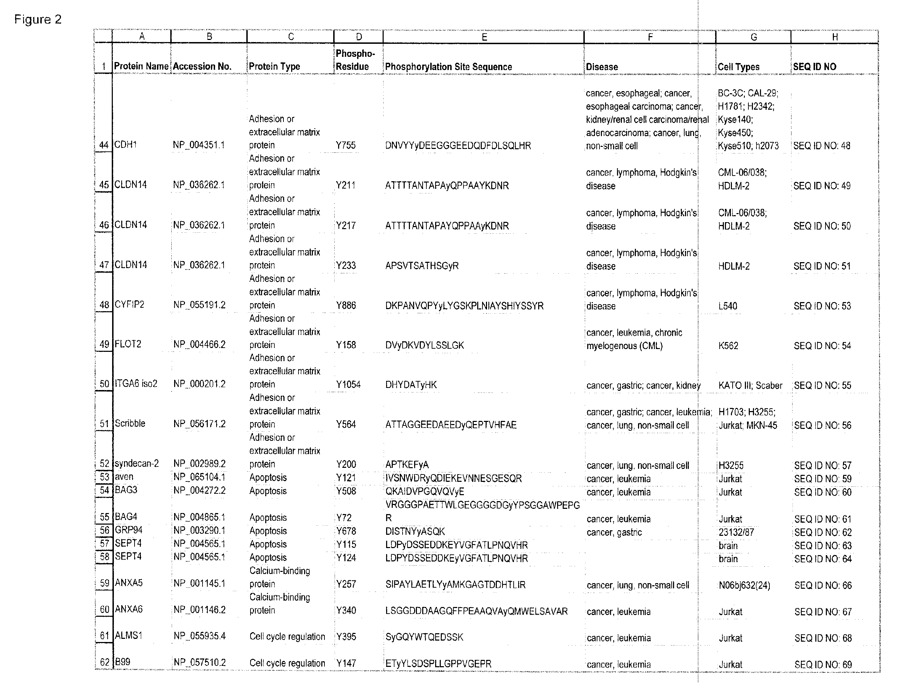

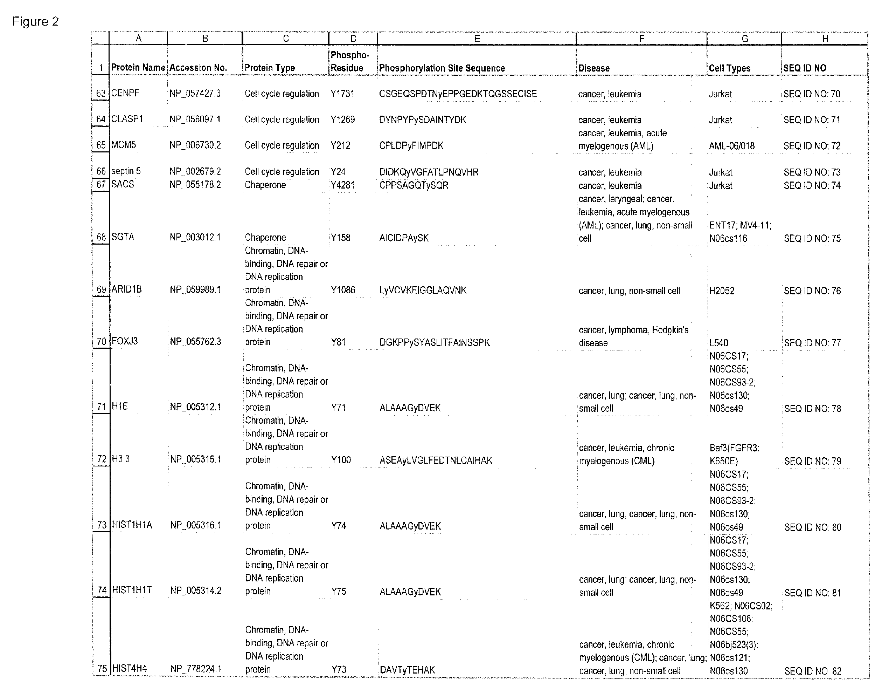

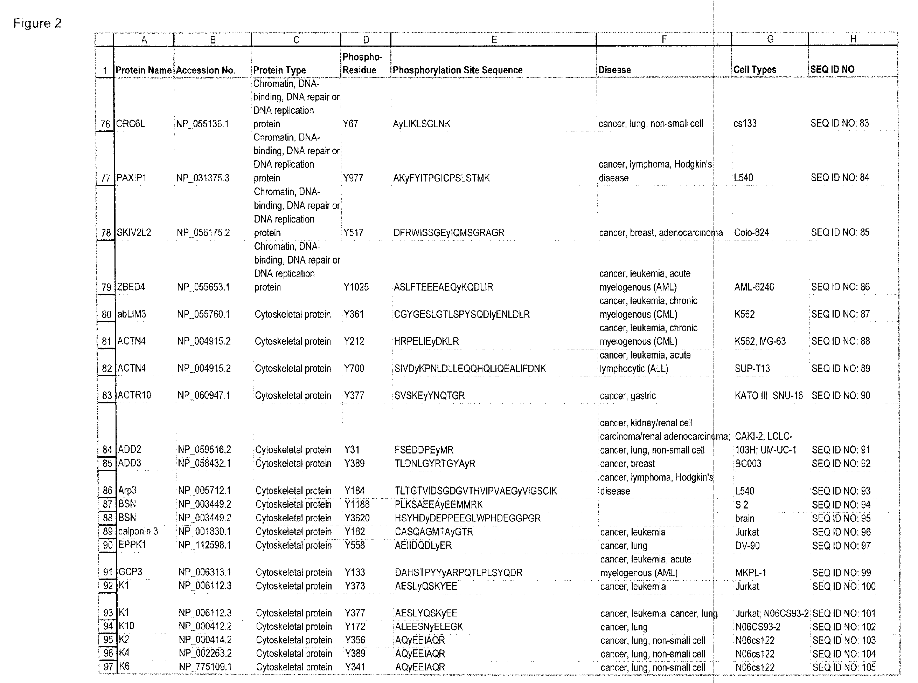

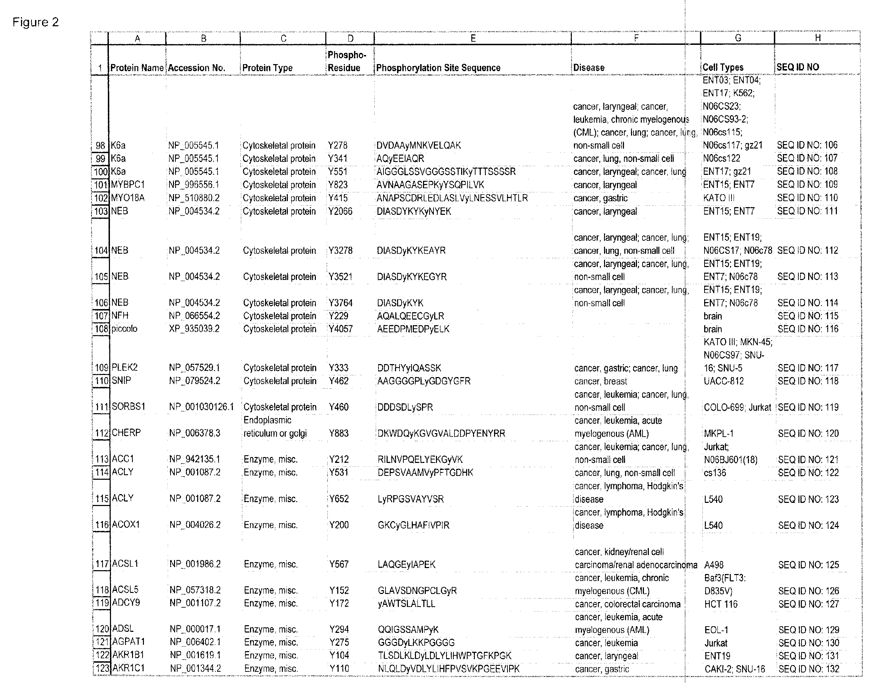

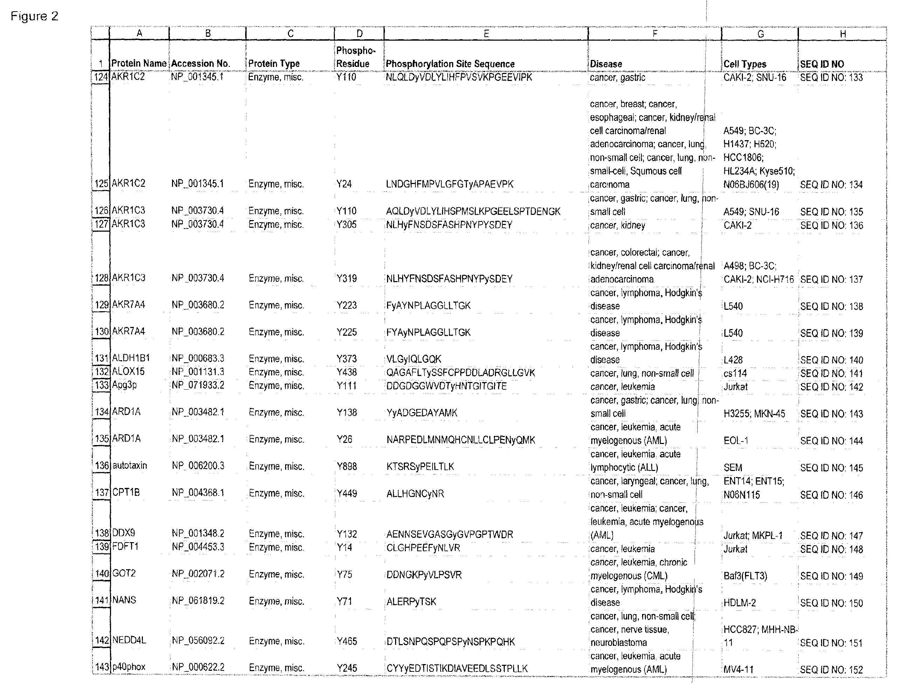

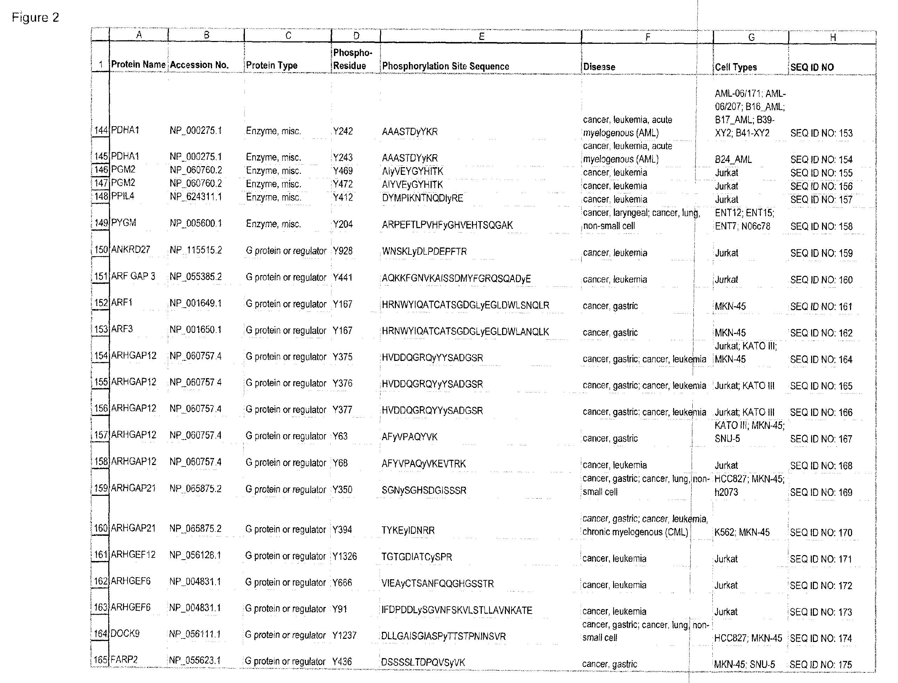

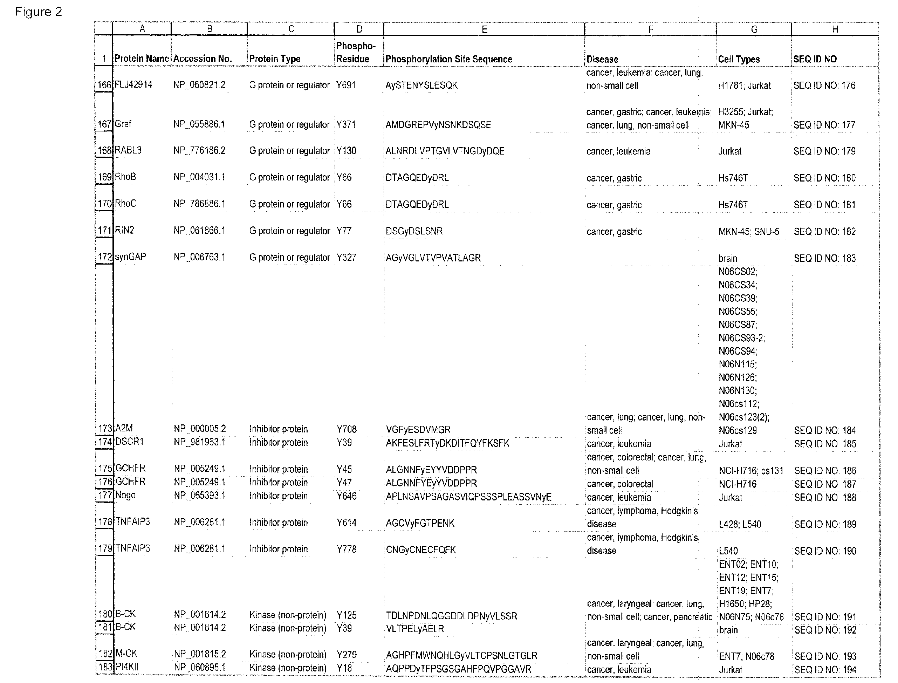

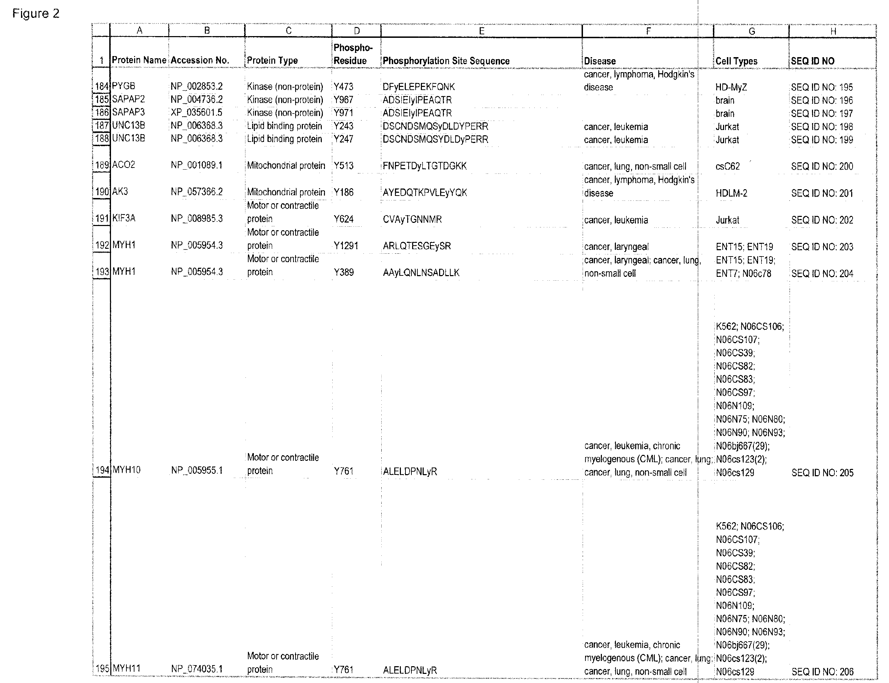

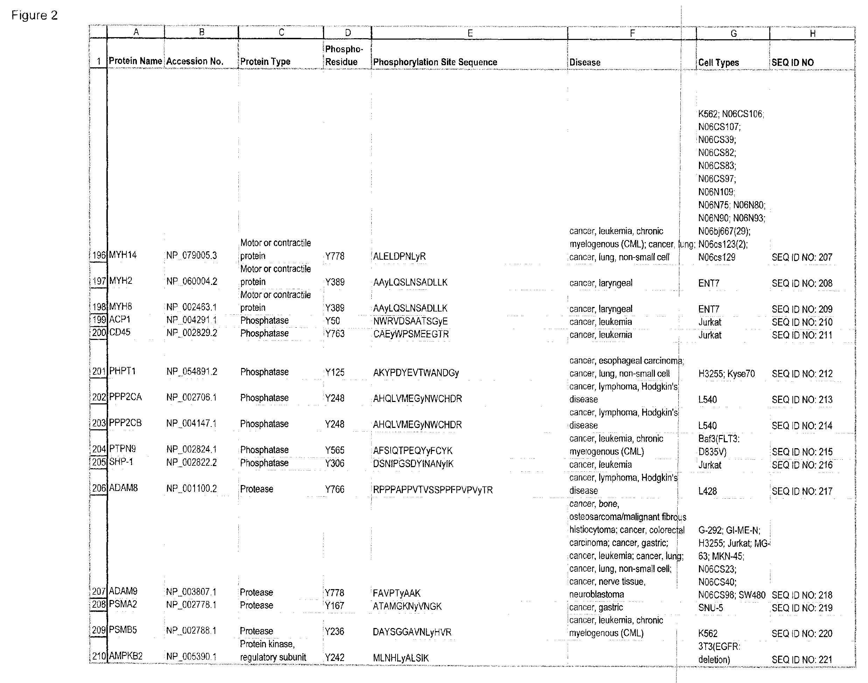

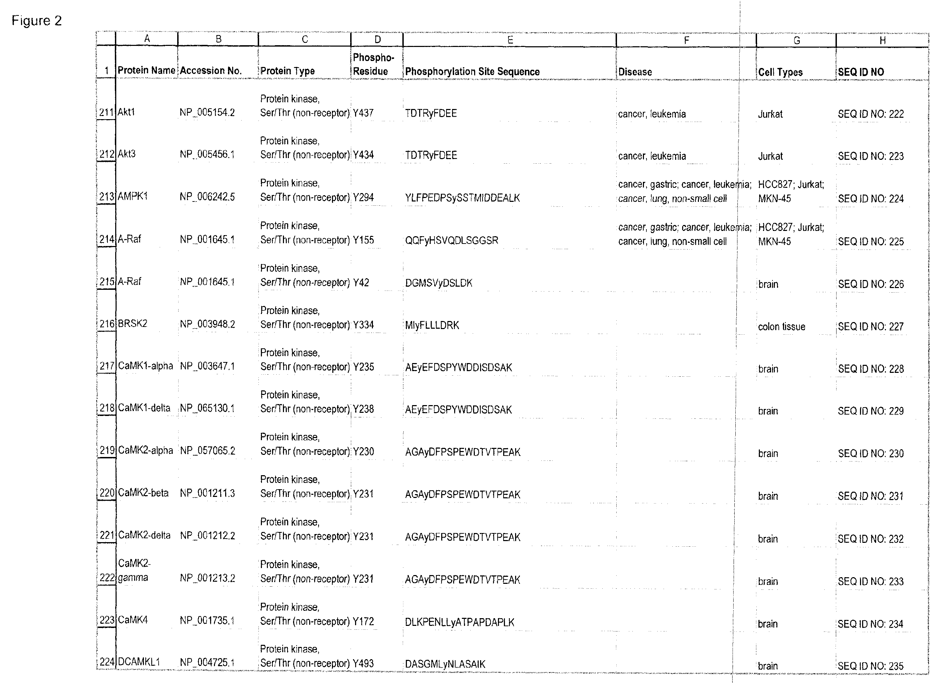

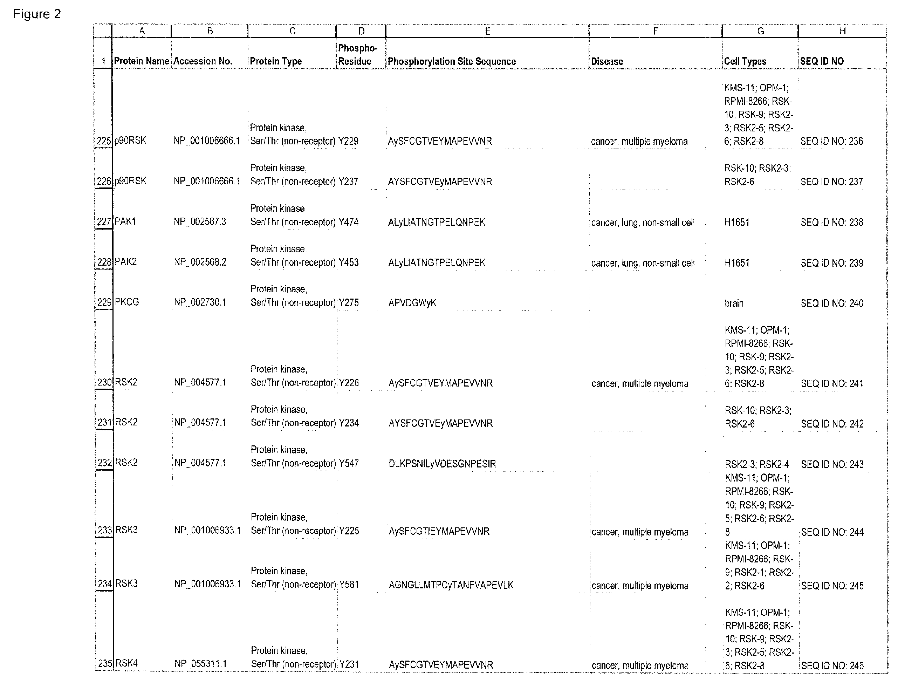

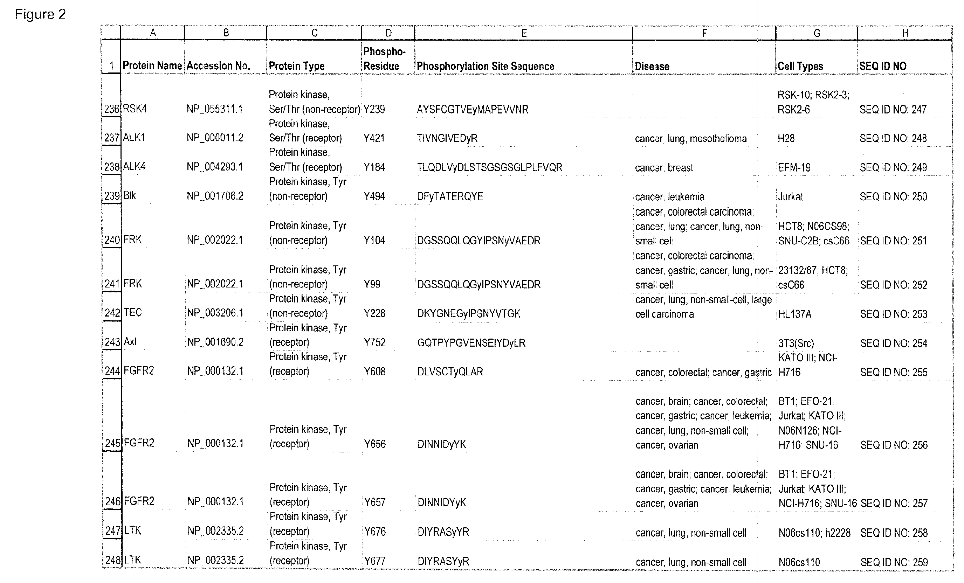

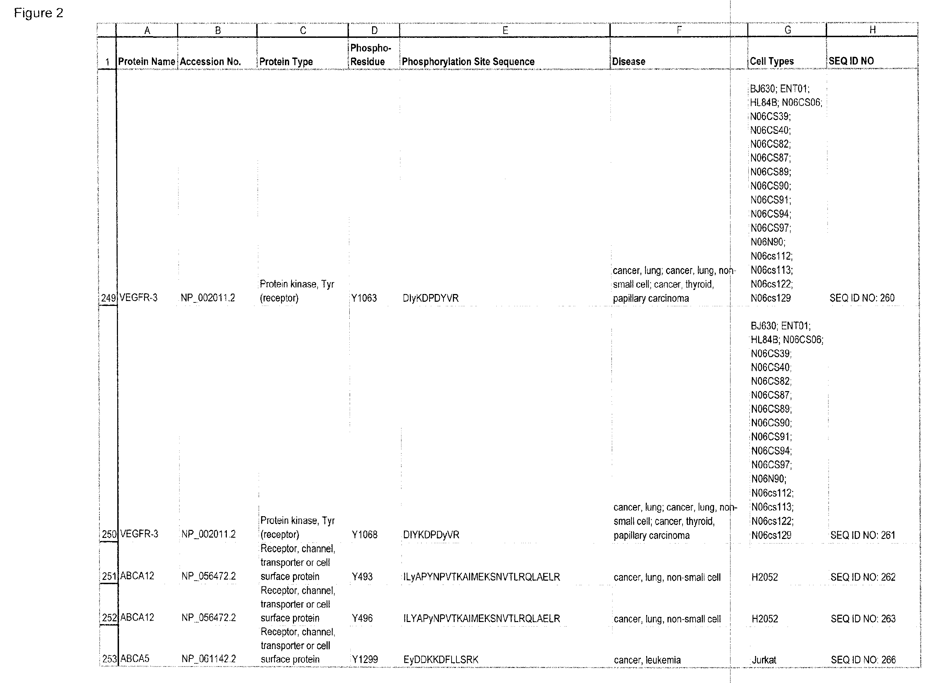

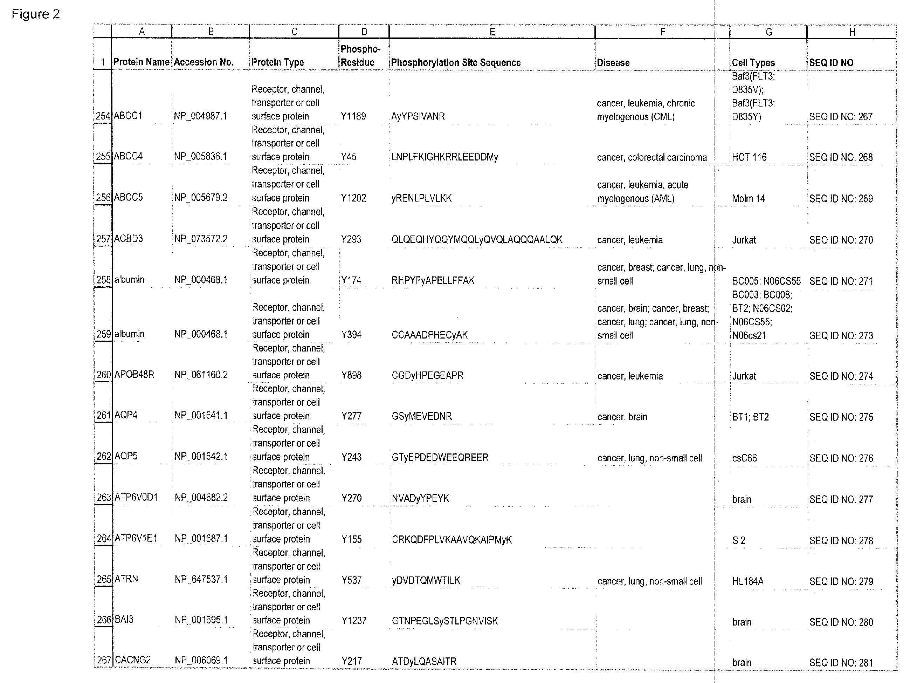

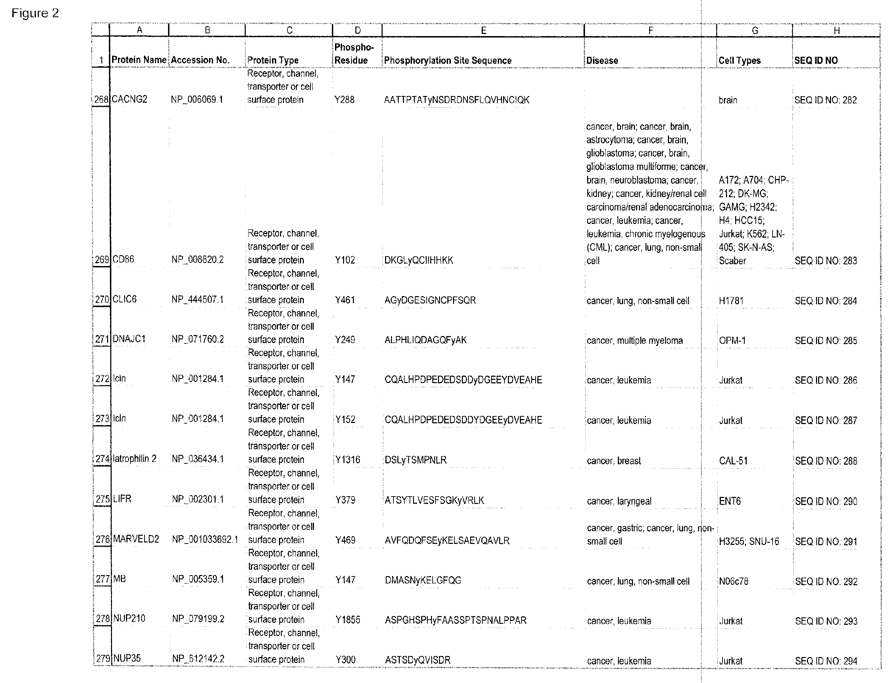

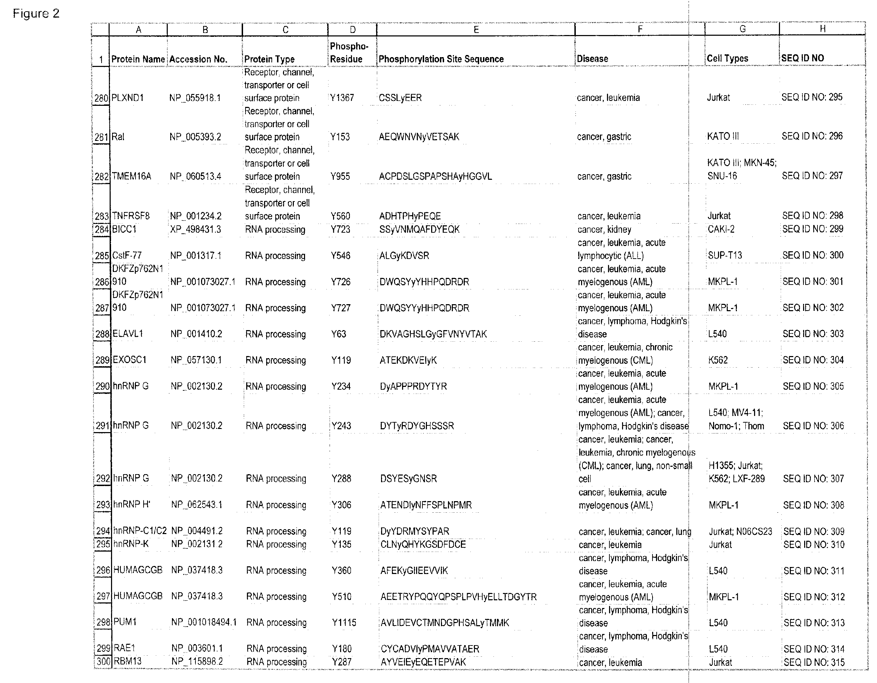

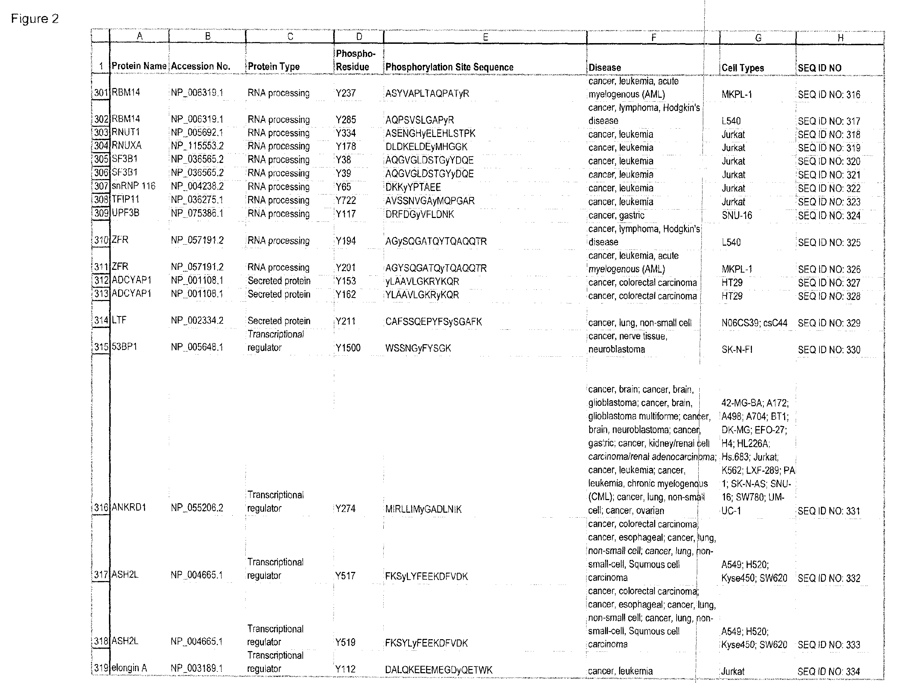

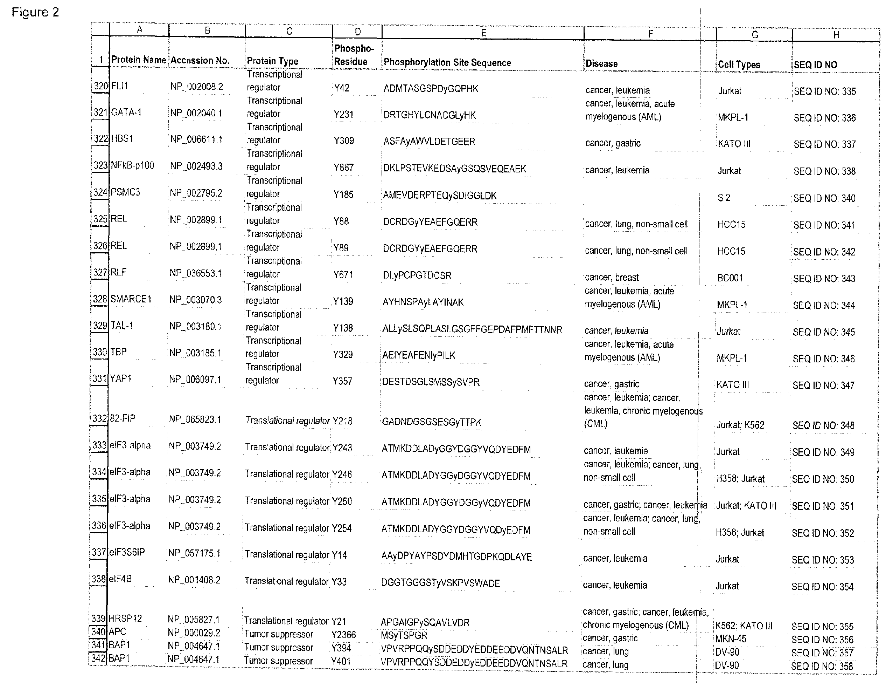

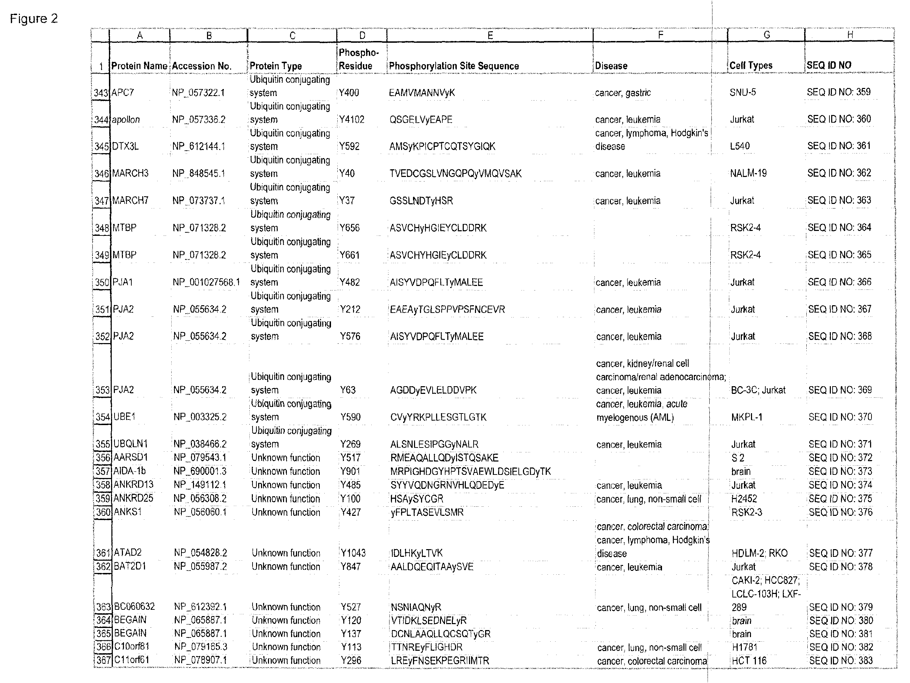

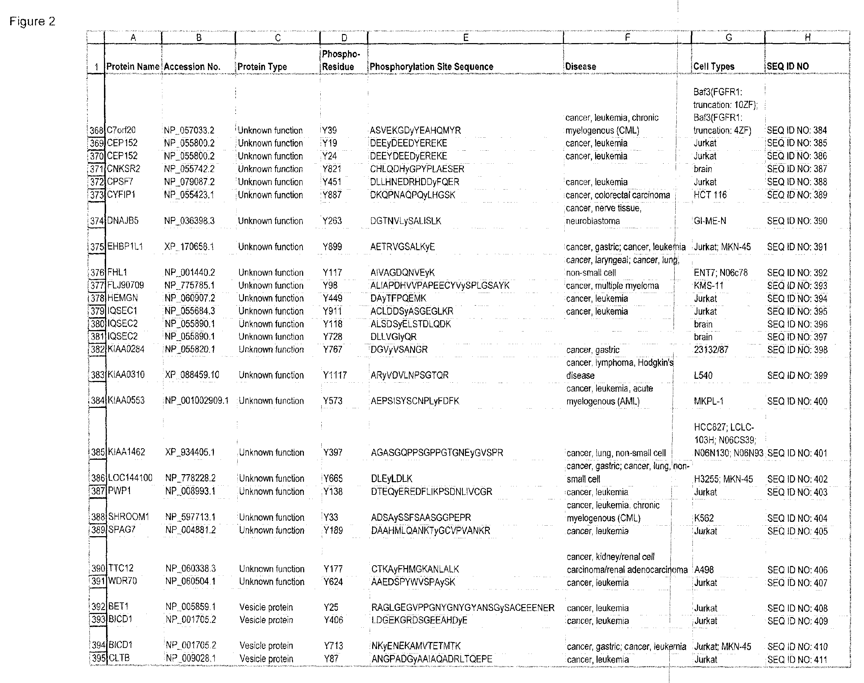

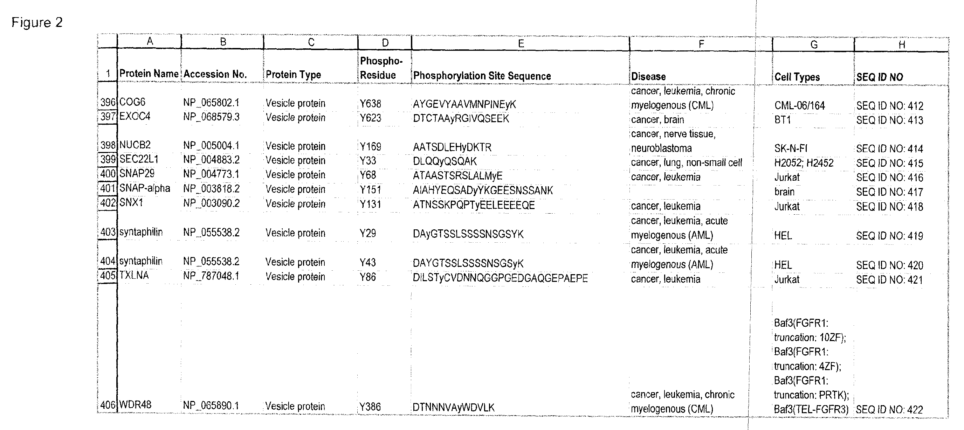

**FIGURE 2** is a table (corresponding to Table 1) summarizing the 405 novel phosphorylation sites of the invention: Column A = the parent proteins from which the phosphorylation sites are derived; Column B = the SwissProt accession number for the human homologue of the identified parent proteins; Column C = the protein type/classification; Column D = the tyrosine residues at which phosphorylation occurs (each number refers to the amino acid residue position of the tyrosine in the parent human protein, according to the published sequence retrieved by the SwissProt accession number); Column E = flanking sequences of the phosphorylatable tyrosine residues; sequences (SEQ ID NOs: 4-12, 14-28, 30-51, 53-57, 59-64, 66-97, 99-127, 129-162, 164-177, 179-263, 266-271, 273-288, 290-338 and 340-422) were identified using Trypsin digestion of the parent proteins; in each sequence, the tyrosine (see corresponding rows in Column D) appears in lowercase; Column F = the type of carcinoma and/or leukemia in which each of the phosphorylation site was discovered; Column G = the cell type(s)/ Tissue/ Patient Sample in which each of the phosphorylation site was discovered; and Column H= the SEQ ID NOs of the trypsin-digested peptides identified in Column E.

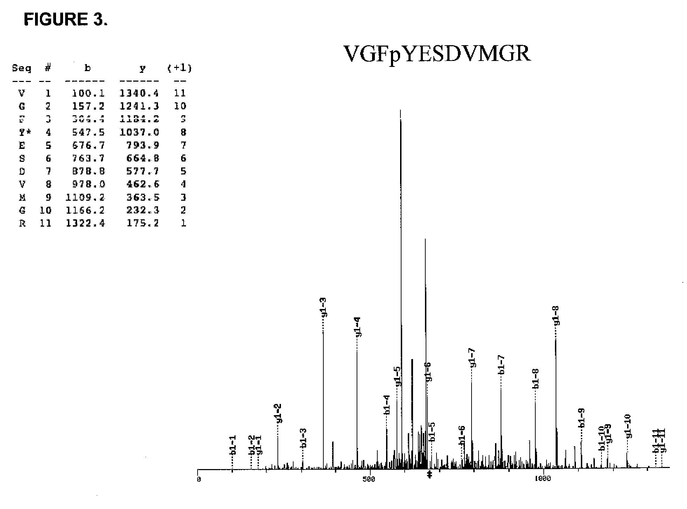

**FIGURE 3** is an exemplary mass spectrograph depicting the detection of the phosphorylation of tyrosine 708 in A2M, as further described in Example 1 (red and blue indicate ions detected in MS/MS spectrum); Y* (and pY) indicates the phosphorylated tyrosine (corresponds to lowercase "y" in Column E of Table 1; SEQ ID NO: 184).

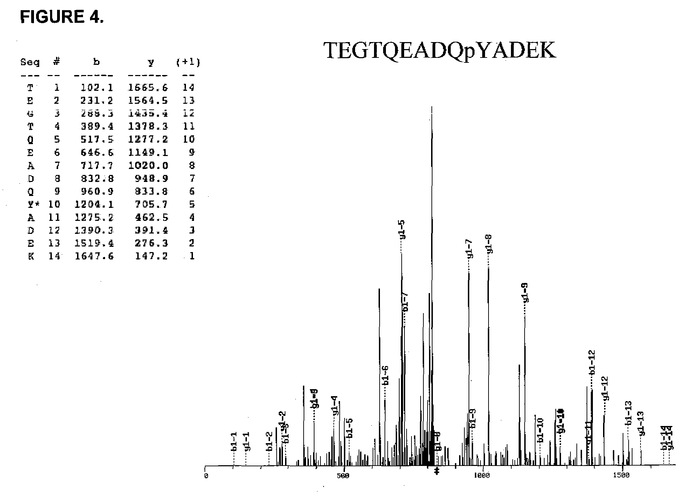

**FIGURE 4** is an exemplary mass spectrograph depicting the detection of the phosphorylation of tyrosine 1270 in AKAP12, as further described in Example 1 (red and blue indicate ions detected in MS/MS spectrum); Y* (and pY) indicates the phosphorylated tyrosine (corresponds to lowercase "y" in Column E of Table 1; SEQ ID NO: 10).

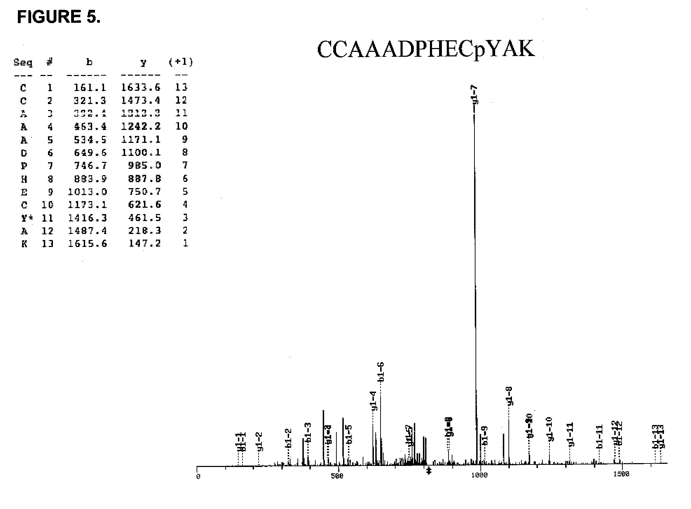

**FIGURE 5** is an exemplary mass spectrograph depicting the detection of the phosphorylation of tyrosine 394 in Albumin, as further described in Example 1 (red and blue indicate ions detected in MS/MS spectrum); Y* (and pY) indicates the phosphorylated tyrosine (corresponds to lowercase "y" in Column E of Table 1; SEQ ID NO: 273).

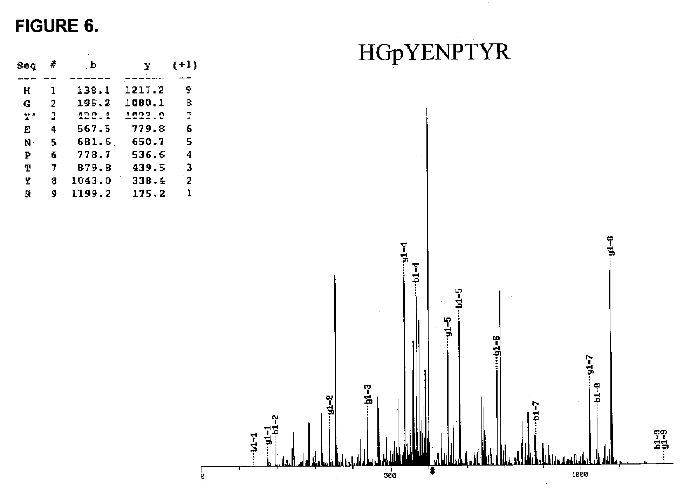

**FIGURE 6** is an exemplary mass spectrograph depicting the detection of the phosphorylation of tyrosine 638 in APLP2, as further described in Example 1 (red and blue indicate ions detected in MS/MS spectrum); Y* (and pY) indicates the phosphorylated tyrosine (corresponds to lowercase "y" in Column E of Table 1; SEQ ID NO: 15).

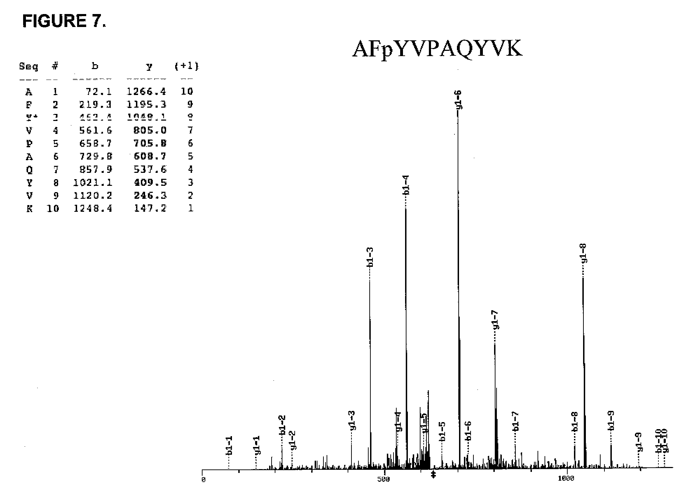

**FIGURE 7** is an exemplary mass spectrograph depicting the detection of the phosphorylation of tyrosine 63 in ARHGAP 12, as further described in Example 1 (red and blue indicate ions detected in MS/MS spectrum); Y* (and pY) indicates the phosphorylated tyrosine (corresponds to lowercase "y" in Column E of Table 1; SEQ ID NO: 167).

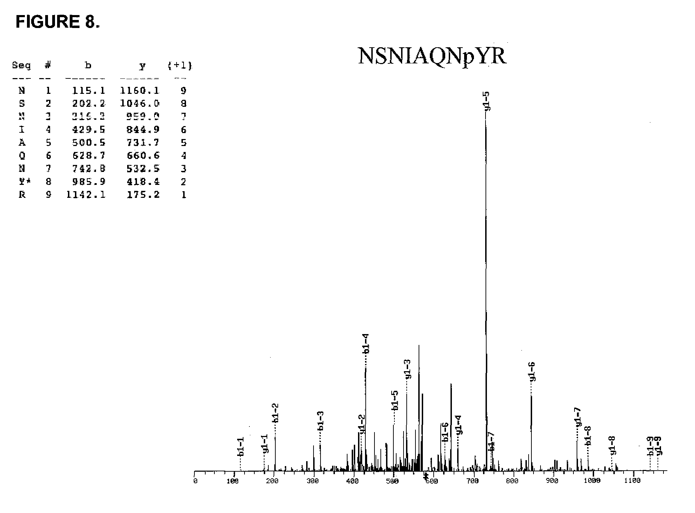

**FIGURE 8** is an exemplary mass spectrograph depicting the detection of the phosphorylation of tyrosine 527 in BC060632, as further described in Example 1 (red and blue indicate ions detected in MS/MS spectrum); Y* (and pY) indicates the phosphorylated tyrosine (corresponds to lowercase "y" in Column E of Table 1; SEQ ID NO: 379).

### DETAILED DESCRIPTION OF THE INVENTION

The inventors have discovered and disclosed herein novel tyrosine phosphorylation sites in signaling proteins extracted from carcinoma and/or leukemia cells. The newly discovered phosphorylation sites significantly extend our knowledge of kinase substrates and of the proteins in which the novel sites occur. The disclosure herein of the novel phosphorylation sites and reagents including peptides and antibodies specific for the sites add important new tools for the elucidation of signaling pathways that are associate with a host of biological processes including cell division, growth, differentiation, develomental changes and disease. Their discovery in carcinoma and/or leukemia cells provides and focuses further elucidation of the disease process. And, the novel sites provide additional diagnostic and therapeutic targets.

### 1. Novel Phosphorylation Sites in Carcinoma and/or leukemia

In one aspect, the invention provides 405 novel tyrosine phosphorylation sites in signaling proteins from cellular extracts from a variety of human carcinoma and/or leukemia-derived cell lines and tissue samples (such as H1993, lung HCC827, etc., as further described below in Examples), identified using the techniques described in "Immunoaffinity Isolation of Modified Peptides From Complex Mixtures," U.S. Patent Publication No. 20030044848, Rush et al.*,* using Table 1 summarizes the identified novel phosphorylation sites.

These phosphorylation sites thus occur in proteins found in carcinoma and/or leukemia. The sequences of the human homologues are publicly available in SwissProt database and their Accession numbers listed in Column B of Table 1. The novel sites occur in proteins such as: enzyme proteins, adaptor/scaffold proteins, protein kinases, receptor/channel/transportercell surface proteins, cytoskeletal proteins, RNA processing proteins, G protein or regulator proteins, transcriptional regulator proteins, adhesion or extracellular matrix proteins and vesicle proteins. (*see* Column C of Table 1).

The novel phosphorylation sites of the invention were identified according to the methods described by Rush et al., U.S. Patent Publication No. 20030044848, which are herein incorporated by reference in its entirety. Briefly, phosphorylation sites were isolated and characterized by immunoaffinity isolation and mass-spectrometric characterization (IAP) (Figure 1), using the following human carcinoma and/or leukemia-derived cell lines and tissue samples: 23132/87; 3T3(EGFR: deletion); 3T3(Src); 42-MG-BA; 5637; A172; A498; A549; A704; AML-06/018; AML-06/171; AML-06/207; AML-6246; B16_AML; B17_AML; B24_AML; B39-XY2; B41-XY2; BC-3C; BC001; BC003; BC005; BC008; BJ630; BT1; BT2; Baf3(FGFR1: truncation: 10ZF); Baf3(FGFR1: truncation: 4ZF); Baf3(FGFR1: truncation: PRTK); Baf3(FGFR3: K650E); Baf3(FLT3); Baf3(FLT3: D835V); Baf3(FLT3: D835Y); Baf3(TEL-FGFR3); CAKI-2; CAL-29; CAL-51; CHP-212; CML-06/038; CML-06/164; COLO-699; Colo-824; DK-MG; DV-90; EFM-19; EFO-21; EFO-27; ENT01; ENT02; ENT03; ENT04; ENT10; ENT12; ENT14; ENT15; ENT17; ENT19; ENT6; ENT7; EOL-1; G-292; GAMG; GI-ME-N; H1355; H1437; H1650; H1651; H1703; H1781; H1838; H2052; H2342; H2452; H28; H3255; H358; H4; H520; HCC15; HCC1806; HCC78; HCC827; HCT 116; HCT8; HD-MyZ; HDLM-2; HEL; HL137A; HL184A; HL226A; HL233B; HL234A; HL84B; HP28; HT29; Hs.683; Hs746T; Jurkat; K562; KATO III; KMS-11; Kyse140; Kyse150; Kyse450; Kyse510; Kyse70; L428; L540; LCLC-103H; LN-405; LXF-289; MG-63; MHH-NB-11; MKN-45; MKPL-1; MV4-11; Molm 14; N06BJ601(18); N06BJ606(19); N06CS02; N06CS06; N06CS106; N06CS107; N06CS17; N06CS23; N06CS34; N06CS39; N06CS40; N06CS55; N06CS82; N06CS83; N06CS87; N06CS89; N06CS90; N06CS91; N06CS93-2; N06CS94; N06CS97; N06CS98; N06N109; N06N115; N06N126; N06IST130; N06N75; N06N80; N06N90; N06N93; N06bj523(3); N06bj632(24); N06bj667(29); N06c78; N06cs 110; N06cs 112; N06cs 113; N06cs 115; N06cs 116; N06cs 117; N06cs121; N06cs122; N06cs123(2); N06cs129; N06cs130; N06cs21; N06cs49; NALM-19; NCI-H716; Nomo-1; OPM-1; PA-1; RKO; RPMI-8266; RSK-10; RSK-9; RSK2-1; RSK2-2; RSK2-3; RSK2-4; RSK2-5; RSK2-6; RSK2-8; S 2; SEM; SK-N-AS; SK-N-FI; SNU-1; SNU-16; SNU-5; SNU-C2B; SUP-T13; SW480; SW620; SW780; Scaber; Thom; UACC-812; UM-UC-1; brain; colon tissue; cs114; cs131; cs133; cs136; csC43; csC44; csC56; csC62; csC66; gz21; h2073; h2228. In addition to the newly discovered phosphorylation sites (all having a phosphorylatable tyrosine), many known phosphorylation sites were also identified.

The immunoaffinity/mass spectrometric technique described in Rush et al, *i.e.,* the "IAP" method, is described in detail in the Examples and briefly summarized below.

The IAP method generally comprises the following steps: (a) a proteinaceous preparation (e.g., a digested cell extract) comprising phosphopeptides from two or more different proteins is obtained from an organism; (b) the preparation is contacted with at least one immobilized general phosphotyrosine-specific antibody; (c) at least one phosphopeptide specifically bound by the immobilized antibody in step (b) is isolated; and (d) the modified peptide isolated in step (c) is characterized by mass spectrometry (MS) and/or tandem mass spectrometry (MS-MS). Subsequently, (e) a search program (e.g., Sequest) may be utilized to substantially match the spectra obtained for the isolated, modified peptide during the characterization of step (d) with the spectra for a known peptide sequence. A quantification step, e.g., using SILAC or AQUA, may also be used to quantify isolated peptides in order to compare peptide levels in a sample to a baseline.

In the IAP method as disclosed herein, a general phosphotyrosine-specific monoclonal antibody (commercially available from Cell Signaling Technology, Inc., Beverly, MA, Cat #9411 (p-Tyr-100)) may be used in the immunoaffinity step to isolate the widest possible number of phospho-tyrosine containing peptides from the cell extracts.

As described in more detail in the Examples, lysates may be prepared from various carcinoma and/or leukemia cell lines or tissue samples and digested with trypsin after treatment with DTT and iodoacetamide to alkylate cysteine residues. Before the immunoaffinity step, peptides may be pre-fractionated (e.g., by reversed-phase solid phase extraction using Sep-Pak C₁₈ columns) to separate peptides from other cellular components. The solid phase extraction cartridges may then be eluted (e.g., with acetonitrile). Each lyophilized peptide fraction can be redissolved and treated with phosphotyrosine-specific antibody (e.g., P-Tyr-100, CST #9411) immobilized on protein Agarose. Immunoaffinity-purified peptides can be eluted and a portion of this fraction may be concentrated (e.g., with Stage or Zip tips) and analyzed by LC-MS/MS (e.g., using a ThermoFinnigan LCQ Deca XP Plus ion trap mass spectrometer or LTQ). MS/MS spectra can be evaluated using, e.g., the program Sequest with the NCBI human protein database.

The novel phosphorylation sites identified are summarized in Table1/Figure 2. Column A lists the parent (signaling) protein in which the phosphorylation site occurs. Column D identifies the tyrosine residue at which phosphorylation occurs (each number refers to the amino acid residue position of the tyrosine in the parent human protein, according to the published sequence retrieved by the SwissProt accession number). Column E shows flanking sequences of the identified tyrosine residues (which are the sequences of trypsin-digested peptides). Figure 2 also shows the particular type of carcinoma and/or leukemia (see Column G) and cell line(s) (see Column F) in which a particular phosphorylation site was discovered.

**Table 1. Novel Phosphorylation Sites in Carcinoma and/or leukemia.**

| 1 | **Protein Name** | **Accession No.** | **Protein Type** | **Phospho-Residue** | **Phosphorylation Site Sequence** | **SEQ ID NO** |
|---|---|---|---|---|---|---|
| 2 | 14-3-3 zeta | NP_003397.1 | Adaptor/scaffold | Y211 | AKTAFDEAIAELDTLSEESyKDSTL | SEQ ID NO: 4 |
| 3 | AFAP | NP_067651.2 | Adaptor/scaffold | Y248 | EAySGCSGPVDSECPPPPSSPVHK | SEQ ID NO: 5 |
| 4 | AFAP | NP_067651.2 | Adaptor/scaffold | Y491 | VISANPYLGGTSNGyAHPSGTALHYDDV PCINGSLK | SEQ ID NO: 6 |
| 5 | AIP1 | NP_036433.2 | Adaptor/scaffold | Y362 | IDDPlyGTYYVDHINR | SEQ ID NO: 7 |
| 6 | AKAP11 | NP_057332.1 | Adaptor/scaffold | Y485 | NHDSVyYTYE | SEQ ID NO: 8 |
| 7 | AKAP11 | NP_057332.1 | Adaptor/scaffold | Y488 | NHDSVYYTyE | SEO ID NO: 9 |
| 8 | AKAP12 | NP_005091.2 | Adaptor/scaffold | Y1270 | TEGTQEADQyADEK | SEQ ID NO: 10 |
| 9 | AKAP2 | NP_009134.1 | Adaptor/scaffold | Y136 | GFSSTDGDAVNyISSQLPDLPILCSR | SEQ ID NO: 11 |
| 10 | ANK1 | NP_000028.3 | Adaptor/scaffold | Y603 | GGSPHSPAWNGyTPLHIAAK | SEQ ID NO: 12 |
| 11 | ANK3 | NP_066267.2 | Adaptor/scaffold | Y484 | yLVQDGAQVEAKAK | SEQ ID NO: 14 |
| 12 | APLP1 | NP_005157.1 | Adaptor/scaffold | Y638 | HGyENPTYR | SEQ ID NO: 15 |
| 13 | APLP1 | NP_005157.1 | Adaptor/scaffold | Y643 | HGYENPTyR | SEQ ID NO: 16 |
| 14 | APPL | NP_036228.1 | Adaptor/scaffold | Y161 | yEVTEDVYTSR | SEQ ID NO: 17 |
| 15 | APPL | NP_036228.1 | Adaptor/scaffold | Y168 | YEVTEDVyTSR | SEQ ID NO: 18 |
| 16 | axin 2 | AA101534.1 | Adaptor/scaffold | Y549 | VHCFCPGGSEyYCYSK | SEQ ID NO: 19 |
| 17 | CASKIN1 | NP_065815.1 | Adaptor/scaffold | Y296 | DYCNNyDLTSLNVK | SEQ ID NO: 20 |
| 18 | Cas-L | NP_006394.1 | Adaptor/scaffold | Y112 | DTIYQVPPSyQNQGIYQVPTGHGTQEQE VYQVPPSVQR | SEQ ID NO: 21 |
| 19 | Cas-L | NP_006394.1 | Adaptor/scaffold | Y132 | DTIYQVPPSYQNQGIYQVPTGHGTQEQE VyQVPPSVQR | SEQ ID NO: 22 |
| 20 | Cbl | NP_005179.2 | Adaptor/scaffold | Y735 | AMyNIQSQAPSITE | SEQ ID NO: 23 |
| 21 | CSDE1 | NP_001007554 .1 | Adaptor/scaffold | Y690 | CVKDQFGFINyE | SEQ ID NO: 24 |
| 22 | DAB2 | NP_001334.1 | Adaptor/scaffold | Y761 | DSFGSSQASVASSQPVSSEMyRDPFGN PFA | SEQ ID NO: 25 |
| 23 | DLG3 | NP_066943.2 | Adaptor/scaffold | Y600 | DFPGLSDDyYGAK | SEQ ID NO: 26 |
| 24 | DLG3 | NP_066943.2 | Adaptor/scaffold | Y601 | DFPGLSDDYyGAK | SEQ ID NO: 27 |
| 25 | DLG5 | NP_004738.3 | Adaptor/scaffold | Y429 | DAVySEYK | SEQ ID NO: 28 |
| 26 | IRTKS | NP_061330.2 | Adaptor/scaffold | Y293 | AyTSPLIDMFNNPATAAPNSQR | SEQ ID NO: 30 |
| 27 | MICAL1 | NP_073602.2 | Adaptor/scaffold | Y483 | DLyDVLAKEPVQR | SEQ ID NO: 31 |
| 28 | PAG | NP_060910.3 | Adaptor/scaffold | Y105 | DSTLTCMQHyEE | SEQ ID NO: 32 |
| 29 | PARD3 | NP_062565.2 | Adaptor/scaffold | Y933 | AAIDKSyDKPAVDDDDEGMETLEEDTEE SSR | SEQ ID NO: 33 |
| 30 | SAPAP 3 | XP_035601.5 | Adaptor/scaffold | Y725 | APTySVFR | SEQ ID NO: 34 |
| 31 | sciellin | NP_003834.2 | Adaptor/scaffold | Y58 | DENyGRVVLNRHNSHDALDR | SEQ ID NO: 35 |
| 32 | SPAG9 | NP_003962.3 | Adaptor/scaffold | Y9 | DGVVyGEEPGGSGAVMSE | SEO ID NO: 36 |
| 33 | TANK | NP_004171.2 | Adaptor/scaffold | Y338 | AACLPPGDHNALyVNSFPLLDPSDAPFP SLDSPGK | SEQ ID NO: 37 |
| 34 | TRAF4 | NP_004286.2 | Adaptor/scaffold | Y166 | CEFCGCDFSGEAYESHEGMCPQESVyC ENK | SEQ ID NO: 38 |
| 35 | VANGL 1 | NP_620409.1 | Adaptor/scaffold | Y290 | DFTlyNPNLLTASK | SEQ ID NO: 39 |
| 36 | ZO2 | NP_004808.2 | Adaptor/scaffold | Y257 | AyDPDYERAYSPEYR | SEQ ID NO: 40 |
| 37 | ZO2 | NP_004808.2 | Adaptor/scaffold | Y269 | AYDPDYERAYSPEyR | SEQ ID NO:41 |
| 38 | afadin | NP_005927.2 | Adhesion or extracellular matrix protein | Y1203 | ITSVSTGNLCTEEQTPPPRPEAyPIPTQT YTR | SEQ ID NO: 42 |
| 39 | afadin | NP_001035090 .1 | Adhesion or extracellular matrix protein | Y1666 | RQEEGyYSR | SEQ ID NO: 43 |
| 40 | afadin | NP_001035090 .1 | Adhesion or extracellular matrix protein | Y1667 | RQEEGYySR | SEQ ID NO: 44 |
| 41 | afadin | NP_005927.2 | Adhesion or extracellular matrix protein | Y262 | IYADSLKPNIPyK | SEQ ID NO: 45 |
| 42 | afadin | NP_005927.2 | Adhesion or extracellular matrix protein | Y374 | ADGSGyGSTLPPEK | SEQ ID NO: 46 |
| 43 | ASAM | NP_079045.1 | Adhesion or extracellular matrix protein | Y333 | TLSTDAAPQPGLATQAySLVGPEVR | SEQ ID NO: 47 |
| 44 | CDH1 | NP_004351.1 | Adhesion or extracellular matrix protein | Y755 | DNVYYyDEEGGGEEDQDFDLSQLHR | SEQ ID NO: 48 |
| 45 | CLDN14 | NP_036262.1 | Adhesion or extracellular matrix protein | Y211 | ATTTTANTAPAyQPPAAYKDNR | SEQ ID NO: 49 |
| 46 | CLDN14 | NP_036262.1 | Adhesion or extracellular matrix protein | Y217 | ATTTTANTAPAYQPPAAyKDNR | SEQ ID NO: 50 |
| 47 | CLDN14 | NP_036262.1 | Adhesion or extracellular matrix protein | Y233 | APSVTSATHSGyR | SEQ ID NO: 51 |
| 48 | CYFIP2 | NP_055191.2 | Adhesion or extracellular matrix protein | Y886 | DKPANVQPYyLYGSKPLNIAYSHIYSSYR | SEQ ID NO: 53 |
| 49 | FLOT2 | NP_004466.2 | Adhesion or extracellular matrix protein | Y158 | DVyDKVDYLSSLGK | SEQ ID NO: 54 |
| 50 | ITGA6 iso2 | NP_000201.2 | Adhesion or extracellular matrix protein | Y1054 | DHYDATyHK | SEQ ID NO: 55 |
| 51 | Scribble | NP_056171.2 | Adhesion or extracellular matrix protein | Y564 | ATTAGGEEDAEEDyQEPTVHFAE | SEQ ID NO: 56 |
| 52 | syndeca n-2 | NP_002989.2 | Adhesion or extracellular matrix protein | Y200 | APTKEFyA | SEQ ID NO: 57 |
| 53 | aven | NP_065104.1 | Apoptosis | Y121 | IVSNWDRyQDIEKEVNNESGESQR | SEQ ID NO: 59 |
| 54 | BAG3 | NP_004272.2 | Apoptosis | Y508 | QKAIDVPGQVQVyE | SEQ ID NO: 60 |
| 55 | BAG4 | NP_004865.1 | Apoptosis | Y72 | VRGGGPAETTWLGEGGGGDGyYPSGG AWPEPGR | SEQ ID NO: 61 |
| 56 | GRP94 | NP_003290.1 | Apoptosis | Y678 | DISTNYyASQK | SEQ ID NO: 62 |
| 57 | SEPT4 | NP_004565.1 | Apoptosis | Y115 | LDPyDSSEDDKEYVGFATLPNQVHR | SEQ ID NO: 63 |
| 58 | SEPT4 | NP_004565.1 | Apoptosis | Y124 | LDPYDSSEDDKEyVGFATLPNQVHR | SEQ ID NO: 64 |
| 59 | ANXA5 | NP_001145.1 | Calcium-binding protein | Y257 | SIPAYLAETLYyAMKGAGTDDHTLIR | SEQ ID NO: 66 |
| 60 | ANXA6 | NP_001146.2 | Calcium-binding protein | Y340 | LSGGDDDAAGQFFPEAAQVAyQMWELS AVAR | SEQ ID NO: 67 |
| 61 | ALMS1 | NP_05935.4 | Cell cycle regulation | Y395 | SyGQYWTQEDSSK | SEQ ID NO: 68 |
| 62 | B99 | NP_057510.2 | Cell cycle regulation | Y147 | ETyYLSDSPLLGPPVGEPR | SEQ ID NO: 69 |
| 63 | CENPF | NP_057427.3 | Cell cycle regulation | Y1731 | CSGEQSPDTNyEPPGEDKTQGSSECISE | SEQ ID NO: 70 |
| 64 | CLASP1 | NP_056097.1 | Cell cycle regulation | Y1269 | DYNPYPySDAINTYDK | SEQ ID NO: 71 |
| 65 | MCM5 | NP_006730.2 | Cell cycle regulation | Y212 | CPLDPyFIMPDK | SEQ ID NO: 72 |
| 66 | septin 5 | NP_002679.2 | Cell cycle regulation | Y24 | DIDKQyVGFATLPNQVHR | SEQ ID NO: 73 |
| 67 | SACS | NP_055178.2 | Chaperone | Y4281 | CPPSAGQTySQR | SEQ ID NO: 74 |
| 68 | SGTA | NP_003012.1 | Chaperone | Y158 | AICIDPAySK | SEQ ID NO: 75 |
| 69 | ARID1B | NP_059989.1 | Chromatin, DNA-binding, DNA repair or DNA replication protein | Y1086 | LyVCVKEIGGLAQVNK | SEQ ID NO: 76 |
| 70 | FOXJ3 | NP_055762.3 | Chromatin, DNA-binding, DNA repair or DNA replication protein | Y81 | DGKPPySYASLITFAINSSPK | SEQ ID NO: 77 |
| 71 | H1E | NP_005312.1 | Chromatin, DNA-binding, DNA repair or DNA replication protein | Y71 | ALAAAGyDVEK | SEQ ID NO: 78 |
| 72 | H3.3 | NP_005315.1 | Chromatin, DNA-binding, DNA repair or DNA replication protein | Y100 | ASEAyLVGLFEDTNLCAIHAK | SEQ ID NO: 79 |
| 73 | HIST1H 1A | NP_005316.1 | Chromatin, DNA-binding, DNA repair or DNA replication protein | Y74 | ALAAAGyDVEK | SEQ ID NO: 80 |
| 74 | HIST1H 1T | NP_005314.2 | Chromatin, DNA-binding, DNA repair or DNA replication protein | Y75 | ALAAAGyDVEK | SEQ ID NO: 81 |
| 75 | HIST4H 4 | NP_778224.1 | Chromatin, DNA-binding, DNA repair or DNA replication protein | Y73 | DAVTyTEHAK | SEQ !D NO: 82 |
| 76 | ORC6L | NP_055136.1 | Chromatin, DNA-binding, DNA repair or DNA replication protein | Y67 | AyLIKLSGLNK | SEQ ID NO: 83 |
| 77 | PAXIP1 | NP_031375.3 | Chromatin, DNA-binding, DNA repair or DNA replication protein | Y977 | AKyFYITPGICPSLSTMK | SEQ ID NO: 84 |
| 78 | SKIV2L 2 | NP_056175.2 | Chromatin, DNA-binding, DNA repair or DNA replication protein | Y517 | DFRWISSGEyIQMSGRAGR | SEQ ID NO: 85 |
| 79 | ZBED4 | NP_055653.1 | Chromatin, DNA-binding, DNA repair or DNA replication protein | Y1025 | ASLFTEEEAEQyKQDLIR | SEQ ID NO: 86 |
| 80 | abLIM3 | NP_055760.1 | Cytoskeletal protein | Y361 | CGYGESLGTLSPYSQDIyENLDLR | SEQ ID NO: 87 |
| 81 | ACTN4 | NP_004915.2 | Cytoskeletal protein | Y212 | HRPELIEyDKLR | SEQ ID NO: 88 |
| 82 | ACTN4 | NP_004915.2 | Cytoskeletal protein | Y700 | SIVDyKPNLDLLEQQHQLIQEALIFDNK | SEQ ID NO: 89 |
| 83 | ACTR10 | NP_060947.1 | Cytoskeletal protein | Y377 | SVSKEyYNQTGR | SEQ ID NO: 90 |
| 84 | ADD2 | NP_059516.2 | Cytoskeletal protein | Y31 | FSEDDPEyMR | SEQ ID NO: 91 |
| 85 | ADD3 | NP_058432.1 | Cytoskeletal protein | Y389 | TLDNLGYRTGYAyR | SEQ ID NO: 92 |
| 86 | Arp3 | NP_005712.1 | Cytoskeletal protein | Y184 | TLTGTVIDSGDGVTHVIPVAEGyVIGSCIK | SEQ ID NO: 93 |
| 87 | BSN | NP_003449.2 | Cytoskeletal protein | Y1188 | PLKSAEEAyEEMMRK | SEQ ID NO: 94 |
| 88 | BSN | NP_003449.2 | Cytoskeletal protein | Y3620 | HSYHDyDEPPEEGLWPHDEGGPGR | SEQ ID NO: 95 |
| 89 | calponin 3 | NP_001830.1 | Cytoskeletal protein | Y182 | CASQAGMTAyGTR | SEQ ID NO: 96 |
| 90 | EPPK1 | NP_112598.1 | Cytoskeletal protein | Y558 | AEIIDQDLyER | SEQ ID NO: 97 |
| 91 | GCP3 | NP_006313.1 | Cytoskeletal protein | Y133 | DAHSTPYYyARPQTLPLSYQDR | SEQ ID NO: 99 |
| 92 | K1 | NP_006112.3 | Cytoskeletal protein | Y373 | AESLyQSKYEE | SEQ ID NO: 100 |
| 93 | K1 | NP_006112.3 | Cytoskeletal protein | Y377 | AESLYQSKyEE | SEQ ID NO: 101 |
| 94 | K10 | NP_000412.2 | Cytoskeletal protein | Y172 | ALEESNyELEGK | SEQ ID NO: 102 |
| 95 | K2 | NP_000414.2 | Cytoskeletal protein | Y356 | AQyEEIAQR | SEQ ID NO: 103 |
| 96 | K4 | NP_002263.2 | Cytoskeletal protein | Y389 | AQyEEIAQR | SEQ ID NO: 104 |
| 97 | K6 | NP_775109.1 | Cytoskeletal protein | Y341 | AQyEEIAQR | SEQ ID NO: 105 |
| 98 | K6a | NP_005545.1 | Cytoskeletal protein | Y278 | DVDAAyMNKVELQAK | SEQ ID NO: 106 |
| 99 | K6a | NP_005545.1 | Cytoskeletal protein | Y341 | AQyEEIAQR | SEQ ID NO: 107 |
| 100 | K6a | NP_005545.1 | Cytoskeletal protein | Y551 | AIGGGLSSVGGGSSTIKyTTTSSSSR | SEQ ID NO: 108 |
| 101 | MYBPC 1 | NP_996556.1 | Cytoskeletal protein | Y823 | AVNAAGASEPKyYSQPILVK | SEQ ID NO: 109 |
| 102 | MYO18 A | NP_510880.2 | Cytoskeletal protein | Y415 | ANAPSCDRLEDLASLVyLNESSVLHTLR | SEQ ID NO: 110 |
| 103 | NEB | NP_004534.2 | Cytoskeletal protein | Y2066 | DIASDYKYKyNYEK | SEQ ID NO: 111 |
| 104 | NEB | NP_004534.2 | Cytoskeletal protein | Y3278 | DIASDyKYKEAYR | SEQ ID NO: 112 |
| 105 | NEB | NP_004534.2 | Cytoskeletal protein | Y3521 | DIASDyKYKEGYR | SEQ ID NO: 113 |
| 106 | NEB | NP_004534.2 | Cytoskeletal protein | Y3764 | DIASDyKYK | SEQ ID NO:114 |
| 107 | NFH | NP_066554.2 | Cytoskeletal protein | Y229 | AQALQEECGyLR | SEQ ID NO:115 |
| 108 | piccolo | XP_935039.2 | Cytoskeletal protein | Y4057 | AEEDPMEDPyELK | SEQ ID NO:116 |
| 109 | PLEK2 | NP_057529.1 | Cytoskeletal protein | Y333 | DDTHYyIQASSK | SEQ ID NO:117 |
| 110 | SNIP | NP_079524.2 | Cytoskeletal protein | Y462 | AAGGGGPLyGDGYGFR | SEQ ID NO:118 |
| 111 | SORBS 1 | NP_001030126 .1 | Cytoskeletal protein | Y460 | DDDSDLySPR | SEQ ID NO:119 |
| 112 | CHERP | NP_006378.3 | Endoplasmic reticulum or golgi | Y883 | DKWDQyKGVGVALDDPYENYRR | SEQ ID NO: 120 |
| 113 | ACC1 | NP_942135.1 | Enzyme, misc. | Y212 | RILNVPQELYEKGyVK | SEQ ID NO: 121 |
| 114 | ACLY | NP_001087.2 | Enzyme, misc. | Y531 | DEPSVAAMVyPFTGDHK | SEQ ID NO: 122 |
| 115 | ACLY | NP_001087.2 | Enzyme, misc. | Y652 | LyRPGSVAYVSR | SEQ ID NO: 123 |
| 116 | ACOX1 | NP_004026.2 | Enzyme, misc. | Y200 | GKCyGLHAFIVPIR | SEQ ID NO:124 |
| 117 | ACSL1 | NP_001986.2 | Enzyme, misc. | Y567 | LAQGEyIAPEK | SEQ ID NO: 125 |
| 118 | ACSL5 | NP_057318.2 | Enzyme, misc. | Y152 | GLAVSDNGPCLGyR | SEQ ID NO: 126 |
| 119 | ADCY9 | NP_001107.2 | Enzyme, misc. | Y172 | yAWTSLALTLL | SEQ ID NO: 127 |
| 120 | ADSL | NP_000017.1 | Enzyme, misc. | Y294 | QQIGSSAMPyK | SEQ ID NO: 129 |
| 121 | AGPAT 1 | NP_006402.1 | Enzyme, misc. | Y275 | GGGDyLKKPGGGG | SEQ ID NO: 130 |
| 122 | AKR1B1 | NP_001619.1 | Enzyme, misc. | Y104 | TLSDLKLDyLDLYLIHWPTGFKPGK | SEQ ID NO: 131 |
| 123 | AKR1C 1 | NP_001344.2 | Enzyme, misc. | Y110 | NLQLDyVDLYLIHFPVSVKPGEEVIPK | SEQ ID NO: 132 |
| 124 | AKR1C 2 | NP_001345.1 | Enzyme, misc. | Y110 | NLQLDyVDLYLIHFPVSVKPGEEVIPK | SEQ ID NO: 133 |
| 125 | AKR1C 2 | NP_001345.1 | Enzyme, misc. | Y24 | LNDGHFMPVLGFGTyAPAEVPK | SEQ ID NO: 134 |
| 126 | AKR1C 3 | NP_003730.4 | Enzyme, misc. | Y110 | AQLDyVDLYLIHSPMSLKPGEELSPTDEN GK | SEQ ID NO: 135 |
| 127 | AKR1C 3 | NP_003730.4 | Enzyme, misc. | Y305 | NLHyFNSDSFASHPNYPYSDEY | SEQ ID NO: 136 |
| 128 | AKR1C 3 | NP_003730.4 | Enzyme, misc. | Y319 | NLHYFNSDSFASHPNYPySDEY | SEQ ID NO: 137 |
| 129 | AKR7A4 | NP_003680.2 | Enzyme, misc. | Y223 | FyAYNPLAGGLLTGK | SEQ ID NO: 138 |
| 130 | AKR7A4 | NP_003680.2 | Enzyme, misc. | Y225 | FYAyNPLAGGLLTGK | SEQ ID NO: 139 |
| 131 | ALDH1 B1 | NP_000683.3 | Enzyme, misc. | Y373 | VLGylQLGQK | SEQ ID NO: 140 |
| 132 | ALOX15 | NP_001131.3 | Enzyme, misc. | Y438 | QAGAFLTySSFCPPDDLADRGLLGVK | SEQ ID NO: 141 |
| 133 | Apg3p | NP_071933.2 | Enzyme, misc. | Y111 | DDGDGGWVDTyHNTGITGITE | SEQ ID NO: 142 |
| 134 | ARD1A | NP_003482.1 | Enzyme, misc. | Y138 | YyADGEDAYAMK | SEQ ID NO: 143 |
| 135 | ARD1A | NP_003482.1 | Enzyme, misc. | Y26 | NARPEDLMNMQHCNLLCLPENyQMK | SEQ ID NO: 144 |
| 136 | autotaxi n | NP_006200.3 | Enzyme, misc. | Y898 | KTSRSyPEILTLK | SEQ ID NO: 145 |
| 137 | CPT1B | NP_004368.1 | Enzyme, misc. | Y449 | ALLHGNCyNR | SEQ ID NO: 146 |
| 138 | DDX9 | NP_001348.2 | Enzyme, misc. | Y132 | AENNSEVGASGyGVPGPTWDR | SEQ ID NO: 147 |
| 139 | FDFT1 | NP_004453.3 | Enzyme, misc. | Y14 | CLGHPEEFyNLVR | SEQ ID NO: 148 |
| 140 | GOT2 | NP_002071.2 | Enzyme, misc. | Y75 | DDNGKPyVLPSVR | SEQ ID NO: 149 |
| 141 | NANS | NP_061819.2 | Enzyme, misc. | Y71 | ALERPyTSK | SEQ ID NO: 150 |
| 142 | NEDD4 L | NP_056092.2 | Enzyme, misc. | Y465 | DTLSNPQSPQPSPyNSPKPQHK | SEQ ID NO: 151 |
| 143 | p40phox | NP_000622.2 | Enzyme, misc. | Y245 | CYYyEDTISTIKDIAVEEDLSSTPLLK | SEQ ID NO: 152 |
| 144 | PDHA1 | NP_000275.1 | Enzyme, misc. | Y242 | AAASTDyYKR | SEQ ID NO: 153 |
| 145 | PDHA1 | NP_000275.1 | Enzyme, misc. | Y243 | AAASTDYyKR | SEQ ID NO: 154 |
| 146 | PGM2 | NP_060760.2 | Enzyme, misc. | Y469 | AlyVEYGYHITK | SEQ ID NO: 155 |
| 147 | PGM2 | NP_060760.2 | Enzyme, misc. | Y472 | AIYVEyGYHITK | SEQ ID NO: 156 |
| 148 | PPIL4 | NP_624311.1 | Enzyme, misc. | Y412 | DYMPIKNTNQDIyRE | SEQ ID NO: 157 |
| 149 | PYGM | NP_005600.1 | Enzyme, misc. | Y204 | ARPEFTLPVHFyGHVEHTSQGAK | SEQ ID NO: 158 |
| 150 | ANKRD 27 | NP_115515.2 | G protein or regulator | Y928 | WNSKLyDLPDEPFTR | SEQ ID NO: 159 |
| 151 | ARF GAP 3 | NP_055385.2 | G protein or regulator | Y441 | AQKKFGNVKAISSDMYFGRQSQADyE | SEQ ID NO: 160 |
| 152 | ARF1 | NP_001649.1 | G protein or regulator | Y167 | HRNWYIQATCATSGDGLyEGLDWLSNQ LR | SEQ ID NO: 161 |
| 153 | ARF3 | NP_001650.1 | G protein or regulator | Y167 | HRNWYIQATCATSGDGLyEGLDWLANQ LK | SEQ ID NO: 162 |
| 154 | ARHGA P12 | NP_060757.4 | G protein or regulator | Y375 | HVDDQGRQyYYSADGSR | SEQ ID NO: 164 |
| 155 | ARHGA P12 | NP_060757.4 | G protein or regulator | Y376 | HVDDQGRQYyYSADGSR | SEQ ID NO: 165 |
| 156 | ARHGA P12 | NP_060757.4 | G protein or regulator | Y377 | HVDDQGRQYYySADGSR | SEQ ID NO: 166 |
| 157 | ARHGA P12 | NP_060757.4 | G protein or regulator | Y63 | AFyVPAQYVK | SEQ ID NO: 167 |
| 158 | ARHGA P12 | NP_060757.4 | G protein or regulator | Y68 | AFYVPAQyVKEVTRK | SEQ ID NO: 168 |
| 159 | ARHGA P21 | NP_065875.2 | G protein or regulator | Y350 | SGNySGHSDGISSSR | SEQ ID NO: 169 |
| 160 | ARHGA P21 | NP_065875.2 | G protein or regulator | Y394 | TYKEyIDNRR | SEQ ID NO: 170 |
| 161 | ARHGE F12 | NP_056128.1 | G protein or regulator | Y1326 | TGTGDIATCySPR | SEQ ID NO: 171 |
| 162 | ARHGE F6 | NP_004831.1 | G protein or regulator | Y666 | VIEAyCTSANFQQGHGSSTR | SEQ ID NO: 172 |
| 163 | ARHGE F6 | NP_004831.1 | G protein or regulator | Y91 | IFDPDDLySGVNFSKVLSTLLAVNKATE | SEQ ID NO: 173 |
| 164 | DOCK9 | NP_056111.1 | G protein or regulator | Y1237 | DLLGAISGIASPyTTSTPNINSVR | SEQ ID NO: 174 |
| 165 | FARP2 | NP_055623.1 | G protein or regulator | Y436 | DSSSSLTDPQVSyVK | SEQ ID NO: 175 |
| 166 | FLJ429 14 | NP_060821.2 | G protein or regulator | Y691 | AySTENYSLESQK | SEQ ID NO: 176 |
| 167 | Graf | NP_055886.1 | G protein or regulator | Y371 | AMDGREPVyNSNKDSQSE | SEQ ID NO: 177 |
| 168 | RABL3 | NP_776186.2 | G protein or regulator | Y130 | ALNRDLVPTGVLVTNGDyDQE | SEQ ID NO: 179 |
| 169 | RhoB | NP_004031.1 | G protein or regulator | Y66 | DTAGQEDyDRL | SEQ ID NO: 180 |
| 170 | RhoC | NP_786886.1 | G protein or regulator | Y66 | DTAGQEDyDRL | SEQ ID NO: 181 |
| 171 | RIN2 | NP_061866.1 | G protein or regulator | Y77 | DSGyDSLSNR | SEQ ID NO: 182 |
| 172 | synGAP | NP_006763.1 | G protein or regulator | Y327 | AGyVGLVTVPVATLAGR | SEQ ID NO: 183 |
| 173 | A2M | NP_000005.2 | Inhibitor protein | Y708 | VGFyESDVMGR | SEQ ID NO: 184 |
| 174 | DSCR1 | NP_981963.1 | Inhibitor protein | Y39 | AKFESLFRTyDKDITFQYFKSFK | SEQ ID NO: 185 |
| 175 | GCHFR | NP_005249.1 | Inhibitor protein | Y45 | ALGNNFyEYYVDDPPR | SEQ ID NO: 186 |
| 176 | GCHFR | NP 005249.1 | Inhibitor protein | Y47 | ALGNNFYEyYVDDPPR | SEQ ID NO: 187 |
| 177 | Nogo | NP_065393.1 | Inhibitor protein | Y646 | APLNSAVPSAGASVIQPSSSPLEASSVNy E | SEQ ID NO: 188 |
| 178 | TNFAIP 3 | NP_006281.1 | Inhibitor protein | Y614 | AGCVyFGTPENK | SEQ ID NO: 189 |
| 179 | TNFAIP 3 | NP_006281.1 | Inhibitor protein | Y778 | CNGyCNECFQFK | SEQ ID NO: 190 |
| 180 | B-CK | NP_001814.2 | Kinase (non-protein) | Y125 | TDLNPDNLQGGDDLDPNyVLSSR | SEQ ID NO: 191 |
| 181 | B-CK | NP_001814.2 | Kinase (non-protein) | Y39 | VLTPELyAELR | SEQ ID NO: 192 |
| 182 | M-CK | NP_001815.2 | Kinase (non-protein) | Y279 | AGHPFMWNQHLGyVLTCPSNLGTGLR | SEQ ID NO: 193 |
| 183 | PI4KII | NP_060895.1 | Kinase (non-protein) | Y18 | AQPPDyTFPSGSGAHFPQVPGGAVR | SEQ ID NO: 194 |
| 184 | PYGB | NP_002853.2 | Kinase (non-protein) | Y473 | DFyELEPEKFQNK | SEQ ID NO: 195 |
| 185 | SAPAP 2 | NP_004736.2 | Kinase (non-protein) | Y967 | ADSIEIyIPEAQTR | SEQ ID NO: 196 |
| 186 | SAPAP 3 | XP_035601.5 | Kinase (non-protein) | Y971 | ADSIEIyIPEAQTR | SEQ ID NO: 197 |
| 187 | UNC13 B | NP_006368.3 | Lipid binding protein | Y243 | DSCNDSMQSyDLDYPERR | SEQ ID NO: 198 |
| 188 | UNC13 B | NP_006368.3 | Lipid binding protein | Y247 | DSCNDSMQSYDLDyPERR | SEQ ID NO: 199 |
| 189 | ACO2 | NP_001089.1 | Mitochondrial protein | Y513 | FNPETDyLTGTDGKK | SEQ ID NO: 200 |
| 190 | AK3 | NP_057366.2 | Mitochondrial protein | Y186 | AYEDQTKPVLEyYQK | SEQ ID NO: 201 |
| 191 | KIF3A | NP_008985.3 | Motor or contractile protein | Y624 | CVAyTGNNMR | SEQ ID NO: 202 |
| 192 | MYH1 | NP_005954.3 | Motor or contractile protein | Y1291 | ARLQTESGEySR | SEQ ID NO: 203 |
| 193 | MYH1 | NP_005954.3 | Motor or contractile protein | Y389 | AAyLQNLNSADLLK | SEQ ID NO: 204 |
| 194 | MYH10 | NP_005955.1 | Motor or contractile protein | Y761 | ALELDPNLyR | SEQ ID NO: 205 |
| 195 | MYH11 | NP_074035.1 | Motor or contractile protein | Y761 | ALELDPNLyR | SEQ ID NO: 206 |
| 196 | MYH14 | NP_079005.3 | Motor or contractile protein | Y778 | ALELDPNLyR | SEQ ID NO: 207 |
| 197 | MYH2 | NP_060004.2 | Motor or contractile protein | Y389 | AAyLQSLNSADLLK | SEQ ID NO: 208 |
| 198 | MYH8 | NP_002463.1 | Motor or contractile protein | Y389 | AAyLQSLNSADLLK | SEQ ID NO: 209 |
| 199 | ACP1 | NP_004291.1 | Phosphatase | Y50 | NWRVDSAATSGyE | SEQ ID NO: 210 |
| 200 | CD45 | NP_002829.2 | Phosphatase | Y763 | CAEyWPSMEEGTR | SEQ ID NO: 211 |
| 201 | PHPT1 | NP_054891.2 | Phosphatase | Y125 | AKYPDYEVTWANDGy | SEQ ID NO: 212 |
| 202 | PPP2C A | NP_002706.1 | Phosphatase | Y248 | AHQLVMEGyNWCHDR | SEQ ID NO: 213 |
| 203 | PPP2C B | NP_004147.1 | Phosphatase | Y248 | AHQLVMEGyNWCHDR | SEQ ID NO: 214 |
| 204 | PTPN9 | NP_002824.1 | Phosphatase | Y565 | AFSIQTPEQYyFCYK | SEQ ID NO: 215 |
| 205 | SHP-1 | NP_002822.2 | Phosphatase | Y306 | DSNIPGSDYINANyIK | SEQ ID NO: 216 |
| 206 | ADAM8 | NP_001100.2 | Protease | Y766 | RPPPAPPVTVSSPPFPVPVyTR | SEQ ID NO: 217 |
| 207 | ADAM9 | NP_003807.1 | Protease | Y778 | FAVPTyAAK | SEQ ID NO: 218 |
| 208 | PSMA2 | NP_002778.1 | Protease | Y167 | ATAMGKNyVNGK | SEQ ID NO: 219 |
| 209 | PSMB5 | NP_002788.1 | Protease | Y236 | DAYSGGAVNLyHVR | SEQ !D NO: 220 |
| 210 | AMPKB 2 | NP_005390.1 | Protein kinase, regulatory subunit | Y242 | MLNHLyALSIK | SEQ ID NO: 221 |
| 211 | Akt1 | NP_005154.2 | Protein kinase, Ser/Thr (non-receptor) | Y437 | TDTRyFDEE | SEQ ID NO: 222 |
| 212 | Akt3 | NP_005456.1 | Protein kinase, Ser/Thr (non-receptor) | Y434 | TDTRyFDEE | SEQ ID NO: 223 |
| 213 | AMPK1 | NP_006242.5 | Protein kinase, Ser/Thr (non-receptor) | Y294 | YLFPEDPSySSTMIDDEALK | SEQ ID NO: 224 |
| 214 | A-Raf | NP_001645.1 | Protein kinase, Ser/Thr(non-receptor) | Y155 | QQFyHSVQDLSGGSR | SEQ ID NO: 225 |
| 215 | A-Raf | NP_001645.1 | Protein kinase, Ser/Thr (non-receptor) | Y42 | DGMSVyDSLDK | SEQ ID NO: 226 |
| 216 | BRSK2 | NP_003948.2 | Protein kinase, Ser/Thr (non-receptor) | Y334 | MlyFLLLDRK | SEQ ID NO: 227 |
| 217 | CaMK1-alpha | NP_003647.1 | Protein kinase, Ser/Thr (non-receptor) | Y235 | AEyEFDSPYWDDISDSAK | SEQ ID NO: 228 |
| 218 | CaMK1-delta | NP_065130.1 | Protein kinase, Ser/Thr (non-receptor) | Y238 | AEyEFDSPYWDDISDSAK | SEQ ID NO: 229 |
| 219 | CaMK2-alpha | NP_057065.2 | Protein kinase, Ser/Thr(non-receptor) | Y230 | AGAyDFPSPEWDTVTPEAK | SEQ ID NO: 230 |
| 220 | CaMK2-beta | NP_001211.3 | Protein kinase, Ser/Thr(non-receptor) | Y231 | AGAyDFPSPEWDTVTPEAK | SEQ ID NO: 231 |
| 221 | CaMK2-delta | NP_001212.2 | Protein kinase, Ser/Thr (non-receptor) | Y231 | AGAyDFPSPEWDTVTPEAK | SEQ ID NO: 232 |
| 222 | CaMK2-gamma | NP_001213.2 | Protein kinase, Ser/Thr (non-receptor) | Y231 | AGAyDFPSPEWDTVTPEAK | SEQ ID NO: 233 |
| 223 | CaMK4 | NP_001735.1 | Protein kinase, Ser/Thr (non-receptor) | Y172 | DLKPENLLyATPAPDAPLK | SEQ ID NO: 234 |
| 224 | DCAMK L1 | NP_004725.1 | Protein kinase, Ser/Thr(non-receptor) | Y493 | DASGMLyNLASAIK | SEQ ID NO: 235 |
| 225 | p90RSK | NP_001006666 .1 | Protein kinase, Ser/Thr (non-receptor) | Y229 | AySFCGTVEYMAPEVVNR | SEQ ID NO: 236 |
| 226 | p90RSK | NP_001006666 .1 | Protein kinase, Ser/Thr (non-receptor) | Y237 | AYSFCGTVEyMAPEWNR | SEQ ID NO: 237 |
| 227 | PAK1 | NP_002567.3 | Protein kinase, Ser/Thr (non-receptor) | Y474 | ALyLIATNGTPELQNPEK | SEQ ID NO: 238 |
| 228 | PAK2 | NP_002568.2 | Protein kinase, Ser/Thr (non-receptor) | Y453 | ALyLIATNGTPELQNPEK | SEQ ID NO: 239 |
| 229 | PKCG | NP_002730.1 | Protein kinase, Ser/Thr (non-receptor) | Y275 | APVDGWyK | SEQ ID NO: 240 |
| 230 | RSK2 | NP_004577.1 | Protein kinase, Ser/Thr(non-receptor) | Y226 | AySFCGTVEYMAPEVVNR | SEQ ID NO: 241 |
| 231 | RSK2 | NP_004577.1 | Protein kinase, Ser/Thr (non-receptor) | Y234 | AYSFCGTVEyMAPEVVNR | SEQ ID NO: 242 |
| 232 | RSK2 | NP_004577.1 | Protein kinase, Ser/Thr(non-receptor) | Y547 | DLKPSNILyVDESGNPESIR | SEQ ID NO: 243 |
| 233 | RSK3 | NP_001006933 .1 | Protein kinase, Ser/Thr(non-receptor) | Y225 | AySFCGTIEYMAPEVVNR | SEQ ID NO: 244 |
| 234 | RSK3 | NP_001006933 .1 | Protein kinase, Ser/Thr (non-receptor) | Y581 | AGNGLLMTPCyTANFVAPEVLK | SEQ ID NO: 245 |
| 235 | RSK4 | NP_055311.1 | Protein kinase, Ser/Thr (non-receptor) | Y231 | AySFCGTVEYMAPEVVNR | SEQ ID NO: 246 |
| 236 | RSK4 | NP_055311.1 | Protein kinase, Ser/Thr (non-receptor) | Y239 | AYSFCGTVEyMAPEVVNR | SEQ ID NO: 247 |
| 237 | ALK1 | NP_000011.2 | Protein kinase, Ser/Thr (receptor) | Y421 | TIVNGIVEDyR | SEQ ID NO: 248 |
| 238 | ALK4 | NP_004293.1 | Protein kinase, Ser/Thr (receptor) | Y184 | TLQDLVyDLSTSGSGSGLPLFVQR | SEQ ID NO: 249 |
| 239 | Blk | NP_001706.2 | Protein kinase, Tyr(non-receptor) | Y494 | DFyTATERQYE | SEQ ID NO: 250 |
| 240 | FRK | NP_002022.1 | Protein kinase, Tyr(non-receptor) | Y104 | DGSSQQLQGYIPSNyVAEDR | SEQ ID NO: 251 |
| 241 | FRK | NP_002022.1 | Protein kinase, Tyr(non-receptor) | Y99 | DGSSQQLQGyIPSNYVAEDR | SEQ ID NO: 252 |
| 242 | TEC | NP_003206.1 | Protein kinase, Tyr(non-receptor) | Y228 | DKYGNEGyIPSNYVTGK | SEQ ID NO: 253 |
| 243 | Axl | NP_001690.2 | Protein kinase, Tyr (receptor) | Y752 | GQTPYPGVENSEIYDyLR | SEQ ID NO: 254 |
| 244 | FGFR2 | NP_000132.1 | Protein kinase, Tyr (receptor) | Y608 | DLVSCTyQLAR | SEQ ID NO: 255 |
| 245 | FGFR2 | NP_000132.1 | Protein kinase, Tyr(receptor) | Y656 | DINNIDyYK | SEQ ID NO: 256 |
| 246 | FGFR2 | NP_000132.1 | Protein kinase, Tyr (receptor) | Y657 | DINNIDYyK | SEQ ID NO: 257 |
| 247 | LTK | NP_002335.2 | Protein kinase, Tyr(receptor) | Y676 | DIYRASyYR | SEQ ID NO: 258 |
| 248 | LTK | NP_002335.2 | Protein kinase, Tyr (receptor) | Y677 | DIYRASYyR | SEQ ID NO: 259 |
| 249 | VEGFR-3 | NP_002011.2 | Protein kinase, Tyr(receptor) | Y1063 | DIyKDPDYVR | SEQ ID NO: 260 |
| 250 | VEGFR-3 | NP_002011.2 | Protein kinase, Tyr (receptor) | Y1068 | DIYKDPDyVR | SEQ ID NO: 261 |
| 251 | ABCA12 | NP_056472.2 | Receptor, channel, transporter or cell surface protein | Y493 | ILyAPYNPVTKAIMEKSNVTLRQLAELR | SEQ ID NO: 262 |
| 252 | ABCA12 | NP_056472.2 | Receptor, channel, transporter or cell surface protein | Y496 | ILYAPyNPVTKAIMEKSNVTLRQLAELR | SEQ ID NO: 263 |
| 253 | ABCA5 | NP_061142.2 | Receptor, channel, transporter or cell surface protein | Y1299 | EyDDKKDFLLSRK | SEQ ID NO: 266 |
| 254 | ABCC1 | NP_004987.1 | Receptor, channel, transporter or cell surface protein | Y1189 | AyYPSIVANR | SEQ ID NO: 267 |
| 255 | ABCC4 | NP_005836.1 | Receptor, channel, transporter or cell surface protein | Y45 | LNPLFKIGHKRRLEEDDMy | SEQ ID NO: 268 |
| 256 | ABCC5 | NP_005679.2 | Receptor, channel, transporter or cell surface protein | Y1202 | yRENLPLVLKK | SEQ ID NO: 269 |
| 257 | ACBD3 | NP_073572.2 | Receptor, channel, transporter or cell surface protein | Y293 | QLQEQHYQQYMQQLyQVQLAQQQAAL QK | SEQ ID NO: 270 |
| 258 | albumin | NP_000468.1 | Receptor, channel, transporter or cell surface protein | Y174 | RHPYFyAPELLFFAK | SEQ ID NO: 271 |
| 259 | albumin | NP_000468.1 | Receptor, channel, transporter or cell surface protein | Y394 | CCAAADPHECyAK | SEO ID NO: 273 |
| 260 | APOB4 8R | NP_061160.2 | Receptor, channel, transporter or cell surface protein | Y898 | CGDyHPEGEAPR | SEQ ID NO: 274 |
| 261 | AQP4 | NP_001641.1 | Receptor, channel, transporter or cell surface protein | Y277 | GSyMEVEDNR | SEQ ID NO: 275 |
| 262 | AQP5 | NP_001642.1 | Receptor, channel, transporter or cell surface protein | Y243 | GTyEPDEDWEEQREER | SEQ ID NO: 276 |
| 263 | ATP6V0 D1 | NP_004632.2 | Receptor, channel, transporter or cell surface protein | Y270 | NVADyYPEYK | SEQ ID NO: 277 |
| 264 | ATP6V1 E1 | NP_001687.1 | Receptor, channel, transporter or cell surface protein | Y155 | CRKQDFPLVKAAVQKAIPMyK | SEQ ID NO: 278 |
| 265 | ATRN | NP_647537.1 | Receptor, channel, transporter or cell surface protein | Y537 | yDVDTQMWTILK | SEQ ID NO: 279 |
| 266 | BA13 | NP_001695.1 | Receptor, channel, transporter or cell surface protein | Y1237 | GTNPEGLSySTLPGNVISK | SEQ ID NO: 280 |
| 267 | CACNG 2 | NP_006069.1 | Receptor, channel, transporter or cell surface protein | Y217 | ATDyLQASAITR | SEQ ID NO: 281 |
| 268 | CACNG 2 | | Receptor, channel, transporter or cell surface protein | Y288 | AATTPTATyNSDRDNSFLQVHNCIQK | SEQ ID NO: 282 |
| 269 | CD86 | NP_008820.2 | Receptor, channel, transporter or cell surface protein | Y102 | DKGLyQCIIHHKK | SEQ ID NO: 283 |
| 270 | CLIC6 | NP_444507.1 | Receptor, channel, transporter or cell surface protein | Y461 | AGyDGESIGNCPFSQR | SEQ ID NO: 284 |
| 271 | DNAJC 1 | NP_071760.2 | Receptor, channel, transporter or cell surface protein | Y249 | ALPHLIQDAGQFyAK | SEQ ID NO: 285 |
| 272 | Icln | NP_001284.1 | Receptor, channel, transporter or cell surface protein | Y147 | CQALHPDPEDEDSDDyDGEEYDVEAHE | SEQ ID NO: 286 |
| 273 | Icln | NP_001284.1 | Receptor, channel, transporter or cell surface protein | Y152 | CQALHPDPEDEDSDDYDGEEyDVEAHE | SEQ ID NO: 287 |
| 274 | latrophilin 2 | NP_036434.1 | Receptor, channel, transporter or cell surface protein | Y1316 | DSLyTSMPNLR | SEQ ID NO: 288 |
| 275 | LIFR | NP_002301.1 | Receptor, channel, transporter or cell surface protein | Y379 | ATSYTLVESFSGKyVRLK | SEQ ID NO: 290 |
| 276 | MARVE LD2 | NP_001033692 .1 | Receptor, channel, transporter or cell surface protein | Y469 | AVFQDQFSEyKELSAEVQAVLR | SEQ ID NO: 291 |
| 277 | MB | NP_005359.1 | Receptor, channel, transporter or cell surface protein | Y147 | DMASNyKELGFQG | SEQ ID NO: 292 |
| 278 | NUP210 | NP_079199.2 | Receptor, channel, transporter or cell surface protein | Y1855 | ASPGHSPHyFAASSPTSPNALPPAR | SEQ ID NO: 293 |
| 279 | NUP35 | NP_612142.2 | Receptor, channel, transporter or cell surface protein | Y300 | ASTSDyQVISDR | SEQ ID NO: 294 |
| 280 | PLXND 1 | NP_055918.1 | Receptor, channel, transporter or cell surface protein | Y1367 | CSSLyEER | SEQ ID NO: 295 |
| 281 | Ral | NP_005393.2 | Receptor, channel, transporter or cell surface protein | Y153 | AEQWNVNyVETSAK | SEQ ID NO: 296 |
| 282 | TMEM1 6A | NP_060513.4 | Receptor, channel, transporter or cell surface protein | Y955 | ACPDSLGSPAPSHAyHGGVL | SEQ ID NO: 297 |
| 283 | TNFRS F8 | NP_001234.2 | Receptor, channel, transporter or cell surface protein | Y560 | ADHTPHyPEQE | SEQ ID NO: 298 |
| 284 | BICC1 | XP_498431.3 | RNA processing | Y723 | SSyVNMQAFDYEQK | SEQ ID NO: 299 |
| 285 | CstF-77 | NP_001317.1 | RNA processing | Y546 | ALGyKDVSR | SEQ ID NO: 300 |
| 286 | DKFZp7 62N191 0 | NP_001073027 .1 | RNA processing | Y726 | DWQSYyYHHPQDRDR | SEQ ID NO: 301 |
| 287 | DKFZp7 62N191 0 | NP_001073027 .1 | RNA processing | Y727 | DWQSYYyHHPQDRDR | SEQ ID NO: 302 |
| 288 | ELAVL1 | NP_001410.2 | RNA processing | Y63 | DKVAGHSLGyGFVNYVTAK | SEQ ID NO: 303 |
| 289 | EXOSC 1 | NP_057130.1 | RNA processing | Y119 | ATEKDKVElyK | SEQ ID NO: 304 |
| 290 | hnRNP G | NP_002130.2 | RNA processing | Y234 | DyAPPPRDYTYR | SEQ ID NO: 305 |
| 291 | hnRNP G | NP_002130.2 | RNA processing | Y243 | DYTyRDYGHSSSR | SEQ ID NO: 306 |
| 292 | hnRNP G | NP_002130.2 | RNA processing | Y288 | DSYESyGNSR | SEQ ID NO: 307 |
| 293 | hnRNP H' | NP_062543.1 | RNA processing | Y306 | ATENDIyNFFSPLNPMR | SEQ ID NO: 308 |
| 294 | hnRNP-C1/C2 | NP_004491.2 | RNA processing | Y119 | DyYDRMYSYPAR | SEQ ID NO: 309 |
| 295 | hnRNP-K | NP_002131.2 | RNA processing | Y135 | CLNyQHYKGSDFDCE | SEQ ID NO: 310 |
| 296 | HUMAG CGB | NP_037418.3 | RNA processing | Y360 | AFEKyGIIEEVVIK | SEQ ID NO: 311 |
| 297 | HUMAG CGB | NP_037418.3 | RNA processing | Y510 | AEETRYPQQYQPSPLPVHyELLTDGYTR | SEQ ID NO: 312 |
| 298 | PUM1 | NP_001018494 .1 | RNA processing | Y1115 | AVLIDEVCTMNDGPHSALyTMMK | SEQ ID NO: 313 |
| 299 | RAE1 | NP_003601.1 | RNA processing | Y180 | CYCADVIyPMAVVATAER | SEQ ID NO: 314 |
| 300 | RBM13 | NP_115898.2 | RNA processing | Y287 | AYVEIEyEQETEPVAK | SEQ ID NO: 315 |
| 301 | RBM14 | NP_006319.1 | RNA processing | Y237 | ASYVAPLTAQPATyR | SEQ ID NO: 316 |
| 302 | RBM14 | NP_006319.1 | RNA processing | Y285 | AQPSVSLGAPyR | SEQ ID NO: 317 |
| 303 | RNUT1 | NP_005692.1 | RNA processing | Y334 | ASENGHyELEHLSTPK | SEQ ID NO: 318 |
| 304 | RNUXA | NP_115553.2 | RNA processing | Y178 | DLDKELDEyMHGGK | SEQ ID NO: 319 |
| 305 | SF3B1 | NP_036565.2 | RNA processing | Y38 | AQGVGLDSTGyYDQE | SEQ ID NO: 320 |
| 306 | SF361 | NP_036565.2 | RNA processing | Y39 | AQGVGLDSTGYyDQE | SEQ ID NO: 321 |
| 307 | snRNP 116 | NP_004238.2 | RNA processing | Y65 | DKKyYPTAEE | SEQ ID NO: 322 |
| 308 | TFIP11 | NP_036275.1 | RNA processing | Y722 | AVSSNVGAyMQPGAR | SEQ ID NO: 323 |
| 309 | UPF3B | NP_075386.1 | RNA processing | Y117 | DRFDGyVFLDNK | SEQ ID NO: 324 |
| 310 | ZFR | NP_057191.2 | RNA processing | Y194 | AGySQGATQYTQAQQTR | SEQ ID NO: 325 |
| 311 | ZFR | NP_057191.2 | RNA processing | Y201 | AGYSQGATQyTQAQQTR | SEQ ID NO: 326 |
| 312 | ADCYAP1 | NP_001108.1 | Secreted protein | Y153 | yLAAVLGKRYKQR | SEQ ID NO: 327 |
| 313 | ADCYAP1 | NP_001108.1 | Secreted protein | Y162 | YLAAVLGKRyKQR | SEQ ID NO: 328 |
| 314 | LTF | NP_002334.2 | Secreted protein | Y211 | CAFSSQEPYFSySGAFK | SEQ ID NO: 329 |
| 315 | 53BP1 | NP_005648.1 | Transcriptional regulator | Y1500 | WSSNGyFYSGK | SEQ ID NO: 330 |
| 316 | ANKRD 1 | NP_055206.2 | Transcriptional regulator | Y274 | MIRLLIMyGADLNIK | SEQ ID NO: 331 |
| 317 | ASH2L | NP_004665.1 | Transcriptional regulator | Y517 | FKSyLYFEEKDFVDK | SEQ ID NO: 332 |
| 318 | ASH2L | NP_004665.1 | Transcriptional regulator | Y519 | FKSYLyFEEKDFVDK | SEQ ID NO: 333 |
| 319 | elongin A | NP_003189.1 | Transcriptional regulator | Y112 | DALQKEEEMEGDyQETWK | SEQ ID NO: 334 |
| 320 | FLI1 | NP_002008.2 | Transcriptional regulator | Y42 | ADMTASGSPDyGQPHK | SEQ ID NO: 335 |
| 321 | GATA-1 | NP_002040.1 | Transcriptional regulator | Y231 | DRTGHYLCNACGLyHK | SEQ ID NO: 336 |
| 322 | HBS1 | NP_006611.1 | Transcriptional regulator | Y309 | ASFAyAWVLDETGEER | SEQ ID NO: 337 |
| 323 | NFkB-p100 | NP_002493.3 | Transcriptional regulator | Y867 | DKLPSTEVKEDSAyGSQSVEQEAEK | SEQ ID NO: 338 |
| 324 | PSMC3 | NP_002795.2 | Transcriptional regulator | Y185 | AMEVDERPTEQySDIGGLDK | SEQ ID NO: 340 |
| 325 | REL | NP_002899.1 | Transcriptional regulator | Y88 | DCRDGyYEAEFGQERR | SEQ ID NO: 341 |
| 326 | REL | NP_002899.1 | Transcriptional regulator | Y89 | DCRDGYyEAEFGQERR | SEQ ID NO: 342 |
| 327 | RLF | NP_036553.1 | Transcriptional regulator | Y671 | DLyPCPGTDCSR | SEQ ID NO: 343 |
| 328 | SMARC E1 | NP_003070.3 | Transcriptional regulator | Y139 | AYHNSPAyLAYINAK | SEQ ID NO: 344 |
| 329 | TAL-1 | NP_003180.1 | Transcriptional regulator | Y138 | ALLySLSQPLASLGSGFFGEPDAFPMFT TNNR | SEQ ID NO: 345 |
| 330 | TBP | NP_003185.1 | Transcriptional regulator | Y329 | AEIYEAFENIyPILK | SEQ ID NO: 346 |
| 331 | YAP1 | NP_006097.1 | Transcriptional regulator | Y357 | DESTDSGLSMSSySVPR | SEQ ID NO: 347 |
| 332 | 82-FIP | NP_065823.1 | Translational regulator | Y218 | GADNDGSGSESGyTTPK | SEQ ID NO: 348 |
| 333 | elF3-alpha | NP_003749.2 | Translational regulator | Y243 | ATMKDDLADyGGYDGGYVQDYEDFM | SEQ ID NO: 349 |
| 334 | elF3-alpha | NP_003749.2 | Translational regulator | Y246 | ATMKDDLADYGGyDGGYVQDYEDFM | SEQ ID NO: 350 |
| 335 | eIF3-alpha | NP_003749.2 | Translational regulator | Y250 | ATMKDDLADYGGYDGGyVQDYEDFM | SEQ ID NO: 351 |
| 336 | eIF3-alpha | NP_003749.2 | Translational regulator | Y254 | ATMKDDLADYGGYDGGYVQDyEDFM | SEQ ID NO: 352 |
| 337 | elF3S6l P | NP_057175.1 | Translational regulator | Y14 | AAyDPYAYPSDYDMHTGDPKQDLAYE | SEQ ID NO: 353 |
| 338 | eIF4B | NP_001408.2 | Translational regulator | Y33 | DGGTGGGSTyVSKPVSWADE | SEQ ID NO: 354 |
| 339 | HRSP1 2 | NP_005827.1 | Translational regulator | Y21 | APGAIGPySQAVLVDR | SEQ ID NO: 355 |
| 340 | APC | NP_000029.2 | Tumor suppressor | Y2366 | MSyTSPGR | SEQ ID NO: 356 |
| 341 | BAP1 | NP_004647.1 | Tumor suppressor | Y394 | VPVRPPQQySDDEDDYEDDEEDDVQNT NSALR | SEQ ID NO: 357 |
| 342 | BAP1 | NP_004647.1 | Tumor suppressor | Y401 | VPVRPPQQYSDDEDDyEDDEEDDVQNT NSALR | SEQ ID NO: 358 |
| 343 | APC7 | NP_057322.1 | Ubiquitin conjugating system | Y400 | EAMVMANNVyK | SEQ ID NO: 359 |
| 344 | apollon | NP_057336.2 | Ubiquitin conjugating system | Y4102 | QSGELVyEAPE | SEQ ID NO: 360 |
| 345 | DTX3L | NP_612144.1 | Ubiquitin conjugating system | Y592 | AMSyKPICPTCQTSYGIQK | SEQ ID NO: 361 |
| 346 | MARCH3 | NP_848545.1 | Ubiquitin conjugating system | Y40 | TVEDCGSLVNGQPQyVMQVSAK | SEQ ID NO: 362 |
| 347 | MARCH7 | NP_073737.1 | Ubiquitin conjugating system | Y37 | GSSLNDTyHSR | SEQ ID NO: 363 |
| 348 | MTBP | NP_071328.2 | Ubiquitin conjugating system | Y656 | ASVCHyHGIEYCLDDRK | SEQ ID NO: 364 |
| 349 | MTBP | NP_071328.2 | Ubiquitin conjugating system | Y661 | ASVCHYHGIEyCLDDRK | SEQ ID NO: 365 |
| 350 | PJA1 | NP_001027568 .1 | Ubiquitin conjugating system | Y482 | AISYVDPQFLTyMALEE | SEQ ID NO: 366 |
| 351 | PJA2 | NP_055634.2 | Ubiquitin conjugating system | Y212 | EAEAyTGLSPPVPSFNCEVR | SEQ ID NO: 367 |
| 352 | PJA2 | NP_055634.2 | Ubiquitin conjugating system | Y576 | AISYVDPQFLTyMALEE | SEQ ID NO: 368 |
| 353 | PJA2 | NP_055634.2 | Ubiquitin conjugating system | Y63 | AGDDyEVLELDDVPK | SEQ ID NO: 369 |
| 354 | UBE1 | NP_003325.2 | Ubiquitin conjugating system | Y590 | CVyYRKPLLESGTLGTK | SEQ ID NO: 370 |
| 355 | UBQLN1 | NP_038466.2 | Ubiquitin conjugating system | Y269 | ALSNLESIPGGyNALR | SEQ ID NO: 371 |
| 356 | AARSD1 | NP_079543.1 | Unknown function | Y517 | RMEAQALLQDyISTQSAKE | SEQ ID NO: 372 |
| 357 | AIDA-1b | NP_690001.3 | Unknown function | Y901 | MRPIGHDGYHPTSVAEWLDSIELGDyTK | SEQ ID NO: 373 |
| 358 | ANKRD13 | NP_149112.1 | Unknown function | Y485 | SYYVQDNGRNVHLQDEDyE | SEQ ID NO: 374 |
| 359 | ANKRD25 | NP_056308.2 | Unknown function | Y100 | HSAySYCGR | SEQ ID NO: 375 |
| 360 | ANKS1 | NP_056060.1 | Unknown function | Y427 | yFPLTASEVLSMR | SEQ ID NO: 376 |
| 361 | ATAD2 | NP_054828.2 | Unknown function | Y1043 | IDLHKyLTVK | SEQ ID NO: 377 |
| 362 | BAT2D1 | NP_055987.2 | Unknown function | Y847 | AALDQEQITAAySVE | SEQ ID NO: 378 |
| 363 | BC0606 32 | NP_612392.1 | Unknown function | Y527 | NSNIAQNyR | SEQ ID NO: 379 |
| 364 | BEGAIN | NP_065887.1 | Unknown function | Y120 | VTIDKLSEDNELyR | SEQ ID NO: 380 |
| 365 | BEGAIN | NP_065887.1 | Unknown function | Y137 | DCNLAAQLLQCSQTyGR | SEQ ID NO: 381 |
| 366 | C10orf81 | NP_079165.3 | Unknown function | Y113 | TTNREyFLIGHDR | SEQ ID NO: 382 |
| 367 | C11orf61 | NP_078907.1 | Unknown function | Y296 | LREyFNSEKPEGRIIMTR | SEQ ID NO: 383 |
| 368 | C7orf20 | NP_057033.2 | Unknown function | Y39 | ASVEKGDyYEAHQMYR | SEQ ID NO: 384 |
| 369 | CEP152 | NP_055800.2 | Unknown function | Y19 | DEEyDEEDYEREKE | SEQ ID NO: 385 |
| 370 | CEP152 | NP_055800.2 | Unknown function | Y24 | DEEYDEEDyEREKE | SEQ ID NO: 386 |
| 371 | CNKSR2 | NP_055742.2 | Unknown function | Y821 | CHLQDHyGPYPLAESER | SEQ ID NO: 387 |
| 372 | CPSF7 | NP_079087.2 | Unknown function | Y451 | DLLHNEDRHDDyFQER | SEQ ID NO: 388 |
| 373 | CYFIP1 | NP_055423.1 | Unknown function | Y887 | DKQPNAQPQyLHGSK | SEQ ID NO: 389 |
| 374 | DNAJB5 | NP_036398.3 | Unknown function | Y263 | DGTNVLySALISLK | SEQ ID NO: 390 |
| 375 | EHBP1L1 | XP_170658.1 | Unknown function | Y899 | AETRVGSALKyE | SEQ ID NO: 391 |
| 376 | FHL1 | NP_001440.2 | Unknown function | Y117 | AIVAGDQNVEyK | SEQ ID NO: 392 |
| 377 | FLJ907 09 | NP_775785.1 | Unknown function | Y98 | ALIAPDHVVPAPEECYVySPLGSAYK | SEQ ID NO: 393 |
| 378 | HEMGN | NP_060907.2 | Unknown function | Y449 | DAyTFPQEMK | SEQ ID NO: 394 |
| 379 | IQSEC1 | NP_055684.3 | Unknown function | Y911 | ACLDDSyASGEGLKR | SEQ ID NO: 395 |
| 380 | IQSEC2 | NP_055890.1 | Unknown function | Y118 | ALSDSyELSTDLQDK | SEQ ID NO: 396 |
| 381 | IQSEC2 | NP_055890.1 | Unknown function | Y728 | DLLVGlyQR | SEO ID NO: 397 |
| 382 | KIAA02 84 | NP_055820.1 | Unknown function | Y767 | DGVyVSANGR | SEQ ID NO: 398 |
| 383 | KIAA03 10 | XP_088459.10 | Unknown function | Y1117 | ARyVDVLNPSGTQR | SEQ ID NO: 399 |
| 384 | KIAA05 53 | NP_001002909 .1 | Unknown function | Y573 | AEPSISYSCNPLyFDFK | SEQ ID NO: 400 |
| 385 | KIAA14 62 | XP_934405.1 | Unknown function | Y397 | AGASGQPPSGPPGTGNEyGVSPR | SEQ ID NO: 401 |
| 386 | LOC144 100 | NP_778228.2 | Unknown function | Y665 | DLEyLDLK | SEQ ID NO: 402 |
| 387 | PWP1 | NP_008993.1 | Unknown function | Y138 | DTEQyEREDFLIKPSDNLIVCGR | SEQ ID NO: 403 |
| 388 | SHROO M1 | NP_597713.1 | Unknown function | Y33 | ADSAySSFSAASGGPEPR | SEQ ID NO: 404 |
| 389 | SPAG7 | NP_004881.2 | Unknown function | Y189 | DAAHMLQANKTyGCVPVANKR | SEQ ID NO: 405 |
| 390 | TTC12 | NP_060338.3 | Unknown function | Y177 | CTKAyFHMGKANLALK | SEQ ID NO: 406 |
| 391 | WDR70 | NP_060504.1 | Unknown function | Y624 | AAEDSPYWVSPAySK | SEQ ID NO: 407 |
| 392 | BET1 | NP_005859.1 | Vesicle protein | Y25 | RAGLGEGVPPGNYGNYGYANSGySACE EENER | SEQ ID NO: 408 |
| 393 | BICD1 | NP_001705.2 | Vesicle protein | Y406 | LDGEKGRDSGEEAHDyE | SEQ ID NO: 409 |
| 394 | BICD1 | NP_001705.2 | Vesicle protein | Y713 | NKyENEKAMVTETMTK | SEQ ID NO: 410 |
| 395 | CLTB | NP_009028.1 | Vesicle protein | Y87 | ANGPADGyAAIAQADRLTQEPE | SEQ ID NO: 411 |
| 396 | COG6 | NP_065802.1 | Vesicle protein | Y638 | AYGEVYAAVMNPINEyK | SEQ ID NO: 412 |
| 397 | EXOC4 | NP_068579.3 | Vesicle protein | Y623 | DTCTAAyRGIVQSEEK | SEQ ID NO: 413 |
| 398 | NUCB2 | NP_005004.1 | Vesicle protein | Y169 | AATSDLEHyDKTR | SEQ ID NO: 414 |
| 399 | SEC22L 1 | NP_004883.2 | Vesicle protein | Y33 | DLQQyQSQAK | SEQ ID NO: 415 |
| 400 | SNAP29 | NP_004773.1 | Vesicle protein | Y68 | ATAASTSRSLALMyE | SEQ ID NO: 416 |
| 401 | SNAP-alpha | NP_003818.2 | Vesicle protein | Y151 | AIAHYEQSADyYKGEESNSSANK | SEQ ID NO: 417 |
| 402 | SNX1 | NP_003090.2 | Vesicle protein | Y131 | ATNSSKPQPTyEELEEEEQE | SEQ ID NO: 418 |
| 403 | syntaphi lin | NP_055538.2 | Vesicle protein | Y29 | DAyGTSSLSSSSNSGSYK | SEQ ID NO: 419 |
| 404 | syntaphi lin | NP_055538.2 | Vesicle protein | Y43 | DAYGTSSLSSSSNSGSyK | SEQ ID NO: 420 |
| 405 | TXLNA | NP_787048.1 | Vesicle protein | Y86 | DILSTyCVDNNQGGPGEDGAQGEPAEP E | SEQ ID NO: 421 |
| 406 | WDR48 | NP_065890.1 | Vesicle protein | Y386 | DTNNNVAyWDVLK | SEQ ID NO: 422 |

One of skill in the art will appreciate that, in many instances the utility of the instant invention is best understood in conjunction with an appreciation of the many biological roles and significance of the various target signaling proteins/polypeptides of the invention. The foregoing is illustrated in the following paragraphs summarizing the knowledge in the art relevant to a few non-limiting representative peptides containing selected phosphorylation sites according to the invention.

CDH1 (Cadherin 1, E-cadherin, uvomorulin), phosphorylated at Y755, is among the proteins listed in this patent. It is a calcium-dependent glycoprotein that mediates cell-cell adhesion and migration and is necessary for epithelial morphogenesis (Nature Cell Biology 2002; 4:E101-E108.). Formation of adherens junctions between cells depends on the CDH1 cytoplasmic domain in which Y755 is located. When situated in an adherens junction, the cytoplasmic domain forms complexes with proteins such as catenins, Cdc42, PAR, atypical PKC, serves as a scaffold for forming adjacent tight junctions, activates PI(3) kinase, promotes actin polymerization and stabilizes microtubules (Nature Cell Biology 2002; 4:E101-E108.). Increased tyrosine phosphorylation of cadherins has been shown to suppress adhesion, suggesting a role for pY755 in regulating the stability of the adherens junction (J Cell Biol. 1995 Aug;130(4):977-86.1. 33-55% of sporadic diffuse-type gastric cancers carry somatic mutations of CDHI, and germline mutations in the gene cause the disease (Cancer Cell 2004 Feb; 5(2):121-125.). In addition, altered expression of CDH1 may be therapeutic for breast and colon cancer (Human PSD™, Biobase Corporation, Beverly, MA). Molecular probes for pY755 and to the CDH1 protein may be useful for diagnostic and/or therapeutic purposes for lung neoplasms (Anticancer Res 2003 Jul-Aug;23(4):3367-71.) and gastric cancer (Cancer Cell 2004 Feb; 5(2):121-125. PhosphoSite®, Cell Signaling Technology, Danvers, MA. Human PSD™, Biobase Corporation, Beverly, MA).

AIP1, phosphorylated at Y362, is among the proteins listed in this patent. Atrophin-1 interacting protein 1 is a tight junction scaffold molecule that couples neurotransmitter receptors and cell adhesion proteins (Biochem Biophys Res Commun. 2003 Feb 21;301(4):1122-8). AIP1 enhances the ability of PTEN to suppress AKT1 activation (Proc Natl Acad Sci U S A. 2000 Apr 11;97(8):4233-8). Molecular probes, such as antibodies, to AIP1 and its post-translational modification, may have diagnostic and/or therapeutic utility based on association with gastrointestinal disorders and its ability to inhibit cell migration and proliferation in hepatocarcinoma (Arch Biochem Biophys. 2007 Nov 1;467(1):1-9. Gut. 2008 Apr;57(4):463-7. PhosphoSite(R), Cell Signaling Technology, Danvers, MA. Human PSD(TM), Biobase Corporation, Beverly, MA).

Two members of the discs large homologue family (DLG3, phosphorylated at Y600 and Y601, and DLG5, phosphorylated at Y429), as well as two of the proteins that are known to interact with them (SAPAP2, phosphorylated at Y967, and SAPAP3, phosphorylated at Y971), are among the proteins listed in this patent. Discs large proteins participate in multi-protein complexes at areas of intercellular contact, such as synapses, where they contribute to cell proliferation, neuron adhesion and synaptic transmission (Genes Dev. 2004 Aug 15;18(16):1909-25). SAPAP proteins bind to DLG proteins at excitatory synapses (J Comp Neurol. 2004 Apr 19;472(1):24-39). Searches for safer and more effective anesthetics have shown that the inhaled anesthetic halothane disrupts interactions between DLG proteins and nNOS and NMDA receptors and that more targeted anesthetics may be developed by designing drugs to disrupt the interactions of specific DLG proteins. DLG5 is associated with Crohn's disease in some populations, and molecular probes to Y429 and to the DLG5 protein may have diagnostic and/or therapeutic utility for this disease (World J Gastroenterol. 2006 Jun 21;12(23):3651-6). Consistent with its role in neuronal function, mutation of DLG3 is associated with X-linked mental retardation, and its chromosomal position correlates with Parkinson disease (Human PSD^{™}, Biobase Corporation, Beverly, MA); antibodies to Y600 and/or Y601 or other parts of the protein may serve as useful tools in dissecting the molecular mechanisms of these diseases (PhosphoSite®, Cell Signaling Technology, Danvers, MA. Human PSD^{™}, Biobase Corporation, Beverly, MA).

PDHA1, phosphorylated at Y242 and Y243, is among the proteins listed in this patent. Pyruvate dehydrogenase (lipoamide) alpha 1, somatic form, oxidatively decarboxylates pyruvate to acetyl-CoA and is the primary link between glycolysis and the tricarboxylic acid cycle (Am J Physiol Endocrinol Metab. 2003 May;284(5):E855-62). Mutation of the corresponding gene causes the majority of pyruvate dehydrogenase deficiencies. One of the known mutations changes Y243 to alanine and causes significantly reduced in the activity of the enzyme accompanied by early onset severe encephalopathy and lactic acidosis in the patient (Brain. 1994 Jun; 117 ( Pt 3):435-43). This phenotype is consistent with Y243 residing nearby the cofactor binding site of the enzyme and suggests that Y242 may have an equally important role in the activity of the protein. Molecular probes to PDHA1 and its modified amino acids would provide insight into the regulation of this critical metabolic enzyme and may well prove useful in the diagnosis and treatment of pyruvate dehydrogenase deficiencies and other metabolic diseases (Am J Hum Genet 1990 Aug;47(2):286-93. PhosphoSite(R), Cell Signaling Technology, Danvers, MA. Human PSD(TM), Biobase Corporation, Beverly, MA).

PYGM, phosphorylated at Y203, and PYGB, phosphorylated at Y472, are among the proteins listed in this patent. The glycogen phosphorylase family catalyzes the rate-limiting step in glycogen catabolism. Mutations in the gene for PYGM cause McArdle disease, the most common disorder of muscle carbohydrate metabolism, which causes exercise intolerance, and the glycogen phosphorylase family has been identified as a potential target for antidiabetic drugs (Protein Sci. 2005 Jul; 14(7):1760-71). Molecular probes to PYGM Y203, PYGB Y472, and to the proteins themselves, would be valuable tools to assess the regulation of glycogen phosphorylases in normal and pathological catabolism of glycogen. In addition, the proteins have potential diagnostic and/or therapeutic utility based on association with hyperparathyroidism and gastric carcinomas (Am J Hum Genet 1994 Jun;54(6):1060-6. PhosphoSite(R), Cell Signaling MA).

B-CK, phosphorylated at Y39 and Y125, and M-CK, phosphorylated at Y279, are among the proteins listed in this patent. These are brain and muscle forms of creatine kinase, which reversibly catalyzes the transfer of phosphate between ATP and various phosphogens (e.g. creatine phosphate). Creatine kinase isoenzymes play a central role in energy transduction in tissues with large, fluctuating energy demands, such as skeletal muscle, heart, brain and spermatozoa. The enzyme may exist either as a homo- or heterodimer of the two forms. Assay of creatine kinase activity and distribution of isoforms are widely used diagnostic tests for stroke, myocardial infarction, and muscle damage. Molecular probes to B-CK Y39, B-CK Y125, and M-CK Y279 would provide valuable tools for more specific diagnoses in common clinical tests. These proteins may also have diagnostic and/or therapeutic implications for neoplasms and myotonic dystrophy (Hum Genet 1991 Mar;86(5):457-62. Anticancer Res 1996 Jan-Feb; 16(1):375-80. Cancer Res. 2006 Jan 15;66(2):763-9. PhosphoSite(R), Cell Signaling Technology, Danvers, MA. Human PSD(TM), Biobase Corporation, Beverly, MA).

The disintegrin and metalloprotease domain containing proteins are a family of membrane-associated proteases. ADAM9, phosphorylated at Y778, and ADAM8, phosphorylated at the paralogous site Y766, are among the proteins listed in this patent. Although their known sites of action are at the extracellular surface of the plasma membrane, the bulk of the ADAM proteins are located inside the cell, perhaps acting as a reservoir for rapid deployment (Genes Dev. 2003 Jan 1;17(1):7-30). The cytoplasmic tails in which ADAM9 Y778 and ADAM8 Y766 reside are thought to be the domains through which signals are transduced across the membrane. Each ADAM protein has a distinct cytoplasmic tail that allows it to bind a unique set of signalling proteins. Interestingly, the phosphorylation sites described here are located adjacent to SH3-binding sites that are likely to be among the interaction sites for the signalling proteins. Of particular interest is the fact that protein kinase C delta binding to the cytoplasmic tail of ADAM9 is essential for induced shedding of HB-EGF, a member of the epidermal growth factor family, in some cell types (EMBO J. 1998 Dec 15;17(24):7260-72). It is not known whether PKCD acts by affecting ADAM9's subcellular localization or by activating it in another way. ADAM9 may also act as an alpha-secretase, potentially ameliorating some effects of Alzheimer's disease (Genes Dev. 2003 Jan 1;17(1):7-30). ADAM8 may play a role in soluble CD23-mediated inflammation and cell migration (J Biol Chem. 2003 Aug 15;278(33):30469-77) although this may not be its primary function (Nat Immunol. 2006 Dec;7(12):1293-8). Because of their ability to control autocrine ligand production and their expression in many types of cancer, the ADAM family proteins have been identified as likely targets for chemotherapy (Cancer Cell. 2006 Jul; 10(1):7-11). Molecular probes to ADAM9 Y778, ADAM8 Y766, and the proteins themselves, would provide valuable reagents in deducing the mechanisms of action of these proteases and their role in the development of cancer (Biochem Biophys Res Commun 1997 Jun 18;235(2):437-42. PhosphoSite(R), Cell Signaling Technology, Danvers, MA. Human PSD(TM), Biobase Corporation, Beverly, MA).

Akt1, phosphorylated at Y437, and Akt3, phosphorylated at Y434, are among the proteins listed in this patent. Normal development relies on selective cell death to eliminate superfluous, defective or transiently useful cells. Signals such as growth factors suppress apoptosis in cells essential to the organism, often via the protein kinase Akt (Genes Dev. 1999 Nov 15;13(22):2905-27). Akt is a member of the AGC serine/threonine kinase family that includes PKA, PKC, PDK1, and the p70 and p90 S6 kinases (Mol Cell. 2002 Jun;9(6): 1227-40). Three separate genomic loci encode Akt kinases in mammals: Akt1, Akt2, and Akt3. The enzyme products of these genes have similar substrate specificities but are regulated differently in various tissues. Akt kinases also participate in the physiological regulation of glucose by insulin (Mol Endocrinol. 1997 Dec; 1 1(13):1881-90). In addition to its normal roles in development and metabolism, Akt has been shown to contribute to oncogenic processes by suppressing cell death in cancer cells (Cancer Cell. 2007 Aug;12(2):104-7. Cancer Cell. 2007 Nov;12(5):411-3). In particular, point mutations in Akt have been found in ≥2% of breast, colorectal, and ovarian tumors studied in a recent publication (Nature. 2007 Jul 26;448(7152):439-44). The Akt1 and 2 genes also are amplified in a small percentage of ovary, pancreas, and breast tumors (Cancer Cell. 2007 Aug; 12(2):104-7). Consistent with its role in cell survival, Akt activity is correlated with drug resistance in some cancers (Cancer Res. 2001 May 15;61(10):3986-97). At least one Akt inhibitor, perifosine, is in clinical trials as a cancer chemotherapeutic (Clin Cancer Res. 2006 Feb 1;12(3 Pt 1):679-89). Molecular probes to Akt1 Y437 and Akt3 Y434, as well as to the Akt proteins, would be valuable tools in assessing Akt regulation in normal and pathological states. If phosphorylation of these sites proves to affect Akt activity, they may well serve as chemotherapeutic targets (PhosphoSite(R), Cell Signaling Technology, Danvers, MA. Human PSD(TM), Biobase Corporation, Beverly, MA).

AMPK1, phosphorylated at Y285, and AMPKB2, phosphorylated at Y242, are among the proteins listed in this patent. AMPK is an AMP-activated protein kinase of the CAMKL family comprised of three subunits: alpha (AMPK1 or AMPK2; catalytic), beta (AMPKB1 or AMPKB2; regulatory), and gamma (AMPKG1, AMPKG2, or AMPKG3; regulatory). Consistent with its sensitivity to cellular energy status, the enzyme is involved in glycolysis, response to starvation, response to hypoxia, and regulation of the cystic fibrosis transmembrane conductance regulator (CFTR). Rat AMPK1 is associated with obesity related insulin resistance. AMPK is activated by at least two anti-diabetic drugs, and potential regulatory sites such as AMPK1 Y285 and AMPKB2 Y242 should be assessed for their utility as either diagnostic markers or therapeutic targets. AMPK is also associated with pancreatic neoplasms, and molecular probes, such as antibodies, to the protein and its modifications sites may be have diagnostic and/or therapeutic implications for this disease (Oncogene 2002 Sep 5;21 (39):6082-90. PhosphoSite(R), Cell Signaling Technology, Danvers, MA. Human PSD(TM), Biobase Corporation, Beverly, MA).

Four ribosomal protein S6 kinase family members are among the proteins listed in this patent: p90RSK, phosphorylated at Y229 and Y237, RSK2, phosphorylated at Y226, Y234, and Y547, RSK3, phosphorylated at Y225 and Y581, and RSK4, phosphorylated at Y231 and Y239. The ribosomal S6 kinases participate in the transduction of signals from extracellular stimuli, such as hormones and neurotransmitters (Mol Cell Endocrinol 1999 May 25; 151(1-2):65-77.) and possess two kinase domains connected by a regulator linker region. p90RSK Y229, RSK2, Y226, RSK3 Y225, and RSK4, Y231 are paralogous sites that flank the first substrate binding pocket. p90RSK Y237, RSK2, Y234, and RSK4 Y239 are paralogous sites within the first substrate binding pocket. RSK2 Y547 resides within the second ATP binding pocket, and RSK3 Y581 resides within the second substrate binding site. The critical positions of these modifications suggest that they may control kinase activity. Mutations in the RSK2 gene cause Coffin-Lowry mental retardation syndrome, and the protein is prominently expressed in brain structures that are essential for cognitive function and learning (Am J Hum Genet 1998 Dec;63(6):1631-40. PhosphoSite®, Cell Signaling Technology, Danvers, MA. Human PSD™, Biobase Corporation, Beverly, MA). Molecular probes to these proteins and their modified amino acids would provide insight into the activation of cells and their effects on development of the nervous system.

FGFR2, phosphorylated at Y608, Y656, and Y657, is among the proteins listed in this patent. Fibroblast growth factor receptor 2 is a receptor tyrosine kinase of the highly-conserved FGFR family that binds fibroblast growth factor (FGF) and acts in induction of apoptosis, skeletal development, cell migration and differentiation (Human PSD™, Biobase Corporation, Beverly, MA). Consistent with its role in development, mutations in the FGFR2 gene are known to cause at least three craniosynostotic conditions--Crouzon syndrome(Nat Genet. 1994 Sep;8(1):98-103. Nat Genet. 1994 Nov;8(3):275-9), Apert syndrome (Science. 2003 Aug 1;301(5633):643-6. Nat Genet. 1996 May;13(1):48-53. Nat Genet. 1995 Feb;9(2):165-72), Pfeiffer syndrome (Eur J Hum Genet. 2006 Mar;14(3):289-98) as well as autosomal dominant lacrimoauriculodentodigital (LADD) syndrome (Nat Genet. 2006 Apr;38(4):414-7). The gene is also associated with gastric cancer (Cancer Res. 2001 May 1;61(9):3541-3) and breast cancer (Nature. 2007 Jun 28;447(7148):1087-93. Nat Genet. 2007 Jul; 39(7):870-4)- Y60-8, Y656, and Y657 are all within the kinase catalytic domain, and their phosphorylation may affect catalytic activity or recognition of substrate. Molecular probes to these and other sites on the protein would provide valuable tools to study the function of this protein in normal and pathological states (PhosphoSite®, Cell Signaling Technology, Danvers, MA).

VEGFR-3, phosphorylated at Y1063 and Y1068, is among the proteins listed in this patent. It is also known as Fms-related tyrosine kinase 4 and receptor for vascular endothelial growth factors C (VEGFC) and D (FIGF). VEGFR-3 is a tyrosine-protein kinase and induces proliferation and migration of lymphatic endothelial cells. It is a relative of FGFR2, also listed in this patent, and Y1068 is paralogous to but distinct from FGFR2 Y657 (SEQ ID NO: 257). Y1063 and Y1068 are both within the tyrosine kinase catalytic domain and are likely to have effects on its function. Mutations in the VEGFR-3 gene are known to cause lymphedema (Am J Hum Genet 2000 Aug;67(2):295-301.), and parasitic infections cause lymphatic filariasis by activating VEGFR-3 (PLoS Pathog. 2006 Sep;2(9):e92). Molecular probes, such as antibodies, to Y1063, Y1068, and this protein may provide insights into diagnostic and/or therapeutic approaches to these diseases (PhosphoSite(R), Cell Signaling Technology, Danvers, MA. Human PSD(TM), Biobase Corporation, Beverly, MA).

CD86, phosphorylated at Y108, is among the proteins listed in this patent. It is a ligand for CD28 and CTLA-4, promotes IL-2 production and phosphatidylinositol-3-kinase activation, and acts in T-cell costimulation. Aberrent expression of CD86 correlates with multiple sclerosis, asthma, Hodgkin disease, and inflammatory bowel disorders. Molecular probes, such as antibodies, to Y108 and to the CD86 proteins have potential diagnostic and/or therapeutic utility based on association with multiple myeloma and nasopharyngeal carcinoma (Blood 1999 Mar 1;93(5):1487-95. BMC Cancer. 2007 May 24;7:88. PhosphoSite(R), Cell Signaling Technology, Danvers, MA. Human PSD(TM), Biobase Corporation, Beverly, MA).

The invention identifies peptide sequences comprising phosphorylation sites useful to profile phosphotyrosine signaling in the analysis of oncogenesis and specifically in lung cancer (e.g., in non-small lung cancer, NSLC). For most solid tumors the tyrosine kinases that drive disease remain unknown, limiting the ability to identify drug targets and pedict response. Tyrosine kinase signaling across 41 NSCLC cells lines and 150 NSCLC tumors have implicated a number of known oncogenic kinases such as EGFR and c-Met as well as novel ALK and ROS fusion proteins, along with others such as PDGFRaand DDR1. The compendium of phosphorylated sites provided herein constitutes a fundamental tool to profile a given sample across many possible target phosphorylation determinants offering a unique tool to characterize a given tumor to identify drug targets and predict response. The invention also provides peptides comprising a novel phosphorylation site of the invention. In one particular embodiment, the peptides comprise any one of the an amino acid sequences as set forth in column E of Table 1 and Figure 2, which are trypsin-digested peptide fragments of the parent proteins. Alternatively, a parent signaling protein listed in Table 1 may be digested with another protease, and the sequence of a peptide fragment comprising a phosphorylation site can be obtained in a similar way. Suitable proteases include, but are not limited to, serine proteases (*e*.*g*. hepsin), metallo proteases (*e*.*g*. PUMP1), chymotrypsin, cathepsin, pepsin, thermolysin, carboxypeptidases, etc.

The invention also provides proteins and peptides that are mutated to eliminate a novel phosphorylation site of the invention. Such proteins and peptides are particular useful as research tools to understand complex signaling transduction pathways of cancer cells, for example, to identify new upstream kinase(s) or phosphatase(s) or other proteins that regulates the activity of a signaling protein; to identify downstream effector molecules that interact with a signaling protein, etc.

Various methods that are well known in the art can be used to eliminate a phosphorylation site. For example, the phosphorylatable tyrosine may be mutated into a non-phosphorylatable residue, such as phenylalanine. A "phosphorylatable" amino acid refers to an amino acid that is capable of being modified by addition of a phosphate group (any includes both phosphorylated form and unphosphorylated form). Alternatively, the tyrosine may be deleted. Residues other than the tyrosine may also be modified (e.g., delete or mutated) if such modification inhibits the phosphorylation of the tyrosine residue. For example, residues flanking the tyrosine may be deleted or mutated, so that a kinase can not recognize/phosphorylate the mutated protein or the peptide. Standard mutagenesis and molecular cloning techniques can be used to create amino acid substitutions or deletions.

### 2. Modulators of the Phosphorylation Sites

In another aspect, the invention provides a modulator that modulates tyrosine phosphorylation at a novel phosphorylation site of the invention, including small molecules, peptides comprising a novel phosphorylation site, and binding molecules that specifically bind at a novel phosphorylation site, including but not limited to antibodies or antigen-binding fragments thereof.

Modulators of a phosphorylation site include any molecules that directly or indirectly counteract, reduce, antagonize or inhibit tyrosine phosphorylation of the site. The modulators may compete or block the binding of the phosphorylation site to its upstream kinase(s) or phosphatase(s), or to its downstream signaling transduction molecule(s).

The modulators may directly interact with a phosphorylation site. The modulator may also be a molecule that does not directly interact with a phosphorylation site. For example, the modulators can be dominant negative mutants, i.e., proteins and peptides that are mutated to eliminate the phosphorylation site. Such mutated proteins or peptides could retain the binding ability to a downstream signaling molecule but lose the ability to trigger downstream signaling transduction of the wild type parent signaling protein.

The modulators include small molecules that modulate the tyrosine phosphorylation at a novel phosphorylation site of the invention. Chemical agents, referred to in the art as "small molecule" compounds are typically organic, non-peptide molecules, having a molecular weight less than 10,000, less than 5,000, less than 1,000, or less than 500 daltons. This class of modulators includes chemically synthesized molecules, for instance, compounds from combinatorial chemical libraries. Synthetic compounds may be rationally designed or identified based on known or inferred properties of a phosphorylation site of the invention or may be identified by screening compound libraries. Alternative appropriate modulators of this class are natural products, particularly secondary metabolites from organisms such as plants or fungi, which can also be identified by screening compound libraries. Methods for generating and obtaining compounds are well known in the art (Schreiber SL, Science 151: 1964-1969(2000); Radmann J. and Gunther J., Science 151: 1947-1948 (2000)).

The modulators also include peptidomimetics, small protein-like chains designed to mimic peptides. Peptidomimetics may be analogues of a peptide comprising a phosphorylation site of the invention. Peptidomimetics may also be analogues of a modified peptide that are mutated to eliminate a phosphorylation site of the invention. Peptidomimetics (both peptide and non-peptidyl analogues) may have improved properties (e.g., decreased proteolysis, increased retention or increased bioavailability). Peptidomimetics generally have improved oral availability, which makes them especially suited to treatment of disorders in a human or animal.

In certain embodiments, the modulators are peptides comprising a novel phosphorylation site of the invention. In certain embodiments, the modulators are antibodies or antigen-binding fragments thereof that specifically bind at a novel phosphorylation site of the invention.

### 3. Heavy-Isotope Labeled Peptides (AQUA Peptides).

In another aspect, the invention provides peptides comprising a novel phosphorylation site of the invention. In a particular embodiment, the invention provides Heavy-Isotype Labeled Peptides (AQUA peptides) comprising a novel phosphorylation site. Such peptides are useful to generate phosphorylation site-specific antibodies for a novel phosphorylation site. Such peptides are also useful as potential diagnostic tools for screening carcinoma and/or leukemia, or as potential therapeutic agents for treating carcinoma and/or leukemia.

The peptides may be of any length, typically six to fifteen amino acids. The novel tyrosine phosphorylation site can occur at any position in the peptide; if the peptide will be used as an immnogen, it preferably is from seven to twenty amino acids in length. In some embodiments, the peptide is labeled with a detectable marker.

"Heavy-isotope labeled peptide" (used interchangeably with AQUA peptide) refers to a peptide comprising at least one heavy-isotope label, as described in WO/03016861, "Absolute Quantification of Proteins and Modified Forms Thereof by Multistage Mass Spectrometry" (Gygi *et al.)* (the teachings of which are hereby incorporated herein by reference, in their entirety). The amino acid sequence of an AQUA peptide is identical to the sequence of a proteolytic fragment of the parent protein in which the novel phosphorylation site occurs. AQUA peptides of the invention are highly useful for detecting, quantitating or modulating a phosphorylation site of the invention (both in phosphorylated and unphosphorylated forms) in a biological sample.

A peptide of the invention, including an AQUA peptides comprises any novel phosphorylation site. Preferably, the peptide or AQUA peptide comprises a novel phosphorylation site of a protein in Table 1 that is a enzyme proteins, adaptor/scaffold proteins, protein kinases, receptor/channel/transportercell surface proteins, cytoskeletal proteins, RNA processing proteins, G protein or regulator proteins, transcriptional regulator proteins, adhesion or extracellular matrix proteins and vesicle proteins.

Particularly preferred peptides and AQUA peptides are these comprising a novel tyrosine phosphorylation site (shown as a lower case "y" in a sequence listed in Table 1) selected from the group consisting of SEQ ID NOs: 123 (ACLY), 125 (ACSL1), 129 (ADSL), 140 (ALDH1B1), 143 (ARD1A), 149 (Got2), 154 (PDHA1), 155 (PDHA1), 213 (PPP2CA), 214 (PPP2CB), 220 (PSMB5), 7 (AIP1), 38 (TRAF4), 221 (AMPKB2), 227 (BRSK2), 236 (p90RSK), 238 (PAK1), 239 (PAK2), 251 (FRK), 256 (FGFR2), 267 (ABCC1), 88 (ACTN4), 93 (Arp3), 304 (EXOSC1), 322 (snRNP 116), 161 (ARF1), 163 (ARF4), 337 (HBS1), 340(PSMC3), 346 (TBP), 45 (afadin), 417 (SNAP), 73 (MCM5), 86 (SKIV2L2), 202 (AK3), 372 (UBQLN1), 385 (C7orf20) and 408 (WDR70).

In some embodiments, the peptide or AQUA peptide comprises the amino acid sequence shown in any one of the above listed SEQ ID NOs. In some embodiments, the peptide or AQUA peptide consists of the amino acid sequence in said SEQ ID NOs. In some embodiments, the peptide or AQUA peptide comprises a fragment of the amino acid sequence in said SEQ ID NOs., wherein the fragment is six to twenty amino acid long and includes the phosphorylatable tyrosine. In some embodiments, the peptide or AQUA peptide consists of a fragment of the amino acid sequence in said SEQ ID NOs., wherein the fragment is six to twenty amino acid long and includes the phosphorylatable tyrosine.

In certain embodiments, the peptide or AQUA peptide comprises any one of the SEQ ID NOs listed in column H, which are trypsin-digested peptide fragments of the parent proteins.

It is understood that parent protein listed in Table 1 may be digested with any suitable protease (e.g., serine proteases (*e.g.* trypsin, hepsin), metallo proteases (*e.g.* PUMP1), chymotrypsin, cathepsin, pepsin, thermolysin, carboxypeptidases, etc), and the resulting peptide sequence comprising a phosphorylated site of the invention may differ from that of trypsin-digested fragments (as set forth in Column E), depending the cleavage site of a particular enzyme. An AQUA peptide for a particular a parent protein sequence should be chosen based on the amino acid sequence of the parent protein and the particular protease for digestion; that is, the AQUA peptide should match the amino acid sequence of a proteolytic fragment of the parent protein in which the novel phosphorylation site occurs.

An AQUA peptide is preferably at least about 6 amino acids long. The preferred ranged is about 7 to 15 amino acids.

The AQUA method detects and quantifies a target protein in a sample by introducing a known quantity of at least one heavy-isotope labeled peptide standard (which has a unique signature detectable by LC-SRM chromatography) into a digested biological sample. By comparing to the peptide standard, one may readily determines the quantity of a peptide having the same sequence and protein modification(s) in the biological sample. Briefly, the AQUA methodology has two stages:(1) peptide internal standard selection and validation; method development; and (2) implementation using validated peptide internal standards to detect and quantify a target protein in a sample. The method is a powerful technique for detecting and quantifying a given peptide/protein within a complex biological mixture, such as a cell lysate, and may be used, *e.g.*, to quantify change in protein phosphorylation as a result of drug treatment, or to quantify a protein in different biological states.

Generally, to develop a suitable internal standard, a particular peptide (or modified peptide) within a target protein sequence is chosen based on its amino acid sequence and a particular protease for digestion. The peptide is then generated by solid-phase peptide synthesis such that one residue is replaced with that same residue containing stable isotopes (¹³C, ¹⁵N). The result is a peptide that is chemically identical to its native counterpart formed by proteolysis, but is easily distinguishable by MS via a mass shift. A newly synthesized AQUA internal standard peptide is then evaluated by LC-MS/MS. This process provides qualitative information about peptide retention by reverse-phase chromatography, ionization efficiency, and fragmentation via collision-induced dissociation. Informative and abundant fragment ions for sets of native and internal standard peptides are chosen and then specifically monitored in rapid succession as a function of chromatographic retention to form a selected reaction monitoring (LC-SRM) method based on the unique profile of the peptide standard.

The second stage of the AQUA strategy is its implementation to measure the amount of a protein or the modified form of the protein from complex mixtures. Whole cell lysates are typically fractionated by SDS-PAGE gel electrophoresis, and regions of the gel consistent with protein migration are excised. This process is followed by in-gel proteolysis in the presence of the AQUA peptides and LC-SRM analysis. (*See* Gerber *et al. supra*.) AQUA peptides are spiked in to the complex peptide mixture obtained by digestion of the whole cell lysate with a proteolytic enzyme and subjected to immunoaffinity purification as described above. The retention time and fragmentation pattern of the native peptide formed by digestion (e.g., trypsinization) is identical to that of the AQUA internal standard peptide determined previously; thus, LC-MS/MS analysis using an SRM experiment results in the highly specific and sensitive measurement of both internal standard and analyte directly from extremely complex peptide mixtures. Because an absolute amount of the AQUA peptide is added (*e.g.* 250 fmol), the ratio of the areas under the curve can be used to determine the precise expression levels of a protein or phosphorylated form of a protein in the original cell lysate. In addition, the internal standard is present during in-gel digestion as native peptides are formed, such that peptide extraction efficiency from gel pieces, absolute losses during sample handling (including vacuum centrifugation), and variability during introduction into the LC-MS system do not affect the determined ratio of native and AQUA peptide abundances.

An AQUA peptide standard may be developed for a known phosphorylation site previously identified by the IAP-LC-MS/MS method within a target protein. One AQUA peptide incorporating the phosphorylated form of the site, and a second AQUA peptide incorporating the unphosphorylated form of site may be developed. In this way, the two standards may be used to detect and quantify both the phosphorylated and unphosphorylated forms of the site in a biological sample.

Peptide internal standards may also be generated by examining the primary amino acid sequence of a protein and determining the boundaries of peptides produced by protease cleavage. Alternatively, a protein may actually be digested with a protease and a particular peptide fragment produced can then sequenced. Suitable proteases include, but are not limited to, serine proteases (*e.g*. trypsin, hepsin), metallo proteases (*e.g.* PUMP1), chymotrypsin, cathepsin, pepsin, thermolysin, carboxypeptidases, etc.

A peptide sequence within a target protein is selected according to one or more criteria to optimize the use of the peptide as an internal standard. Preferably, the size of the peptide is selected to minimize the chances that the peptide sequence will be repeated elsewhere in other non-target proteins. Thus, a peptide is preferably at least about 6 amino acids. The size of the peptide is also optimized to maximize ionization frequency. Thus, peptides longer than about 20 amino acids are not preferred. The preferred ranged is about 7 to 15 amino acids. A peptide sequence is also selected that is not likely to be chemically reactive during mass spectrometry, thus sequences comprising cysteine, tryptophan, or methionine are avoided.

A peptide sequence that is outside a phosphorylation site may be selected as internal standard to determine the quantity of all forms of the target protein. Alternatively, a peptide encompassing a phosphorylated site may be selected as internal standard to detect and quantify only the phosphorylated form of the target protein. Peptide standards for both phosphorylated form and unphosphorylated form can be used together, to determine the extent of phosphorylation in a particular sample.

The peptide is labeled using one or more labeled amino acids (*i.e*. the label is an actual part of the peptide) or less preferably, labels may be attached after synthesis according to standard methods. Preferably, the label is a mass-altering label selected based on the following considerations: The mass should be unique to shift fragment masses produced by MS analysis to regions of the spectrum with low background; the ion mass signature component is the portion of the labeling moiety that preferably exhibits a unique ion mass signature in MS analysis; the sum of the masses of the constituent atoms of the label is preferably uniquely different than the fragments of all the possible amino acids. As a result, the labeled amino acids and peptides are readily distinguished from unlabeled ones by the ion/mass pattern in the resulting mass spectrum. Preferably, the ion mass signature component imparts a mass to a protein fragment that does not match the residue mass for any of the 20 natural amino acids.

The label should be robust under the fragmentation conditions of MS and not undergo unfavorable fragmentation. Labeling chemistry should be efficient under a range of conditions, particularly denaturing conditions, and the labeled tag preferably remains soluble in the MS buffer system of choice. The label preferably does not suppress the ionization efficiency of the protein and is not chemically reactive. The label may contain a mixture of two or more isotopically distinct species to generate a unique mass spectrometric pattern at each labeled fragment position. Stable isotopes, such as ¹³C, ¹⁵N, ¹⁷O, ¹⁸O, or ³⁴S, are among preferred labels. Pairs of peptide internal standards that incorporate a different isotope label may also be prepared. Preferred amino acid residues into which a heavy isotope label may be incorporated include leucine, proline, valine, and phenylalanine.

Peptide internal standards are characterized according to their mass-to-charge (m/z) ratio, and preferably, also according to their retention time on a chromatographic column (*e.g.* an HPLC column). Internal standards that coelute with unlabeled peptides of identical sequence are selected as optimal internal standards. The internal standard is then analyzed by fragmenting the peptide by any suitable means, for example by collision-induced dissociation (CID) using, e.g., argon or helium as a collision gas. The fragments are then analyzed, for example by multi-stage mass spectrometry (MSⁿ) to obtain a fragment ion spectrum, to obtain a peptide fragmentation signature. Preferably, peptide fragments have significant differences in m/z ratios to enable peaks corresponding to each fragment to be well separated, and a signature that is unique for the target peptide is obtained. If a suitable fragment signature is not obtained at the first stage, additional stages of MS are performed until a unique signature is obtained.

Fragment ions in the MS/MS and MS³ spectra are typically highly specific for the peptide of interest, and, in conjunction with LC methods, allow a highly selective means of detecting and quantifying a target peptide/protein in a complex protein mixture, such as a cell lysate, containing many thousands or tens of thousands of proteins. Any biological sample potentially containing a target protein/peptide of interest may be assayed. Crude or partially purified cell extracts are preferably used. Generally, the sample has at least 0.01 mg of protein, typically a concentration of 0.1-10 mg/mL, and may be adjusted to a desired buffer concentration and pH.

A known amount of a labeled peptide internal standard, preferably about 10 femtomoles, corresponding to a target protein to be detected/quantified is then added to a biological sample, such as a cell lysate. The spiked sample is then digested with one or more protease(s) for a suitable time period to allow digestion. A separation is then performed (e.g., by HPLC, reverse-phase HPLC, capillary electrophoresis, ion exchange chromatography, etc.) to isolate the labeled internal standard and its corresponding target peptide from other peptides in the sample. Microcapillary LC is a preferred method.

Each isolated peptide is then examined by monitoring of a selected reaction in the MS. This involves using the prior knowledge gained by the characterization of the peptide internal standard and then requiring the MS to continuously monitor a specific ion in the MS/MS or MSⁿ spectrum for both the peptide of interest and the internal standard. After elution, the area under the curve (AUC) for both peptide standard and target peptide peaks are calculated. The ratio of the two areas provides the absolute quantification that can be normalized for the number of cells used in the analysis and the protein's molecular weight, to provide the precise number of copies of the protein per cell. Further details of the AQUA methodology are described in Gygi *et al. ,* and Gerber *et al. supra.*

Accordingly, AQUA internal peptide standards (heavy-isotope labeled peptides) may be produced, as described above, for any of the 405 novel phosphorylation sites of the invention (see Table 1/Figure 2). For example, peptide standards for a given phosphorylation site (e.g., an AQUA peptide having the sequence NHDSVyYTYE (SEQ ID NO: 8), wherein "y" corresponds to phosphorylatable tyrosine 485 of AKAP11) may be produced for both the phosphorylated and unphosphorylated forms of the sequence. Such standards may be used to detect and quantify both phosphorylated form and unphosphorylated form of the parent signaling protein (e.g., AKAP11) in a biological sample.

Heavy-isotope labeled equivalents of a phosphorylation site of the invention, both in phosphorylated and unphosphorylated form, can be readily synthesized and their unique MS and LC-SRM signature determined, so that the peptides are validated as AQUA peptides and ready for use in quantification.

The novel phosphorylation sites of the invention are particularly well suited for development of corresponding AQUA peptides, since the IAP method by which they were identified (see Part A above and Example 1) inherently confirmed that such peptides are in fact produced by enzymatic digestion (e.g., trypsinization) and are in fact suitably fractionated/ionized in MS/MS. Thus, heavy-isotope labeled equivalents of these peptides (both in phosphorylated and unphosphorylated form) can be readily synthesized and their unique MS and LC-SRM signature determined, so that the peptides are validated as AQUA peptides and ready for use in quantification experiments.

Accordingly, the invention provides heavy-isotope labeled peptides (AQUA peptides) that may be used for detecting, quantitating, or modulating any of the phosphorylation sites of the invention (Table 1). For example, an AQUA peptide having the sequence DAVySEYK (SEQ ID NO: 28), wherein y (Tyr 429) may be either phosphotyrosine or tyrosine, and wherein V = labeled valine (e.g., ¹⁴C)) is provided for the quantification of phosphorylated (or unphosphorylated) form of DLG5 (an adaptor/scaffold protein) in a biological sample.

Example 4 is provided to further illustrate the construction and use, by standard methods described above, of exemplary AQUA peptides provided by the invention. For example, AQUA peptides corresponding to both the phosphorylated and unphosphorylated forms of SEQ ID NO:28 (a trypsin-digested fragment of DLG5, with a tyrosine 429 phosphorylation site) may be used to quantify the amount of phosphorylated DLG5 in a biological sample, e.g., a tumor cell sample or a sample before or after treatment with a therapeutic agent.

Peptides and AQUA peptides provided by the invention will be highly useful in the further study of signal transduction anomalies underlying cancer, including carcinoma and/or leukemias. Peptides and AQUA peptides of the invention may also be used for identifying diagnostic/bio-markers of carcinoma and/or leukemias, identifying new potential drug targets, and/or monitoring the effects of test therapeutic agents on signaling proteins and pathways.

### 4. Phosphorylation Site-Specific Antibodies

In another aspect, the invention discloses phosphorylation site-specific binding molecules that specifically bind at a novel tyrosine phosphorylation site of the invention, and that distinguish between the phosphorylated and unphosphorylated forms. In one embodiment, the binding molecule is an antibody or an antigen-binding fragment thereof. The antibody may specifically bind to an amino acid sequence comprising a phosphorylation site identified in Table 1.

In some embodiments, the antibody or antigen-binding fragment thereof specifically binds the phosphorylated site. In other embodiments, the antibody or antigen-binding fragment thereof specially binds the unphosphorylated site. An antibody or antigen-binding fragment thereof specially binds an amino acid sequence comprising a novel tyrosine phosphorylation site in Table 1 when it does not significantly bind any other site in the parent protein and does not significantly bind a protein other than the parent protein. An antibody of the invention is sometimes referred to herein as a"phospho-specific" antibody.

An antibody or antigen-binding fragment thereof specially binds an antigen when the dissociation constant is ≤ 1mM, preferably ≤ 100nM, and more preferably ≤ 10nM.

In some embodiments, the antibody or antigen-binding fragment of the invention binds an amino acid sequence that comprises a novel phosphorylation site of a protein in Table 1 that is a enzyme proteins, adaptor/scaffold proteins, protein kinases, receptor/channel/transportercell surface proteins, cytoskeletal proteins, RNA processing proteins, G protein or regulator proteins, transcriptional regulator proteins, adhesion or extracellular matrix proteins and vesicle proteins.

In particularly preferred embodiments, an antibody or antigen-binding fragment thereof of the invention specially binds an amino acid sequence comprising a novel tyrosine phosphorylation site shown as a lower case "y" in a sequence listed in Table 1 selected from the group consisting of SEQ ID NOS: 123 (ACLY), 125 (ACSL1), 129 (ADSL), 140 (ALDH1B1), 143 (ARD1A), 149 (Got2), 154 (PDHA1), 155 (PDHA1), 213 (PPP2CA), 214 (PPP2CB), 220 (PSMB5), 7 (AIP1), 38 (TRAF4), 221 (AMPKB2), 227 (BRSK2), 236 (p90RSK), 238 (PAK1), 239 (PAK2), 251 (FRK), 256 (FGFR2), 267 (ABCC1), 88 (ACTN4), 93 (Arp3), 304 (EXOSC1), 322 (snRNP 116), 161 (ARF1), 163 (ARF4), 337 (HBS1), 340(PSMC3), 346 (TBP), 45 (afadin), 417 (SNAP), 73 (MCMS), 86 (SKIV2L2), 202 (AK3), 372 (UBQLN1), 385 (C7orf20) and 408 (WDR70).

In some embodiments, an antibody or antigen-binding fragment thereof of the invention specifically binds an amino acid sequence comprising any one of the above listed SEQ ID NOs. In some embodiments, an antibody or antigen-binding fragment thereof of the invention especially binds an amino acid sequence comprises a fragment of one of said SEQ ID NOs., wherein the fragment is four to twenty amino acid long and includes the phosphorylatable tyrosine.

In certain embodiments, an antibody or antigen-binding fragment thereof of the invention specially binds an amino acid sequence that comprises a peptide produced by proteolysis of the parent protein with a protease wherein said peptide comprises a novel tyrosine phosphorylation site of the invention. In some embodiments, the peptides are produced from trypsin digestion of the parent protein. The parent protein comprising the novel tyrosine phosphorylation site can be from any species, preferably from a mammal including but not limited to non-human primates, rabbits, mice, rats, goats, cows, sheep, and guinea pigs. In some embodiments, the parent protein is a human protein and the antibody binds an epitope comprising the novel tyrosine phosphorylation site shown by a lower case "y" in Column E of Table 1. Such peptides include any one of the SEQ ID NOs.

An antibody of the invention can be an intact, four immunoglobulin chain antibody comprising two heavy chains and two light chains. The heavy chain of the antibody can be of any isotype including IgM, IgG, IgE, IgG, IgA or IgD or sub-isotype including IgG1, IgG2, IgG3, IgG4, IgE1, IgE2, etc. The light chain can be a kappa light chain or a lambda light chain.

Also within the invention are antibody molecules with fewer than 4 chains, including single chain antibodies, Camelid antibodies and the like and components of the antibody, including a heavy chain or a light chain. The term "antibody" (or "antibodies") refers to all types of immunoglobulins. The term "an antigen-binding fragment of an antibody" refers to any portion of an antibody that retains specific binding of the intact antibody. An exemplary antigen-binding fragment of an antibody is the heavy chain and/or light chain CDR, or the heavy and/or light chain variable region. The term "does not bind," when appeared in context of an antibody's binding to one phospho-form (e.g., phosphorylated form) of a sequence, means that the antibody does not substantially react with the other phospho-form (e.g., non-phosphorylated form) of the same sequence. One of skill in the art will appreciate that the expression may be applicable in those instances when (1) a phospho-specific antibody either does not apparently bind to the non-phospho form of the antigen as ascertained in commonly used experimental detection systems (Western blotting, IHC, Immunofluorescence, etc.); (2) where there is some reactivity with the surrounding amino acid sequence, but that the phosphorylated residue is an immunodominant feature of the reaction. In cases such as these, there is an apparent difference in affinities for the two sequences. Dilutional analyses of such antibodies indicates that the antibodies apparent affinity for the phosphorylated form is at least 10-100 fold higher than for the non-phosphorylated form; or where (3) the phospho-specific antibody reacts no more than an appropriate control antibody would react under identical experimental conditions. A control antibody preparation might be, for instance, purified immunoglobulin from a pre-immune animal of the same species, an isotype- and species-matched monoclonal antibody. Tests using control antibodies to demonstrate specificity are recognized by one of skill in the art as appropriate and definitive.

In some embodiments an immunoglobulin chain may comprise in order from 5' to 3', a variable region and a constant region. The variable region may comprise three complementarity determining regions (CDRs), with interspersed framework (FR) regions for a structure FR1, CDR1, FR2, CDR2, FR3, CDR3 and FR4. Also within the invention are heavy or light chain variable regions, framework regions and CDRs. An antibody of the invention may comprise a heavy chain constant region that comprises some or all of a CH1 region, hinge, CH2 and CH3 region.

An antibody of the invention may have an binding affinity (K_{D}) of 1x 10⁻⁷ M or less. In other embodiments, the antibody binds with a K_{D} of 1 x10⁻⁸ M, 1 × 10⁻⁹ M, 1 × 10⁻¹⁰ M, 1 x 10⁻¹¹ M, 1 x 10⁻¹² M or less. In certain embodiments, the K_{D} is 1 pM to 500 pM, between 500 pM to 1 µM, between 1 µM to 100 nM, or between 100 mM to 10 nM.

Antibodies of the invention can be derived from any species of animal, preferably a mammal. Non-limiting exemplary natural antibodies include antibodies derived from human, chicken, goats, and rodents (e.g., rats, mice, hamsters and rabbits), including transgenic rodents genetically engineered to produce human antibodies (see, e.g., Lonberg et al., WO93/12227; U.S. Pat. No. 5,545,806; and Kucherlapati, et al., WO91/10741; U.S. Pat. No. 6,150,584, which are herein incorporated by reference in their entirety). Natural antibodies are the antibodies produced by a host animal. "Genetically altered antibodies" refer to antibodies wherein the amino acid sequence has been varied from that of a native antibody. Because of the relevance of recombinant DNA techniques to this application, one need not be confined to the sequences of amino acids found in natural antibodies; antibodies can be redesigned to obtain desired characteristics. The possible variations are many and range from the changing of just one or a few amino acids to the complete redesign of, for example, the variable or constant region. Changes in the constant region will, in general, be made in order to improve or alter characteristics, such as complement fixation, interaction with membranes and other effector functions. Changes in the variable region will be made in order to improve the antigen binding characteristics.

The antibodies of the invention include antibodies of any isotype including IgM, IgG, IgD, IgA and IgE, and any sub-isotype, including IgG1, IgG2a, IgG2b, IgG3 and IgG4, IgE1, IgE2 etc.. The light chains of the antibodies can either be kappa light chains or lambda light chains.

Antibodies disclosed in the invention may be polyclonal or monoclonal. As used herein, the term "epitope" refers to the smallest portion of a protein capable of selectively binding to the antigen binding site of an antibody. It is well accepted by those skilled in the art that the minimal size of a protein epitope capable of selectively binding to the antigen binding site of an antibody is about five or six to seven amino acids.

Other antibodies specifically contemplated are oligoclonal antibodies. As used herein, the phrase "oligoclonal antibodies" refers to a predetermined mixture of distinct monoclonal antibodies. *See,* e.g., PCT publication WO 95/20401; U.S. Patent Nos. 5,789,208 and 6,335,163. In one embodiment, oligoclonal antibodies consisting of a predetermined mixture of antibodies against one or more epitopes are generated in a single cell. In other embodiments, oligoclonal antibodies comprise a plurality of heavy chains capable of pairing with a common light chain to generate antibodies with multiple specificities (e.g., PCT publication WO 04/009618). Oligoclonal antibodies are particularly useful when it is desired to target multiple epitopes on a single target molecule. In view of the assays and epitopes disclosed herein, those skilled in the art can generate or select antibodies or mixtures of antibodies that are applicable for an intended purpose and desired need.

Recombinant antibodies against the phosphorylation sites identified in the invention are also included in the present application. These recombinant antibodies have the same amino acid sequence as the natural antibodies or have altered amino acid sequences of the natural antibodies in the present application. They can be made in any expression systems including both prokaryotic and eukaryotic expression systems or using phage display methods (see, e.g., Dower et al., WO91/17271 and McCafferty et al., WO92/01047; U.S. Pat. No. 5,969,108, which are herein incorporated by reference in their entirety).

Antibodies can be engineered in numerous ways. They can be made as single-chain antibodies (including small modular immunopharmaceuticals or SMIPs^{™}), Fab and F(ab')₂ fragments, etc. Antibodies can be humanized, chimerized, deimmunized, or fully human. Numerous publications set forth the many types of antibodies and the methods of engineering such antibodies. For example, see U.S. Patent Nos. 6,355,245; 6,180,370; 5,693,762; 6,407,213; 6,548,640; 5,565,332; 5,225,539; 6,103,889; and 5,260,203.

The genetically altered antibodies should be functionally equivalent to the above-mentioned natural antibodies. In certain embodiments, modified antibodies provide improved stability or/and therapeutic efficacy. Examples of modified antibodies include those with conservative substitutions of amino acid residues, and one or more deletions or additions of amino acids that do not significantly deleteriously alter the antigen binding utility. Substitutions can range from changing or modifying one or more amino acid residues to complete redesign of a region as long as the therapeutic utility is maintained. Antibodies of this application can be modified post-translationally (e.g., acetylation, and/or phosphorylation) or can be modified synthetically (e.g., the attachment of a labeling group).

Antibodies with engineered or variant constant or Fc regions can be useful in modulating effector functions, such as, for example, antigen-dependent cytotoxicity (ADCC) and complement-dependent cytotoxicity (CDC). Such antibodies with engineered or variant constant or Fc regions may be useful in instances where a parent singling protein (Table 1) is expressed in normal tissue; variant antibodies without effector function in these instances may elicit the desired therapeutic response while not damaging normal tissue. Accordingly, certain aspects and methods of the present disclosure relate to antibodies with altered effector functions that comprise one or more amino acid substitutions, insertions, and/or deletions.

In certain embodiments, genetically altered antibodies are chimeric antibodies and humanized antibodies.

The chimeric antibody is an antibody having portions derived from different antibodies. For example, a chimeric antibody may have a variable region and a constant region derived from two different antibodies. The donor antibodies may be from different species. In certain embodiments, the variable region of a chimeric antibody is non-human, e.g., murine, and the constant region is human.

The genetically altered antibodies used in the invention include CDR grafted humanized antibodies. In one embodiment, the humanized antibody comprises heavy and/or light chain CDRs of a non-human donor immunoglobulin and heavy chain and light chain frameworks and constant regions of a human acceptor immunoglobulin. The method of making humanized antibody is disclosed in U.S. Pat. Nos: 5,530,101; 5,585,089; 5,693,761; 5,693,762; and 6,180,370 each of which is incorporated herein by reference in its entirety.

Antigen-binding fragments of the antibodies of the invention, which retain the binding specificity of the intact antibody, are also included in the invention. Examples of these antigen-binding fragments include, but are not limited to, partial or full heavy chains or light chains, variable regions, or CDR regions of any phosphorylation site-specific antibodies described herein.

In one embodiment of the application, the antibody fragments are truncated chains (truncated at the carboxyl end). In certain embodiments, these truncated chains possess one or more immunoglobulin activities (e.g., complement fixation activity). Examples of truncated chains include, but are not limited to, Fab fragments (consisting of the VL, VH, CL and CH1 domains); Fd fragments (consisting of the VH and CH1 domains); Fv fragments (consisting of VL and VH domains of a single chain of an antibody); dAb fragments (consisting of a VH domain); isolated CDR regions; (Fab')₂ fragments, bivalent fragments (comprising two Fab fragments linked by a disulphide bridge at the hinge region). The truncated chains can be produced by conventional biochemical techniques, such as enzyme cleavage, or recombinant DNA techniques, each of which is known in the art. These polypeptide fragments may be produced by proteolytic cleavage of intact antibodies by methods well known in the art, or by inserting stop codons at the desired locations in the vectors using site-directed mutagenesis, such as after CH1 to produce Fab fragments or after the hinge region to produce (Fab')₂ fragments. Single chain antibodies may be produced by joining VL- and VH-coding regions with a DNA that encodes a peptide linker connecting the VL and VH protein fragments

Papain digestion of antibodies produces two identical antigen-binding fragments, called "Fab" fragments, each with a single antigen-binding site, and a residual "Fc" fragment, whose name reflects its ability to crystallize readily. Pepsin treatment of an antibody yields an F(ab')₂ fragment that has two antigen-combining sites and is still capable of cross-linking antigen.

"Fv" usually refers to the minimum antibody fragment that contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the CDRs confer antigen-binding specificity to the antibody. However, even a single variable domain (or half of an Fv comprising three CDRs specific for an antigen) has the ability to recognize and bind antigen, although likely at a lower affinity than the entire binding site.

Thus, in certain embodiments, the antibodies of the application may comprise 1, 2, 3, 4, 5, 6, or more CDRs that recognize the phosphorylation sites identified in Column E of Table 1.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments that have hinge cysteines between them. Other chemical couplings of antibody fragments are also knovm.

"Single-chain Fv" or "scFv" antibody fragments comprise the V_{H} and V_{L} domains of an antibody, wherein these domains are present in a single polypeptide chain. In certain embodiments, the Fv polypeptide further comprises a polypeptide linker between the V_{H} and V_{L} domains that enables the scFv to form the desired structure for antigen binding. For a review of scFv see Pluckthun in The Pharmacology of Monoclonal Antibodies, vol. 113, Rosenburg and Moore, eds. (Springer-Verlag: New York, 1994), pp. 269-315.

SMIPs are a class of single-chain peptides engineered to include a target binding region and effector domain (CH2 and CH3 domains). *See,* e.g., U.S. Patent Application Publication No. 20050238646. The target binding region may be derived from the variable region or CDRs of an antibody, e.g., a phosphorylation site-specific antibody of the application. Alternatively, the target binding region is derived from a protein that binds a phosphorylation site.

Bispecific antibodies may be monoclonal, human or humanized antibodies that have binding specificities for at least two different antigens. In the present case, one of the binding specificities is for the phosphorylation site, the other one is for any other antigen, such as for example, a cell-surface protein or receptor or receptor subunit. Alternatively, a therapeutic agent may be placed on one arm. The therapeutic agent can be a drug, toxin, enzyme, DNA, radionuclide, etc.

In some embodiments, the antigen-binding fragment can be a diabody. The term "diabody" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

Camelid antibodies refer to a unique type of antibodies that are devoid of light chain, initially discovered from animals of the camelid family. The heavy chains of these so-called heavy-chain antibodies bind their antigen by one single domain, the variable domain of the heavy immunoglobulin chain, referred to as VHH. VHHs show homology with the variable domain of heavy chains of the human VHIII family. The VHHs obtained from an immunized camel, dromedary, or llama have a number of advantages, such as effective production in microorganisms such as Saccharomyces cerevisiae.

In certain embodiments, single chain antibodies, and chimeric, humanized or primatized (CDR-grafted) antibodies, as well as chimeric or CDR-grafted single chain antibodies, comprising portions derived from different species, are also encompassed by the present disclosure as antigen-binding fragments of an antibody. The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., U.S. Pat. Nos. 4,816,567 and 6,331,415; U.S. Pat. No. 4,816,397; European Patent No. 0,120,694; WO 86/01533; European Patent No. 0,194,276 B1; U.S. Pat. No. 5,225,539; and European Patent No. 0,239,400 B1. See also, Newman et al., BioTechnology, 10: 1455-1460 (1992), regarding primatized antibody. See, e.g., Ladner et al., U.S. Pat. No. 4,946,778; and Bird et al., Science, 242: 423-426 (1988)), regarding single chain antibodies.

In addition, functional fragments of antibodies, including fragments of chimeric, humanized, primatized or single chain antibodies, can also be produced. Functional fragments of the subject antibodies retain at least one binding function and/or modulation function of the full-length antibody from which they are derived.

Since the immunoglobulin-related genes contain separate functional regions, each having one or more distinct biological activities, the genes of the antibody fragments may be fused to functional regions from other genes (e.g., enzymes, U.S. Pat. No. 5,004,692, which is incorporated by reference in its entirety) to produce fusion proteins or conjugates having novel properties.

Non-immunoglobulin binding polypeptides are also contemplated. For example, CDRs from an antibody disclosed herein may be inserted into a suitable non-immunoglobulin scaffold to create a non-immunoglobulin binding polypeptide. Suitable candidate scaffold structures may be derived from, for example, members of fibronectin type III and cadherin superfamilies.

Also contemplated are other equivalent non-antibody molecules, such as protein binding domains or aptamers, which bind, in a phospho-specific manner, to an amino acid sequence comprising a novel phosphorylation site of the invention. *See,* e.g., Neuberger et al., Nature 312: 604 (1984). Aptamers are oligonucleic acid or peptide molecules that bind a specific target molecule. DNA or RNA aptamers are typically short oligonucleotides, engineered through repeated rounds of selection to bind to a molecular target. Peptide aptamers typically consist of a variable peptide loop attached at both ends to a protein scaffold. This double structural constraint generally increases the binding affinity of the peptide aptamer to levels comparable to an antibody (nanomolar range).

The invention also discloses the use of the phosphorylation site-specific antibodies with immunotoxins. Conjugates that are immunotoxins including antibodies have been widely described in the art. The toxins may be coupled to the antibodies by conventional coupling techniques or immunotoxins containing protein toxin portions can be produced as fusion proteins. In certain embodiments, antibody conjugates may comprise stable linkers and may release cytotoxic agents inside cells (see U.S. Patent Nos. 6,867,007 and 6,884,869). The conjugates of the present application can be used in a corresponding way to obtain such immunotoxins. Illustrative of such immunotoxins are those described by Byers et al., Seminars Cell Biol 2:59-70 (1991) and by Fanger et al., Immunol Today 12:51-54 (1991). Exemplary immunotoxins include radiotherapeutic agents, ribosome-inactivating proteins (RIPs), chemotherapeutic agents, toxic peptides, or toxic proteins.

The phosphorylation site-specific antibodies disclosed in the invention may be used singly or in combination. The antibodies may also be used in an array format for high throughput uses. An antibody microarray is a collection of immobolized antibodies, typically spotted and fixed on a solid surface (such as glass, plastic and silicon chip).

In another aspect, the antibodies of the invention modulate at least one, or all, biological activities of a parent protein identified in Column A of Table 1. The biological activities of a parent protein identified in Column A of Table 1 include: 1) ligand binding activities (for instance, these neutralizing antibodies may be capable of competing with or completely blocking the binding of a parent signaling protein to at least one, or all, of its ligands; 2) signaling transduction activities, such as receptor dimerization, or tyrosine phosphorylation; and 3) cellular responses induced by a parent signaling protein, such as oncogenic activities (e.g., cancer cell proliferation mediated by a parent signaling protein), and/or angiogenic activities.

In certain embodiments, the antibodies of the invention may have at least one activity selected from the group consisting of: 1) inhibiting cancer cell growth or proliferation; 2) inhibiting cancer cell survival; 3) inhibiting angiogenesis; 4) inhibiting cancer cell metastasis, adhesion, migration or invasion; 5) inducing apoptosis of cancer cells; 6) incorporating a toxic conjugate; and 7) acting as a diagnostic marker.

In certain embodiments, the phosphorylation site specific antibodies disclosed in the invention are especially indicated for diagnostic and therapeutic applications as described herein. Accordingly, the antibodies may be used in therapies, including combination therapies, in the diagnosis and prognosis of disease, as well as in the monitoring of disease progression. The invention, thus, further includes compositions comprising one or more embodiments of an antibody or an antigen binding portion of the invention as described herein. The composition may further comprise a pharmaceutically acceptable carrier. The composition may comprise two or more antibodies or antigen-binding portions, each with specificity for a different novel tyrosine phosphorylation site of the invention or two or more different antibodies or antigen-binding portions all of which are specific for the same novel tyrosine phosphorylation site of the invention. A composition of the invention may comprise one or more antibodies or antigen-binding portions of the invention and one or more additional reagents, diagnostic agents or therapeutic agents.

The present application provides for the polynucleotide molecules encoding the antibodies and antibody fragments and their analogs described herein. Because of the degeneracy of the genetic code, a variety of nucleic acid sequences encode each antibody amino acid sequence. The desired nucleic acid sequences can be produced by de novo solid-phase DNA synthesis or by PCR mutagenesis of an earlier prepared variant of the desired polynucleotide. In one embodiment, the codons that are used comprise those that are typical for human or mouse (see, e.g., Nakamura, Y., Nucleic Acids Res. 28: 292 (2000)).

The invention also provides immortalized cell lines that produce an antibody of the invention. For example, hybridoma clones, constructed as described above, that produce monoclonal antibodies to the targeted signaling protein phosphorylation sties disclosed herein are also provided. Similarly, the invention includes recombinant cells producing an antibody of the invention, which cells may be constructed by well known techniques; for example the antigen combining site of the monoclonal antibody can be cloned by PCR and single-chain antibodies produced as phage-displayed recombinant antibodies or soluble antibodies in *E. coli (see,* e.g., ANTIBODY ENGINEERING PROTOCOLS, 1995, Humana Press, Sudhir Paul editor.)

### 5. Methods of Making Phosphorylation site-Specific Antibodies

In another aspect, the invention provides a method for making phosphorylation site-specific antibodies.

Polyclonal antibodies of the invention may be produced according to standard techniques by immunizing a suitable animal (e.g., rabbit, goat, etc.) with an antigen comprising a novel tyrosine phosphorylation site of the invention. (*i.e*. a phosphorylation site shown in Table 1) in either the phosphorylated or unphosphorylated state, depending upon the desired specificity of the antibody, collecting immune serum from the animal, and separating the polyclonal antibodies from the immune serum, in accordance with known procedures and screening and isolating a polyclonal antibody specific for the novel tyrosine phosphorylation site of interest as further described below. Methods for immunizing non-human animals such as mice, rats, sheep, goats, pigs, cattle and horses are well known in the art. See, e.g., Harlow and Lane, Antibodies: A Laboratory Manual, New York: Cold Spring Harbor Press, 1990.

The immunogen may be the full length protein or a peptide comprising the novel tyrosine phosphorylation site of interest. In some embodiments the immunogen is a peptide of from 7 to 20 amino acids in length, preferably about 8 to 17 amino acids in length. In some embodiments, the peptide antigen desirably will comprise about 3 to 8 amino acids on each side of the phosphorylatable tyrosine. In yet other embodiments, the peptide antigen desirably will comprise four or more amino acids flanking each side of the phosphorylatable amino acid and encompassing it. Peptide antigens suitable for producing antibodies of the invention may be designed, constructed and employed in accordance with well-known techniques. *See,* e.g., Antibodies: A Laboratory Manual, Chapter 5, p. 75-76, Harlow & Lane Eds., Cold Spring Harbor Laboratory (1988); Czemik, Methods In Enzymology, 201: 264-283 (1991); Merrifield, J. Am. Chem. Soc. 85: 21-49 (1962)).

Suitable peptide antigens may comprise all or partial sequence of a trypsin-digested fragment as set forth in Column E of Table 1/Figure 2. Suitable peptide antigens may also comprise all or partial sequence of a peptide fragment produced by another protease digestion.

Preferred immunogens are those that comprise a novel phosphorylation site of a protein in Table 1 that is a enzyme proteins, adaptor/scaffold proteins, protein kinases, receptor/channel/transportercell surface proteins, cytoskeletal proteins, RNA processing proteins, G protein or regulator proteins, transcriptional regulator proteins, adhesion or extracellular matrix proteins and vesicle proteins. In some embodiments, the peptide immunogen is an AQUA peptide, for example, any one of SEQ ID NOS: 4-12, 14-28, 30-51, 53-57, 59-64, 66-97, 99-127, 129-162, 164-177, 179-263, 266-271, 273-288, 290-338 and 340-422.

Particularly preferred immunogens are peptides comprising any one of the novel tyrosine phosphorylation site shown as a lower case "y" in a sequence listed in Table 1 selected from the group consisting of SEQ ID NOS: 123 (ACLY), 125 (ACSL1), 129 (ADSL), 140 (ALDH1B1), 143 (ARD1A), 149 (Got2), 154 (PDHA1), 155 (PDHA1), 213 (PPP2CA), 214 (PPP2CB), 220 (PSMBS), 7 (AIP1), 38 (TRAF4), 221 (AMPKB2), 227 (BRSK2), 236 (p90RSK), 238 (PAK1), 239 (PAK2), 251 (FRK), 256 (FGFR2), 267 (ABCC1), 88 (ACTN4), 93 (Arp3), 304 (EXOSC1), 322 (snRNP 116), 161 (ARF1), 163 (ARF4), 337 (HBS1), 340(PSMC3), 346 (TBP), 45 (afadin), 417 (SNAP), 73 (MCMS), 86 (SKIV2L2), 202 (AK3), 372 (UBQLN1), 385 (C7orf20) and 408 (WDR70).

In some embodiments the immunogen is administered with an adjuvant. Suitable adjuvants will be well known to those of skill in the art. Exemplary adjuvants include complete or incomplete Freund's adjuvant, RIBI (muramyl dipeptides) or ISCOM (immunostimulating complexes).

For example, a peptide antigen comprising the novel adaptor/scaffold protein phosphorylation site in SEQ ID NO: 4 shown by the lower case "y" in Table 1 may be used to produce antibodies that specifically bind the novel tyrosine phosphorylation site.

When the above-described methods are used for producing polyclonal antibodies, following immunization, the polyclonal antibodies which secreted into the bloodstream can be recovered using known techniques. Purified forms of these antibodies can, of course, be readily prepared by standard purification techniques, such as for example, affinity chromatography with Protein A, anti-immunoglobulin, or the antigen itself. In any case, in order to monitor the success of immunization, the antibody levels with respect to the antigen in serum will be monitored using standard techniques such as ELISA, RIA and the like.

Monoclonal antibodies of the invention may be produced by any of a number of means that are well-known in the art. In some embodiments, antibody-producing B cells are isolated from an animal immunized with a peptide antigen as described above. The B cells may be from the spleen, lymph nodes or peripheral blood. Individual B cells are isolated and screened as described below to identify cells producing an antibody specific for the novel tyrosine phosphorylation site of interest. Identified cells are then cultured to produce a monoclonal antibody of the invention.

Alternatively, a monoclonal phosphorylation site-specific antibody of the invention may be produced using standard hybridoma technology, in a hybridoma cell line according to the well-known technique of Kohler and Milstein. See Nature 265: 495-97 (1975); Kohler and Milstein, Eur. J. Immunol. 6: 511 (1976); *see also,* Current Protocols in Molecular Biology, Ausubel et al. Eds. (1989). Monoclonal antibodies so produced are highly specific, and improve the selectivity and specificity of diagnostic assay methods provided by the invention. For example, a solution containing the appropriate antigen may be injected into a mouse or other species and, after a sufficient time (in keeping with conventional techniques), the animal is sacrificed and spleen cells obtained. The spleen cells are then immortalized by any of a number of standard means. Methods of immortalizing cells include, but are not limited to, transfecting them with oncogenes, infecting them with an oncogenic virus and cultivating them under conditions that select for immortalized cells, subjecting them to carcinogenic or mutating compounds, fusing them with an immortalized cell, e.g., a myeloma cell, and inactivating a tumor suppressor gene. See, e.g., Harlow and Lane, supra. If fusion with myeloma cells is used, the myeloma cells preferably do not secrete immunoglobulin polypeptides (a non-secretory cell line). Typically the antibody producing cell and the immortalized cell (such as but not limited to myeloma cells) with which it is fused are from the same species. Rabbit fusion hybridomas, for example, may be produced as described in U.S Patent No. 5,675,063, C. Knight, Issued October 7, 1997. The immortalized antibody producing cells, such as hybridoma cells, are then grown in a suitable selection media, such as hypoxanthine-aminopterin-thymidine (HAT), and the supernatant screened for monoclonal antibodies having the desired specificity, as described below. The secreted antibody may be recovered from tissue culture supernatant by conventional methods such as precipitation, ion exchange or affinity chromatography, or the like.

The invention also encompasses antibody-producing cells and cell lines, such as hybridomas, as described above.

Polyclonal or monoclonal antibodies may also be obtained through in vitro immunization. For example, phage display techniques can be used to provide libraries containing a repertoire of antibodies with varying affinities for a particular antigen. Techniques for the identification of high affinity human antibodies from such libraries are described by Griffiths et al., (1994) EMBO J., 13:3245-3260; Nissim et al., ibid, pp. 692-698 and by Griffiths et al., ibid, 12:725-734, which are incorporated by reference.

The antibodies may be produced recombinantly using methods well known in the art for example, according to the methods disclosed in U.S. Pat. No. 4,349,893 (Reading) or U.S. Pat. No. 4,816,567 (Cabilly et al.) The antibodies may also be chemically constructed by specific antibodies made according to the method disclosed in U.S. Pat. No. 4,676,980 (Segel et al.*)*

Once a desired phosphorylation site-specific antibody is identified, polynucleotides encoding the antibody, such as heavy, light chains or both (or single chains in the case of a single chain antibody) or portions thereof such as those encoding the variable region, may be cloned and isolated from antibody-producing cells using means that are well known in the art. For example, the antigen combining site of the monoclonal antibody can be cloned by PCR and single-chain antibodies produced as phage-displayed recombinant antibodies or soluble antibodies in *E. coli (see,* e.g., Antibody Engineering Protocols, 1995, Humana Press, Sudhir Paul editor.)

Accordingly, in a further aspect, the invention provides such nucleic acids encoding the heavy chain, the light chain, a variable region, a framework region or a CDR of an antibody of the invention. In some embodiments, the nucleic acids are operably linked to expression control sequences. The invention, thus, also provides vectors and expression control sequences useful for the recombinant expression of an antibody or antigen-binding portion thereof of the invention. Those of skill in the art will be able to choose vectors and expression systems that are suitable for the host cell in which the antibody or antigen-binding portion is to be expressed.

Monoclonal antibodies of the invention may be produced recombinantly by expressing the encoding nucleic acids in a suitable host cell under suitable conditions. Accordingly, the invention further provides host cells comprising the nucleic acids and vectors described above.

Monoclonal Fab fragments may also be produced in *Escherichia coli* by recombinant techniques known to those skilled in the art. *See,* e.g., W. Huse, Science 246: 1275-81 (1989); Mullinax et al., Proc. Nat'l Acad. Sci. 87: 8095 (1990).

If monoclonal antibodies of a single desired isotype are preferred for a particular application, particular isotypes can be prepared directly, by selecting from the initial fusion, or prepared secondarily, from a parental hybridoma secreting a monoclonal antibody of different isotype by using the sib selection technique to isolate class-switch variants (Steplewski, et al., Proc. Nat'l. Acad. Sci., 82: 8653 (1985); Spira et al., J. Immunol. Methods, 74: 307 (1984)). Alternatively, the isotype of a monoclonal antibody with desirable propertied can be changed using antibody engineering techniques that are well-known in the art.

Phosphorylation site-specific antibodies of the invention, whether polyclonal or monoclonal, may be screened for epitope and phospho-specificity according to standard techniques. *See,* e.g., Czernik et al., Methods in Enzymology, 201: 264-283 (1991). For example, the antibodies may be screened against the phosphorylated and/or unphosphosphorylated peptide library by ELISA to ensure specificity for both the desired antigen (*i*.*e*. that epitope including a phosphorylation site of the invention and for reactivity only with the phosphorylated (or unphosphorylated) form of the antigen. Peptide competition assays may be carried out to confirm lack of reactivity with other phospho-epitopes on the parent protein. The antibodies may also be tested by Western blotting against cell preparations containing the parent signaling protein, e.g., cell lines over-expressing the parent protein, to confirm reactivity with the desired phosphorylated epitope/target.

Specificity against the desired phosphorylated epitope may also be examined by constructing mutants lacking phosphorylatable residues at positions outside the desired epitope that are known to be phosphorylated, or by mutating the desired phospho-epitope and confirming lack of reactivity. Phosphorylation site-specific antibodies of the invention may exhibit some limited cross-reactivity to related epitopes in non-target proteins. This is not unexpected as most antibodies exhibit some degree of cross-reactivity, and antipeptide antibodies will often cross-react with epitopes having high homology to the immunizing peptide. *See,* e.g., *Czernik, supra.* Cross-reactivity with non-target proteins is readily characterized by Western blotting alongside markers of known molecular weight. Amino acid sequences of cross-reacting proteins may be examined to identify phosphorylation sites with flanking sequences that are highly homologous to that of a phosphorylation site of the invention.

In certain cases, polyclonal antisera may exhibit some undesirable general cross-reactivity to phosphotyrosine itself, which may be removed by further purification of antisera, e.g., over a phosphotyramine column. Antibodies of the invention specifically bind their target protein (*i.e.* a protein listed in Column A of Table 1) only when phosphorylated (or only when not phosphorylated, as the case may be) at the site disclosed in corresponding Columns D/E, and do not (substantially) bind to the other form (as compared to the form for which the antibody is specific).

Antibodies may be further characterized via immunohistochemical (IHC) staining using normal and diseased tissues to examine phosphorylation and activation state and level of a phosphorylation site in diseased tissue. IHC may be carried out according to well-known techniques. *See,* e.g., Antibodies: A Laboratory Manual, Chapter 10, Harlow & Lane Eds., Cold Spring Harbor Laboratory (1988). Briefly, paraffin-embedded tissue (e.g., tumor tissue) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody and secondary antibody; and finally detecting using ABC avidin/biotin method according to manufacturer's instructions.

Antibodies may be further characterized by flow cytometry carried out according to standard methods. *See* Chow et al., Cytometry (Communications in Clinical Cytometry) 46: 72-78 (2001). Briefly and by way of example , the following protocol for cytometric analysis may be employed:
samples may be centrifuged on Ficoll gradients to remove lysed erythrocytes and cell debris. Adherring cells may be scrapped off plates and washed with PBS. Cells may then be fixed with 2% paraformaldehyde for 10 minutes at 37 °C followed by permeabilization in 90% methanol for 30 minutes on ice. Cells may then be stained with the primary phosphorylation site-specific antibody of the invention (which detects a parent signaling protein enumerated in Table 1), washed and labeled with a fluorescent-labeled secondary antibody. Additional fluorochrome-conjugated marker antibodies (e.g., CD45, CD34) may also be added at this time to aid in the subsequent identification of specific hematopoietic cell types. The cells would then be analyzed on a flow cytometer (*e.g.* a Beckman Coulter FC500) according to the specific protocols of the instrument used.

Antibodies of the invention may also be advantageously conjugated to fluorescent dyes (*e.g.* Alexa488, PE) for use in multi-parametric analyses along with other signal transduction (phospho-CrkL, phospho-Erk 1/2) and/or cell marker (CD34) antibodies.

Phosphorylation site-specific antibodies of the invention may specifically bind to a signaling protein or polypeptide listed in Table 1 only when phosphorylated at the specified tyrosine residue, but are not limited only to binding to the listed signaling proteins of human species, *per se.* The invention includes antibodies that also bind conserved and highly homologous or identical phosphorylation sites in respective signaling proteins from other species (e.g., mouse, rat, monkey, yeast), in addition to binding the phosphorylation site of the human homologue. The term "homologous" refers to two or more sequences or subsequences that have at least about 85%, at least 90%, at least 95%, or higher nucleotide or amino acid residue identity, when compared and aligned for maximum correspondence, as measured using sequence comparison method (e.g., BLAST) and/or by visual inspection. Highly homologous or identical sites conserved in other species can readily be identified by standard sequence comparisons (such as BLAST).

Methods for making bispecific antibodies are within the purview of those skilled in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Antibody variable domains with the desired binding specificities (antibody-antigen combining sites) can be fused to immunoglobulin constant domain sequences. In certain embodiments, the fusion is with an immunoglobulin heavy-chain constant domain, including at least part of the hinge, CH2, and CH3 regions. DNAs encoding the immunoglobulin heavy-chain fusions and, if desired, the immunoglobulin light chain, are inserted into separate expression vectors, and are co-transfected into a suitable host organism. For further details of illustrative currently known methods for generating bispecific antibodies see, for example, Suresh et al., Methods in Enzymology, 121:210 (1986); WO 96/27011; Brennan et al., Science 229:81 (1985); Shalaby et al., J. Exp. Med. 175:217-225 (1992); Kostelny et al., J. Immunol. 148(5):1547-1553 (1992); Hollinger et al., Proc. Natl. Acad. Sci. USA 90:6444-6448 (1993); Gruber et al., J. Immunol. 152:5368 (1994); and Tutt et al., J. Immunol. 147:60 (1991). Bispecific antibodies also include cross-linked or heteroconjugate antibodies. Heteroconjugate antibodies may be made using any convenient cross-linking methods. Suitable cross-linking agents are well known in the art, and are disclosed in U.S. Pat. No. 4,676,980, along with a number of cross-linking techniques.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins may be linked to the Fab' portions of two different antibodies by gene fusion. The antibody homodimers may be reduced at the hinge region to form monomers and then re-oxidized to form the antibody heterodimers. This method can also be utilized for the production of antibody homodimers. A strategy for making bispecific antibody fragments by the use of single-chain Fv (scFv) dimers has also been reported. See Gruber et al., J. Immunol., 152:5368 (1994). Alternatively, the antibodies can be "linear antibodies" as described in Zapata et al. Protein Eng. 8(10):1057-1062 (1995). Briefly, these antibodies comprise a pair of tandem Fd segments (V_{H} -C_{H}1-V_{H} - C_{H}1) which form a pair of antigen binding regions. Linear antibodies can be bispecific or monospecific.

To produce the chimeric antibodies, the portions derived from two different species (e.g., human constant region and murine variable or binding region) can be joined together chemically by conventional techniques or can be prepared as single contiguous proteins using genetic engineering techniques. The DNA molecules encoding the proteins of both the light chain and heavy chain portions of the chimeric antibody can be expressed as contiguous proteins. The method of making chimeric antibodies is disclosed in U.S. Pat. No. 5,677,427; U.S. Pat. No. 6,120,767; and U.S. Pat. No. 6,329,508, each of which is incorporated by reference in its entirety.

Fully human antibodies may be produced by a variety of techniques. One example is trioma methodology. The basic approach and an exemplary cell fusion partner, SPAZ-4, for use in this approach have been described by Oestberg et al., Hybridoma 2:361-367 (1983); Oestberg, U.S. Pat. No. 4,634,664; and Engleman et al., U.S. Pat. No. 4,634,666 (each of which is incorporated by reference in its entirety).

Human antibodies can also be produced from non-human transgenic animals having transgenes encoding at least a segment of the human immunoglobulin locus. The production and properties of animals having these properties are described in detail by, see, e.g., Lonberg et al., WO93/12227; U.S. Pat. No. 5,545,806; and Kucherlapati, et al., WO91/10741; U.S. Pat. No. 6,150,584, which are herein incorporated by reference in their entirety.

Various recombinant antibody library technologies may also be utilized to produce fully human antibodies. For example, one approach is to screen a DNA library from human B cells according to the general protocol outlined by Huse et al., Science 246:1275-1281 (1989). The protocol described by Huse is rendered more efficient in combination with phage-display technology. See, e.g., Dower et al., WO 91/17271 and McCafferty et al., WO 92/01047; U.S. Pat. No. 5,969,108, (each of which is incorporated by reference in its entirety).

Eukaryotic ribosome can also be used as means to display a library of antibodies and isolate the binding human antibodies by screening against the target antigen, as described in Coia G, et al., J. Immunol. Methods 1: 254 (1-2):191-7 (2001); Hanes J. et al., Nat. Biotechnol. 18(12):1287-92 (2000); Proc. Natl. Acad. Sci. U. S. A. 95(24):14130-5 (1998); Proc. Natl. Acad. Sci. U. S. A. 94(10):4937-42 (1997), each which is incorporated by reference in its entirety.

The yeast system is also suitable for screening mammalian cell-surface or secreted proteins, such as antibodies. Antibody libraries may be displayed on the surface of yeast cells for the purpose of obtaining the human antibodies against a target antigen. This approach is described by Yeung, et al., Biotechnol. Prog. 18(2):212-20 (2002); Boeder, E. T., et al., Nat. Biotechnol. 15(6):553-7 (1997), each of which is herein incorporated by reference in its entirety. Alternatively, human antibody libraries may be expressed intracellularly and screened via the yeast two-hybrid system (WO0200729A2, which is incorporated by reference in its entirety).

Recombinant DNA techniques can be used to produce the recombinant phosphorylation site-specific antibodies described herein, as well as the chimeric or humanized phosphorylation site-specific antibodies, or any other genetically-altered antibodies and the fragments or conjugate thereof in any expression systems including both prokaryotic and eukaryotic expression systems, such as bacteria, yeast, insect cells, plant cells, mammalian cells (for example, NSO cells).

Once produced, the whole antibodies, their dimers, individual light and heavy chains, or other immunoglobulin forms of the present application can be purified according to standard procedures of the art, including ammonium sulfate precipitation, affinity columns, column chromatography, gel electrophoresis and the like (see, generally, Scopes, R., Protein Purification (Springer-Verlag, N.Y., 1982)). Once purified, partially or to homogeneity as desired, the polypeptides may then be used therapeutically (including extracorporeally) or in developing and performing assay procedures, immunofluorescent staining, and the like. (See, generally, Immunological Methods, Vols. I and II (Lefkovits and Pernis, eds., Academic Press, NY, 1979 and 1981).

### 6. Therapeutic Uses

In a further aspect, the invention provides methods and compositions for therapeutic uses of the peptides or proteins comprising a phosphorylation site of the invention, and phosphorylation site-specific antibodies of the invention.

In one embodiment, the invention provides for a method of treating or preventing carcinoma and/or leukemia in a subject, wherein the carcinoma and/or leukemia is associated with the phosphorylation state of a novel phosphorylation site in Table 1, whether phosphorylated or dephosphorylated, comprising: administering to a subject in need thereof a therapeutically effective amount of a peptide comprising a novel phosphorylation site (Table 1) and/or an antibody or antigen-binding fragment thereof that specifically bind a novel phosphorylation site of the invention (Table 1). The antibodies maybe full-length antibodies, genetically engineered antibodies, antibody fragments, and antibody conjugates of the invention.

The term "subject" refers to a vertebrate, such as for example, a mammal, or a human. Although present application are primarily concerned with the treatment of human subjects, the disclosed methods may also be used for the treatment of other mammalian subjects such as dogs and cats for veterinary purposes.

In one aspect, the disclosure provides a method of treating carcinoma and/or leukemia in which a peptide or an antibody that reduces at least one biological activity of a targeted signaling protein is administered to a subject. For example, the peptide or the antibody administered may disrupt or modulate the interaction of the target signaling protein with its ligand. Alternatively, the peptide or the antibody may interfere with, thereby reducing, the down-stream signal transduction of the parent signaling protein. An antibody that specifically binds the novel tyrosine phosphorylation site only when the tyrosine is phosphorylated, and that does not substantially bind to the same sequence when the tyrosine is not phosphorylated, thereby prevents downstream signal transduction triggered by a phospho-tyrosine. Alternatively, an antibody that specifically binds the unphosphorylated target phosphorylation site reduces the phosphorylation at that site and thus reduces activation of the protein mediated by phosphorylation of that site. Similarly, an unphosphorylated peptide may compete with an endogenous phosphorylation site for same kinases, thereby preventing or reducing the phosphorylation of the endogenous target protein. Alternatively, a peptide comprising a phosphorylated novel tyrosine site of the invention but lacking the ability to trigger signal transduction may competitively inhibit interaction of the endogenous protein with the same down-stream ligand(s).

The antibodies of the invention may also be used to target cancer cells for effector-mediated cell death. The antibody disclosed herein may be administered as a fusion molecule that includes a phosphorylation site-targeting portion joined to a cytotoxic moiety to directly kill cancer cells. Alternatively, the antibody may directly kill the cancer cells through complement-mediated or antibody-dependent cellular cytotoxicity.

Accordingly in one embodiment, the antibodies of the present disclosure may be used to deliver a variety of cytotoxic compounds. Any cytotoxic compound can be fused to the present antibodies. The fusion can be achieved chemically or genetically (e.g., via expression as a single, fused molecule). The cytotoxic compound can be a biological, such as a polypeptide, or a small molecule. As those skilled in the art will appreciate, for small molecules, chemical fusion is used, while for biological compounds, either chemical or genetic fusion can be used.

Non-limiting examples of cytotoxic compounds include therapeutic drugs, radiotherapeutic agents, ribosome-inactivating proteins (RIPs), chemotherapeutic agents, toxic peptides, toxic proteins, and mixtures thereof. The cytotoxic drugs can be intracellularly acting cytotoxic drugs, such as short-range radiation emitters, including, for example, short-range, high-energy α-emitters. Enzymatically active toxins and fragments thereof, including ribosome-inactivating proteins, are exemplified by saporin, luffin, momordins, ricin, trichosanthin, gelonin, abrin, etc. Procedures for preparing enzymatically active polypeptides of the immunotoxins are described in WO84/03508 and WO85/03508, which are hereby incorporated by reference. Certain cytotoxic moieties are derived from adriamycin, chlorambucil, daunomycin, methotrexate, neocarzinostatin, and platinum, for example.

Exemplary chemotherapeutic agents that may be attached to an antibody or antigen-binding fragment thereof include taxol, doxorubicin, verapamil, podophyllotoxin, procarbazine, mechlorethamine, cyclophosphamide, camptothecin, ifosfamide, melphalan, chlorambucil, bisulfan, nitrosurea, dactinomycin, daunorubicin, doxorubicin, bleomycin, plicomycin, mitomycin, etoposide (VP16), tamoxifen, transplatinum, 5-fluorouracil, vincristin, vinblastin, or methotrexate.

Procedures for conjugating the antibodies with the cytotoxic agents have been previously described and are within the purview of one skilled in the art.

Alternatively, the antibody can be coupled to high energy radiation emitters, for example, a radioisotope, such as ¹³¹I, a γ-emitter, which, when localized at the tumor site, results in a killing of several cell diameters. See, e.g., S. E. Order, "Analysis, Results, and Future Prospective of the Therapeutic Use of Radiolabeled Antibody in Cancer Therapy", Monoclonal Antibodies for Cancer Detection and Therapy, Baldwin et al. (eds.), pp. 303-316 (Academic Press 1985), which is hereby incorporated by reference. Other suitable radioisotopes include α-emitters, such as ²¹²Bi, ²¹³Bi, and ²¹¹At, and β-emitters, such as ¹⁸⁶Re and ⁹⁰Y.

Because many of the signaling proteins in which novel tyrosine phosphorylation sites of the invention occur also are expressed in normal cells and tissues, it may also be advantageous to administer a phosphorylation site-specific antibody with a constant region modified to reduce or eliminate ADCC or CDC to limit damage to normal cells. For example, effector function of an antibodies may be reduced or eliminated by utilizing an IgG1 constant domain instead of an IgG2/4 fusion domain. Other ways of eliminating effector function can be envisioned such as, e.g., mutation of the sites known to interact with FcR or insertion of a peptide in the hinge region, thereby eliminating critical sites required for FcR interaction. Variant antibodies with reduced or no effector function also include variants as described previously herein.

The peptides and antibodies of the invention may be used in combination with other therapies or with other agents. Other agents include but are not limited to polypeptides, small molecules, chemicals, metals, organometallic compounds, inorganic compounds, nucleic acid molecules, oligonucleotides, aptamers, spiegelmers, antisense nucleic acids, locked nucleic acid (LNA) inhibitors, peptide nucleic acid (PNA) inhibitors, immunomodulatory agents, antigen-binding fragments, prodrugs, and peptidomimetic compounds. In certain embodiments, the antibodies and peptides of the invention may be used in combination with cancer therapies known to one of skill in the art.

In certain aspects, the present disclosure relates to combination treatments comprising a phosphorylation site-specific antibody described herein and immunomodulatory compounds, vaccines or chemotherapy. Illustrative examples of suitable immunomodulatory agents that may be used in such combination therapies include agents that block negative regulation of T cells or antigen presenting cells (e.g., anti-CTLA4 antibodies, anti-PD-L1 antibodies, anti-PDL-2 antibodies, anti-PD-1 antibodies and the like) or agents that enhance positive co-stimulation of T cells (e.g., anti-CD40 antibodies or anti 4-1BB antibodies) or agents that increase NK cell number or T-cell activity (e.g., inhibitors such as IMiDs, thalidomide, or thalidomide analogs). Furthermore, immunomodulatory therapy could include cancer vaccines such as dendritic cells loaded with tumor cells, proteins, peptides, RNA, or DNA derived from such cells, patient derived heat-shock proteins (hsp's) or general adjuvants stimulating the immune system at various levels such as CpG, Luivac®, Biostim®, Ribomunyl®, Imudon®, Bronchovaxom® or any other compound or other adjuvant activating receptors of the innate immune system (e.g., toll like receptor agonist, anti-CTLA-4 antibodies, etc.). Also, immunomodulatory therapy could include treatment with cytokines such as IL-2, GM-CSF and IFN-gamma.

Furthermore, combination of antibody therapy with chemotherapeutics could be particularly useful to reduce overall tumor burden, to limit angiogenesis, to enhance tumor accessibility, to enhance susceptibility to ADCC, to result in increased immune function by providing more tumor antigen, or to increase the expression of the T cell attractant LIGHT.

Pharmaceutical compounds that may be used for combinatory anti-tumor therapy include, merely to illustrate: aminoglutethimide, amsacrine, anastrozole, asparaginase, bcg, bicalutamide, bleomycin, buserelin, busulfan, camptothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

These chemotherapeutic anti-tumor compounds may be categorized by their mechanism of action into groups, including, for example, the following classes of agents: anti-metabolites/anti-cancer agents, such as pyrimidine analogs (5-fluorouracil, floxuridine, capecitabine, gemcitabine and cytarabine) and purine analogs, folate inhibitors and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristine, vinblastine, nocodazole, epothilones and navelbine, epidipodophyllotoxins (etoposide, teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethylmelamineoxaliplatin, iphosphamide, melphalan, mechlorethamine, mitomycin, mitoxantrone, nitrosourea, plicamycin, procarbazine, taxol, taxotere, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes - dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); immunomodulatory agents (thalidomide and analogs thereof such as lenalidomide (Revlimid, CC-5013) and CC-4047 (Actimid)), cyclophosphamide; anti-angiogenic compounds (TNP-470, genistein) and growth factor inhibitors (vascular endothelial growth factor (VEGF) inhibitors, fibroblast growth factor (FGF) inhibitors); angiotensin receptor blocker; nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin and mitoxantrone, topotecan, irinotecan), corticosteroids (cortisone, dexamethasone, hydrocortisone, methylprednisolone, prednisone, and prenisolone); growth factor signal transduction kinase inhibitors; mitochondrial dysfunction inducers and caspase activators; and chromatin disruptors.

In certain embodiments, pharmaceutical compounds that may be used for combinatory anti-angiogenesis therapy include: (1) inhibitors of release of "angiogenic molecules," such as bFGF (basic fibroblast growth factor); (2) neutralizers of angiogenic molecules, such as anti-βbFGF antibodies; and (3) inhibitors of endothelial cell response to angiogenic stimuli, including collagenase inhibitor, basement membrane turnover inhibitors, angiostatic steroids, fungal-derived angiogenesis inhibitors, platelet factor 4, thrombospondin, arthritis drugs such as D-penicillamine and gold thiomalate, vitamin D₃ analogs, alpha-interferon, and the like. For additional proposed inhibitors of angiogenesis, see Blood et al., Biochim. Biophys. Acta, 1032:89-118 (1990), Moses et al., Science, 248:1408-1410 (1990), Ingber et al., Lab. Invest., 59:44-51 (1988), and U.S. Pat. Nos. 5,092,885, 5,112,946, 5,192,744, 5,202,352, and 6,573,256. In addition, there are a wide variety of compounds that can be used to inhibit angiogenesis, for example, peptides or agents that block the VEGF-mediated angiogenesis pathway, endostatin protein or derivatives, lysine binding fragments of angiostatin, melanin or melanin-promoting compounds, plasminogen fragments (e.g., Kringles 1-3 of plasminogen), troponin subunits, inhibitors of vitronectin αᵥβ₃, peptides derived from Saposin B, antibiotics or analogs (e.g., tetracycline or neomycin), dienogest-containing compositions, compounds comprising a MetAP-2 inhibitory core coupled to a peptide, the compound EM-138, chalcone and its analogs, and naaladase inhibitors. See, for example, U.S. Pat. Nos. 6,395,718, 6,462,075, 6,465,431, 6,475,784, 6,405,802, 6,405,810, 6,500,431, 6,500,924, 6,518,298, 6,521,439, 6,525,019, 6,538,103, 6,544,758, 6,544,947, 6,548,477, 6,559,126, and 6,569,845.

### 7. Diagnostic Uses

In a further aspect, the invention provides methods for detecting and quantitating phosphoyrlation at a novel tyrosine phosphorylation site of the invention. For example, peptides, including AQUA peptides of the invention, and antibodies of the invention are useful in diagnostic and prognostic evaluation of carcinoma and/or leukemias, wherein the carcinoma and/or leukemia is associated with the phosphorylation state of a novel phosphorylation site in Table 1, whether phosphorylated or dephosphorylated.

Methods of diagnosis can be performed *in vitro* using a biological sample (e.g., blood sample, lymph node biopsy or tissue) from a subject, or in vivo. The phosphorylation state or level at the tyrosine residue identified in the corresponding row in Column D of Table 1 may be assessed. A change in the phosphorylation state or level at the phosphorylation site, as compared to a control, indicates that the subject is suffering from, or susceptible to, carcinoma and/or leukemia.

In one embodiment, the phosphorylation state or level at a novel phosphorylation site is determined by an AQUA peptide comprising the phosphorylation site. The AQUA peptide may be phosphorylated or unphosphorylated at the specified tyrosine position.

In another embodiment, the phosphorylation state or level at a phosphorylation site is determined by an antibody or antigen binding fragment thereof, wherein the antibody specifically binds the phosphorylation site. The antibody may be one that only binds to the phosphorylation site when the tyrosine residue is phosphorylated, but does not bind to the same sequence when the tyrosine is not phosphorylated; or vice versa.

In particular embodiments, the antibodies of the present application are attached to labeling moieties, such as a detectable marker. One or more detectable labels can be attached to the antibodies. Exemplary labeling moieties include radiopaque dyes, radiocontrast agents, fluorescent molecules, spin-labeled molecules, enzymes, or other labeling moieties of diagnostic value, particularly in radiologic or magnetic resonance imaging techniques.

A radiolabeled antibody in accordance with this disclosure can be used for in vitro diagnostic tests. The specific activity of an antibody, binding portion thereof, probe, or ligand, depends upon the half-life, the isotopic purity of the radioactive label, and how the label is incorporated into the biological agent. In immunoassay tests, the higher the specific activity, in general, the better the sensitivity. Radioisotopes useful as labels, e.g., for use in diagnostics, include iodine (¹³¹I or ¹²⁵I), indium (¹¹¹In), technetium ⁹⁹Tc), phosphorus (³²P), carbon (¹⁴C), and tritium (³H), or one of the therapeutic isotopes listed above.

Fluorophore and chromophore labeled biological agents can be prepared from standard moieties known in the art. Since antibodies and other proteins absorb light having wavelengths up to about 310 nm, the fluorescent moieties may be selected to have substantial absorption at wavelengths above 310 nm, such as for example, above 400 nm. A variety of suitable fluorescers and chromophores are described by Stryer, Science, 162:526 (1968) and Brand et al., Annual Review of Biochemistry, 41:843-868 (1972), which are hereby incorporated by reference. The antibodies can be labeled with fluorescent chromophore groups by conventional procedures such as those disclosed in U.S. Patent Nos. 3,940,475, 4,289,747, and 4,376, 110, which are hereby incorporated by reference.

The control may be parallel samples providing a basis for comparison, for example, biological samples drawn from a healthy subject, or biological samples drawn from healthy tissues of the same subject. Alternatively, the control may be a pre-determined reference or threshold amount. If the subject is being treated with a therapeutic agent, and the progress of the treatment is monitored by detecting the tyrosine phosphorylation state level at a phosphorylation site of the invention, a control may be derived from biological samples drawn from the subject prior to, or during the course of the treatment.

In certain embodiments, antibody conjugates for diagnostic use in the present application are intended for use in vitro, where the antibody is linked to a secondary binding ligand or to an enzyme (an enzyme tag) that will generate a colored product upon contact with a chromogenic substrate. Examples of suitable enzymes include urease, alkaline phosphatase, (horseradish) hydrogen peroxidase and glucose oxidase. In certain embodiments, secondary binding ligands are biotin and avidin or streptavidin compounds.

Antibodies of the invention may also be optimized for use in a flow cytometry (FC) assay to determine the activation/phosphorylation status of a target signaling protein in subjects before, during, and after treatment with a therapeutic agent targeted at inhibiting tyrosine phosphorylation at the phosphorylation site disclosed herein. For example, bone marrow cells or peripheral blood cells from patients may be analyzed by flow cytometry for target signaling protein phosphorylation, as well as for markers identifying various hematopoietic cell types. In this manner, activation status of the malignant cells may be specifically characterized. Flow cytometry may be carried out according to standard methods. *See,* e.g., Chow et al., Cytometry (Communications in Clinical Cytometry) 46: 72-78 (2001).

Alternatively, antibodies of the invention may be used in immunohistochemical (IHC) staining to detect differences in signal transduction or protein activity using normal and diseased tissues. IHC may be carried out according to well-known techniques. *See,* e.g., Antibodies: A Laboratory Manual, supra.

Peptides and antibodies of the invention may be also be optimized for use in other clinically-suitable applications, for example bead-based multiplex-type assays, such as IGEN, Luminex^{™} and/or Bioplex^{™} assay formats, or otherwise optimized for antibody arrays formats, such as reversed-phase array applications *(see,* e.g. Paweletz et al., Oncogene 20(16): 1981-89 (2001)). Accordingly, in another embodiment, the invention provides a method for the multiplex detection of the phosphorylation state or level at two or more phosphorylation sites of the invention (Table 1) in a biological sample, the method comprising utilizing two or more antibodies or AQUA peptides of the invention. In one preferred embodiment, two to five antibodies or AQUA peptides of the invention are used. In another preferred embodiment, six to ten antibodies or AQUA peptides of the invention are used, while in another preferred embodiment eleven to twenty antibodies or AQUA peptides of the invention are used.

In certain embodiments the diagnostic methods of the application may be used in combination with other cancer diagnostic tests.

The biological sample analyzed may be any sample that is suspected of having abnormal tyrosine phosphorylation at a novel phosphorylation site of the invention, such as a homogenized neoplastic tissue sample.

### 8. Screening assays

In another aspect, the invention provides a method for identifying an agent that modulates tyrosine phosphorylation at a novel phosphorylation site of the invention, comprising: a) contacting a candidate agent with a peptide or protein comprising a novel phosphorylation site of the invention; and b) determining the phosphorylation state or level at the novel phosphorylation site. A change in the phosphorylation level of the specified tyrosine in the presence of the test agent, as compared to a control, indicates that the candidate agent potentially modulates tyrosine phosphorylation at a novel phosphorylation site of the invention.

In one embodiment, the phosphorylation state or level at a novel phosphorylation site is determined by an AQUA peptide comprising the phosphorylation site. The AQUA peptide may be phosphorylated or unphosphorylated at the specified tyrosine position.

In another embodiment, the phosphorylation state or level at a phosphorylation site is determined by an antibody or antigen-binding fragment thereof, wherein the antibody specifically binds the phosphorylation site. The antibody may be one that only binds to the phosphorylation site when the tyrosine residue is phosphorylated, but does not bind to the same sequence when the tyrosine is not phosphorylated; or vice versa.

In particular embodiments, the antibodies of the present application are attached to labeling moieties, such as a detectable marker.

The control may be parallel samples providing a basis for comparison, for example, the phosphorylation level of the target protein or peptide in absence of the testing agent. Alternatively, the control may be a pre-determined reference or threshold amount.

### 9. Immunoassays

In another aspect, the present application concerns immunoassays for binding, purifying, quantifying and otherwise generally detecting the phosphorylation state or level at a novel phosphorylation site of the invention.

Assays may be homogeneous assays or heterogeneous assays. In a homogeneous assay the immunological reaction usually involves a phosphorylation site-specific antibody of the invention, a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels that may be used include free radicals, radioisotopes, fluorescent dyes, enzymes, bacteriophages, coenzymes, and so forth.

In a heterogeneous assay approach, the reagents are usually the specimen, a phosphorylation site-specific antibody of the invention, and suitable means for producing a detectable signal. Similar specimens as described above may be used. The antibody is generally immobilized on a support, such as a bead, plate or slide, and contacted with the specimen suspected of containing the antigen in a liquid phase. The support is then separated from the liquid phase and either the support phase or the liquid phase is examined for a detectable signal using means for producing such signal. The signal is related to the presence of the analyte in the specimen. Means for producing a detectable signal include the use of radioactive labels, fluorescent labels, enzyme labels, and so forth.

Phosphorylation site-specific antibodies disclosed herein may be conjugated to a solid support suitable for a diagnostic assay (e.g., beads, plates, slides or wells formed from materials such as latex or polystyrene) in accordance with known techniques, such as precipitation.

In certain embodiments, immunoassays are the various types of enzyme linked immunoadsorbent assays (ELISAs) and radioimmunoassays (RIA) known in the art. Immunohistochemical detection using tissue sections is also particularly useful. However, it will be readily appreciated that detection is not limited to such techniques, and Western blotting, dot and slot blotting, FACS analyses, and the like may also be used. The steps of various useful immunoassays have been described in the scientific literature, such as, e.g., Nakamura et al., in Enzyme Immunoassays: Heterogeneous and Homogeneous Systems, Chapter 27 (1987), incorporated herein by reference.

In general, the detection of immunocomplex formation is well known in the art and may be achieved through the application of numerous approaches. These methods are based upon the detection of radioactive, fluorescent, biological or enzymatic tags. Of course, one may find additional advantages through the use of a secondary binding ligand such as a second antibody or a biotin/avidin ligand binding arrangement, as is known in the art.

The antibody used in the detection may itself be conjugated to a detectable label, wherein one would then simply detect this label. The amount of the primary immune complexes in the composition would, thereby, be determined.

Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under conditions effective and for a period of time sufficient to allow the formation of secondary immune complexes. The secondary immune complexes are washed extensively to remove any non-specifically bound labeled secondary antibodies or ligands, and the remaining label in the secondary immune complex is detected.

An enzyme linked immunoadsorbent assay (ELISA) is a type of binding assay. In one type of ELISA, phosphorylation site-specific antibodies disclosed herein are immobilized onto a selected surface exhibiting protein affinity, such as a well in a polystyrene microtiter plate. Then, a suspected neoplastic tissue sample is added to the wells. After binding and washing to remove non-specifically bound immune complexes, the bound target signaling protein may be detected.

In another type of ELISA, the neoplastic tissue samples are immobilized onto the well surface and then contacted with the phosphorylation site-specific antibodies disclosed herein. After binding and washing to remove non-specifically bound immune complexes, the bound phosphorylation site-specific antibodies are detected.

Irrespective of the format used, ELISAs have certain features in common, such as coating, incubating or binding, washing to remove non-specifically bound species, and detecting the bound immune complexes.

The radioimmunoassay (RIA) is an analytical technique which depends on the competition (affinity) of an antigen for antigen-binding sites on antibody molecules. Standard curves are constructed from data gathered from a series of samples each containing the same known concentration of labeled antigen, and various, but known, concentrations of unlabeled antigen. Antigens are labeled with a radioactive isotope tracer. The mixture is incubated in contact with an antibody. Then the free antigen is separated from the antibody and the antigen bound thereto. Then, by use of a suitable detector, such as a gamma or beta radiation detector, the percent of either the bound or free labeled antigen or both is determined. This procedure is repeated for a number of samples containing various known concentrations of unlabeled antigens and the results are plotted as a standard graph. The percent of bound tracer antigens is plotted as a function of the antigen concentration. Typically, as the total antigen concentration increases the relative amount of the tracer antigen bound to the antibody decreases. After the standard graph is prepared, it is thereafter used to determine the concentration of antigen in samples undergoing analysis.

In an analysis, the sample in which the concentration of antigen is to be determined is mixed with a known amount of tracer antigen. Tracer antigen is the same antigen known to be in the sample but which has been labeled with a suitable radioactive isotope. The sample with tracer is then incubated in contact with the antibody. Then it can be counted in a suitable detector which counts the free antigen remaining in the sample. The antigen bound to the antibody or immunoadsorbent may also be similarly counted. Then, from the standard curve, the concentration of antigen in the original sample is determined.

### 10. Pharmaceutical Formulations and Methods of Administration

Methods of administration of therapeutic agents, particularly peptide and antibody therapeutics, are well-known to those of skill in the art.

Peptides of the invention can be administered in the same manner as conventional peptide type pharmaceuticals. Preferably, peptides are administered parenterally, for example, intravenously, intramuscularly, intraperitoneally, or subcutaneously. When administered orally, peptides may be proteolytically hydrolyzed. Therefore, oral application may not be usually effective. However, peptides can be administered orally as a formulation wherein peptides are not easily hydrolyzed in a digestive tract, such as liposome-microcapsules. Peptides may be also administered in suppositories, sublingual tablets, or intranasal spray.

If administered parenterally, a preferred pharmaceutical composition is an aqueous solution that, in addition to a peptide of the invention as an active ingredient, may contain for example, buffers such as phosphate, acetate, etc., osmotic pressure-adjusting agents such as sodium chloride, sucrose, and sorbitol, etc., antioxidative or antioxygenic agents, such as ascorbic acid or tocopherol and preservatives, such as antibiotics. The parenterally administered composition also may be a solution readily usable or in a lyophilized form which is dissolved in sterile water before administration.

The pharmaceutical formulations, dosage forms, and uses described below generally apply to antibody-based therapeutic agents, but are also useful and can be modified, where necessary, for making and using therapeutic agents of the disclosure that are not antibodies.

To achieve the desired therapeutic effect, the phosphorylation site-specific antibodies or antigen-binding fragments thereof can be administered in a variety of unit dosage forms. The dose will vary according to the particular antibody. For example, different antibodies may have different masses and/or affinities, and thus require different dosage levels. Antibodies prepared as Fab or other fragments will also require differing dosages than the equivalent intact immunoglobulins, as they are of considerably smaller mass than intact immunoglobulins, and thus require lower dosages to reach the same molar levels in the patient's blood. The dose will also vary depending on the manner of administration, the particular symptoms of the patient being treated, the overall health, condition, size, and age of the patient, and the judgment of the prescribing physician. Dosage levels of the antibodies for human subjects are generally between about 1 mg per kg and about 100 mg per kg per patient per treatment, such as for example, between about 5 mg per kg and about 50 mg per kg per patient per treatment. In terms of plasma concentrations, the antibody concentrations may be in the range from about 25 µg/mL to about 500 µg/mL. However, greater amounts may be required for extreme cases and smaller amounts may be sufficient for milder cases.

Administration of an antibody will generally be performed by a parenteral route, typically via injection such as intra-articular or intravascular injection (e.g., intravenous infusion) or intramuscular injection. Other routes of administration, e.g., oral (p.o.), may be used if desired and practicable for the particular antibody to be administered. An antibody can also be administered in a variety of unit dosage forms and their dosages will also vary with the size, potency, and in vivo half-life of the particular antibody being administered. Doses of a phosphorylation site-specific antibody will also vary depending on the manner of administration, the particular symptoms of the patient being treated, the overall health, condition, size, and age of the patient, and the judgment of the prescribing physician.

The frequency of administration may also be adjusted according to various parameters. These include the clinical response, the plasma half-life of the antibody, and the levels of the antibody in a body fluid, such as, blood, plasma, serum, or synovial fluid. To guide adjustment of the frequency of administration, levels of the antibody in the body fluid may be monitored during the course of treatment.

Formulations particularly useful for antibody-based therapeutic agents are also described in U.S. Patent App. Publication Nos. 20030202972, 20040091490 and 20050158316. In certain embodiments, the liquid formulations of the application are substantially free of surfactant and/or inorganic salts. In another specific embodiment, the liquid formulations have a pH ranging from about 5.0 to about 7.0. In yet another specific embodiment, the liquid formulations comprise histidine at a concentration ranging from about 1 mM to about 100 mM. In still another specific embodiment, the liquid formulations comprise histidine at a concentration ranging from 1 mM to 100 mM. It is also contemplated that the liquid formulations may further comprise one or more excipients such as a saccharide, an amino acid (e.g., arginine, lysine, and methionine) and a polyol. Additional descriptions and methods of preparing and analyzing liquid formulations can be found, for example, in PCT publications WO 03/106644, WO 04/066957, and WO 04/091658.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the pharmaceutical compositions of the application.

In certain embodiments, formulations of the subject antibodies are pyrogen-free formulations which are substantially free of endotoxins and/or related pyrogenic substances. Endotoxins include toxins that are confined inside microorganisms and are released when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, it is advantageous to remove even low amounts of endotoxins from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight, as can be the case with monoclonal antibodies, it is advantageous to remove even trace amounts of endotoxin.

The amount of the formulation which will be therapeutically effective can be determined by standard clinical techniques. In addition, *in vitro* assays may optionally be used to help identify optimal dosage ranges. The precise dose to be used in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. The dosage of the compositions to be administered can be determined by the skilled artisan without undue experimentation in conjunction with standard dose-response studies. Relevant circumstances to be considered in making those determinations include the condition or conditions to be treated, the choice of composition to be administered, the age, weight, and response of the individual patient, and the severity of the patient's symptoms. For example, the actual patient body weight may be used to calculate the dose of the formulations in milliliters (mL) to be administered. There may be no downward adjustment to "ideal" weight. In such a situation, an appropriate dose may be calculated by the following formula:

Dose (mL) = [patient weight (kg) x dose level (mg/kg)/ drug concentration (mg/mL)]

For the purpose of treatment of disease, the appropriate dosage of the compounds (for example, antibodies) will depend on the severity and course of disease, the patient's clinical history and response, the toxicity of the antibodies, and the discretion of the attending physician. The initial candidate dosage may be administered to a patient. The proper dosage and treatment regimen can be established by monitoring the progress of therapy using conventional techniques known to those of skill in the art.

The formulations of the application can be distributed as articles of manufacture comprising packaging material and a pharmaceutical agent which comprises, e.g., the antibody and a pharmaceutically acceptable carrier as appropriate to the mode of administration. The packaging material will include a label which indicates that the formulation is for use in the treatment of prostate cancer.

### 11. Kits

Antibodies and peptides (including AQUA peptides) of the invention may also be used within a kit for detecting the phosphorylation state or level at a novel phosphorylation site of the invention, comprising at least one of the following: an AQUA peptide comprising the phosphorylation site, or an antibody or an antigen-binding fragment thereof that binds to an amino acid sequence comprising the phosphorylation site. Such a kit may further comprise a packaged combination of reagents in predetermined amounts with instructions for performing the diagnostic assay. Where the antibody is labeled with an enzyme, the kit will include substrates and co-factors required by the enzyme. In addition, other additives may be included such as stabilizers, buffers and the like. The relative amounts of the various reagents may be varied widely to provide for concentrations in solution of the reagents that substantially optimize the sensitivity of the assay. Particularly, the reagents may be provided as dry powders, usually lyophilized, including excipients that, on dissolution, will provide a reagent solution having the appropriate concentration.

The following Examples are provided only to further illustrate the invention, and are not intended to limit its scope, except as provided in the claims appended hereto. The invention encompasses modifications and variations of the methods taught herein which would be obvious to one of ordinary skill in the art.

### EXAMPLE 1

### Isolation of Phosphotyrosine-Containing Peptides from Extracts of Carcinoma and/or leukemia Cell Lines and Identification of Novel Phosphorylation Sites.

In order to discover novel tyrosine phosphorylation sites in leukemia, IAP isolation techniques were used to identify phosphotyrosine-containing peptides in cell extracts from human leukemia cell lines and patient cell lines identified in Column G of Table 1 including: 23132/87; 3T3(EGFR: deletion); 3T3(Src); 42-MG-BA; 5637; A172; A498; A549; A704; AML-06/018; AML-06/171; AML-06/207; AML-6246; B16_AML; B17_AML; B24_AML; B39-XY2; B41-XY2; BC-3C; BC001; BC003; BC005; BC008; BJ630; BT1; BT2; Baf3(FGFR1: truncation: 10ZF); Baf3(FGFR1: truncation: 4ZF); Baf3(FGFR1: truncation: PRTK); Baf3(FGFR3: K650E); Baf3(FLT3); Baf3(FLT3: D835V); Baf3(FLT3: D835Y); Baf3(TEL-FGFR3); CAKI-2; CAL-29; CAL-51; CHP-212; CML-06/038; CML-06/164; COLO-699; Colo-824; DK-MG; DV-90; EFM-19; EFO-21; EFO-27; ENT01; ENT02; ENT03; ENT04; ENT10; ENT12; ENT14; ENT15; ENT17; ENT19; ENT6; ENT7; EOL-1; G-292; GAMG; GI-ME-N; H1355; H1437; H1650; H1651; H1703; H1781; H1838; H2052; H2342; H2452; H28; H3255; H358; H4; H520; HCC15; HCC1806; HCC78; HCC827; HCT 116; HCT8; HD-MyZ; HDLM-2; HEL; HL137A; HL184A; HL226A; HL233B; HL234A; HL84B; HP28; HT29; Hs.683; Hs746T; Jurkat; K562; KATO III; KMS-11; Kyse140; Kyse150; Kyse450; Kyse510; Kyse70; L428; L540; LCLC-103H; LN-405; LXF-289; MG-63; MHH-NB-11; MKN-45; MKPL-1; MV4-11; Molm 14; N06BJ601(18); N06BJ606(19); N06CS02; N06CS06; N06CS106; N06CS107; N06CS17; N06CS23; N06CS34; N06CS39; N06CS40; N06CS55; N06CS82; N06CS83; N06CS87; N06CS89; N06CS90; N06CS91; N06CS93-2; N06CS94; N06CS97; N06CS98; N06N109; N06N115; N06N126; N06N130; N06N75; N06N80; N06N90; N06N93; N06bj523(3); N06bj632(24); N06bj667(29); N06c78; N06cs110; N06cs112; N06cs 113; N06cs 115; N06cs 116; N06cs 117; N06cs 121; N06cs 122; N06cs123(2); N06cs129; N06cs130; N06cs21; N06cs49; NALM-19; NCI-H716; Nomo-1; OPM-1; PA-1; RKO; RPMI-8266; RSK-10; RSK-9; RSK2-1; RSK2-2; RSK2-3; RSK2-4; RSK2-5; RSK2-6; RSK2-8; S 2; SEM; SK-N-AS; SK-N-FI; SNU-1; SNU-16; SNU-5; SNU-C2B; SUP-T13; SW480; SW620; SW780; Scaber; Thom; UACC-812; UM-UC-1; brain; colon tissue; cs114; cs131; cs133; cs136; csC43; csC44; csC56; csC62; csC66; gz21; h2073; h2228.
Tryptic phosphotyrosine-containing peptides were purified and analyzed from extracts of each of the cell lines mentioned above, as follows. Cells were cultured in DMEM medium or RPMI 1640 medium supplemented with 10% fetal bovine serum and penicillin/streptomycin.

Suspension cells were harvested by low speed centrifugation. After complete aspiration of medium, cells were resuspended in 1 mL lysis buffer per 1.25 x 10⁸ cells (20 mM HEPES pH 8.0, 9 M urea, 1 mM sodium vanadate, supplemented or not with 2.5 mM sodium pyro-phosphate, 1 mM ß-glycerol-phosphate) and sonicated.

Adherent cells at about 70-80 % confluency were starved in medium without serum overnight and stimulated, with ligand depending on the cell type or not stimulated. After complete aspiration of medium from the plates, cells were scraped off the plate in 10 ml lysis buffer per 2 x 10⁸ cells (20 mM HEPES pH 8.0, 9 M urea, 1 mM sodium vanadate, supplemented with 2.5 mM sodium pyrophosphate, 1 mM β-glycerol-phosphate) and sonicated.

Frozen tissue samples were cut to small pieces, homogenize in lysis buffer (20 mM HEPES pH 8.0, 9 M Urea, 1 mM sodium vanadate, supplemented with 2.5 mM sodium pyrophosphate, 1 mM ß-glycerol-phosphate, 1 ml lysis buffer for 100 mg of frozen tissue) using a polytron for 2 times of 20 sec. each time. Homogenate is then briefly sonicated.

Sonicated cell lysates were cleared by centrifugation at 20,000 x g, and proteins were reduced with DTT at a final concentration of 4.1 mM and alkylated with iodoacetamide at 8.3 mM. For digestion with trypsin, protein extracts were diluted in 20 mM HEPES pH 8.0 to a final concentration of 2 M urea and soluble TLCK-trypsin (Worthington) was added at 10-20 µg/mL. Digestion was performed for 1 day at room temperature.

Trifluoroacetic acid (TFA) was added to protein digests to a final concentration of 1%, precipitate was removed by centrifugation, and digests were loaded onto Sep-Pak C₁₈ columns (Waters) equilibrated with 0.1% TFA. A column volume of 0.7-1.0 ml was used per 2 x 10⁸ cells. Columns were washed with 15 volumes of 0.1% TFA, followed by 4 volumes of 5% acetonitrile (MeCN) in 0.1 % TFA. Peptide fraction I was obtained by eluting columns with 2 volumes each of 8, 12, and 15% MeCN in 0.1% TFA and combining the eluates. Fractions II and III were a combination of eluates after eluting columns with 18, 22, 25% MeCN in 0.1% TFA and with 30, 35, 40% MeCN in 0.1% TFA, respectively. All peptide fractions were lyophilized.

Peptides from each fraction corresponding to 2 x 10⁸ cells were dissolved in 1 ml of IAP buffer (20 mM Tris/HCl or 50 mM MOPS pH 7.2, 10 mM sodium phosphate, 50 mM NaCl) and insoluble matter (mainly in peptide fractions III) was removed by centrifugation. IAP was performed on each peptide fraction separately. The phosphotyrosine monoclonal antibody P-Tyr-100 (Cell Signaling Technology, Inc., catalog number 9411) was coupled at 4 mg/ml beads to protein G (Roche), respectively. Immobilized antibody (15 µl, 60 µg) was added as 1:1 slurry in IAP buffer to 1 ml of each peptide fraction, and the mixture was incubated overnight at 4° C with gentle rotation. The immobilized antibody beads were washed three times with 1 ml IAP buffer and twice with 1 ml water, all at 4° C. Peptides were eluted from beads by incubation with 75 µl of 0.1% TFA at room temperature for 10 minutes.

Alternatively, one single peptide fraction was obtained from Sep-Pak C18 columns by elution with 2 volumes each of 10%, 15%, 20 %, 25 %, 30 %, 35 % and 40 % acetonitirile in 0.1% TFA and combination of all eluates. IAP on this peptide fraction was performed as follows: After

lyophilization, peptide was dissolved in 1.4 ml IAP buffer (MOPS pH 7.2,

10 mM sodium phosphate, 50 mM NaCl) and insoluble matter was removed by centrifugation. Immobilized antibody (40 µl, 160 µg) was added as 1:1 slurry in IAP buffer, and the mixture was incubated overnight at 4° C with gentle shaking. The immobilized antibody beads were washed three times with 1 ml IAP buffer and twice with 1 ml water, all at 4° C. Peptides were eluted from beads by incubation with 55 µl of 0.15% TFA at room temperature for 10 min (eluate 1), followed by a wash of the beads (eluate 2) with 45 µl of 0.15% TFA. Both eluates were combined.

### Analysis by LC-MS/MS Mass Spectrometry.

40 µl or more of IAP eluate were purified by 0.2 µl C18 microtips (StageTips or ZipTips). Peptides were eluted from the microcolumns with 1 µl of 40% MeCN, 0.1% TFA (fractions I and II) or 1 µl of 60% MeCN, 0.1% TFA (fraction III) into 7.6-9.0 µl of 0.4% acetic acid/0.005% heptafluorobutyric acid. For single fraction analysis, 1 µl of 60% MeCN, 0.1 % TFA, was used for elution from the microcolumns. This sample was loaded onto a 10 cm x 75 µm PicoFrit capillary column (New Objective) packed with Magic C 18 AQ reversed-phase resin (Michrom Bioresources) using a Famos autosampler with an inert sample injection valve (Dionex). The column was then developed with a 45-min linear gradient of acetonitrile delivered at 200 nl/min (Ultimate, Dionex), and tandem mass spectra were collected in a data-dependent manner with an LTQ ion trap mass spectrometer essentially as described by Gygi *et al., supra.*

### Database Analysis & Assignments.

MS/MS spectra were evaluated using TurboSequest in the Sequest Browser package (v. 27, rev. 12) supplied as part of BioWorks 3.0 (ThermoFinnigan). Individual MS/MS spectra were extracted from the raw data file using the Sequest Browser program CreateDta, with the following settings: bottom MW, 700; top MW, 4,500; minimum number of ions, 40; minimum TIC, 2 x 10³; and precursor charge state, unspecified. Spectra were extracted from the beginning of the raw data file before sample injection to the end of the eluting gradient. The IonQuest and VuDta programs were not used to further select MS/MS spectra for Sequest analysis. MS/MS spectra were evaluated with the following TurboSequest parameters: peptide mass tolerance, 2.5; fragment ion tolerance, 1.0; maximum number of differential amino acids per modification, 4; mass type parent, average; mass type fragment, average; maximum number of internal cleavage sites, 10; neutral losses of water and ammonia from b and y ions were considered in the correlation analysis. Proteolytic enzyme was specified except for spectra collected from elastase digests.

Searches were performed against the then current NCBI human protein database. Cysteine carboxamidomethylation was specified as a static modification, and phosphorylation was allowed as a variable modification on serine, threonine, and tyrosine residues or on tyrosine residues alone. It was determined that restricting phosphorylation to tyrosine residues had little effect on the number of phosphorylation sites assigned.

In proteomics research, it is desirable to validate protein identifications based solely on the observation of a single peptide in one experimental result, in order to indicate that the protein is, in fact, present in a sample. This has led to the development of statistical methods for validating peptide assignments, which are not yet universally accepted, and guidelines for the publication of protein and peptide identification results *(see* Carr et al., Mol. Cell Proteomics 3: 531-533 (2004)), which were followed in this Example. However, because the immunoaffinity strategy separates phosphorylated peptides from unphosphorylated peptides, observing just one phosphopeptide from a protein is a common result, since many phosphorylated proteins have only one tyrosine-phosphorylated site. For this reason, it is appropriate to use additional criteria to validate phosphopeptide assignments. Assignments are likely to be correct if any of these additional criteria are met: (i) the same phosphopeptide sequence is assigned to co-eluting ions with different charge states, since the MS/MS spectrum changes markedly with charge state; (ii) the phosphorylation site is found in more than one peptide sequence context due to sequence overlaps from incomplete proteolysis or use of proteases other than trypsin; (iii) the phosphorylation site is found in more than one peptide sequence context due to homologous but not identical protein isoforms; (iv) the phosphorylation site is found in more than one peptide sequence context due to homologous but not identical proteins among species; and (v) phosphorylation sites validated by MS/MS analysis of synthetic phosphopeptides corresponding to assigned sequences, since the ion trap mass spectrometer produces highly reproducible MS/MS spectra. The last criterion is routinely used to confirm novel site assignments of particular interest.

All spectra and all sequence assignments made by Sequest were imported into a relational database. The following Sequest scoring thresholds were used to select phosphopeptide assignments that are likely to be correct: RSp < 6, XCorr ≥ 2.2, and DeltaCN > 0.099. Further, the sequence assignments could be accepted or rejected with respect to accuracy by using the following conservative, two-step process.

In the first step, a subset of high-scoring sequence assignments should be selected by filtering for XCorr values of at least 1.5 for a charge state of +1, 2.2 for +2, and 3.3 for +3, allowing a maximum RSp value of 10. Assignments in this subset should be rejected if any of the following criteria are satisfied: (i) the spectrum contains at least one major peak (at least 10% as intense as the most intense ion in the spectrum) that can not be mapped to the assigned sequence as an *a, b,* or y ion, as an ion arising from neutral-loss of water or ammonia from a b or y ion, or as a multiply protonated ion; (ii) the spectrum does not contain a series of *b* or y ions equivalent to at least six uninterrupted residues; or (iii) the sequence is not observed at least five times in all the studies conducted (except for overlapping sequences due to incomplete proteolysis or use of proteases other than trypsin).

In the second step, assignments with below-threshold scores should be accepted if the low-scoring spectrum shows a high degree of similarity to a high-scoring spectrum collected in another study, which simulates a true reference library-searching strategy.

### EXAMPLE 2

### Production of Phosphorylation site-Specific Polyclonal Antibodies

Polyclonal antibodies that specifically bind a novel phosphorylation site of the invention (Table 1/Figure 2) only when the tyrosine residue is phosphorylated (and does not bind to the same sequence when the tyrosine is not phosphorylated), and vice versa, are produced according to standard methods by first constructing a synthetic peptide antigen comprising the phosphorylation site and then immunizing an animal to raise antibodies against the antigen, as further described below. Production of exemplary polyclonal antibodies is provided below.

### A. AIP1 (tyrosine 362).

A 16 amino acid phospho-peptide antigen, IDDPIy*GTYYVDHINR (SEQ NO: 35; y*= phosphotyrosine), which comprises the phosphorylation site derived from human PSD-95 (an adaptor/scaffold protein, Tyr 362 being the phosphorylatable residue), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phosphorylation site-specific polyclonal antibodies as described in Immunization/Screening below.

### B. afadin (tyrosine 262).

An 13 amino acid phospho-peptide antigen, IYADSLKPNIPy*K (SEQ ID NO: 45; y*= phosphotyrosine), which comprises the phosphorylation site derived from human afadin (a cytoskeletal protein, Tyr 262 being the phosphorylatable residue), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phosphorylation site-specific polyclonal antibodies as described in Immunization/Screening below.

### C. MCM5 (tyr 212).

A 9 amino acid phospho-peptide antigen, GMEy*LASKK (SEQ ID NO: 72; y*= phosphotyrosine, which comprises the phosphorylation site derived from human MCM5 (a cell cycle regulation protein, Tyr 212 being the phosphorylatable residue), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phosphorylation site-specific polyclonal antibodies as described in Immunization/Screening below.

### Immunization/Screening.

A synthetic phospho-peptide antigen as described in A-C above is coupled to KLH, and rabbits are injected intradermally (ID) on the back with antigen in complete Freunds adjuvant (500 µg antigen per rabbit). The rabbits are boosted with same antigen in incomplete Freund adjuvant (250 µg antigen per rabbit) every three weeks. After the fifth boost, bleeds are collected. The sera are purified by Protein A-affinity chromatography by standard methods (*see* ANTIBODIES: A LABORATORY MANUAL, Cold Spring Harbor, *supra.).* The eluted immunoglobulins are further loaded onto an unphosphorylated synthetic peptide antigen-resin Knotes column to pull out antibodies that bind the unphosphorylated form of the phosphorylation sites. The flow through fraction is collected and applied onto a phospho-synthetic peptide antigen-resin column to isolate antibodies that bind the phosphorylated form of the phosphorylation sites. After washing the column extensively, the bound antibodies (*i.e.* antibodies that bind the phosphorylated peptides described in A-C above, but do not bind the unphosphorylated form of the peptides) are eluted and kept in antibody storage buffer.

The isolated antibody is then tested for phospho-specificity using Western blot assay using an appropriate cell line that expresses (or overexpresses) target phospho-protein (*i*.*e*. phosphorylated AIP1, afadin or MCM5), for example, H1838 cells, gastric cancer tisse or leukemia cells. Cells are cultured in DMEM or RPMI supplemented with 10% FCS. Cell are collected, washed with PBS and directly lysed in cell lysis buffer. The protein concentration of cell lysates is then measured. The loading buffer is added into cell lysate and the mixture is boiled at 100 °C for 5 minutes. 20 µl (10 µg protein) of sample is then added onto 7.5% SDS-PAGE gel.

A standard Western blot may be performed according to the Immunoblotting Protocol set out in the CELL SIGNALING TECHNOLOGY, INC. 2003-04 Catalogue, p. 390. The isolated phosphorylation site-specific antibody is used at dilution 1:1000. Phospho-specificity of the antibody will be shown by binding of only the phosphorylated form of the target amino acid sequence. Isolated phosphorylation site-specific polyclonal antibody does not (substantially) recognize the same target sequence when not phosphorylated at the specified tyrosine position (e.g., the antibody does not bind to MCM5 in the non-stimulated cells, when tyrosine 212 is not phosphorylated).

In order to confirm the specificity of the isolated antibody, different cell lysates containing various phosphorylated signaling proteins other than the target protein are prepared. The Western blot assay is performed again using these cell lysates. The phosphorylation site-specific polyclonal antibody isolated as described above is used (1:1000 dilution) to test reactivity with the different phosphorylated non-target proteins. The phosphorylation site-specific antibody does not significantly cross-react with other phosphorylated signaling proteins that do not have the described phosphorylation site, although occasionally slight binding to a highly homologous sequence on another protein may be observed. In such case the antibody may be further purified using affinity chromatography, or the specific immunoreactivity cloned by rabbit hybridoma technology.

### EXAMPLE 3

### Production of Phosphorylation site-specific Monoclonal Antibodies

Monoclonal antibodies that specifically bind a novel phosphorylation site of the invention (Table 1) only when the tyrosine residue is phosphorylated (and does not bind to the same sequence when the tyrosine is not phosphorylated) are produced according to standard methods by first constructing a synthetic peptide antigen comprising the phosphorylation site and then immunizing an animal to raise antibodies against the antigen, and harvesting spleen cells from such animals to produce fusion hybridomas, as further described below. Production of exemplary monoclonal antibodies is provided below.

### A. ACTN4 (tyr 212).

A 12 amino acid phospho-peptide antigen, HRPELIEy*DKLR (SEQ ID NO: 88; y*= phosphotyrosine), which comprises the phosphorylation site derived from human ACTN4 (a cytoskeletal protein, Tyr 212 being the phosphorylatable residue), plus cysteine on the C-terminal for coupling, is constructed according to standard synthesis techniques using, *e.g.,* a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Screening below.

### B. SKIV2L2 (tyrosine 517).

An 18 amino acid phospho-peptide antigen, DFRWISSGEy*QMSGRAGR (SEQ ID NO: 85; y*= phosphotyrosine), which comprises the phosphorylation site derived from human SKIV2L2 (a chromatin or DNA binding/repair/replication protein, Tyr 517 being the phosphorylatable residue), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Screening below.

### C. AGSL1 (tyrosine 567).

An 11 amino acid phospho-peptide antigen, LAQGEy*IAPEK (SEQ ID NO: 125; y*= phosphotyrosines), which comprises the phosphorylation site derived from human ACSL1 (an enzyme protein, Tyr 567 being the phosphorylatable residue), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phosphorylation site-specific monoclonal antibodies as described in Immunization/Fusion/Screening below.

### Immunization/Fusion/Screening.

A synthetic phospho-peptide antigen as described in A-C above is coupled to KLH, and BALB/C mice are injected intradermally (ID) on the back with antigen in complete Freunds adjuvant (e.g., 50 µg antigen per mouse). The mice are boosted with same antigen in incomplete Freund adjuvant (e.g. 25 µg antigen per mouse) every three weeks. After the fifth boost, the animals are sacrificed and spleens are harvested.

Harvested spleen cells are fused to SP2/0 mouse myeloma fusion partner cells according to the standard protocol of Kohler and Milstein (1975). Colonies originating from the fusion are screened by ELISA for reactivity to the phospho-peptide and non-phospho-peptide forms of the antigen and by Western blot analysis (as described in Example 1 above). Colonies found to be positive by ELISA to the phospho-peptide while negative to the non-phospho-peptide are further characterized by Western blot analysis. Colonies found to be positive by Western blot analysis are subcloned by limited dilution. Mouse ascites are produced from a single clone obtained from subcloning, and tested for phospho-specificity (against the ACTN4, SKIV2L2 and ACSL1) phospho-peptide antigen, as the case may be) on ELISA. Clones identified as positive on Western blot analysis using cell culture supernatant as having phospho-specificity, as indicated by a strong band in the induced lane and a weak band in the uninduced lane of the blot, are isolated and subcloned as clones producing monoclonal antibodies with the desired specificity.

Ascites fluid from isolated clones may be further tested by Western blot analysis. The ascites fluid should produce similar results on Western blot analysis as observed previously with the cell culture supernatant, indicating phospho-specificity against the phosphorylated target.

### EXAMPLE 4

### Production and Use of AQUA Peptides for Detecting and Quantitating Phosphorylation at a Novel Phosphorylation Site

Heavy-isotope labeled peptides (AQUA peptides (internal standards)) for the detecting and quantitating a novel phosphorylation site of the invention (Table 1) only when the tyrosine residue is phosphorylated are produced according to the standard AQUA methodology *(see* Gygi *et al.,* Gerber *et al., supra.)* methods by first constructing a synthetic peptide standard corresponding to the phosphorylation site sequence and incorporating a heavy-isotope label. Subsequently, the MSⁿ and LC-SRM signature of the peptide standard is validated, and the AQUA peptide is used to quantify native peptide in a biological sample, such as a digested cell extract. Production and use of exemplary AQUA peptides is provided below.

### A. ALDH1B1 (tyrosine 373).

An AQUA peptide comprising the sequence, VLCTy*IQLGQK (SEQ ID NO: 140; y*= phosphotyrosine; Valine being ¹⁴C/¹⁵N-labeled, as indicated in bold), which comprises the phosphorylation site derived from ALDH1B1 (an enzyme protein, Tyr 373 being the phosphorylatable residue), is constructed according to standard synthesis techniques using, e.g., a Rainin/Protein Technologies, Inc., Symphony peptide synthesizer (*see* Merrifield, *supra.)* as further described below in Synthesis & MS/MS Signature. The ALDH1B1 (tyr 373) AQUA peptide is then spiked into a biological sample to quantify the amount of phosphorylated ALDH1B (tyr 373) in the sample, as further described below in Analysis & Quantification.

### B. GOT2 (tyrosine 75).

An AQUA peptide comprising the sequence DDNGKPy*VLPSVR (SEQ ID NO: 149 y*= phosphotyrosine; Proline being ¹⁴C/¹⁵N-labeled, as indicated in bold), which comprises the phosphorylation site derived from human GOT2 (Tyr 75) being the phosphorylatable residue), is constructed according to standard synthesis techniques using, e.g., a Rainin/Protein Technologies, Inc., Symphony peptide synthesizer (*see* Merrifield, *supra.)* as further described below in Synthesis & MS/MS Signature. The GOT2 (Tyr 75) AQUA peptide is then spiked into a biological sample to quantify the amount of phosphorylated GOT2 (Tyr 75) in the sample, as further described below in Analysis & Quantification.

### C. AMPKB2 (tyrosine 242).

An AQUA peptide comprising the sequence MLNHLy*ALSIK (SEQ ID NO: 221; y*= phosphotyrosine; Leucine being ¹⁴C/¹⁵N-labeled, as indicated in bold), which comprises the phosphorylation site derived from human AMPKB2 (Tyr 242 being the phosphorylatable residue), is constructed according to standard synthesis techniques using, e.g., a Rainin/Protein Technologies, Inc., Symphony peptide synthesizer (*see* Merrifield, *supra.)* as further described below in Synthesis & MS/MS Signature. The AMPKB2 (Tyr 242) AQUA peptide is then spiked into a biological sample to quantify the amount of phosphorylated AMPKB2 (Tyr 242) in the sample, as further described below in Analysis & Quantification.

### D. AK3 (tyrosine 186).

An AQUA peptide comprising the sequence AYEDQTKPVLEy*YQK (SEQ ID NO: 201; y*= phosphotyrosine; valine being ¹⁴C/¹⁵N-labeled, as indicated in bold), which comprises the phosphorylation site derived from human AK3 (Tyr 186 being the phosphorylatable residue), is constructed according to standard synthesis techniques using, e.g., a Rainin/Protein Technologies, Inc., Symphony peptide synthesizer (*see* Merrifield, *supra.)* as further described below in Synthesis & MS/MS Signature. The AK3 (Tyr 186) AQUA peptide is then spiked into a biological sample to quantify the amount of phosphorylated AK3 (Tyr 186) in the sample, as further described below in Analysis & Quantification.

### Synthesis & MS/MS Spectra.

Fluorenylmethoxycarbonyl (Fmoc)-derivatized amino acid monomers may be obtained from AnaSpec (San Jose, CA). Fmoc-derivatized stable-isotope monomers containing one ¹⁵N and five to nine ¹³C atoms may be obtained from Cambridge Isotope Laboratories (Andover, MA). Preloaded Wang resins may be obtained from Applied Biosystems. Synthesis scales may vary from 5 to 25 µmol. Amino acids are activated *in situ* with 1-H-benzotriazolium, 1-bis(dimethylamino) methylene]-hexafluorophosphate (1-),3-oxide:1-hydroxybenzotriazole hydrate and coupled at a 5-fold molar excess over peptide. Each coupling cycle is followed by capping with acetic anhydride to avoid accumulation of one-residue deletion peptide by-products. After synthesis peptide-resins are treated with a standard scavenger-containing trifluoroacetic acid (TFA)-water cleavage solution, and the peptides are precipitated by addition to cold ether. Peptides (*i.e.* a desired AQUA peptide described in A-D above) are purified by reversed-phase C18 HPLC using standard TFA/acetonitrile gradients and characterized by matrix-assisted laser desorption ionization-time of flight (Biflex III, Bruker Daltonics, Billerica, MA) and ion-trap (ThermoFinnigan, LCQ DecaXP or LTQ) MS.

MS/MS spectra for each AQUA peptide should exhibit a strong *y*-type ion peak as the most intense fragment ion that is suitable for use in an SRM monitoring/analysis. Reverse-phase microcapillary columns (0.1 Å~ 150-220 mm) are prepared according to standard methods. An Agilent 1100 liquid chromatograph may be used to develop and deliver a solvent gradient [0.4% acetic acid/0.005% heptafluorobutyric acid (HFBA)/7% methanol and 0.4% acetic acid/0.005% HFBA/65% methanol/35% acetonitrile] to the microcapillary column by means of a flow splitter. Samples are then directly loaded onto the microcapillary column by using a FAMOS inert capillary autosampler (LC Packings, San Francisco) after the flow split. Peptides are reconstituted in 6% acetic acid/0.01% TFA before injection.

### Analysis & Quantification.

Target protein (e.g. a phosphorylated proteins of A-D above) in a biological sample is quantified using a validated AQUA peptide (as described above). The IAP method is then applied to the complex mixture of peptides derived from proteolytic cleavage of crude cell extracts to which the AQUA peptides have been spiked in.

LC-SRM of the entire sample is then carried out. MS/MS may be performed by using a ThermoFinnigan (San Jose, CA) mass spectrometer (LCQ DecaXP ion trap or TSQ Quantum triple quadrupole or LTQ). On the DecaXP, parent ions are isolated at 1.6 m/z width, the ion injection time being limited to 150 ms per microscan, with two microscans per peptide averaged, and with an AGC setting of 1x10⁸; on the Quantum, Q 1 is kept at 0.4 and Q3 at 0.8 m/z with a scan time of 200 ms per peptide. On both instruments, analyte and internal standard are analyzed in alternation within a previously known reverse-phase retention window; well-resolved pairs of internal standard and analyte are analyzed in separate retention segments to improve duty cycle. Data are processed by integrating the appropriate peaks in an extracted ion chromatogram (60.15 m/z from the fragment monitored) for the native and internal standard, followed by calculation of the ratio of peak areas multiplied by the absolute amount of internal standard (e.g., 500 fmol).

## Claims

1. An isolated phosphorylation site-specific antibody that specifically binds a human carcinoma and/or leukemia-related signaling protein selected from Column A of Table 1 only when phosphorylated at the tyrosine listed in corresponding Column D of Table 1, comprised within the phosphorylatable peptide sequence listed in corresponding Column E of Table 1 (SEQ ID NOs: 4-12, 14-28, 30-51, 53-57, 59-64, 66-97, 99-127, 129-162, 164-177, 179-263, 266-271, 273-288, 290-338 and 340-422), wherein said antibody does not bind said signaling protein when not phosphorylated at said tyrosine.

2. An isolated phosphorylation site-specific antibody that specifically binds a human carcinoma and/or leukemia-related signaling protein selected from Column A of Table 1 only when not phosphorylated at the tyrosine listed in corresponding Column D of Table 1, comprised within the phosphorylatable peptide sequence listed in corresponding Column E of Table 1 (SEQ ID NOs: 4-12, 14-28, 30-51, 53-57, 59-64, 66-97, 99-127, 129-162, 164-177, 179-263, 266-271, 273-288, 290-338 and 340-422), wherein said antibody does not bind said signaling protein when phosphorylated at said tyrosine.

3. (New) A method selected from the group consisting of:
(a) a method for detecting a human carcinoma and/or leukemia-related signaling protein selected from Column A of Table 1, wherein said human carcinoma and/or leukemia-related signaling protein is phosphorylated at the tyrosine listed in corresponding Column D of Table 1, comprised within the phosphorylatable peptide sequence listed in corresponding Column E of Table 1 (SEQ ID NOs: 4-12, 14-28, 30-51, 53-57, 59-64, 66-97, 99-127, 129-162, 164-177, 179-263, 266-271, 273-288, 290-338 and 340-422), comprising the step of adding an isolated phosphorylation-specific antibody according to claim 1, to a sample comprising said human carcinoma and/or leukemia-related signaling protein under conditions that permit the binding of said antibody to said human carcinoma and/or leukemia-related signaling protein, and detecting bound antibody;
(b) a method for quantifying the amount of a human carcinoma and/or leukemia-related signaling protein listed in Column A of Table 1 that is phosphorylated at the corresponding tyrosine listed in Column D of Table 1, comprised within the phosphorylatable peptide sequence listed in corresponding Column E of Table 1 (SEQ ID NOs: 4-12, 14-28, 30-51, 53-57, 59-64, 66-97, 99-127, 129-162, 164-177, 179-263, 266-271, 273-288, 290-338 and 340-422), in a sample using a heavy-isotope labeled peptide (AQUA^{™} peptide), said labeled peptide comprising the phosphorylated tyrosine listed in corresponding Column D of Table 1, comprised within the phosphorylatable peptide sequence listed in corresponding Column E of Table 1 as an internal standard; and
(c) a method comprising step (a) followed by step (b).

4. The method of claim 3, wherein said isolated phosphorylation-specific antibody is capable of specifically binding p90RSK only when phosphorylated at Y237, comprised within the phosphorylatable peptide sequence listed in Column E, Row 226, of Table 1 (SEQ ID NO: 237), wherein said antibody does not bind said protein when not phosphorylated at said tyrosine.

5. The method of claim 3, wherein said isolated phosphorylation-specific antibody is capable of specifically binding RSK2 only when phosphorylated at Y226, comprised within the phosphorylatable peptide sequence listed in Column E, Row 230, of Table 1 (SEQ ID NO: 241), wherein said antibody does not bind said protein when not phosphorylated at said threonine.

6. The method of claim 3, wherein said isolated phosphorylation-specific antibody is capable of specifically binding FGFR2 only when phosphorylated at 656, comprised within the phosphorylatable peptide sequence listed in Column E, Row 245, of Table 1 (SEQ ID NO: 256), wherein said antibody does not bind said protein when not phosphorylated at said tyrosine.

7. An isolated phosphorylation site-specific antibody capable of specifically binding CDH1 only when phosphorylated at Y755, comprised within the phosphorylatable peptide sequence listed in Column E, Row 44, of Table 1 (SEQ ID NO: 48), wherein said antibody does not bind said protein when not phosphorylated at said tyrosine.

8. An isolated phosphorylation site-specific antibody capable of specifically binding CDH1 only when not phosphorylated at Y755, comprised within the phosphorylatable peptide sequence listed in Column E, Row 44, of Table 1 (SEQ ID NO: 48), wherein said antibody does not bind said protein when phosphorylated at said tyrosine.

9. An isolated phosphorylation site-specific antibody capable of specifically binding p90RSK only when phosphorylated at Y229, comprised within the phosphorylatable peptide sequence listed in Column E, Row 225, of Table 1 (SEQ ID NO: 236), wherein said antibody does not bind said protein when not phosphorylated at said tyrosine.

10. An isolated phosphorylation site-specific antibody capable of specifically binding p90RSK only when not phosphorylated at Y229, comprised within the phosphorylatable peptide sequence listed in Column E, Row 225, of Table 1 (SEQ ID NO: 236), wherein said antibody does not bind said protein when phosphorylated at said tyrosine.
